(19) Europäisches Patentamt
European Patent Office
Office européen des brevets

(11) Publication number: **0 235 449 B1**

(12) # EUROPEAN PATENT SPECIFICATION

(45) Date of publication of patent specification: **11.09.91**

(51) Int. Cl.5: **C07D 239/42**, C07D 239/47, C07D 239/48, C07D 251/18, C07D 251/22, C07D 251/46, C07C 311/15, C07C 311/51, C07C 311/65, A01N 47/36, //C07D239/52,C07D251/48, C07D251/50,C07D251/52

(21) Application number: **86309138.5**

(22) Date of filing: **21.11.86**

The file contains technical information submitted after the application was filed and not included in this specification

(54) **Herbicidal sulfonamides.**

(30) Priority: **22.11.85 US 801165**
**22.11.85 US 801120**
**10.02.86 US 826682**

(43) Date of publication of application:
**09.09.87 Bulletin 87/37**

(45) Publication of the grant of the patent:
**11.09.91 Bulletin 91/37**

(84) Designated Contracting States:
**BE CH DE FR GB GR IT LI LU NL SE**

(56) References cited:
**EP-A- 0 007 687**
**EP-A- 0 046 626**
**EP-A- 0 084 020**
**EP-A- 0 096 002**
**EP-A- 0 124 295**
**EP-A- 0 162 723**
**EP-A- 0 165 003**

(73) Proprietor: **E.I. DU PONT DE NEMOURS AND COMPANY**
**1007 Market Street**
**Wilmington Delaware 19898(US)**

(72) Inventor: **Hillemann, Craig Lee**
**1229 Crestover Road**
**Wilmington Delaware 19803(US)**

(74) Representative: **Hildyard, Edward Martin et al**
**Frank B. Dehn & Co. European Patent Attorneys Imperial House 15-19 Kingsway**
**London WC2B 6UZ(GB)**

EP 0 235 449 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

This invention relates to certain herbicidal sulfonamides, agriculturally suitable compositions thereof and a method for their use as a general or selective preemergent or postemergent herbicide or as a plant growth regulant.

New compounds effective for controlling the growth of undesired vegetation are in constant demand. In the most common situation, such compounds are sought to selectively control the growth of weeds in useful crops such as cotton, rice, corn, wheat and soybeans, to name a few. Unchecked weed growth in such crops can cause significant losses, reducing profit to the farmer and increasing costs to the consumer. In other situations, herbicides are desired which will control all plant growth. Examples of areas in which complete control of all vegetation is desired are areas around fuel storage tanks, ammunition depots and industrial storage areas. There are many products commercially available for these purposes, but the search continues for products which are more effective, less costly and environmentally safe.

The "sulfonylurea" herbicides are an extremely potent class of herbicides discovered within the last few years which generally consist of a sulfonylurea bridge, $-SO_2NHCONH-$, linking two aromatic or heteroaromatic rings.

U.S. 4,394,506 discloses herbicidal ortho-alkoxycarbonylbenzenesulfonamides such as

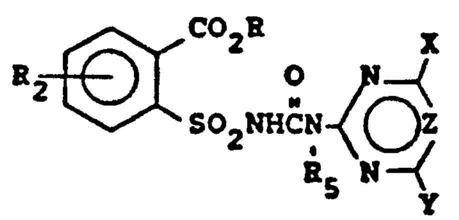

wherein

$R_2$ is H, F, Cl, Br, $C_1$-$C_3$ alkyl, $NO_2$, $SO_2CH_3$, $OCH_3$, $SCH_3$, $CF_3$, $N(CH_3)_2$, $NH_2$ or CN;

X is H, Cl, $CH_3$, $OCH_3$, $OCH_2CH_3$ or $OCH_2CH_2OCH_3$;and

Y is H, halogen, $C_1$-$C_4$ alkyl, $C_1$-$C_4$ substituted alkyl, $C_1$-$C_4$ alkoxy, etc.

South African Patent Application 81/4874 (EP-A-44,807) discloses herbicidal sulfonamides of formula

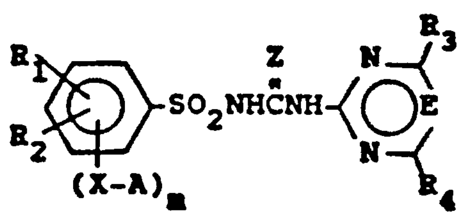

wherein

A is a $C_1$-$C_6$ alkyl radical which is substituted by halogen or various other organic substituents or a $C_2$-$C_6$ alkenyl radical which is substituted or unsubstituted;

X is O, S, SO or $SO_2$;

$R_1$ is H, halogen, $C_1$-$C_5$ alkyl, $C_2$-$C_5$ alkenyl or $YR_5$;

$R_2$ is H, halogen, $C_1$-$C_5$ alkyl, $C_2$-$C_5$ alkenyl, $C_1$-$C_4$ haloalkyl, $CO_2R_6$, $YR_5$, $NO_2$ or $CONR_7R_8$; and

$R_3$ and $R_4$, each independently of the other. are hydrogen, $C_1$-$C_4$ alkyl, $C_1$-$C_4$ alkoxy, $C_1$-$C_4$ alkylthio, $C_1$-$C_4$ haloalkyl, halogen or alkoxyalkyl of at most 4 carbon atoms.

South African Patent Application 82/5042 (EP-A-70,802) discloses herbicidal sulfonamides of formula

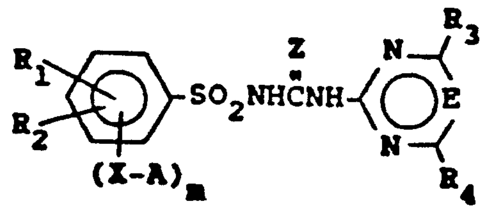

wherein

| | |
|---|---|
| A | is $C_3$-$C_6$ alkynyl; |
| X | is O, S, SO or $SO_2$; |
| $R_1$ | is H, halogen, $C_1$-$C_5$ alkyl, $C_2$-$C_5$ alkenyl or $YR_5$; |
| $R_2$ | is H, halogen, $C_1$-$C_5$ alkyl, $C_2$-$C_5$ alkenyl, $C_1$-$C_4$ haloalkyl, $CO_2R_6$, $YR_5$, $NO_2$ or $CONR_7R_8$; and |
| $R_3$ and $R_4$, | independently of one another, are hydrogen, $C_1$-$C_4$ alkyl, $C_1$-$C_4$ alkoxy, $C_1$-$C_4$ alkylthio, $C_1$-$C_4$ haloalkyl, $C_1$-$C_4$ haloalkoxy, halogen or alkoxyalkyl of at most 4 carbon atoms. |

South African Patent Application 82/5671 (EP-A-72,347) discloses herbicidal sulfonamides of formula

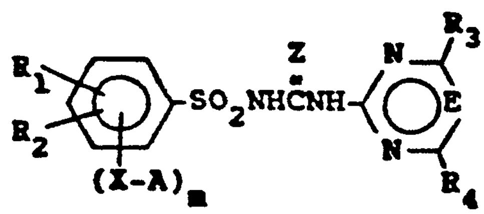

wherein

| | |
|---|---|
| A | is a $C_1$-$C_6$ alkyl radical or a $C_2$-$C_6$ alkenyl radical which is substituted by halogen or various other organic substituents; |
| X | is O, S, SO or $SO_2$; |
| $R_1$ | is H, halogen, $C_1$-$C_5$ alkyl, $C_2$-$C_5$ alkenyl or $YR_5$; |
| $R_2$ | is H, halogen, $C_1$-$C_5$ alkyl, $C_2$-$C_5$ alkenyl, $C_1$-$C_4$ haloalkyl, $CO_2R_6$, $YR_5$, $NO_2$ or $CONR_7R_8$; |
| $R_3$ | is hydrogen, $C_1$-$C_4$ alkyl, $C_1$-$C_4$ alkoxy, $C_1$-$C_4$ alkylthio, $C_1$-$C_4$ haloalkyl, $C_1$-$C_4$ haloalkoxy, halogen or alkoxyalkyl of at mcst 4 carbon atoms; and |
| $R_4$ | is $C_1$-$C_4$ haloalkoxy or $C_1$-$C_4$ haloalkylthio. |

South African Patent Application 83/0127 (EP-A-84,020) discloses herbicidal sulfonamides of formula

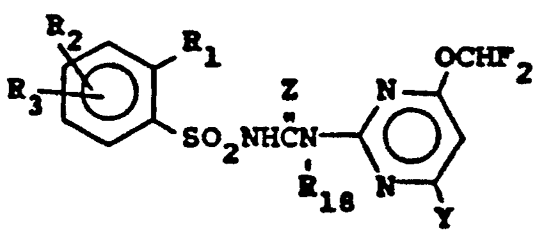

wherein

| | |
|---|---|
| $R_1$ | is hydrogen, halogen, cyano, nitro, $C_1$-$C_4$ haloalkyl, $C_1$-$C_4$ alkyl, $C_1$-$C_4$ alkoxy, $COR_6$, $NR_7R_8$, S-$(O)_m$-$C_1$-$C_4$ alkyl or $SO_2R_9$; |
| $R_2$ | is H, F, Cl, Br, $NO_2$, $CF_3$, $NR_{20}R_{21}$, $C_1$-$C_2$ alkyl, $C_1$-$C_2$ alkoxy or $S(O)_m$-$C_1$-$C_4$ alkyl; |
| $R_3$ | is H, F, Cl, Br, $NH_2$, $NO_2$ or $OCH_3$; |
| $R_6$ | is hydrogen, $C_1$-$C_4$ alkyl, $C_3$-$C_5$ alkenyloxy, $C_3$-$C_5$ alkynyloxy, $C_1$-$C_4$ haloalkyl, $C_1$-$C_5$ alkylthio, phenoxy, benzyloxy, $NR_{10}R_{11}$ or $C_1$-$C_5$ alkoxy which is unsubstituted or substituted by 1-3 halogen atoms or $C_1$-$C_3$ alkoxy; |
| $R_{18}$ | is hydrogen, $C_1$-$C_3$ alkyl or $C_1$-$C_3$ alkoxy; |
| m | is 0, 1 or 2; and |
| Y | is $C_1$-$C_3$ alkyl, $C_1$-$C_3$ haloalkyl, $C_1$-$C_3$ alkoxy, $C_1$-$C_3$ haloalkoxy, $C_2$-$C_3$ alkoxyalkyl, $C_1$-$C_3$ alkylthio, halogen or $NR_{16}R_{17}$. |

South African Patent Application 84/2245 (EP-A-120,814) discloses herbicidal sulfonamides of formula

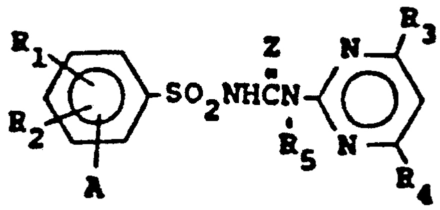

wherein

- A is $C_1$-$C_6$ haloalkyl;
- $R_1$ is hydrogen, halogen, nitro, cyano, $C_1$-$C_4$ alkyl, $C_1$-$C_4$ haloalkyl, $C_1$-$C_4$ alkoxy, $C_1$-$C_4$ alkylthio, $C_1$-$C_4$ alkylsulfinyl, $C_1$-$C_4$ alkylsulfonyl, $COR_6$, $NR_7R_8$, $CONR_9R_{10}$ or $SO_2NR_{11}R_{12}$;
- $R_2$ is hydrogen, halogen, $C_1$-$C_4$ alkyl, $C_1$-$C_4$ alkoxy, $C_1$-$C_4$ alkylthio, $C_1$-$C_4$ alkylsulfinyl or $C_1$-$C_4$ alkylsulfonyl; and
- $R_3$ and $R_4$, independently of one another, are each hydrogen, halogen, $C_1$-$C_4$ alkyl, $C_1$-$C_4$ haloalkyl, $C_2$-$C_4$ haloalkoxy, $C_1$-$C_4$ alkylthio, $C_1$-$C_4$ haloalkylthio, $C_2$-$C_4$ alkoxyalkyl, $C_1$-$C_4$ alkoxy or $NR_{12}R_{13}$.

South African Patent Application 84/2722 (EP-A-125,205) discloses herbicidal benzenesulfonamides of formula

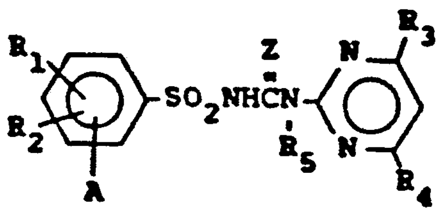

wherein

- A is a radical of the formula $CR_6R_7XR_8$, $CR_9R_{10}R_{11}$ or $CHR_7SCQR_{21}$;
- $R_1$ is hydrogen, halogen, nitro, cyano, $C_1$-$C_4$ alkyl, $C_1$-$C_4$ haloalkyl, $YR_{14}$, $CONR_{12}R_{13}$, $NR_{12}R_{13}$, $SONR_{15}R_{16}$, $OSO_2R_{17}$ or $COR_{18}$;
- $R_2$ is hydrogen, halogen, $C_1$-$C_4$ alkyl, $C_1$-$C_4$ alkoxy, $C_1$-$C_4$ alkylthio, $C_1$-$C_4$ alkylsulfinyl or $C_1$-$C_4$ alkylsulfonyl;
- $R_3$ and $R_4$, independently of one another, are each hydrogen, halogen, $C_1$-$C_4$ alkyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ haloalkylthio, $C_2$-$C_5$ alkoxyalkyl or $NR_{19}R_{20}$;
- $R_9$ is hydrogen, halogen, $C_1$-$C_4$ alkyl, $C_1$-$C_4$ alkoxy, $C_1$-$C_4$ alkylthio, $C_1$-$C_4$ alkylsulfinyl or $C_1$-$C_4$ alkylsulfonyl;
- $R_{10}$ is hydrogen, halogen or methyl;
- $R_{11}$ is a radical $COR_{24}$ or a $C_1$-$C_4$ alkyl group that is mono- or polysubstituted by substituents selected from the group: cyano, nitro, hydroxyl, $C_1$-$C_4$ alkoxy, $C_1$-$C_4$ alkylthio, etc.
- $R_{18}$ is H, $C_1$-$C_4$ alkoxy and various other organic radicals.

South African Patent Application 83/0441 (EP-A-85,028) discloses herbicidal benzenesulfonamides of formula

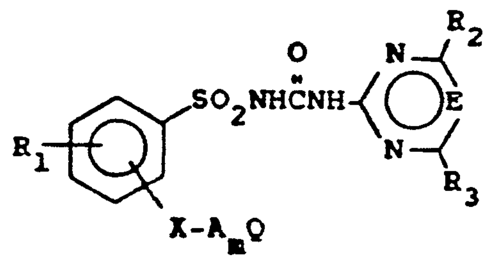

wherein

- $R_1$ is H, halogen, $NO_2$, $C_1$-$C_4$ haloalkyl, $C_1$-$C_4$ alkyl, $C_1$-$C_4$ alkoxy, $C_2$-$C_5$ alkenyl or $C_1$-$C_4$ alkoxycarbonyl;
- $R_2$ is $C_1$-$C_3$ alkyl or $C_1$-$C_3$ alkoxy, each unsubstituted or substituted by 1 to 3 halogen atoms;
- $R_3$ is halogen, H, $NR_4R_5$, $C_1$-$C_3$ alkyl, unsubstituted or substituted by 1 to 3 halogen atoms or $C_1$-$C_4$ alkoxy, or is $C_1$-$C_3$ alkoxy, unsubstituted or substituted by methoxy, ethoxy, or 1 to 3 halogen atoms;
- A is $C_1$-$C_4$ alkylene or $C_2$-$c_4$ alkenylene, each unsubstituted or substituted by $C_1$-$C_4$ alkyl;
- m is 0 or 1;
- E is N or CH;
- X is oxygen, sulfur, SO or $SO_2$; and
- Q is, in part, OH, CN, $NR_6R_7$, $SO_2R_8$, cycloalkyl or $COC_1$-$C_6$ alkyl.

South African Patent Application 83/3779 (EP-A-96,002) discloses herbicidal benzenesulfonamides of formula

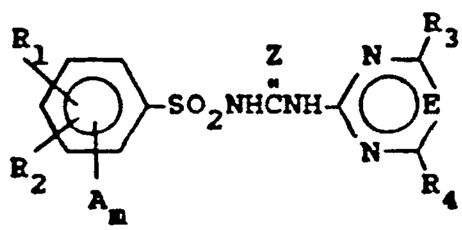

wherein

- A is C≡CR;
- m is 1 or 2;
- E is CH or N;
- Z is oxygen or sulfur,
- R is, in part, H, $C_1$-$C_9$ alkyl or $C_1$-$C_9$ haloalkyl;
- $R_1$ is H, halogen, $C_1$-$C_5$ alkyl, $C_2$-$C_5$ alkenyl or $YR_5$;
- $R_2$ is H, halogen, $C_1$-$C_5$ alkyl, $C_2$-$C_5$ alkenyl, $C_1$-$C_4$ haloalkyl, $YR_5$, $CO_2R_6$, $NO_2$ or $CONR_7R_8$;
- $R_3$ and $R_4$, each independently of the other, are H, $C_1$-$C_4$ alkyl, $C_1$-$C_4$ alkoxy, $C_1$-$C_4$ alkylthio, $C_1$-$C_4$ haloalkyl, $C_1$-$C_4$ haloalkoxy, $C_1$-$C_4$ haloalkylthio, halogen, $C_2$-$C_5$ alkoxyalkyl, $NR_9R_{10}$ or $OCH_2CH_2NR_9R_{10}$.

South African Patent Application 82/7439 (EP-A-82,108) discloses herbicidal sulfonamides of formula

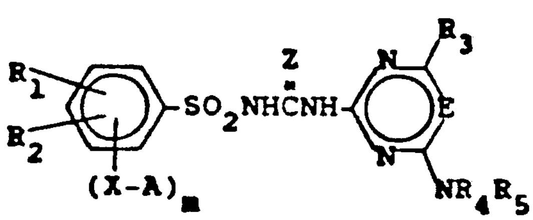

wherein

A is a $C_3$-$C_6$ alkynyl group, a $C_1$-$C_6$ alkyl group which is substituted by halogen, $C_1$-$C_4$ alkoxy, $C_1$-$C_4$ alkylthio, $C_1$-$C_4$ alkylsulfinyl, $C_1$-$C_4$ alkylsulfonyl, $C_1$-$C_4$ haloalkoxy, $C_1$-$C_4$ haloalkylthio, $C_1$-$C_4$ haloalkylsulfinyl or $C_1$-$C_4$ haloalkylsulfonyl, or it is a $C_2$-$C_6$ alkenyl group substituted by one of the above substituents;

X is O, S, SO or $SO_2$;

$R_1$ is hydrogen, halogen, $C_1$-$C_5$ alkyl, $C_2$-$C_5$ alkenyl or a $YR_6$ group;

$R_2$ is hydrogen, halogen, $C_1$-$C_5$ alkyl, $C_2$-$C_5$ alkenyl, $C_1$-$C_4$ haloalkyl, $YR_6$, $CO_2R_7$, $NO_2$ or $CONR_8R_9$;

$R_3$ is hydrogen, $C_1$-$C_4$ alkyl, $C_1$-$C_4$ alkoxy, $C_1$-$C_4$ alkylthio, $C_1$-$C_4$ haloalkyl, $C_1$-$C_4$ haloalkoxy, halogen or alkoxyalkyl having at most 4 carbon atoms;

$R_4$ is hydrogen, methyl or ethyl;

$R_5$ is hydrogen, $C_1$-$C_4$ alkyl, $C_1$-$C_3$ alkoxy, methoxymethyl, cyanomethyl or cyanoethyl;

$R_6$ and $R_7$ are each $C_1$-$C_5$ alkyl, $C_2$-$C_5$ alkenyl or $C_2$-$C_6$ alkynyl.

South African Patent Application 83/6449 (Swiss priority 01.09.82; EP-A-102,925) discloses herbicidal benzenesulfonamides of formula

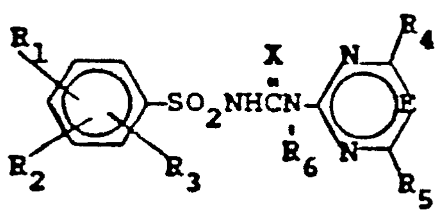

wherein

$R_1$ is H, halogen, $NO_2$, amino, $C_1$-$C_5$ alkyl, $C_1$-$C_4$ haloalkyl or a $QR_7$, $CO_2R_8$ or $(CO)_nNR_9R_{10}$ radical;

$R_2$ is H, $C_1$-$C_4$ alkyl, $C_1$-$C_4$ alkoxy, $C_1$-$C_4$ alkylthio, $C_1$-$C_4$ haloalkyl, $C_1$-$C_4$ haloalkoxy, halogen or alkoxyalkyl containing not more than 4 carbon atoms;

$R_3$ is $C_2$-$C_{10}$ alkenyl which is substituted by one or more fluorine or bromine atoms or by one or more hydroxyl, cyano, nitro, $(Y)_mCO(Z)_nR_8$, $SO_2NR_{11}R_{12}$, $S(O)_pC_1$-$C_3$ haloalkyl or $S(O)_nC_1$-$C_3$ alkyl groups and which may additionally be substituted by one or more chlorine atoms;

$R_4$ is $C_1$-$C_3$ alkyl, $C_1$-$C_3$ haloalkyl, $C_1$-$C_3$ alkoxy or $C_1$-$C_3$ haloalkoxy;

$R_5$ is H, halogen, $NR_{13}R_{14}$, $C_1$-$C_3$ alkyl, $C_1$-$C_3$ haloalkyl, $C_1$-$C_3$ alkoxy or $C_1$-$C_2$ haloalkoxy: and

E is CH or N.

Other herbicidal sulfonylureas are diclosed in EP-A-162,723; EP-A-46,676; and EP-A-165,003.

There is however a continuing need to find herbicides that are more effective and more selective toward agricultural products.

## Summary of the Invention

This invention relates to certain compounds of the structural formula:

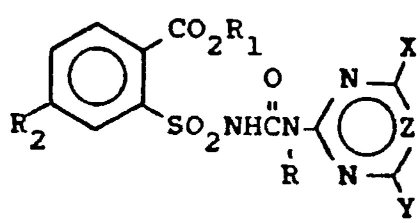

I

wherein

R is H or $CH_3$;

$R_1$ is $C_1$-$C_3$ alkyl, $C_3$-$C_4$ alkoxyalkyl, $C_2$-$C_4$ haloalkyl, $C_3$-$C_4$ alkenyl or $C_3$-$C_4$ alkynyl;

$R_2$ is $C_2$-$C_6$ alkoxy, $C_3$-$C_6$ cycloalkoxy, $C_4$-$C_6$ cycloalkylalkoxy, $C_1$-$C_6$ haloalkoxy, $C_2$-$C_6$ alkenyloxy, $C_2$-$C_6$ haloalkenyloxy, $C_3$-$C_6$ alkynyloxy, $C_3$-$C_6$ haloalkynyloxy, $C_2$-$C_4$ alko-

xyalkoxy, $C_2$-$C_4$ haloalkoxyalkoxy, $C_2$-$C_4$ alkylthioalkoxy, $C_2$-$C_4$ haloalkylthioalkoxy, $C_2$-$C_4$ alkylsulfinylalkoxy, $C_2$-$C_4$ haloalkylsulfinylalkoxy, $C_2$-$C_4$ alkylsulfonylalkoxy, $C_2$-$C_4$ haloalkylsulfonylalkoxy, $C_2$-$C_4$ cyanoalkoxy, $OCH_2C(O)CH_3$,$OCH_2CH_2C(O)CH_3$, $C_2$-$C_4$ aminoalkoxy, $C_1$-$C_8$ alkylthio, $C_3$-$C_6$ cycloalkylthio, $C_4$-$C_6$ cycloalkylalkylthio, $C_1$-$C_8$ haloalkylthio, $C_2$-$C_6$ alkenylthio, $C_2$-$C_6$ haloalkenylthio, $C_3$-$C_6$ alkynylthio, $C_3$-$C_6$ haloalkynylthio, $C_2$-$C_4$ alkoxyalkylthio, $C_2$-$C_4$ haloalkoxyalkylthio, $C_2$-$C_4$ alkylthioalkylthio, $C_2$-$C_4$ haloalkylthioalkylthio, $C_2$-$C_4$ cyanoalkylthio, $SCH_2C(O)CH_3$, $SCH_2CH_2C(O)CH_3$, $C_2$-$C_4$ aminoalkylthio, $SC_6H_5$, $SCH_2C_6H_5$, $C_1$-$C_8$ alkylsulfinyl, $C_3$-$C_6$ cycloalkylsulfinyl, $C_4$-$C_6$ cycloalkylalkylsulfinyl, $C_1$-$C_8$ haloalkylsulfinyl, $C_2$-$C_6$ alkenylsulfinyl, $C_2$-$C_6$ haloalkenylsulfinyl, $C_3$-$C_6$ alkynylsulfinyl, $C_3$-$C_6$ haloalkynylsulfinyl, $C_2$-$C_4$ alkoxyalkylsulfinyl, $C_2$-$C_4$ haloalkoxyalkylsulfinyl, $C_2$-$C_4$ cyanoalkylsulfinyl. $S(O)CH_2C(O)CH_3$, $S(O)CH_2CH_2C(O)CH_3$, $C_2$-$C_4$ aminoalkylsulfinyl, $C_2$-$C_8$ alkylsulfonyl, $C_3$-$C_6$ cycloalkylsulfonyl, $C_4$-$C_6$ cycloalkylalkylsulfonyl, $C_1$-$C_8$ haloalkylsulfonyl, $C_2$-$C_6$ alkenylsulfonyl, $C_2$-$C_6$ haloalkenylsulfonyl, $C_3$-$C_6$ alkynylsulfonyl, $C_3$-$C_6$ haloalkynylsulfonyl, $C_2$-$C_4$ alkoxyalkylsulfonyl, $C_2$-$C_4$ haloalkoxyalkylsulfonyl, $C_2$-$C_4$ cyanoalkylsulfonyl, $SO_2CH_2C(O)CH_3$, $SO_2CH_2CH_2C(O)CH_3$, $C_2$-$C_4$ aminoalkylsulfonyl, $CH_2F$, $CHF_2$, $CH_2Cl$, $CHCl_2$, $CH_2Br$, $CHBr_2$, $C_2$-$C_6$ alkyl substituted with 1-3 atoms of F, Cl or Br, $C_2$-$C_6$ alkenyl, $C_2$-$C_6$ haloalkenyl, $C_2$-$C_6$ haloalkynyl, $OC(O)$-$C_1$-$C_4$ alkyl, $CH_2C(O)NR_aR_b$, $NHCH_3$, $NR_bR_c$ or $C_1$-$C_4$ alkyl substituted with $C_1$-$C_4$ alkoxy, $C_3$-$C_4$ cycloalkoxy, cyclopropylmethoxy, $C_1$-$C_4$ haloalkoxy, $C_2$-$C_4$ alkenyloxy, $C_2$-$C_4$ haloalkenyloxy, $C_3$-$C_4$ alkynyloxy, $C_3$-$C_4$ haloalkynyloxy, $C_2$-$C_4$ alkoxyalkoxy, $C_2$-$C_4$ aminoalkoxy, $C_1$-$C_4$ alkylcarbonyloxy, $C_1$-$C_4$ haloalkylcarbonyloxy, $C_1$-$C_4$ carbamoyloxy, $C_1$-$C_4$ alkoxycarbonyloxy, OH, $OP(O)(OC_1$-$C_2$ alkyl$)_2$, $C_1$-$C_4$ alkylsulfonyloxy, $C_1$-$C_2$ haloalkylsulfonyloxy, $OSi(CH_3)_3$, $OSi(CH_3)_2C(CH_3)_3$, $C_1$-$C_4$ alkylthio, $C_3$-$C_4$ cycloalkylthio, cyclopropylmethylthio, $C_1$-$C_4$ haloalkylthio, $C_2$-$C_4$ alkenylthio, $C_2$-$C_4$ haloalkenylthio, $C_3$-$C_4$ alkynylthio, $C_3$-$C_4$ haloalkynylthio, $C_2$-$C_4$ alkoxyalkylthio, $C_2$-$C_4$ aminoalkylthio, SH, $SP(O)(OC_1$-$C_2$ alkyl$)_2$, $C_1$-$C_4$ alkylsulfinyl, $C_3$-$C_4$ cycloalkylsulfonyl, cyclopropylmethylsulfinyl, $C_1$-$C_4$ haloalkylsulfinyl, $C_2$-$C_4$ alkenylsulfinyl, $C_2$-$C_4$ haloalkenylsulfinyl, $C_3$-$C_4$ alkynylsulfinyl, $C_3$-$C_4$ haloalkynylsulfinyl, $C_2$-$C_4$ alkoxyalkylsulfinyl, $C_2$-$C_4$ aminoalkylsulfinyl, $C_1$-$C_4$ alkylsulfonyl, $C_3$-$C_4$ cycloalkylsulfonyl, cyclopropylmethylsulfonyl, $C_1$-$C_4$ haloalkylsulfonyl, $C_2$-$C_4$ alkenylsulfonyl, $C_2$-$C_4$ haloalkenylsulfonyl, $C_3$-$C_4$ alkynylsulfonyl, $C_3$-$C_4$ haloalkynylsulfonyl, $C_2$-$C_4$ alkoxyalkylsulfonyl or $C_2$-$C_4$ aminoalkylsulfonyl;

$R_a$ and $R_b$ are independently H or $C_1$-$C_3$ alkyl;

$R_c$ is $C_2$-$C_4$ alkyl, cyclopropylmethyl, $C_2$-$C_4$ cyanoalkyl, $CH_2C(O)CH_3$, $CH_2CH_2C(O)CH_3$, $C_1$-$C_4$ haloalkyl, $C_3$-$C_4$ alkenyl, $C_3$-$C_4$ haloalkenyl, $C_3$-$C_4$ alkynyl, $C_3$-$C_4$ haloalkynyl, $C_1$-$C_4$ alkyl substituted with $C_1$-$C_4$ alkoxy, $C_1$-$C_4$ alkylthio, $C_1$-$C_4$ alkylsulfinyl; $C_1$-$C_4$ alkylsulfonyl, OH, $NH_2$, $NHCH_3$ or $N(CH_3)_2$;

X is $CH_3$, $OCH_3$, $OC_2H_5$, Cl or Br;

Y is $C_1$-$C_2$ alkyl, $C_1$-$C_2$ alkoxy, $OCH_2CH_2F$, $OCH_2CHF_2$,$OCH_2CF_3$, $NHCH_3$ or $N(CH_3)_2$; and

Z is CH or N; and

their agriculturally suitable salts;

provided that

1) when X is Cl or Br, then Z is CH and Y is $C_1$-$C_2$ alkoxy, $NHCH_3$ or $N(CH_3)_2$; and

2) when $R_1$ is $C_1$-$C_3$ alkyl then $R_2$ is other than $C_2$-$C_6$ alkoxy, $C_1$-$C_8$ alkylthio, $C_1$-$C_8$ alkylsulfinyl, $C_2$-$C_8$ alkylsulfonyl, $C_3$-$C_6$ alkenyloxy, $C_3$-$C_6$ alkynyloxy, $C_3$-$C_6$ alkenylthio, $C_3$-$C_6$ alkenylsulfinyl, $C_3$-$C_6$ alkenylsulfonyl, $C_3$-$C_6$ alkynylthio, $C_3$-$C_6$ alkynylsulfinyl, $C_3$-$C_6$ alkynylsulfonyl, $OCH_2CH_2OCH_3$, $OCH_2CH_2SCH_3$, $OCH_2CH_2S(O)CH_3$, $OCH_2CH_2SO_2CH_3$, $C_2$-$C_6$ alkyl substituted with 1-3 atoms of F, Cl or Br, $CH_2F$, $CHF_2$, $C_1$-$C_4$ alkyl substituted with $C_1$-$C_2$ alkoxy, $C_1$-$C_2$ alkylthio, $C_1$-$C_2$ alkylsulfinyl or $C_1$-$C_2$ alkylsulfonyl, $OCF_2H$, $OCH_2CH_2F$, $OCH_2CHF_2$, $OCH_2CF_3$, $OCH_2CH_2Cl$, S-cyclohexyl, S-$C_6H_5$ or $SCH_2C_6H_5$.

In the above definitions, the term "alkyl", used either alone or in compound words such as "alkylthio" or "haloalkyl", denotes straight chain or branched alkyl, e.g., methyl, ethyl, n-propyl, isopropyl or the different butyl, pentyl, hexyl, heptyl and octyl isomers.

Alkoxy denotes methoxy, ethoxy, n-propoxy, isopropoxy and the different butoxy, pentoxy or hexoxy isomers.

Alkenyl denotes straight chain or branched alkenes, e.g. vinyl, 1-propenyl, 2-propenyl, 3-propenyl and the different butenyl, pentenyl and hexenyl isomers.

Alkynyl denotes straight chain or branched alkynes, e.g., ethynyl, 1-propynyl, 2-propynyl and the different butynyl, pentynyl and hexynyl isomers.

Cycloalkyl denotes cyclopropyl, cyclobutyl, cyclopentyl and cyclohexyl.

$C_4$-$C_6$ cycloalkylalkyl means cyclopropylmethyl through cyclopropylpropyl or cyclopentylmethyl.

The term "halogen", either alone or in compound words such as "haloalkyl", denotes fluorine, chlorine, bromine or iodine. Further, when used in compound words such as "haloalkyl" said alkyl may be partially or fully substituted with halogen atoms, which may be the same or different. Examples of haloalkyl include $CH_2CH_2F$, $CF_2CF_3$ and $CH_2CHFCl$.

The total number of carbon atoms in a substituent group is indicated by the $C_i$-$C_j$ prefix where i and j are numbers from 1 to 8. For example, $C_1$-$C_3$ alkylsulfonyl would designate methylsulfonyl through propylsulfonyl, $C_2$ alkoxyalkoxy would designate $OCH_2OCH_3$; $C_4$ alkoxyalkoxy would designate the various isomers of an alkoxy group substituted with a second alkoxy group containing a total of 4 carbon atoms, examples including $OCH_2OCH_2CH_2CH_3$ and $OCH_2CH_2OCH_2CH_3$; $C_2$ cyanoalkyl would designate $CH_2CN$ and $C_3$ cyanoalkyl would designate $CH_2CH_2CN$ and $CH(CN)CH_3$; $C_4$ aminoalkoxy would designate the various isomers of an alkoxy group substituted by an amino, alkylamino or dialkylamino group containing a total of 4 carbon atoms, examples including $OCH_2CH_2CH_2CH_2NH_2$ and $OCH_2CH_2N(CH_3)_2$; as further example, CH(H, $C_1$-$C_3$ alkyl)O$C_1$-$C_2$ alkyl would designate the various isomers of a methyl group substituted by a $C_1$-$C_2$ alkoxy group and optionally substituted by a $C_1$-$C_3$ alkyl group, examples including $CH_2OCH_3$ and $CH(CH_2CH_2CH_3)OCH_2CH_3$.

Compounds of the invention which are preferred for their higher herbicidal activity, greater plant growth regulant activity and/or more favorable ease of synthesis are:

1) Compounds of Formula I wherein

$R_2$ is $C_2$-$C_6$ alkoxy, $C_1$-$C_8$ alkylthio, $C_1$-$C_8$ alkylsulfinyl, $C_2$-$C_8$ alkylsulfonyl, $C_3$-$C_6$ alkenyloxy, $C_3$-$C_6$ alkynyloxy, $C_3$-$C_6$ alkenylthio, $C_3$-$C_6$ alkenylsulfinyl, $C_3$-$C_6$ alkenylsulfonyl, $C_3$-$C_6$ alkynylthio, $C_3$-$C_6$ alkynylsulfinyl, $C_3$-$C_6$ alkynylsulfonyl, $OCH_2CH_2OCH_3$, $OCH_2CH_2SCH_3$, $OCH_2CH_2S(O)CH_3$, $OCH_2CH_2SO_2CH_3$,$C_2$-$C_6$ alkyl substituted with 1-3 atoms of F, Cl or Br, $CH_2F$, $CHF_2$, $C_1$-$C_4$ alkyl substituted with $C_1$-$C_2$ alkoxy, $C_1$-$C_2$ alkylthio, $C_1$-$C_2$ alkylsulfinyl or $C_1$-$C_2$ alkylsulfonyl, $OCF_2H$, $OCH_2CH_2F$, $OCH_2CHF_2$, $OCH_2CF_3$, $OCH_2CH_2Cl$, S-cyclohexyl, $SC_6H_5$ or $SCH_2C_6H_5$;

2) Compounds of Preferred (1) where

$R_2$ is $CH_3S$, $C_3$-$C_6$ alkenyloxy alkynyloxy, $C_3$-$C_6$ alkenylthio, $C_3$-$C_6$ alkenylsulfinyl, $C_3$-$C_6$ alkenylsulfonyl, $C_3$-$C_6$ alkynylthio, $C_3$-$C_6$ alkynylsulfinyl, $C_3$-$C_6$ alkynylsulfonyl, $C_2$-$C_6$ alkyl substituted with 1-3 atoms of F, Cl or Br, $CH_2F$, $CHF_2$, $C_1$-$C_4$ alkyl substituted in a nonbenzylic position with $C_1$-$C_2$ alkoxy, $C_1$-$C_2$ alkylthio, $C_1$-$C_2$ alkyl sulfinyl or $C_1$-$C_2$ alkylsulfonyl, $OCH_2CH_2OCH_3$, $OCH_2CH_2SCH_3$, $OCH_2CH_2S(O)CH_3$, $OCH_2CH_2SO_2CH_3$,$OCF_2H$, $OCH_2CH_2F$, $OCH_2CHF_2$, $OCH_2CF_3$, $OCH_2CH_2Cl$, S-cyclohexyl, $SC_6H_5$ or $SCH_2C_6H_5$.

3) Compounds of Preferred (2) wherein R is H;

4) Compounds of Preferred (3) wherein

$R_2$ is $CH_3S$, $C_3$-$C_4$ alkenyloxy, $C_3$-$C_4$ alkenylthio, $C_3$-$C_4$ alkenylsulfinyl, $C_3$-$C_4$ alkenylsulfonyl, $C_2$-$C_3$ alkyl substituted with 1-3 atoms of F or Cl, $CH_2F$, $CHF_2$, $OCHF_2$, $OCH_2CH_2F$, $OCH_2CF_3$ or $OCH_2CH_2Cl$.

5) Compounds of Preferred (4) wherein

$R_1$ is $CH_3$ or $CH_2CH_3$;

X is $CH_3$, $OCH_3$ or Cl; and

Y is $CH_3$, $OCH_3$, $C_2H_5$ or $OC_2H_5$.

6) Compounds of Preferred (5) wherein

$R_2$ is allyloxy, allylthio, propargyloxy, propargylthio, $CH_2F$, $OCHF_2$, $OCH_2CH_2F$ or $CH_3S$.

7) Compounds of Preferred (1) wherein

$R_2$ is $C_2$-$C_6$ alkoxy, $C_2$-$C_8$ alkylthio, $C_2$-$C_8$ alkylsulfonyl, $C_1$-$C_8$ alkylsulfinyl, CH(H, $C_1$-$C_3$ alkyl)-O$C_1$-$C_2$ alkyl, CH(H, $C_1$-$C_3$ alkyl)-S$C_1$-$C_2$ alkyl, CH(H, $C_1$-$C_3$ alkyl)S(O)$C_1$-$C_2$ alkyl or CH(H, $C_1$-$C_3$ alkyl)$SO_2C_1$-$C_2$ alkyl.

8) Compounds of Preferred (7) wherein R is H.

9) Compounds of Preferred (8) wherein

$R_2$ is $C_2$-$C_4$ alkoxy, $C_2$-$C_4$ alkylthio, $C_2$-$C_4$ alkylsulfonyl, $C_1$-$C_4$ alkylsulfinyl, $CH_2OC_1$-$C_2$ alkyl, $CH_2SC_1$-$C_2$ alkyl, $CH_2S(O)C_1$-$C_2$ alkylor $CH_2SO_2C_1$-$C_2$ alkyl.

10) Compounds of Preferred (9) wherein

$R_1$ is $CH_3$ or $C_2H_5$

X is $CH_3$, $OCH_3$ or Cl; and

Y is $CH_3$, $OCH_3$, $C_2H_5$ or $OC_2H_5$.

11) Compounds of Preferred (10) wherein

$R_2$ is $C_2$-$C_3$ alkoxy, $C_2$-$C_3$ alkylthio, $C_1$-$C_3$ alkylsulfinyl, $CH_2OCH_3$, $CH_2SCH_3$, $CH_2S(O)CH_3$ or $CH_2SO_2CH_3$.

12) Compounds of Formula I wherein

$R_2$ is $C_3$-$C_6$ cycloalkoxy, $C_4$-$C_6$ cycloalkylalkoxy, $OCF_2Cl$, $OCF_2Br$, $OCF_3$, $OCF_2CF_2H$, $OCF_2CHFCl$, $OCF_2CHFBr$, $OCF_2CF_3$, $OCH_2CH_2Br$, $OCH_2CCl_3$, $C_3$-$C_6$ haloalkoxy, vinyloxy, $C_2$-$C_6$ haloalkenyloxy, $C_3$-$C_6$ haloalkynyloxy, $OCH_2OCH_3$, $OCH_2OCH_2CH_3$, $OCH(CH_3)OCH_3$, $C_4$ alkoxyalkoxy, $C_2$-$C_4$ haloalkoxyalkoxy, $OCH_2SCH_3$, $OCH_2SCH_2CH_3$, $OCH(CH_3)SCH_3$, $C_4$ alkylthioalkoxy, $C_2$-$C_4$ haloalkylthioalkoxy, $OCH_2S(O)CH_3$, $OCH_2S(O)CH_2CH_3$, $OCH(CH_3)S(O)CH_3$, $C_4$ alkylsulfinylalkoxy, $C_2$-$C_4$ haloalkylsulfinylalkoxy, $OCH_2SO_2CH_3$, $OCH_2SO_2CH_2CH_3$, $OCH(CH_3)SO_2CH_3$, $C_4$ alkylsulfonylalkoxy, $C_2$-$C_4$ haloalkylsulfonylalkoxy, $C_2$-$C_4$ cyanoalkoxy, $OCH_2C(O)CH_3$, $OCH_2CH_2C(O)CH_3$, $C_2$-$C_4$ aminoalkoxy, $C_3$-$C_5$ cycloalkylthio, $C_4$-$C_6$ cycloalkylalkylthio, $SCF_2H$, $SCF_2Cl$, $SCF_2Br$, $SCF_3$, $C_2$-$C_8$ haloalkylthio, vinylthio, $C_2$-$C_6$ haloalkenylthio, $C_3$-$C_6$ haloalkynylthio, $C_2$-$C_4$ alkoxyalkylthio, $C_2$-$C_4$ haloalkoxyalkylthio, $C_2$-$C_4$ alkylthioalkylthio, $C_2$-$C_4$ haloalkylthioalkylthio, $C_2$-$C_4$ cyanoalkylthio, $SCH_2C(O)CH_3$, $SCH_2CH_2C(O)CH_3$, $C_2$-$C_4$ aminoalkylthio, $C_3$-$C_6$ cycloalkylsulfinyl, $C_4$-$C_6$ cycloalkylalkylsulfinyl, $C_1$-$C_8$ haloalkylsulfinyl, vinylsulfinyl, $C_2$-$C_6$ haloalkenylsulfinyl, $C_3$-$C_6$ haloalkynylsulfinyl, $C_2$-$C_4$ alkoxyalkylsulfinyl, $C_2$-$C_4$ haloalkoxyalkylsulfinyl, $C_2$-$C_4$ cyanoalkylsulfinyl, $S(O)CH_2C(O)CH_3$, $S(O)CH_2CH_2C(O)CH_3$, $C_2$-$C_4$ aminoalkylsulfinyl, $C_3$-$C_6$ cycloalkylsulfonyl, $C_4$-$C_6$ cycloalkylalkylsulfonyl, $C_1$-$C_8$ haloalkylsulfonyl, vinylsulfonyl, $C_2$-$C_6$ haloalkenylsulfonyl, $C_3$-$C_6$ haloalkynylsulfonyl, $C_2$-$C_4$ alkoxyalkylsulfonyl, $C_2$-$C_4$ haloalkoxysulfonyl, $C_2$-$C_4$ cyanoalkylsulfonyl, $SO_2CH_2C(O)CH_3$, $SO_2CH_2CH_2C(O)CH_3$, $C_2$-$C_4$ aminoalkylsulfonyl, $CH_2Cl$, $CHCl_2$, $CH_2Br$, $CHBr_2$, $C_2$-$C_6$ alkenyl, $C_2$-$C_6$ haloalkenyl, $C_2$-$C_6$ haloalkynyl, $OC(O)C_1$-$C_4$ alkyl, $CH_2C(O)NR_aR_b$,$NHCH_3$, $NR_bR_c$ or $C_1$-$C_4$ alkyl substituted with $C_3$-$C_4$ alkoxy, $C_3$-$C_4$ cycloalkoxy, cyclopropylmethoxy, $C_1$-$C_4$ haloalkoxy, $C_2$-$C_4$ alkenyloxy, $C_2$-$C_4$ haloalkenyloxy, $C_3$-$C_4$ alkynyloxy, $C_3$-$C_4$ haloalkynyloxy, $C_2$-$C_4$ alkoxyalkoxy, $C_2$-$C_4$ aminoalkoxy, $C_1$-$C_4$ alkylcarbonyloxy, $C_1$-$C_4$ haloalkylcarbonyloxy, $C_1$-$C_4$ carbamoyloxy, $C_1$-$C_4$ alkoxycarbonyloxy, OH, $OP(O)(OC_1$-$C_2$ alkyl$)_2$, $C_1$-$C_4$ alkylsulfonyloxy, $C_1$-$C_2$ haloalkylsulfonyloxy, $OSi(CH_3)_3$, $OSi(CH_3)_2C(CH_3)_3$, $C_3$-$C_4$ alkylthio, $C_3$-$C_4$ cycloalkylthio, cyclopropylmethylthio, $C_1$-$C_4$ haloalkylthio, $C_2$-$C_4$ alkenylthio, $C_2$-$C_4$ haloalkenylthio, $C_3$-$C_4$ alkynylthio, $C_3$-$C_4$ haloalkynylthio, $C_2$-$C_4$ alkoxyalkylthio, $C_2$-$C_4$ aminoalkylthio, SH, $SP(O)(OC_1$-$C_2$ alkyl$)_2$,$C_3$-$C_4$ alkylsulfinyl, $C_3$-$C_4$ cycloalkylsulfinyl, cyclopropylmethylsulfinyl, $C_1$-$C_4$ haloalkylsulfinyl, $C_2$-$C_4$ alkenylsulfinyl, $C_2$-$C_4$ haloalkenylsulfinyl, $C_3$-$C_4$ alkynylsulfinyl, $C_3$-$C_4$ haloalkynylsulfinyl, $C_2$-$C_4$ alkoxyalkylsulfinyl, $C_2$-$C_4$ aminoalkylsulfinyl, $C_3$-$C_4$ alkylsulfonyl, $C_3$-$C_4$ cycloalkylsulfonyl, cyclopropylmethylsulfonyl, $C_1$-$C_4$ haloalkylsulfonyl, $C_2$-$C_4$ alkenylsulfonyl, $C_2$-$C_4$ haloalkenylsulfonyl, $C_3$-$C_4$ alkynylsulfonyl, $C_3$-$C_4$ haloalkynylsulfonyl, $C_2$-$C_4$ alkoxyalkylsulfonyl or $C_2$-$C_4$ aminoalkylsulfonyl.

13) Compounds of Preferred (12) wherein

$R_2$ is $C_3$-$C_6$ cycloalkoxy, $C_4$-$C_6$ cycloalkylalkoxy, $OCF_2Cl$, $OCF_2Br$, $OCF_3$, $OCF_2CF_2H$, $OCF_2CHFCl$, $OCF_2CHFBr$, $OCF_2CF_3$, $OCH_2CH_2Br$, $OCH_2CCl_3$, $C_3$-$C_6$ haloalkoxy, vinyloxy, $C_2$-$C_6$ haloalkenyloxy, $OCH_2OCH_3$, $OCH_2OCH_2CH_3$,$OCH(CH_3)OCH_3$, $C_4$ alkoxyalkoxy, $C_2$-$C_4$ haloalkoxyalkoxy, $OCH_2SCH_3$, $OCH_2SCH_2CH_3$,$OCH(CH_3)SCH_3$, $C_4$ alkylthioalkoxy, $C_2$-$C_4$ haloalkylthioalkoxy, $OCH_2S(O)CH_3$, $OCH_2S(O)CH_2CH_3$, $OCH(CH_3)S(O)CH_3$, $C_4$ alkylsulfinylalkoxy, $C_2$-$C_4$ haloalkylsulfinylalkoxy, $OCH_2SO_2CH_3$, $OCH_2SO_2CH_2CH_3$, $OCH(CH_3)SO_2CH_3$, $C_4$ alkylsulfonylalkoxy, $C_2$-$C_4$ haloalkylsulfonylalkoxy, $C_2$-$C_4$ cyanoalkoxy, $OCH_2C(O)CH_3$, $OCH_2CH_2C(O)CH_3$, $C_2$-$C_4$ aminoalkoxy, $C_3$-$C_5$ cycloalkylthio, $SCF_3$, $SCF_2Cl$, $SCF_2Br$, $C_4$-$C_6$ cycloalkylalkylthio, $SCF_2H$, $C_2$-$C_6$ haloalkylthio, vinylthio, $C_2$-$C_6$ haloalkenylthio, $C_2$-$C_4$ alkoxyalkylthio, $C_2$-$C_4$ haloalkoxyalkylthio, $C_2$-$C_4$ alkylthioalkylthio, $C_2$-$C_4$ haloalkylthioalkylthio, $C_2$-$C_4$ cyanoalkylthio, $SCH_2C(O)CH_3$, $SCH_2CH_2C(O)CH_3$, $C_2$-$C_4$ aminoalkylthio, $C_3$-$C_6$ cycloalkylsulfinyl, $C_4$-$C_6$ cycloalkylalkylsulfinyl, $C_1$-$C_6$ haloalkylsulfinyl, vinylsulfinyl, $C_2$-$C_6$ haloalkenylsulfinyl, $C_2$-$C_4$ alkoxyalkylsulfinyl, $C_2$-$C_4$ haloalkoxyalkylsulfinyl, $C_2$-$C_4$ cyanoalkylsulfinyl, $S(O)CH_2C(O)CH_3$, $S(O)CH_2CH_2C(O)CH_3$, $C_2$-$C_4$ aminoalkylsulfinyl, $C_3$-$C_6$ cycloalkylsulfonyl, $C_4$-$C_6$ cycloalkylalkylsulfonyl, $C_1$-$C_6$ haloalkylsulfonyl, vinylsulfonyl, $C_2$-$C_6$ haloalkenylsulfonyl, $C_2$-$C_4$ alkoxyalkylsulfonyl, $C_2$-$C_4$ haloalkoxysulfonyl, $C_2$-$C_4$ cyanoalkylsulfonyl, $SO_2CH_2C(O)CH_3$, $SO_2CH_2CH_2(O)CH_3$, $C_2$-$C_4$ aminoalkylsulfonyl, $CHBr_2$, $C_2$-$C_6$ alkenyl substituted by one or more fluorine or bromine atoms. $C_3$-$C_6$ haloalkynyl, $NR_bR_c$, CH(H, $C_1$-$C_3$ alkyl)$OC_1$-$C_4$ haloalkyl having 1 or 2 halogen atoms, CH(H, $C_1$-$C_3$ alkyl)$OC_2$-$C_4$ haloalkenyl, CH(H, $C_1$-$C_3$ alkyl)$OC_3$-$C_5$ alkynyl, CH(H, $C_1$-$C_3$ alkyl)$OC_2$-$C_4$ alkoxyalkyl, CH(H, $C_1$-$C_3$ alkyl)$SC_1$-$C_4$ haloalkyl having 1 or 2 halogen atoms, CH(H, $C_1$-$C_3$ alkyl)$SC_2$-$C_4$ haloalkenyl, CH(H, $C_1$-$C_3$ alkyl)$SC_3$-$C_4$ alkynyl, CH(H, $C_1$-$C_3$ alkyl)$SC_2$-$C_4$ alkoxyalkyl, CH(H, $C_1$-$C_3$ alkyl)$S(O)C_1$-$C_4$

haloalkyl having 1 or 2 halogen atoms, CH(H, $C_1$-$C_3$ alkyl)S(O)$C_2$-$C_4$ haloalkenyl, CH(H, $C_1$-$C_3$ alkyl)S(O)$C_3$-$C_4$ alkynyl, CH(H, $C_1$-$C_3$ alkyl)S(O)$C_2$-$C_4$ alkoxyalkyl, CH(H, $C_1$-$C_3$ alkyl)$SO_2C_1$-$C_4$ haloalkyl having 1 or 2 halogen atoms, CH(H, $C_1$-$C_3$ alkyl)$SO_2C_2$-$C_4$ haloalkenyl, CH(H, $C_1$-$C_3$ alkyl)$SO_2C_3$-$C_4$ alkynyl, CH(H, $C_1$-$C_3$ alkyl)$SO_2C_2$-$C_4$ alkoxyalkyl, or $C_1$-$C_4$ alkyl substituted in a nonbenzylic position with $C_3$-$C_4$ alkoxy, $C_1$-$C_4$ haloalkoxy, $C_1$-$C_4$ alkylcarbonyloxy, $C_1$-$C_4$ haloalkylcarbonyloxy, $C_1$-$C_4$ carbamoyloxy, alkoxycarbonyloxy, $C_1$-$C_4$ alkylsulfonyloxy, $C_1$-$C_2$ haloalkylsulfonyloxy, $C_3$-$C_4$ alkylthio, $C_1$-$C_4$ haloalkylthio, $C_3$-$C_4$ alkylsulfinyl or $C_3$-$C_4$ alkylsulfonyl; and

$R_c$ is $C_2$-$C_4$ cyanoalkyl, $CH_2C(O)CH_3$, $CH_2CH_2C(O)CH_3$, $C_1$-$C_4$ haloalkyl, $C_3$-$C_4$ alkenyl, $C_3$-$C_4$ haloalkenyl, $C_3$-$C_4$ alkynyl or $C_3$-$C_4$ haloalkynyl:

14) Compounds of Preferred (13) wherein R is H.

15) Compounds of Preferred (14) wherein

$R_2$ is $OCF_3$, $OCF_2CF_3$, $OCH_2CH_2Br$, $C_3$ haloalkoxy, $OCH_2CH_2OCH_2CH_3$, $SCF_2H$, $SCF_3$, $C_2$ haloalkylthio, $C_2$-$C_3$ alkoxyalkylthio, $C_1$-$C_2$ haloalkylsulfinyl, $C_1$-$C_2$ haloalkylsulfonyl, $CH_2OC_1$-$C_2$ haloalkyl having 1 or 2 halogen atoms, $CH_2SC_1$-$C_2$ haloalkyl having 1 or 2 halogen atoms, $CH_2S(O)C_1$-$C_2$ haloalkyl having 1 or 2 halogen atoms, or $CH_2SO_2C_1$-$C_2$ haloalkyl having 1 or 2 halogen atoms.

16) Compounds of Preferred (15) wherein

$R_1$ is $CH_3$ or $CH_2CH_3$;

X is $CH_3$, $OCH_3$ or Cl; and

Y is $CH_3$, $OCH_3$, $C_2H_5$ or $OC_2H_5$.

17) Compounds of Preferred (16) wherein

$R_2$ is $OCF_2CF_3$, $SCF_2H$, $SCH_2CH_2OCH_3$, $S(O)CF_2H$, $SO_2CF_2H$, $CH_2OCH_2CH_2F$ or $CH_2OCH_2CHF_2$.

18) Compounds of Preferred (12) wherein

$R_2$ is $C_3$-$C_6$ haloalkynyloxy, $C_7$-$C_8$ haloalkylthio, $C_3$-$C_6$ haloalkynylthio, $C_7$-$C_8$ haloalkylsulfinyl, $C_3$-$C_6$ haloalkynylsulfinyl, $C_7$-$C_8$ haloalkylsulfonyl, $C_3$-$C_6$ haloalkynylsulfonyl, $CH_2Cl$, $CHCl_2$, $CH_2Br$, $C_2$-$C_6$ alkenyl, $C_2$-$C_6$ alkenyl substituted with one or more chlorine atoms, $C_2$ haloalkynyl, $OC(O)C_1$-$C_4$ alkyl,$CH_2C(O)NR_aR_b$, $NHCH_3$, $NR_bR_c$, CH(H, $C_1$-$C_3$ alkyl)$OC_3$-$C_4$ alkyl, CH(H, $C_1$-$C_3$ alkyl)$OC_1$-$C_4$ haloalkyl having 3 or more halogen atoms, CH(H, $C_1$-$C_3$ alkyl)OC(O)$C_1$-$C_3$ alkyl, CH(H, $C_1$-$C_3$ alkyl)OC(O)$C_1$-$C_3$ haloalkyl, CH(H, $C_1$-$C_3$ alkyl)$OC_1$-$C_4$ carbamoyl, CH(H, $C_1$-$C_3$ alkyl)-OC(O)$OC_1$-$C_3$ alkyl, CH(H, $C_1$-$C_3$ alkyl)$OSO_2C_1$-$C_4$ alkyl, CH(H, $C_1$-$C_3$ alkyl)$OSO_2C_1$-$C_2$ haloalkyl, CH(H, $C_1$-$C_3$ alkyl)$SC_3$-$C_4$ alkyl, CH(H, $C_1$-$C_3$ alkyl)$SC_1$-$C_4$ haloalkyl having 3 or more halogen atoms, CH(H, $C_1$-$C_3$ alkyl)S(O)$C_3$-$C_4$ alkyl, CH(H, $C_1$-$C_3$ alkyl)S(O)$C_1$-$C_4$ haloalkyl having 3 or more halogen atoms, CH(H, $C_1$-$C_3$ alkyl)-$SO_2C_3$-$C_4$ alkyl, CH(H, $C_1$-$C_3$ alkyl)$SO_2C_1$-$C_4$ haloalkyl having 3 or more halogen atoms, $C_1$-$C_4$ alkyl substituted in a nonbenzylic position by $C_2$-$C_4$ haloalkenyloxy, $C_3$-$C_4$ alkynyloxy, $C_2$-$C_4$ alkoxyalkoxy, $C_2$-$C_4$ haloalkenylthio, $C_3$-$C_4$ alkynylthio, $C_2$-$C_4$ alkoxyalkylthio, $C_1$-$C_4$ haloalkylsulfinyl, $C_2$-$C_4$ haloalkenylsulfinyl, $C_3$-$C_4$ alkynylsulfinyl, $C_2$-$C_4$ alkoxyalkylsulfinyl, $C_1$-$C_4$ haloalkylsulfonyl, $C_2$-$C_4$ haloalkenylsulfonyl, $C_3$-$C_4$ alkynylsulfonyl, or $C_2$-$C_4$ alkoxyalkylsulfonyl, or $C_1$-$C_4$ alkyl substituted by $C_3$-$C_4$ cycloalkoxy, cyclopropylmethoxy, $C_2$-$C_4$ alkenyloxy, $C_3$-$C_4$ haloalkynyloxy, $C_2$-$C_4$ aminoalkoxy, OH, $OP(O)(OC_1$-$C_2$ alkyl$)_2$, $OSi(CH_3)_3$, $OSi(CH_3)_2C(CH_3)_3$, cycloalkylthio, cyclopropylmethylthio, $C_2$-$C_4$ alkenylthio, $C_3$-$C_4$ haloalkynylthio, $C_2$-$C_4$ aminoalkylthio, SH, $SP(O)(OC_1$-$C_2$ alkyl$)_2$, $C_3$-$C_4$ cycloalkylsulfinyl, cyclopropylmethylsulfinyl, $C_2$-$C_4$ alkenylsulfinyl, $C_3$-$C_4$ haloalkynylsulfinyl, $C_2$-$C_4$ aminoalkylsulfinyl, $C_3$-$C_4$ cycloalkylsulfonyl, cyclopropylmethylsulfonyl, $C_2$-$C_4$ alkenylsulfonyl, $C_3$-$C_4$ haloalkynylsulfonyl, or $C_2$-$C_4$ aminoalkylsulfonyl; and

$R_c$ is $C_2$-$C_4$ alkyl, cyclopropylmethyl, or $C_1$-$C_4$ alkyl substituted with $C_1$-$C_4$ alkoxy, $C_1$-$C_4$ alkylthio, $C_1$-$C_4$ alkylsulfinyl, $C_1$-$C_4$ alkyl sulfonyl, OH, $NH_2$, $NHCH_3$ or $N(CH_3)_2$.

19) Compounds of Preferred (18) wherein R is H.

20) Compounds of Preferred (19) wherein

$R_2$ is $OCF_2CF_2H$, $C_2$-$C_3$ cyanoalkoxy, $C_2$-$C_3$ aminoalkoxy, $C_2$-$C_4$ cyanoalkylthio, $C_2$-$C_3$ alkenyl, $CH_2C(O)NR_aR_b$, $NHCH_3$, $NR_bR_c$, $CH_2OC_1$-$C_2$ haloalkyl having 3 or more halogen atoms, $CH_2OC(O)C_1$-$C_2$ alkyl, $CH_2OSO_2C_1$-$C_2$ alkyl, $CH_2SC_1$-$C_2$ haloalkyl having 3 or more halogen atoms, $CH_2S(O)C_1$-$C_2$ haloalkyl having 3 or more halogen atoms or $CH_2SO_2C_1$-$C_2$ haloalkyl having 3 or more halogen atoms.

21) Compounds of Preferred (20) wherein

$R_1$ is $CH_3$ or $C_2H_5$;

X is $CH_3$, $OCH_3$ or Cl; and

Y is $CH_3$, $OCH_3$, $C_2H_5$ or $OC_2H_5$.

22) Compounds of Preferred (21) wherein

$R_2$ is $OCF_2CF_2H$, $OCH_2CH_2CN$, $SCH_2CH_2CN$, $CH=CH_2$, $CH_2C(O)NH_2$, $NHCH_3$, $CH_2OCH_2CF_3$, $CH_2OC(O)CH_3$ or $CH_2OSO_2CH_3$.

This invention further relates to the method of use of the compound

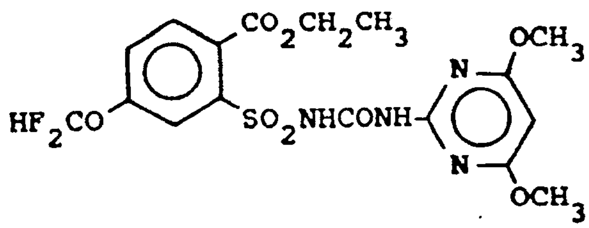

for weed control on a rice crop because of its selectivity to rice, especially Japonica rice or paddy rice, and activity against undesired plant growth and also the method of use for blackgrass control on wheat and barley with safety to the crop.

## DETAILED DESCRIPTION OF THE INVENTION

### Synthesis

The compounds of Formula I can be prepared by one or more of the following methods described in Equations 1 to 3 and 14. Reagents and reaction conditions are given by way of illustration.

As shown in Equation 1. compounds of Formula I can be prepared by reacting an appropriately substituted sulfonyl isocyanate of Formula (1) with an appropriate amino or methylamino heterocycle of Formula (2).

A is 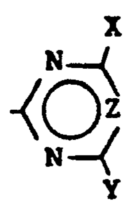 , and R, $R_1$, $R_2$, X, Y and Z are as previously defined.

**Equation 1**

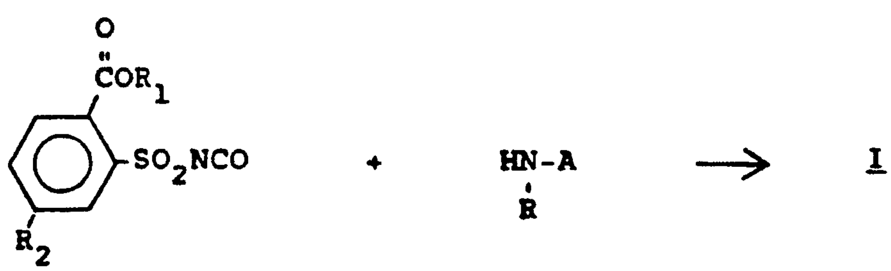

(1) (2)

The reaction is best carried out in inert aprotic organic solvents such as dichloromethane, 1,2-dichloroethane, tetrahydrofuran, or acetonitrile, at a temperature between 20° and 85° C. The order of addition is not critical; however, it is often convenient to add the sulfonyl isocyanate or a solution of it in the reaction solvent to a stirred suspension of the amine.

In some cases, the desired product is insoluble in the reaction solvent at ambient temperature and crystallizes from it in pure form. Products soluble in the reaction solvent are isolated by evaporation of the solvent. Compounds of Formula I then may be purified by trituration of the evaporation residue with solvents such as 1-chlorobutane or ethyl ether and filtration, by recrystallization from mixtures of solvents such as 1,2-dichloroethane, 1-chlorobutane, and heptane, or by chromatography on silica gel.

Many of the compounds of Formula I can be prepared by the procedure shown in Equation 2, where A, R, $R_1$, and $R_2$ are as previously defined.

Equation 2

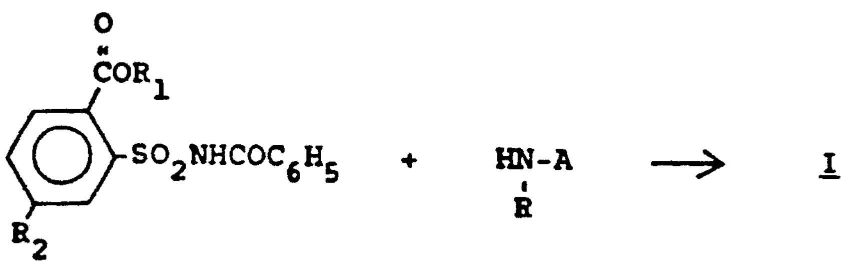

(3) (2)

The reaction shown in Equation 2 is carried out by contacting phenyl carbamates of Formula (3) with aminoheterocycles of Formula (2) in an inert organic solvent such as dioxane or tetrahydrofuran at temperatures of about 20-100 °C for a period of about one-half to twenty-four hours. The product can be isolated by evaporation of the reaction solvent and purified by methods previously described.

Phenyl carbamates of Formula (3) can be prepared by the methods described, or modifications thereof known to those skilled in the art, in European Patent Application 81810282.4 (Publication No. 44.808), published January 27, 1982; or South African Patent Application 825042 (EP-A-70,802).

Alternatively, many of the compounds of Formula I can be prepared by the method described in Equation 3, where A, $R_1$, and $R_2$ are as previously defined and R is H.

Equation 3

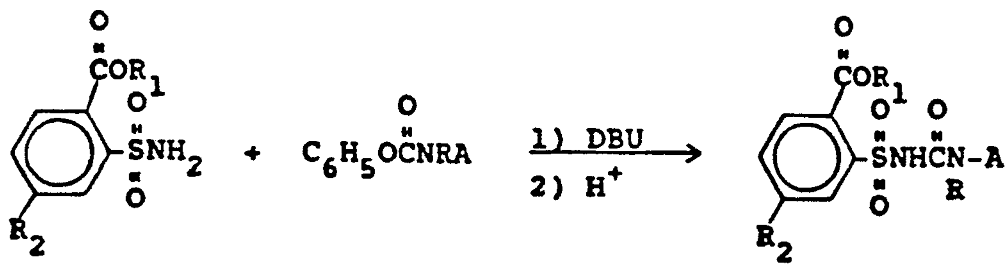


(4) (5. R=H) I, R=H

The reaction of Equation 3 can be carried out by contacting equimolar amounts of a sulfonamide of Formula (4) with a heterocyclic phenyl carbamate of Formula (5) in the presence of an equimolar amount of 1,8-diazabicyclo-[5.4.0]undec-7-ene (DBU), by methods analogous to those described in South African Patent Application 830441 (EP-A-85,028). The phenyl carbamate of Formula (5) can be prepared by methods, or modifications thereof known to those skilled in the art, described in South African Patent Application 825671 (EP-A-72,347) and South African Patent Application 825045 (EP-A-70,804).

Some of the compounds of Formula I may be prepared from compounds of Formula I where $R_2$ is OH, $NR_bH$ or SH. See Equation 14 for a discussion of this method.

For compounds of Formula I, the unsubstituted and substituted alkoxy, allyloxy and propargyloxy benzenesulfonamide intermediates of Formula (4a) can be prepared by one or more of the following general methods.

As shown in Equation 4, one highly useful general route starts from the phenol (6).

Equation 4

(a)

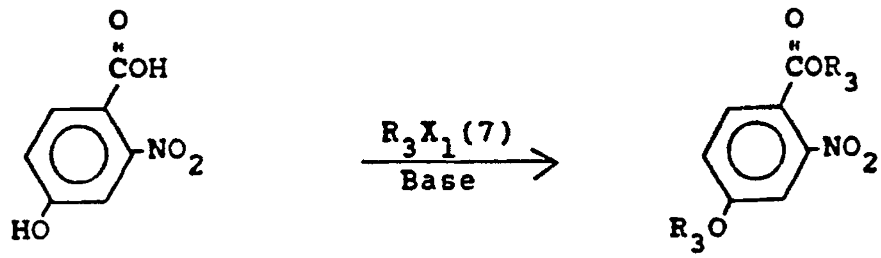


(6) (8)

(b)

(8)

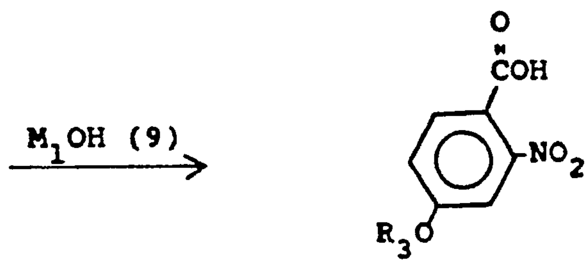


(10)

(c)

(10)

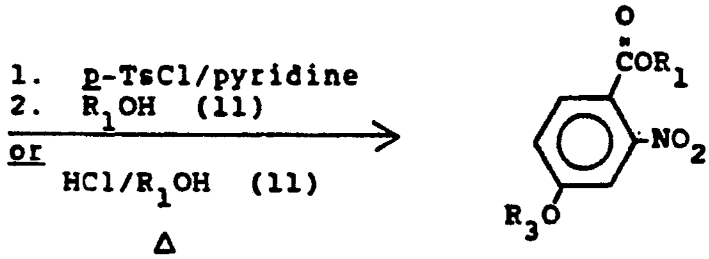


(12)

(d)

(12)

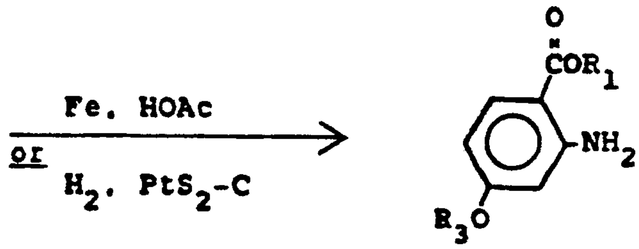


(13)

(e)

(13)

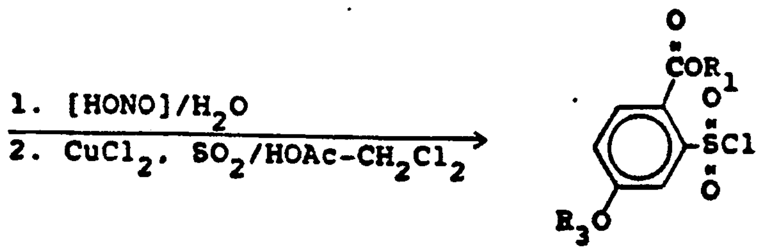


(14)

(f)

(14)

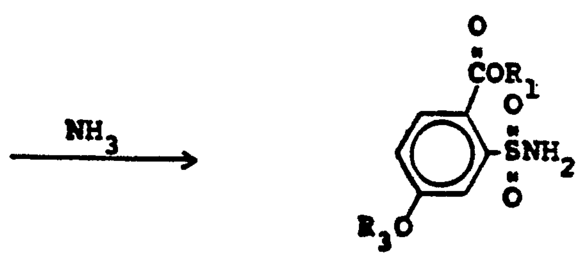


(4a)

where $R_1$ is as previously defined, and $R_3$ is $C_2$-$C_6$ alkyl, $C_3$-$C_6$ cycloalkyl, $C_4$-$C_6$ cycloalkylalkyl, $C_1$-$C_6$haloalkyl, $C_3$-$C_6$ alkenyl, $C_3$-$C_6$ haloalkenyl, $C_3$-$C_6$ haloalkynyl, $C_3$-$C_6$ alkynyl, $C_2$-$C_4$ alkoxyalkyl, $C_2$-$C_4$ haloalkoxyalkyl, $C_2$-$C_4$ alkylthioalkyl, $C_2$-$C_4$ haloalkylthioalkyl, $CH_2C(O)CH_3$, $CH_2CH_2C(O)CH_3$, or benzyl. $M_1$ is

Na or K. $X_1$ is Cl, Br, I, $O\overset{O}{\underset{O}{S}}CH_3$, 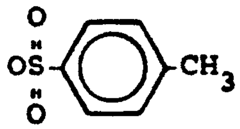, $O\overset{O}{\underset{O}{S}}OR_3$,

or $\overset{\oplus}{O}(R_3)_2$ $\overset{\ominus}{BF_4}$, or $O\overset{O}{\underset{O}{S}}CF_3$.

A solution or suspension of 4-hydroxy-2-nitrobenzoic acid (6) in a suitable Polar aprotic solvent, such as dichloromethane or N,N-dimethylformamide (DMF), is treated with at least two equivalents of $R_3X_1$ (7) in the presence of at least two equivalents of a suitable base such as N,N-diisopropylethylamine or potassium carbonate and at a temperature between 20 and 155 °C for four to sixteen hours (Equation 4a). If the solvent is miscible with water, it is then evaporated, and the residue is taken up in dichloromethane. The product solution is washed with aqueous sodium or potassium carbonate solution and aqueous hydrochloric acid, and then is dried over a suitable desiccant such as magnesium sulfate. Filtration and evaporation of the solvent leaves (8) in semipurified form. It may be further purified through recrystallization or chromatography on a column of silica gel. The requisite alkylating, alkenylating, and alkynylating agents $R_3X_1$ (7) are either known or may be made by a wide variety of methods known in the art.

In cases where $R_3$ is not the same as $R_1$, the ester (8) is treated with at least one equivalent of sodium or potassium hydroxide in a mixture of water and a suitable organic cosolvent such as ethanol or p-dioxane at a temperature between 20 and 100 °C for four to sixteen hours (Equation 4b). The reaction mixture is then acidified with concentrated hydrochloric acid. If the product separates out in crystalline form, it is collected. Otherwise the aqueous solution is extracted with ether. The ether solution is dried over sodium sulfate and filtered, and the solvent is evaporated to afford the carboxylic acid (10).

To convert carboxylic acid (10) to ester (12), a solution of it in pyridine is treated sequentially with p-toluenesulfonyl chloride and $R_1OH$ (11) according to the general procedure of J. H. Brewster and C. J. Ciotti, Jr., J. Am. Chem. Soc. 1955, 77, 6214.

Alternatively, the carboxylic acid (10) may be converted to ester (12) through the use of excess $R_1OH$ (11) and a strong acid catalyst such as hydrogen chloride as reviewed by C. A. Buehler and D. E. Pearson, Survey of Organic Syntheses, Wiley-Interscience, New York, 1970, pp 802-807 (Equation 4c).

The nitrobenzene (12) is reduced to the aniline (13) either by use of iron in acetic acid as reviewed by C. A. Buehler and D. E. Pearson (ibid, pp 413-414) or by hydrogenation using platinum sulfide as catalyst and the conditions of F. S. Dovel and H. Greenfield, J. Am. Chem. Soc., 87, 2767 (1965) (Equation 4d). When the other substituents present are not potentially susceptible to hydrogenation or hydrogenolysis, palladium may replace platinum sulfide as catalyst.

The aniline (13) is converted to the sulfonyl chloride (14) using the general procedures of H. Meerwein, G. Dittmar, R. Gollner, K. Hafner, F. Mensch, O. Steinfort, Chem. Ber., 90, 841 (1957) (Equation 4e). To limit the hydrolysis of the product during the coupling step, the use of dichloromethane as a cosolvent is advantageous.

Finally, the sulfonyl chloride (14) is aminated to give (4a) using two equivalents of ammonia in dichloromethane solution at a temperature between -30 and -10 °C (Equation 4f).

Alternatively, many of the sulfonamides of Formulae (4a) or (4c') can be prepared via the route shown in Equation 5.

Equation 5

(a)

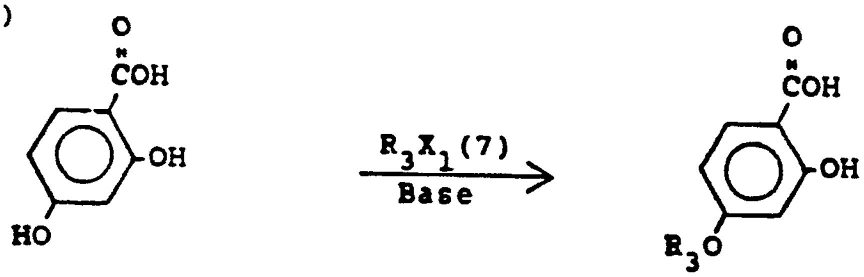


(15) (16)

(b)

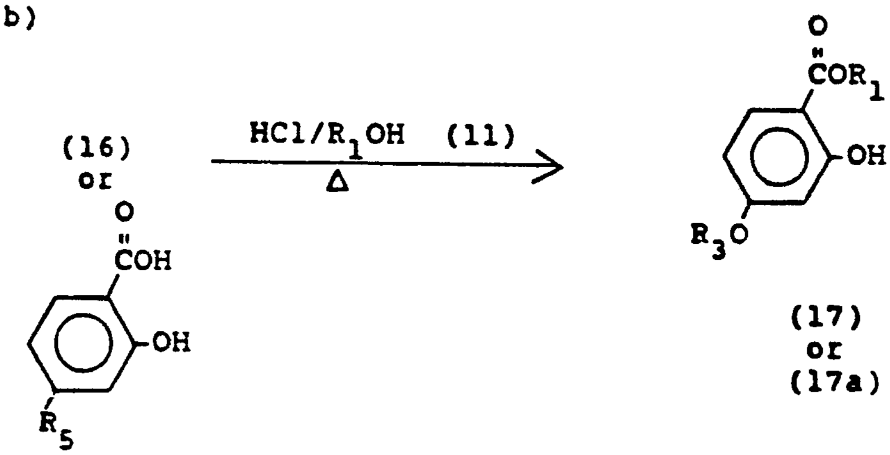


(16) or

(17) or (17a)

(16a)

(c)

(17) or (17a)

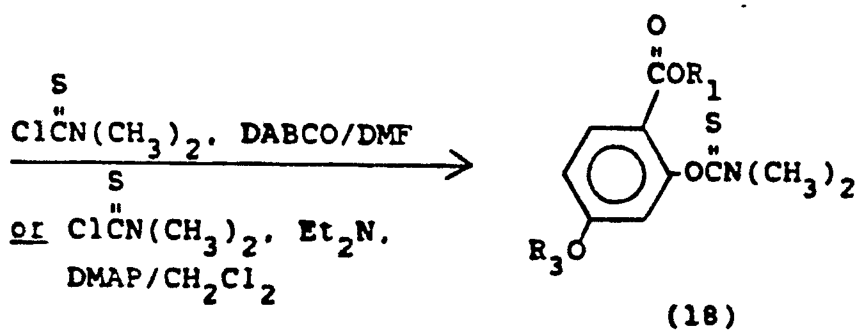


(18) or (18a)

(d)

(18)
or
(18a)

Δ

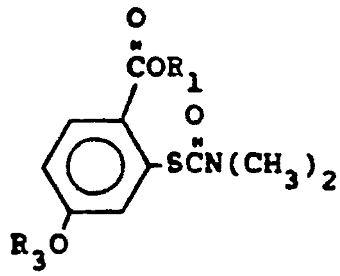

(19)
or
(19a)

(e)

(19)
or
(19a)

1. $NaOR_1$ (20)
2. HCl

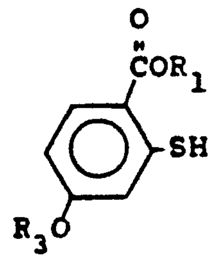

(21)
or
(21a)

(f)

(21)
or
(21a)

1. $H_2O_2$. $NaO\overset{O}{\overset{\|}{C}}H/HO\overset{O}{\overset{\|}{C}}H$
2. $DMF/SOCl_2$

(14)
or
(14a)

(g)

(14)
or
(14a)

$NH_3$

(4a) or

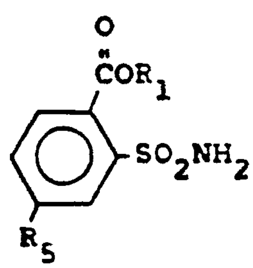

(4c')

where $M_1$, $R_1$, $R_a$, $R_b$, and $X_1$ are as previously defined; $R_5$ is $C_1$-$C_6$ alkyl, $C_1$-$C_6$ haloalkyl or $CH_2C(O)$-$NR_aR_b$; and $R_3$ is as previously defined or $C_2$-$C_4$ aminoalkyl but not $C_2$-$C_6$ alkenyl, $C_2$-$C_6$ haloalkenyl, $C_3$-$C_6$ alkynyl or $C_3$-$C_6$ haloalkynyl.

Phenol (15) is added to a solution of two equivalents of sodium in anhydrous methanol, ethanol, or amyl alcohol. A little more than one equivalent of $R_3X_1$ (7) is added and the mixture is heated at reflux for 4 to 24 hours. The solvent is removed in vacuo and the residue is partitioned between 1N hydrochloric acid and ether. The ether phase is dried over sodium sulfate, and filtered. Evaporation of the ether leaves the phenol (16) (Equation 5a).

In Equations 5b, c, d, e and f compounds 17a, 18a, 19a, 21a and 14a have the same structure as 17, 18, 19, 21, and 14 respectively except that the $OR_3$ substituent is replaced by $R_5$.

The carboxylic acid (16/16a) is converted to ester (17/17a) through the use of excess $R_1OH$ and a

strong acid catalyst such as hydrogen chloride as reviewed by C. A. Buehler and D. E. Pearson (op.cit., pp 802-807) (Equation 5b).

The phenol (17/17a) is converted to the thiocarbamate (18/18a) through the use of dimethylthiocarbamoyl chloride and 1,4-diazabicyclo[2.2.2]octane (DABCO) in N,N-dimethylformamide according to the general procedure of M. S. Newman and H. A. Karnes, J. Org. Chem. 1966, 31, 3890 (Equation 5c). Alternatively a mixture of the phenol (17/17a), at least one equivalent of dimethylthiocarbamoyl chloride, at least one equivalent of triethylamine ($Et_3N$), and a catalytic amount of 4-dimethylaminopyridine (DMAP) in dichloromethane is heated at reflux for one to seven days. The reaction mixture is washed with 1N hydrochloric acid and 10% aqueous sodium hydroxide solution, then dried over magnesium sulfate and filtered. Evaporation of the solvent leaves crude (18/18a) which may be purified by chromatography on silica gel or by recrystallization if it is crystalline.

The thiocarbamate (18/18a) is converted to its isomer (19/19a) by heating according to the general procedure of M. S. Newman and H. A. Karnes (op. cit.) (Equation 5d).

The solution of (19/19a) in anhydrous $R_1OH$ (11) is added little more than one equivalent of $NaOR_1$ - (20). The mixture is heated at 60-70° C for 0.5-1 hour. The solvent is removed in vacuo, and the residue is partitioned between dichloromethane and water. The aqueous phase is washed with dichloromethane, acidified with 12N hydrochloric acid, and extracted with dichloromethane. The dichloromethane extracts are dried over sodium sulfate and filtered. Evaporation of the solvent leaves thiol (21/21a) (Equation 5e).

To a mixture of the thiol (21/21a) and at least one equivalent of sodium formate in formic acid is added at least three equivalents of hydrogen peroxide at such a rate as to keep the temperature between 40 and 50° C. The reaction mixture is then heated at 45-55° C for 1 to 5 hours. The excess peroxide is destroyed within sodium sulfite, and the solvent is evaporated. The residue is added to excess thionyl chloride, and a catalytic amount of N,N-dimethylformamide (DMF) is added. The mixture is heated at reflux for 8 to 24 hours, and then the solvent is evaporated. The residue is partitioned between water and ether. The ether solution is washed with aqueous sodium bicarbonate solution, dried over magnesium sulfate and filtered. Evaporation of the solvent leaves the sulfonyl chloride (14/14a) (Equation 5f).

Finally the sulfonyl chloride (14) is aminated giving the sulfonamide (4a) and likewise (14a) gives (4c′) as already described for Equation 4f (Equation 5g).

Most of the sulfonamides (4a) can be prepared via the route shown in Equation 6 starting from 6-hydroxysaccharin (22).

Equation 6

(a)

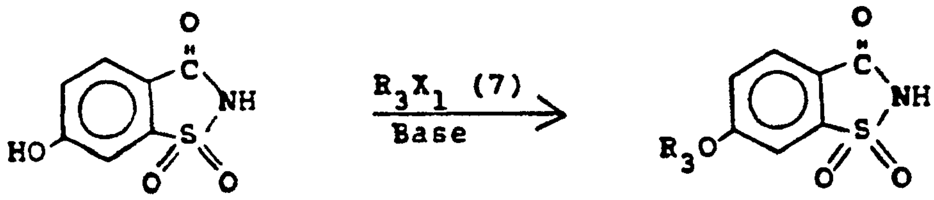


(22) (23)

(b)

(23)

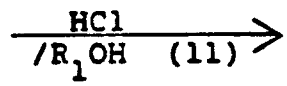


(4a)

(c)

(22)

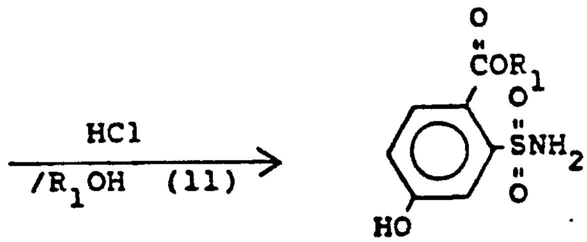


(24)

(d)

(24)

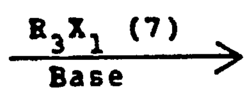


(4a)

where $R_1$ and $X_1$ are as previously defined and $R_3$ is as defined for Equation 4 or $C_2$-$C_4$ cyanoalkyl.

6-Hydroxysaccharin (22) can be prepared as described by C. Finzi and M. Colonna, Atti. accad. Lincei, Classe, sci. fis., mat. mat., 1937, 26, 19 (Chem. Abst. 1938, 32, 3762). Alternatively, (4a), where $R_1 = CH_3$, $R_3 = C_6H_5CH_2$ may be prepared via the route described by Equation 5. A solution of this sulfonamide in dichloromethane is treated with one equivalent of 1,8-diazabicyclo[5.4.0]undec-7-ene (DBU). The solvent is evaporated, and the residue is dissolved in minimal water and acidified with concentrated hydrochloric acid to precipitate 6-benzyloxysaccharin. This is dissolved in ethanol containing palladium catalyst. The mixture is hydrogenated at 20-40°C and 1-10 psi until hydrogen uptake ceases. Filtration and evaporation of the solvent affords 6-hydroxysaccharin (22).

In the method described by Equation 6a, 6-hydroxysaccharin (22) is added to a solution of two equivalents sodium in anhydrous methanol, ethanol, or amyl alcohol. A little more than one equivalent of $R_3X_1$ (7) is added and the mixture is heated at reflux for 4 to 24 hours. The solvent is removed in vacuo and the residue is dissolved in minimal water. It is acidified with concentrated hydrochloric acid. If the product crystallizes, it is collected, washed with dilute hydrochloric acid, and dried. If it does not crystallize, the aqueous mixture is extracted with dichloromethane. The dichloromethane phase is dried over sodium sulfate and filtered. Evaporation of the solvent leaves the saccharin (23) (Equation 6a).

A solution or suspension of the saccharin (23) in $R_1OH$ (11) is saturated with hydrogen chloride. The mixture is heated at 65-80°C for 1-6 hours. The solvent and hydrogen chloride are evaporated. The residue

is dissolved in dichloromethane, washed with aqueous sodium bicarbonate solution, dried over sodium sulfate, and filtered. Evaporation of the solvent affords the sulfonamide (4a) (Equation 6b).

Alternatively, saccharin (22) may be opened to the sulfonamide (24) using the conditions already described for the conversion of (23) to (4a) in Equation 6b (Equation 6c). To a solution of the sulfonamide (24) in anhydrous methanol, ethanol, amyl alcohol, or a suitable aprotic solvent such as N,N-dimethylformamide is added one equivalent of sodium or potassium methoxide, ethoxide, or tert-butoxide followed by the alkylating agent $R_3X_1$ (7). The mixture is held at 20-80 °C for 1-8 hours. The solvent is removed in vacuo, and the residue is dissolved in dichloromethane, washed with aqueous sodium bicarbonate solution, dried over $Na_2SO_4$, and filtered. Evaporation of the solvent affords the sulfonamide (4a) (Equation 6d).

Many sulfonamides of Formulae (4a), (4b) and (4e) can be prepared by the methods shown in Equation 7.

Equation 7

(a)

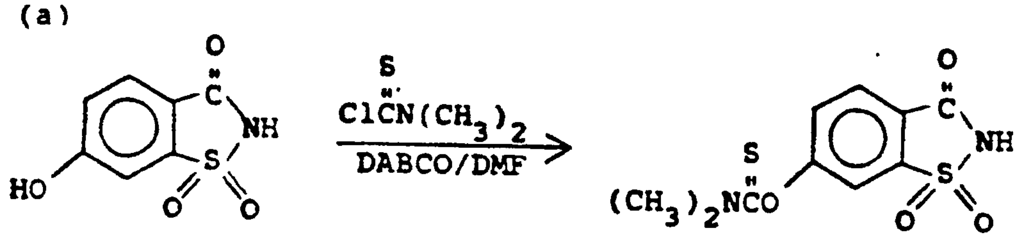


(22) (25)

(b)

(25)

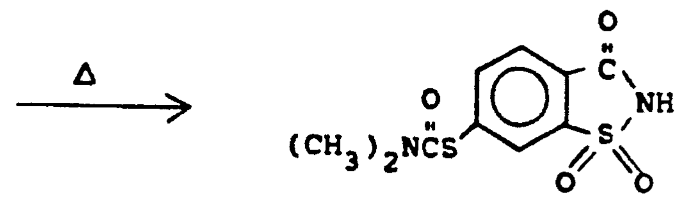


(26)

(c)

(26)

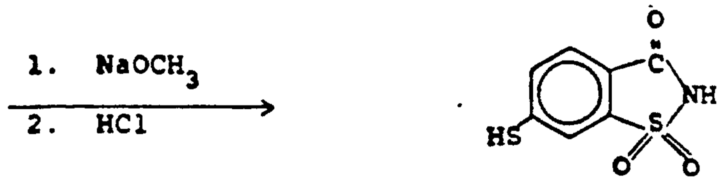


(27)

(d)

(27)

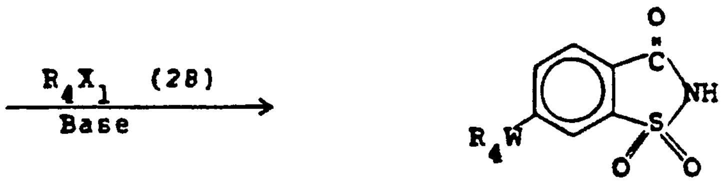


(29), (W=S)

(e)

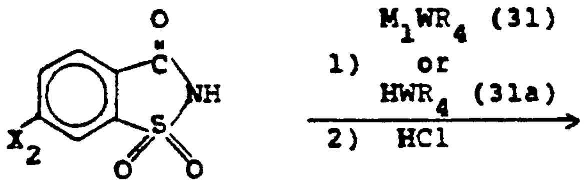


(29), (W=S)
(29a), (W=$NR_b$)
(29b), (W=O)

(30)

(f)

(29)
or
(29a)
or
(29b)

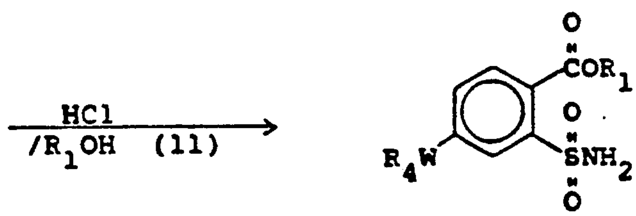


(4a), (W=O)
(4b), (W=S)
(4e), (W=$NR_b$)

(g)

(27)
or
(29a)

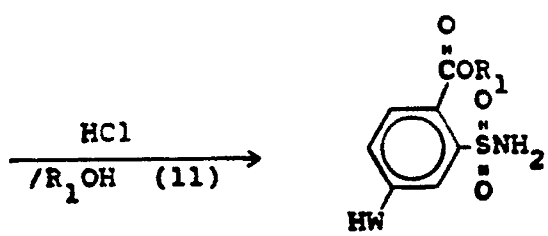


(32), (W=S)
(32a), (W=$NR_b$)

(h)

(32) or (32a) $\xrightarrow[\text{Base}]{R_4X_1\ (28)}$ (4b), (W=S)
(4e), (W=$NR_b$)

where $M_1$, $R_1$ and $X_1$ are as previously defined, $X_2$ is F, Cl, Br or I, W is O, S, or $NR_b$; $R_b$ is as previously defined; and $R_4$ is $R_3$ as defined for Equation 6 or H, $CH_3$, cyclohexyl, or phenyl.

The hydroxysaccharin (22) is converted to the thiocarbamate (25) through the use of dimethylthiocarbamoyl chloride and 1,4-diazabicyclo[2.2.2]octane (DABCO) in N,N-dimethylformamide according to the general procedure, or modifications thereof known to those skilled in the art, of M.S. Newman and H. A. Karnes (op. cit.) (Equation 7a).

The thiocarbamate (25) is converted to its isomer (26) by heating according to the general procedure, or modifications thereof known to those skilled in the art, of M. S. Newman and H. A. Karnes (ibid.) (Equation 7b).

To a solution of (26) in anhydrous methanol is added a little more than two equivalents of sodium methoxide. The mixture is heated at 60-70 °C for 0.5-1 hour. The solvent is removed in vacuo, and the residue is partitioned between dichloromethane and water. The aqueous phase is washed with dichloromethane and acidified with concentrated hydrochloric acid. If the saccharin (27) crystallizes, it is collected, rinsed with dilute cold hydrochloric acid, and dried. If it does not crystallize, the aqueous phase is extracted with dichloromethane. The dichloromethane extracts are dried over sodium sulfate and filtered. Evaporation of the solvent leaves saccharin (27) (Equation 7c).

The mercaptan (27) is added to a solution of two equivalents sodium in anhydrous methanol, ethanol, or amyl alcohol. A little more than one equivalent of $R_4X_1$ (28) is added, and the mixture is heated at reflux for 1 to 8 hours. The requisite alkylating, alkenylating, and alkynylating agents $R_4X_1$ (28) are either known in the art or may be made by a wide variety of methods known in the art.

The solvent is removed in vacuo, and the residue is dissolved in minimal water. It is acidified with concentrated hydrochloric acid. If the saccharin (29) crystallizes, it is collected, washed with cold dilute hydrochloric acid, and dried. If it does not crystallize, the aqueous mixture is extracted with dichloromethane. The dichloromethane phase is dried over sodium sulfate and filtered. Evaporation of the solvent leaves the saccharin (29) (Equation 7d).

Alternatively, in many cases, the saccharins of Formula (29) or (29b) may be prepared by treating a halosaccharin (30) in a polar aprotic solvent such as N-N-dimethylformamide at 20-100 °C with at least two equivalents of the corresponding thiolate or alkoxide of Formula (31) for 4-24 hours. The reaction is worked up by evaporation of the solvent. The residue is dissolved in minimal water and acidified with concentrated hydrochloric acid. If the saccharin (29/29b) crystallizes, it is filtered. Otherwise the aqueous solution is extracted with dichloromethane, and evaporation of the solvent leaves the saccharin (29/29b) (Equation 7e).

Many 5-aminosaccharins (29a) can be prepared by heating the halosaccharin (30) in N,N-dimethylformamide under pressure at 125-190 °C in the presence of amines for 8-16 hours. The reaction is worked up as described in the preceding paragraph.

In addition, aminosaccharins (29a) wherein $R_4$ is H can be alkylated in the same fashion as described previously for alkoxy and mercapto saccharins in Equations (6a) and (7d). The conversion of aminosaccharins (29a) into aminosulfonamides (4e) or (32a) can be effected using conditions described previously in Equations (6b and c) (Equation 7f).

The requisite precursor halosaccharins of Formula (30) may be prepared by conversion of the corresponding 4-halo-2-nitrobenzoic acids to esters of 2-(aminosulfonyl)-4-halobenzoic acids by use of methods analogous to those described for Equation 4. These esters are closed to the corresponding saccharins of Formula (30) by treatment with DBU according to the general method already described for the preparation of 6-benzyloxysaccharin.

Saccharins (29/29b) are similarly opened to the corresponding sulfonamides (4b/4a) using the conditions already described for the conversion of (23) to (4a) in Equation 6b (Equation 7f).

Alternatively, saccharin (27/29a) may be opened to the sulfonamide (32/32a) using the conditions already described for the conversion of (23) to (4a) in Equation 6b (Equation 7g). The thiol (32) or aniline (32a) is then converted to (4b/4e) using the method already described for the conversion of (24) to (4a) in Equation 6d (Equation 7h).

Most sulfonamides of Formula (4b) ($R_1$ and $R_4$ as previously defined) can also be prepared by synthetic routes analogous to that described for $R_1 = R_4 = CH_3$ in Examples 9 through 16.

Sulfonamides of Formula (4c) can be prepared by the method shown in Equation 8.

Equation 8

(a)

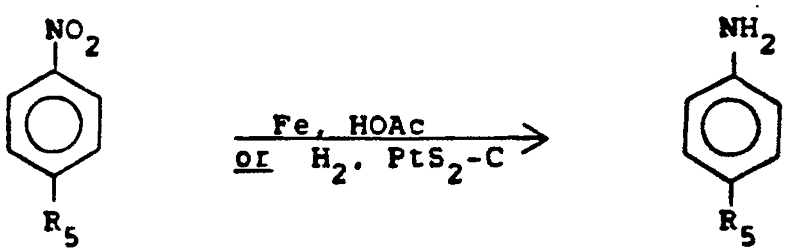


(33) (34)

(b)

(34)

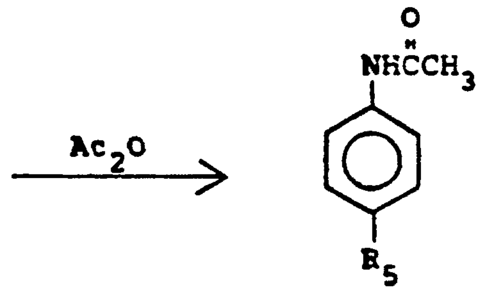


(35)

(c)

(35)

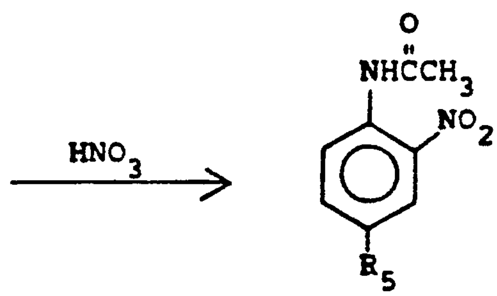


(36)

(d)

(36)

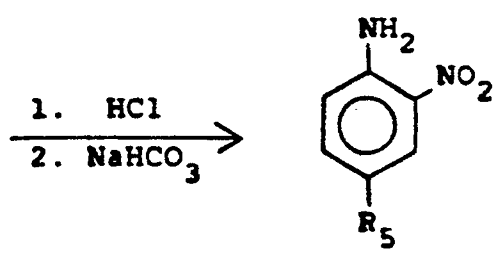


(37)

(e)

(37)

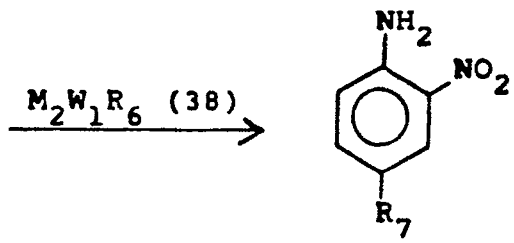


(39)

(f)

(39)
or
(37)

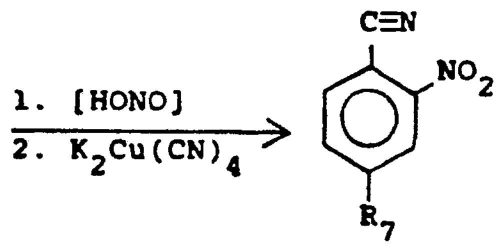


(40) or (40a, $R_7$=$R_5$)

(g)

(40)
or
(40a
$R_7$=$R_5$)

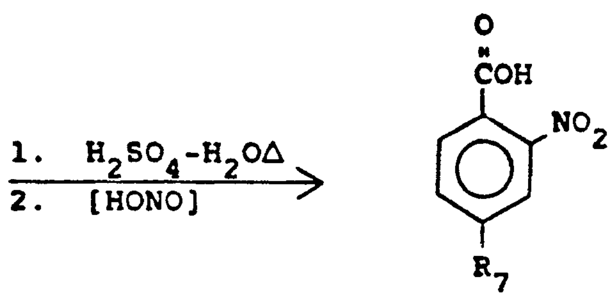


(41) or (41a, $R_7$=$R_5$)

(h)

(41)
or
(41a, $R_7$=$R_5$)

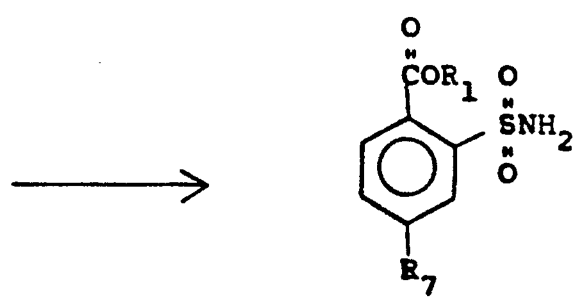

(4c) or (4c', $R_7$=$R_5$)

(i)

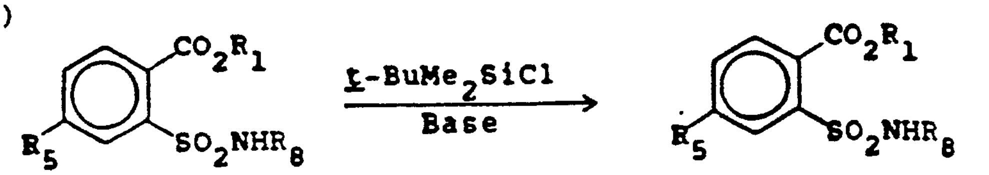


(4c', $R_8$=H) (4c', $R_8$=SiMe$_2$-t-Bu)

(j)

(4c', $R_8$=SiMe$_2$-t-Bu)

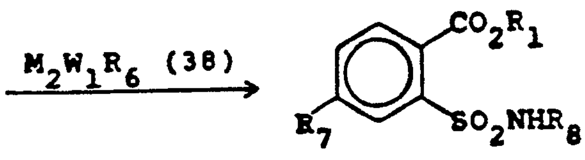


(4c, $R_8$=SiMe$_2$-t-Bu)

where $R_1$ is as previously defined; $R_5$ is alkyl, haloalkyl, alkenyl, haloalkenyl, alkynyl, haloalkynyl or $CH_2C$-(O)$NR_aR_b$; $R_a$ and $R_b$ are as previously defined; $W_1$ is O or S; $M_2$ is Na or K; $R_6$ is H, $C_1$-$C_4$ alkyl, $C_1$-$C_4$ haloalkyl, $C_3$-$C_4$ cycloalkyl, cyclopropylmethyl, $C_3$-$C_4$ alkenyl, $C_3$-$C_4$ haloalkenyl, $C_3$-$C_4$ alkynyl, $C_3$-$C_4$ haloalkynyl, $C_2$-$C_4$ alkoxyalkyl, or $C_2$-$C_4$ aminoalkyl; and $R_7$ is $C_1$-$C_4$ alkyl substituted with $W_1R_6$.

The starting nitrobenzenes (33) are either known in the art or may be made by a wide variety of methods known in the art. Some of these methods include the nitration (for a review, see G. Lehmann. H. Eichmann "Formation of Carbon-Nitrogen Bonds" in Preparative Organic Chemistry, G. Hilgetag, A. Martini ed., Wiley-Interscience, New York, 1972) of the coupling product obtained from reaction of the appropriate halide with diphenylcuprate (for a review, see G. H. Posner "Substitution Reactions Using Organo-copper Reagents" in Organic Reactions, Vol. 22, W. G. Dauben ed., Wiley, New York, 1975).

Another method is the Friedel-Crafts acylation of benzene with the appropriate acid halide or anhydride (for a review, see G. A. Olah, Friedel-Crafts and Related Reactions, Volumes I-IV, Wiley-Interscience, New York, 1963-1965), followed by reductive removal of the carbonyl oxidation (for a review, see C. Bischoff, P. -G. Dietrich, E. Höft, D. Murowski, "Formation of Carbon-Hydrogen Bonds" in Preparative Organic Chemistry, G. Hilgetag, A. Martini ed., Wiley-Interscience, New York, 1972) and then nitration.

Still another method involves conversion of the appropriate 4-nitrobenzyl alcohols, aldehydes, or ketones to their mono or difluoro alkyl homologues using the conditions reviewed by G. A. Boswell, Jr., W. C. Ripka, R. M. Scribner, C. W. Tullock, "Fluorination by Sulfur Tetrafluoride" and C. M. Sharts, W. A. Sheppard "Modern Methods to Prepare Monofluoroaliphatic Compounds" in Organic Reactions, Vol. 21, W. G. Dauben ed., Wiley, New York, 1974).

The nitrobenzene (33) is reduced to the aniline (34) using the conditions already described for the conversion of (12) to (13) in Equation 4d (Equation 8a).

The aniline (34) in a suitable solvent such as benzene is treated with at least one equivalent of acetic anhydride and heated at reflux for 8-24 hours. The reaction mixture is cooled, and if the acetanilide (35) crystallizes, it is collected and dried. If it does not crystallize, the benzene solution is washed with 1N hydrochloric acid and aqueous sodium bicarbonate solution, dried over magnesium sulfate, and filtered. Evaporation of the solvent leaves the acetanilide (35) (Equation 8b).

The acetanilide (35) is then nitrated using the general conditions reviewed by G. Lehmann and H. Teichmann (op.cit.) to give nitroacetanilide (36) (Equation 8c).

A slurry of acetanilide (36) in 1N hydrochloric acid is heated at reflux until all of the solid dissolves. The solution is then made slightly basic with sodium bicarbonate and is extracted with dichloromethane. The dichloromethane extracts are dried over sodium sulfate and filtered. Evaporation of the solvent leaves the aniline (37) (Equation 8d).

Many of the substituents described within $R_7$ are prepared from aniline (37) having an $R_5$ substituent containing a displaceable halogen atom at the position where the substituent $W_1R_6$ is to be placed. Thus the halide (37) in a suitable solvent such as N,N-dimethylformamide is treated with a little more than one equivalent of the appropriate sodium or potassium salt (38).

The mixture is held at 20-100°C for 1-8 hours. The solvent is then evaporated, and the residue is

partitioned between water and dichloromethane. The dichloromethane solution is dried over sodium sulfate and filtered. Evaporation leaves the desired compound (39) (Equation 8e).

The aniline (39/37) is then diazotized and subjected to the Sandmeyer reaction according to the general experimental procedure of G. T. Morgan and E. A. Coulson, J. Chem. Soc. 1929, 2551 to give nitrile (40/40a) (Equation 8f).

A suspension of the nitrile (40/40a) in 75-80% aqueous sulfuric acid is heated at 95-100°C for 2-5 hours. Then over 1-2 hours and at a temperature of 80-100°C 1.5-2.5 equivalents of sodium nitrite is added in small portions. The heating is continued 0.5-1 hour longer, then the mixture is cooled and poured onto excess ice. If the carboxylic acid (41/41a) crystallizes, it is collected, rinsed with ice water and dried. If it does not crystallize, the aqueous mixture is extracted with dichloromethane. The dichloromethane solution is extracted with aqueous 10% sodium carbonate solution. The aqueous extract is made acidic with concentrated hydrochloric acid. If the carboxylic acid (41/41a) then crystallizes, it is collected, rinsed with ice water and dried. If it does not crystallize, the aqueous solution is extracted with ether. The ether extracts are dried over magnesium sulfate and filtered. Evaporation of the solvent leaves carboxylic acid (41/41a) (Equation 8g).

By use of the methods previously described, carboxylic acid (41) can be converted to sulfonamide (4c) and likewise (41a) give (4c') (Equation 8h).

In the case where $R_7$ substituents of sulfonamide (4c) are incompatible with the strongly acidic conditions described in Equation 8g, an alternative scheme is used.

Sulfonamide of Formula (4c') wherein $R_5$ is $C_1$-$C_4$ alkyl substituted with Cl, Br, or I in a suitable solvent such as acetonitrile is treated with one equivalent of tert-butyldimethylsilyl chloride in the presence of a mild base such as pyridine at 15-25°C for 1-8 hours. when the reaction is judged complete by thin layer chromatography the mixture is poured onto ice water. If the silylsulfonamide crystallizes, it is collected, rinsed with water and dried. If not, the aqueous solution is extracted with ether. The ether extracts are dried and concentrated to furnish the silylsulfonamide of (4c') (Equation 8i).

A stirred solution of the silylsulfonamide of (4c') in a solvent such as DMF is treated at 0°-80°C with at least one equivalent of (38) for 1-8 hours. When the reaction is judged complete by thin layer chromatography the mixture is poured onto ice water and the reaction is worked up as described in the previous paragraph (Equation 8j).

The silylsulfonamide of (4c) can then be converted directly into sulfonylurea (I) using the same conditions described in Equation 3, with the exception that tetra-n-butyl ammonium fluoride is used as base in lieu of 1,8-diazabicyclo[5.4.0]undec-7-ene (DBU).

Sulfonamides of Formula (4) where $R_2$ contains sulfinyl or sulfonyl groups can be prepared by peracid oxidation of the corresponding mercapto sulfonamides using methods analogous to those described for the preparation of sulfinyl sulfonamides of Formula (4d.n = 1) and sulfonyl sulfonamides of Formula (4d.n = 2) in Equation 9 (vide infra).

For example, the sulfinyl sulfonamides of Formula (4d.n = 1) and sulfonyl sulfonamides of Formula (4d.n = 2) can be be prepared from the corresponding thio sulfonamides of Formula (4b) as shown in Equation 9.

Equation 9

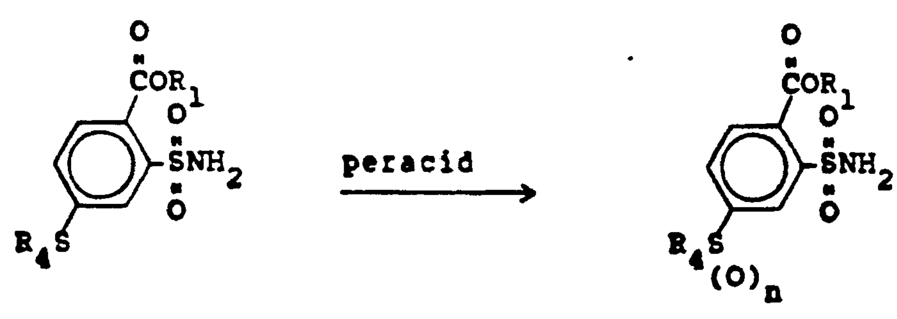


(4b) (4d)

where $R_1$ and $R_4$ are as previously defined, except that $R_4$ cannot be alkylthioalkyl or haloalkylthioalkyl, and n is 1 or 2.

To prepare the sulfinyl sulfonamides of Formula (4d,n=1) a solution of one equivalent of a peracid, such as 3-chloroperoxybenzoic acid, in an inert solvent, such as dichloromethane, is added to a stirred solution of the appropriate thio sulfonamide (4b) in an inert solvent, such as a mixture of dichloromethane and tetrahydrofuran, at 0-5° C. The mixture is then warmed to 20-40° C. When thin layer chromatography has revealed the sulfonamide (4b) to have been oxidized, the mixture is washed in turn with 5% aqueous sodium sulfite solution, saturated aqueous sodium bicarbonate solution, water, and brine, and then dried ($MgSO_4$). Evaporation of the solvent then leaves the sulfinyl sulfcnamide of formula (4d, n=1).

To prepare sulfonyl sulfonamides of Formula (4d, n=2) a solution of more than two equivalents (only two equivalents when $R_4$ is alkenyl or alkynyl) of a peracid, such as 3-chloroperoxybenzoic acid, in an inert solvent, such as 1,2-dichloroethane, is added to a stirred mixture of the appropriate thio sulfonamide (4b) in an inert solvent, such as 1,2-dichloroethane, containing 1-5 mol % of a free radical inhibitor, such as 4,4-thiobis(6-tert-butyl-m-cresol). The mixture is heated to 50-80° C. When thin layer chromatography shows the thio sulfonamide (4b) and intermediate sulfinyl sulfonamide (4d, n=1) to have been consumed, the mixture is cooled and diluted with tetrahydrofuran to maintain sulfonamide solubility. Using the same work up method as already described for the preparation of sulfinyl sulfonamides (4d, n=1), one obtains the sulfonyl sulfonamides of formula (4d, n=2).

Sulfonamides of Formula (4d) where $R_4$ is alkylthioalkyl or haloalkylthioalkyl and n is 1 or 2 can be prepared from the homologous sulfonamides of Formula (4d) having a $R_4$ monohaloalkyl substituent with a displaceable halogen atom at the position where the alkylthioalkyl or haloalkylthioalkyl group is to be placed. These monohaloalkyl sulfonamides are first closed to the corresponding saccharins by use of DBU as previously described for the preparation of 6-benzyloxysaccharin. The halogen atom is then replaced with the appropriate alkylthio or haloalkylthio group using the displacement conditions previously described for the conversion of halide (37) to thioether (39) in Equation 8. When the appropriate haloalkylthiolate reagent is unstable to self-condensation, the corresponding hydroxy-, carbonyl-, and/or carboxy-containing alkylthiolate can be used. After formation of the thioether bridge, these oxygen-containing groups can be converted to the desired halogen substitution pattern by use of a wide variety of methods known in the art. Finally, the saccharins are opened to the desired sulfonamides of Formula (4d) where $R_4$ is alkylthioalkyl or haloalkylthioalkyl and n is 1 or 2 by the method already described for the conversion of (23) to (4a) in Equation 6b.

The amino sulfonamides of Formula (4e) are prepared as shown in Equation 9.1 and discussed below.

**Equation 9.1**

**(a)**

(30)

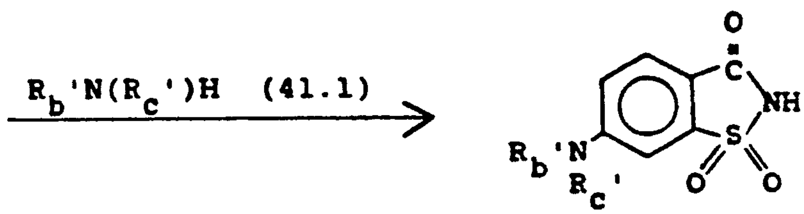


(41.2)

**(b)**

(41.2)

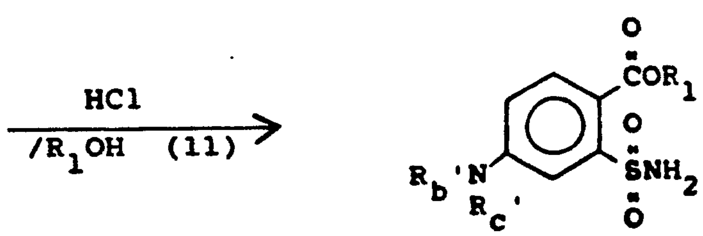


(4e)

where $R_1$ and $X_2$ are as previously defined, $R_b$' is H or alkyl, and $R_c$' is H, alkyl, or haloalkyl.

In most cases the amino saccharins of Formula (41.2) are prepared by treating halo saccharins of Formula (30) with one or more equivalents of the corresponding amine derivatives of Formula (41.1) using

one or more of a wide variety of experimental conditions known in the art. In some cases the reaction can be run using an excess of the amine derivative (41.1) as the only solvent and heating the mixture to 30-200 °C at atmospheric pressure or, if the boiling point of the amine derivative makes necessary. at above atmospheric pressure. In other cases the use of a polar inert solvent such as N,N-dimethylformamide is advantageous. When the amine derivative is itself difficult or expensive to prepare, the amount of it used can be reduced to little more than one equivalent if at least one equivalent of another base such as triethylamine, N,N-diisopropyl-N-ethylamine, diazabicyclo[5.4.0]undec-7-ene, sodium hydride, or n-butyllithium is added to the reaction mixture. Besides reducing the amount of amine derivative needed, the use of at least two equivalents of very strong bases, such as n-butyllithium or sodium hydride, capable of deprotonating the amine derivative can accelerate the rate of the displacement reaction and allow it to proceed at lower temperatures as is well known in the art. The requisite amine derivatives (41.1) are either known or can be made by methods known in the art.

After the displacement reaction is complete the solvent and excess of any volatile amine derivative (41.1) is evaporated and the residue is dissolved in 5% aqueous sodium hydroxide solution. Concentrated hydrochloric acid is added until the pH is 7. If the saccharin (41.2) crystallizes, it is filtered. Otherwise the aqueous solution is saturated with salt and then extracted with dichloromethane, and in most cases evaporation of the dichloromethane leaves the saccharin (41.2). If it does not, the aqueous solution is evaporated to dryness and the residue is continuously extracted with tetrahydrofuran. Evaporation of the solvent then leaves the saccharin (41.2) (Equation 9.1a).

In some cases saccharins of formula (41.2) may be prepared from saccharins of Formula (41.2) having fewer substituents on the amino group. A variety of methods are well-known in the art for the alkylation of aniline amino groups and their derivatives.

To prepare the sulfonamide of Formula (4e) a solution or suspension of the saccharin (41.2) in the appropriate alcohol $R_1OH$ (11) is saturated with hydrogen chloride. The mixture is heated at 65-80 °C for 1-12 hours. The solvent and hydrogen chloride are evaporated. The residue is dissolved in a mixture of brine and 5:1 (vol.) dichloromethane-tetrahydrofuran. Sodium carbonate is added to the vigorously stirred mixture until it is made basic. The dichloromethane-tetrahydrofuran phase is then separated, and evaporation of the solvent leaves the sulfonamide of Formula (4e) (Equation 9.1b).

Sulfonyl isocyanates (1) are prepared from the corresponding sulfonamides (4) with compatible $R_2$ substituents by one of the following two general methods.

<u>Equation 10</u>

$$(4) \quad \xrightarrow[COCl_2,\ Cat]{CH_3(CH_2)_3NCO} \quad (1)$$

where $R_1$ and $R_2$ are as previously defined.

The sulfonamide (4) and an alkyl isocyanate (e.g., n-butyl isocyanate) in xylene or other solvent boiling above 135 °C are mixed in the presence or absence of a catalytic amount of 1.4-diaza[2.2.2]bicyclooctane (DABCO) and heated to 135-140 °C. After 5-60 minutes phosgene is slowly added to the heated mixture at such a rate that the temperature remains between 133-135 °C. When the consumption of phosgene has ceased, the mixture is cooled and filtered to removed insoluble material. Finally, the solvent, alkyl isocyanate, and excess phosgene are evaporated, leaving the sulfonyl isocyanate (1).

If desired, the alkyl isocyanate-sulfonamide adduct can be made and isolated before reaction with the phosgene. In this case, the sulfonamide (4), alkyl isocyanate, and anhydrous base (e.g., $K_2CO_3$) in a polar, aprotic solvent (e.g. acetone, butanone, or acetonitrile) are mixed and heated under reflux for 1 to 6 hours. The reaction mixture is then diluted with water, and the pH is adjusted to about 3 with acid (e.g. HCl, $H_2SO_4$). The adduct is filtered out and dried, and then reacted with phosgene as described above. This procedure modification is especially useful when sulfonamide (4) is high melting and has low solubility in the phosgenation solvent.

Sulfonyl isocyanates (1) can also be prepared by the following method.

Equation 11

(a)

(4)
(4A)

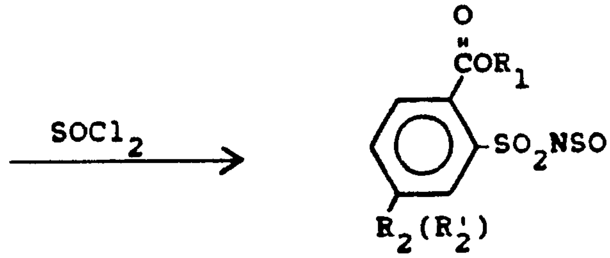


(42)

(b)

(42)

$COCl_2$,
pyridine cat.

(1)

where $R_1$ and $R_2$ are as previously defined.

The sulfonamide (4) or (4A) is heated at reflux in an excess of thionyl chloride. The reaction is continued until the sulfonamide protons are no longer detectable in the proton magnetic resonance spectrum. From 16 hours to 5 days is typically sufficient for complete conversion to the thionylamide (42) (Equation 11a).

The thionyl chloride is evaporated and the residue is treated with an inert solvent (e.g. toluene) containing at least one equivalent (typically 2-3 equivalents) of phosgene. A catalytic amount of pyridine (typically 0.1 equivalent) is added, and the mixture is heated to about 60-140 °C, with 80-100 ° preferred. Conversion to the isocyanate (1) is usually substantially complete within 15 minutes to 3 hours (Equation 11b). The mixture is then cooled and filtered, and the solvent is evaporated, leaving the sulfonyl isocyanate (1).

The heterocyclic amines of Formula (2) below are either known, disclosed in this application, or can be prepared by obvious methods by one skilled in the art.

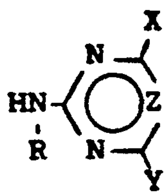

(2)

For a review of the synthesis and reactions of 2-amino and 2-methylaminopyrimidines (2, Z = CH) see The Chemistry of Heterocyclic Compounds, Vol. 16, Wiley-Interscience, New York (1962). For a review of the synthesis and reactions 2-amino- and 2-methylamino-s-triazines (2, Z = N) see The Chemistry of Heterocyclic Compounds, Vol. 13, Wiley-Interscience, New York (1959), F. C. Schaefer, U.S. Patent 3,154,537 and F. C. Schaefer and K. R. Huffman J. Org. Chem., 28, 1812 (1963).

In some cases, heterocycles of Formula (2) may be more easily prepared with R being H than with R being $CH_3$. Many heterocycles (2, R = $CH_3$) can be prepared from the corresponding heterocycles (2, R = H) by one or more of the following two methods.

Equation 12

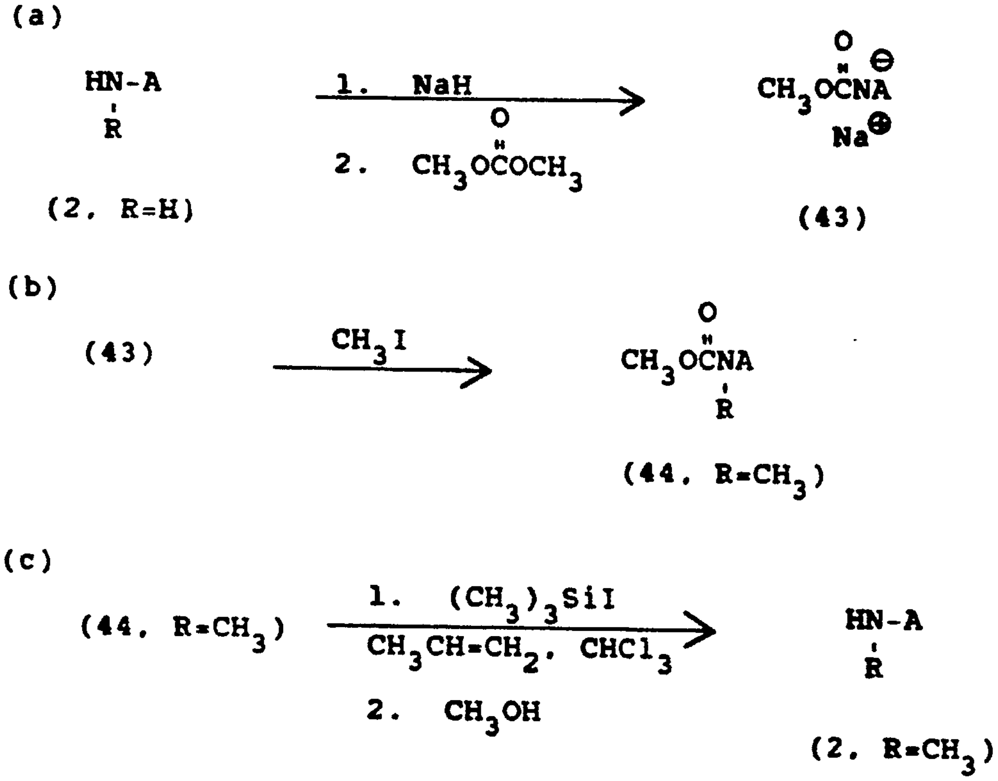


(2, R=H) (43)

(43) (44, $R=CH_3$)

(44, $R=CH_3$) (2, $R=CH_3$)

where A is as previously defined.

In this method, a solution or slurry of the appropriate heterocycle (2, R=H) in a suitable aprotic solvent (e.g., tetrahydrofuran, dioxane, glyme) at 0-30° C is treated with two equivalents of sodium hydride. After gas evolution ceases, the reaction mixture is treated with one equivalent of dimethyl carbonate and stirred at 20-30° C for 8 to 24 hours to provide a suspension of the sodium salt (43) (Equation 12a).

The reaction mixture containing (43) is treated with at least two equivalents of iodomethane and then heated at 60-80° C for 8 to 24 hours. The mixture is cooled and filtered, and the solvent is evaporated. The residue is taken up in dichloromethane, washed with water, and the solvent is evaporated, leaving the N-methyl carbamate (44, $R=CH_3$) (Equation 12b).

The carbamate (44, $R=CH_3$) is dissolved in anhydrous, alcohol-free chloroform saturated with propylene gas. Slightly more than one equivalent (typically 1.1-1.2 equivalents) of iodotrimethylsilane) is added and the stirred solution is heated at 50-60° C for 2 to 4 hours. The mixture is cooled and two equivalents of methanol is added. The solvent is evaporated and the residue is taken up in methanol. The mixture is carefully neutralized with 10% sodium methoxide in methanol, and then the solvent is evaporated. The residue is triturated with ice water. If a precipitate forms, it is filtered out, rinsed with ice water and dried to provide (2, $R=CH_3$). If no precipitate forms, the solution is saturated with sodium chloride and extracted with ethyl acetate. Evaporation of the solvent leaves heterocycle (2, $R=CH_3$)(Equation 12c).

Alternatively, the following two-step procedure is useful in many cases.

Equation 13

(a)

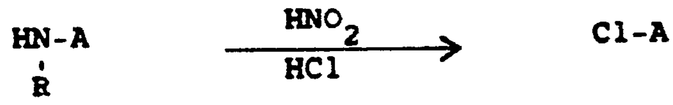


(2. R=H) (45)

(b)

(45)

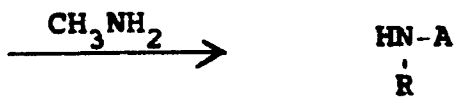


(2. R=CH3)

where A is as previously defined.

A solution of the amine (2, R = H) in concentrated hydrochloric acid is treated with sodium nitrite solution and the chloro compound (45) is isolated in the usual manner by filtration of the acidic solution (Equation 13a). A representative procedure is described by Bee and Rose in J. Chem. Soc. C. 1966, 2031, for the case in which Z = CH, and X = Y = $OCH_3$.

The heterocycle (45) is then treated with at least two equivalents of methylamine in a suitable inert solvent (e.g. tetrahydrofuran, glyme, or diglyme) at a temperature between 20° and 80° C for 1-18 hours (Equation 13b). The reaction mixture is then cooled and filtered. Evaporation of the solvent leaves (2, R = $CH_3$) contaminated with a little $CH_3NH_3^+$ $Cl^-$ salt. The product may be purified by trituration with ice water or by dissolution in dichloromethane, followed by washing with a small amount of water, drying, and evaporation of solvent. Further purification may be accomplished by recrystallization or column chromatography on silica gel.

As an alternative to the methods already described in which the sulfonamides of Formulae (4a), (4b), (4d), and (4e) are fully elaborated prior to construction of the urea bridge of compounds of Formula I, many compounds of Formula Ia may be prepared by the method described in Equation 14.

Equation 14

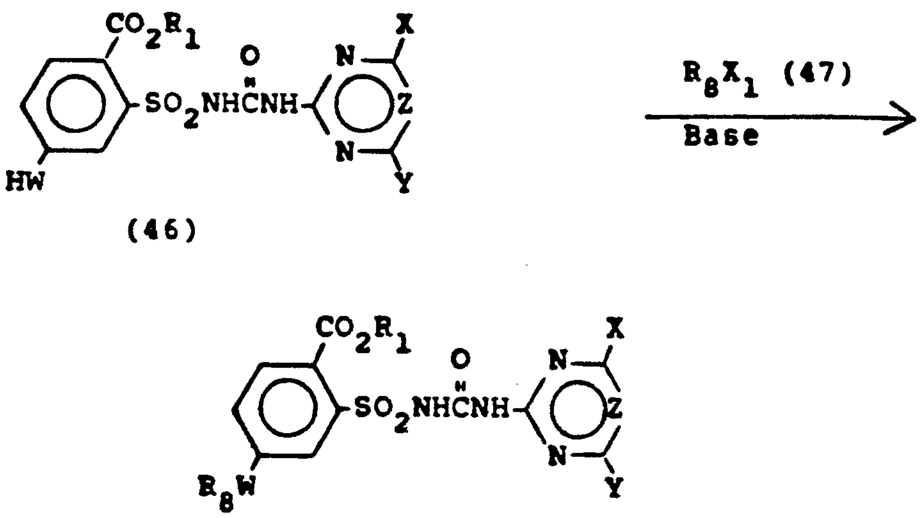


(46)

Ia

where $R_1$, X, $X_1$, Y and Z are as previously defined; W is S, O or $NR_b$; $R_b$ is as previously defined; $R_8$ is $C_4$-$C_6$ cycloalkylalkyl, $C_2$-$C_6$ alkenyl, $C_2$-$C_6$ haloalkenyl, $C_3$-$C_6$ alkynyl, $C_3$-$C_6$ haloalkynyl, $C_2$-$C_8$ alkoxyalkyl, $C_2$-$C_4$ haloalkoxyalkyl, $C_2$-$C_8$ alkylthioalkyl, $C_2$-$C_4$ haloalkylthioalkyl, $C_2$-$C_4$ cyanoalkyl, $CH_2C(O)CH_3$, $CH_2CH_2C(O)CH_3$ or $C_1$-$C_6$ alkyl.

In this method a solution of the appropriate compound of Formula (46) in a polar solvent such as a mixture of acetonitrile and N,N-dimethylformamide is treated with two equivalents of a strong base such as sodium methoxide or sodium hydride with catalytic methanol followed by a little more than one equivalent of the appropriate alkylating, alkenylating, or alkynylating agent (47). The mixture is held at 20-60 °C for 2-24 hours. Then the mixture is poured into excess hydrochloric acid. If the compound of Formula Ia crystallizes, it is filtered. Otherwise the aqueous mixture is extracted with dichloromethane, and evaporation of the solvent leaves the compound of Formula Ia. The compounds of Formula (46) are in turn prepared by coupling the appropriate sulfonamides of Formulae (4a, 4b or 4e) with the appropriate heterocyclic phenyl carbamates of Formula (5,R = H) according to the general method described for Equation 3.

Many of the compounds of Formula Ia where $R_8$ is alkyl, cycloalkyl, cycloalkylalkyl, haloalkyl, alkenyl, haloalkenyl, alkynyl, haloalkynyl, alkoxyalkyl, haloalkoxyalkyl, cyanoalkyl, $-CH_2C(O)CH_3$, or $-CH_2CH_2C(O)CH_3$ and W is S(O) or $S(O)_2$ can be made by oxidation of the corresponding compounds of Formula Ia where W is S using methods similar to those already described for the conversion of (4b) to (4d) in Equation 9.

Compounds of Formula I where $R_2$ contains a carbonyloxy, carbamoyloxy, phosphoryloxy, sulfonyloxy, silyloxy, or phosphorylthio group are prepared from the corresponding compounds of Formula I where $R_2$ contains a hydroxyl or thiol group by use of a wide variety of methods known in the art.

The following examples further illustrate the synthesis of this invention.

## Example 1
## 6-Hydroxy-1,2-benzisothiazol-3(2H)-one, 1,1-dioxide

To methyl 2-(aminosulfonyl)-4-(methoxy)benzoate (34 g, 0.139 mol) in dry N,N-dimethylformamide (DMF) (500 mL) was added potassium tert-butoxide (46.6 g, 0.42 mol) portionwise. The mixture was stirred 15 minutes at room temperature then 2-propanethiol (25 mL, 0.28 mol) was added dropwise. The reaction exothermed to 37° and became homogeneous. The mixture was heated at reflux for 4 hours and then was cooled to room temperature. The mixture was diluted with water and acidified with concentrated HCl until the pH was approximately 2. The homogeneous solution was extracted with ethyl acetate. The organic layer was washed with water and brine and dried with magnesium sulfate. Concentration furnished 28 g of the subject compound as a white solid, m.p. 148-150 °C. NMR (90 MHz) $D_6$-acetone; $\delta$ 9.8 (bs. 2H + water), 8.0 (d, 1H) and 7.5 (m, 2H).

## Example 2
## 6-(Difluoromethoxy)-1,2-benzisothiazol-3(2H)-one, 1,1-dioxide

To a solution of 6-hydroxysaccharin (1.0 g, 5.0 mmol) in aqueous KOH (0.85 g, 15 mmol in water (10mL)) was added methylene chloride (25 mL) and tetrabutyl ammonium bromide (0.2 g, catalytic). An excess of Freon(TM) was added and the reaction was heated to reflux using a dry-ice/acetone condenser. After 3 hours the reaction was filtered and the filtrate was acidified with concentrated HCl to pH 2. The solids were collected by filtration, washed with water and dried to give 0.32 g of the desired compound as a white solid, m.p. 183-185 °C. A second crop of 0.16 g was obtained from the filtrate and was combined with the first. NMR (90 MHz) $D_6$-acetone: $\delta$ 8.2 (d, 1H), 7.9 (d, 1H), 7.7 (dd, 1H) and 7.3 (t, 1H, J = 72 Hz).

## Example 3
## 2-(Aminosulfonyl)-4-(difluoromethoxy)benzoic acid, 2-methoxyethyl ester

A solution of 6-difluoromethoxysaccharin (1.5 g) in 2-methoxyethanol (25 mL) was cooled to 0° and HCl gas was bubbled into the reaction for 30 minutes. The mixture was heated at reflux for 20 minutes and cooled to 25°. After 3 hours, there was no change as judged by thin layer chromatography (TLC) so the reaction was heated an additional 12 hours. The reaction was judged complete and was cooled to room temperature and concentrated. The resultant mixture was taken up in ethyl acetate and washed with 5% sodium bicarbonate followed by brine and then dried with magnesium sulfate. Concentration furnished 0.8 g of the desired compound as a white solid, m.p. 80-81 °C NMR (200 MHz) $D_6$-acetone: $\delta$ 7.9 (d, 1H), 7.8 (d, 1H), 7.5 (dd, 1H), 7.2 (t, 1H, J = 72 Hz), 4.4 (t, 2H), 3.8 (t, 2H) and 3.4 (s, 3H).

Example 4

4-(Difluoromethoxy)-2-[[(4,6-dimethoxypyrimidin-2-yl)aminocarbonyl]aminosulfonyl]benzoic acid, (2-methoxyethyl)ester

To 2-methoxyethyl 2-(aminosulfonyl)-4-difluoromethoxy)benzoate (0.1 g, 0.31 mmol) and dimethoxypyrimidine phenylcarbamate (0.09 g, 0.31 mmol) in dry acetonitrile (5 mL) was added 1,8-diazabicyclo-[5.4.0]-undec-7-ene (DBU) (0.5 mL). The mixture was allowed to stir for 1 hour, then water and 1N HCl (to pH 3) was added. The resultant solid was collected, washed with water and dried to furnish 0.08 g of the desired compound as a white solid, m.p. 121-125° C. NMR (200 MHz) $D_6$-acetone: δ 13.2 (bs. 1H), 9.2 (bs, 1H), 8.1 (d, 1H), 7.9 (d, 1H), 7.6 (dd, 1H), 7.2 (t, 1H), 5.8 (s, 1H), 4.5 (t, 2H), 4.0 (s, 6H), 3.7 (t, 2H) and 3.3 (s, 3H).

Example 5

6-(4-Methoxybenzylthio)-1,2-benzisothiazol-3(2H)-one, 1,1-dioxide

To 6-chlorosaccharin (10 g, 46 mmol) and 4-methoxybenzyl mercaptan (7.8 g, 50 mmol) in N,N-dimethylformamide (DMF) (50 mL) was added potassium tert-butoxide (14 g, 125 mmol) portionwise. The reaction was allowed to exotherm to 60° and slowly cool to room temperature. After stirring for 14 hours, the mixture was poured onto ice-water (100 g) and acidified with concentrated HCl to pH 2. The desired compound was collected by filtration, washed with water and dried to furnish 13 g of a white solid, m.p. 162-165° C. NMR (90 MHz) $D_6$-acetone: δ 8.2 (s, 1H), 8.0 (s, 2H), 7.7 (d, 2H), 7.2 (d, 2H), 4.6 (s, 2H) and 3,8 (s, 3H).

Example 6

6-(Thio)-1,2-benzisothiazol-3(2H)-one,1,1-dioxide

A solution of 6-(4-methoxybenzyl)thiosaccharin in trifluoroacetic acid (50 mL) was heated at reflux for 14 hours. The heterogeneous reaction mixture was cooled and the solids were collected by filtration. The solids were washed with butyl chloride and air dried and 6.3 g of the desired compound was obtained as a greenish solid, m.p. 216-218° C. NMR (90 MHz) $D_6$-acetone: δ 8.2 (s, 1H), 7.95 (s, 2H), 5.3 (bs, 1H) and 5.1 (bs, 1H).

Example 7

6-(2,2,2-Trifluoroethylthio)-1,2-benzisothiazol-3)2H)-one, 1,1-dioxide

To 6-thiosaccharin (5.0 g, 27.3 mmol) in N,N-dimethylformamide (50 mL) was added potassium tert-butoxide (6.7 g, 60 mol) portionwise. Then 2,2,2-trifluoroethanol toluene sulfonate (7.2 g, 28 mmol) was added in one portion. The mixture stirred for 14 hours at room temperature and was poured onto ice-water. The solution was acidified to pH 2 with concentrated HCl and the solid was collected by filtration, washed with water and air-dried. The desired compound was obtained as a white solid, m.p. 170-173° C. NMR (90 MHz) $D_6$-acetone: δ 8.3 (s, 1H), 8.1 (s, 2H), 7.5 (bs, 1H) and 4.2 (q, 2H).

Example 8

2-(Aminosulfonyl)-4-(2,2,2-trifluoroethylthio)benzoicacid, methyl ester

Into 6-trifluoroethoxy-thiosaccharin (1.5 g) in methanol (50 mL) at 0° was bubbled HCl gas for 0.5 hours. The reaction was allowed to warm to room temperature and stirred for 14 hours. The solvent was removed on the rotary evaporator and the solids were taken up in ethyl acetate and washed with 5% sodium bicarbonate, brine and dried with magnesium sulfate. After filtration, the filtrate was concentrated and the desired compound was obtained as a white solid, m.p. 117-120° C. NMR (200 MHz) $D_6$-acetone: δ 8.1 (s, 1H), 7.95 (s, 2H), 6.7 (bs, 2H), 4.1 (q, 2H) and 3.95 (s, 3H).

Example 9

4-(2,2,2-Trifluoroethylthio)-2-[[(4,6-dimethoxy-pyrimidin-2-yl)aminocarbonyl]aminosulfonyl]benzoic acid, methyl ester

To methyl 2-(aminosulfonyl)-4-(2,2,2-trifluoroethylthio)benzoate (0.1 g, 0.3 mmol) and dimethoxypyrimidine phenylcarbamate (0.08 g, 0.31 mmol) in dry acetonitrile (5 mL) was added 1,8-diazabicyclo-[5.4.0]-undec-7-ene (DBU) (0.5 mL). The mixture was allowed to stir for 1 hour, then water and 1N HCl (to pH 3) was added. The resultant solid was collected, washed with water and dried to furnish 0.1 g of the desired subject as a white solid, m.p. 171-173° C. NMR (200 MHz) $D_6$-acetone: δ 12.3 (bs, 1H), 9.8 (bs, 1H), 8.3 (d, 1H), 8.0 (dd, 1H), 7.8 (d, 1H), 5.9 (s, 1H), 4.1 (q, 2H), 4.0 (s, 6H) and 3.9 (s, 3H).

Using the procedures and examples shown above, the following compounds can be prepared.

It will be understood that the substituents X and Y are indistinguishable in the final product, and the compounds below wherein X is $OCH_3$ and Y is Cl should be regarded as compounds wherein X is Cl and Y is $OCH_3$, and are thus within the scope of our invention.

**Table I**

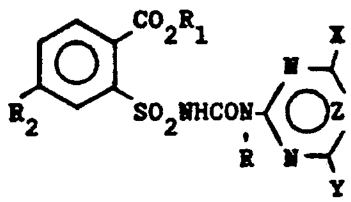

| $R_1$ | $R_2$ | R | X | Y | Z | m.p.(°C) |
|---|---|---|---|---|---|---|
| $CH_3$ | O-cyclopropyl | H | $CH_3$ | $CH_3$ | CH | |
| $CH_3$ | O-cyclopropyl | H | $OCH_3$ | $CH_3$ | CH | |
| $CH_3$ | O-cyclopropyl | H | $OCH_3$ | $OCH_3$ | CH | |
| $CH_3$ | O-cyclopropyl | H | Cl | $OCH_3$ | CH | |
| $CH_3$ | O-cyclopropyl | H | $OCH_3$ | $N(CH_3)_2$ | CH | |
| $CH_3$ | O-cyclopropyl | H | $CH_3$ | $CH_3$ | N | |
| $CH_3$ | O-cyclopropyl | H | $OCH_3$ | $CH_3$ | N | |
| $CH_3$ | O-cyclopropyl | H | $OCH_3$ | $OCH_3$ | N | |
| $CH_3$ | O-cyclopropyl | H | $OCH_3$ | $N(CH_3)_2$ | N | |
| $CH_3$ | O-cyclopropyl | H | $OCH_3$ | $OCH_2CF_3$ | N | |
| $CH_3$ | O-cyclopropyl | $CH_3$ | $OCH_3$ | $CH_3$ | N | |
| $CH_3$ | O-cyclopropyl | $CH_3$ | $OCH_3$ | $OCH_3$ | N | |
| $CH_3$ | O-cyclopropyl | $CH_3$ | $CH_3$ | $OCH_3$ | CH | |
| $CH_3$ | O-cyclopropyl | $CH_3$ | $OCH_3$ | $OCH_3$ | CH | |
| $CH_3$ | O-cyclopropyl | H | Br | $OCH_3$ | CH | |
| $CH_3$ | O-cyclopropyl | $CH_3$ | $OCH_3$ | $N(CH_3)_2$ | N | |
| $CH_3$ | O-cyclopropyl | H | $CH_3$ | $OCH_2CF_3$ | N | |
| $CH_3$ | O-cyclopropyl | H | $CH_3$ | $OCH_2CF_3$ | CH | |
| $CH_3$ | O-cyclopropyl | H | $OCH_3$ | $OCH_2CF_3$ | CH | |
| $CH_3$ | O-cyclopropyl | H | $OCH_3$ | $C_2H_5$ | CH | |
| $CH_3$ | O-cyclopropyl | H | $OCH_3$ | $C_2H_5$ | N | |
| $CH_3$ | O-cyclopropyl | H | $OCH_3$ | $NHCH_3$ | CH | |
| $CH_3$ | O-cyclopropyl | H | $OCH_3$ | $NHCH_3$ | N | |
| $CH_3$ | O-cyclopropyl | H | $CH_3$ | $OC_2H_5$ | CH | |
| $CH_3$ | O-cyclopropyl | H | $OCH_3$ | $OC_2H_5$ | CH | |
| $CH_3$ | O-cyclopropyl | H | $CH_3$ | $OC_2H_5$ | N | |

Table I (Continued)

| $R_1$ | $R_2$ | R | X | Y | Z | m.p.(°C) |
|---|---|---|---|---|---|---|
| $CH_3$ | O-cyclopropyl | H | $OCH_3$ | $OC_2H_5$ | N | |
| $CH_3$ | O-cyclopropyl | H | $CH_3$ | $OCH_2CHF_2$ | CH | |
| $CH_3$ | O-cyclopropyl | H | $OCH_3$ | $OCH_2CHF_2$ | CH | |
| $CH_3$ | O-cyclopropyl | H | $CH_3$ | $OCH_2CHF_2$ | N | |
| $CH_3$ | O-cyclopropyl | H | $OCH_3$ | $OCH_2CHF_2$ | N | |
| $CH_3$ | O-cyclopropyl | H | $CH_3$ | $OCH_2CH_2F$ | CH | |
| $CH_3$ | O-cyclopropyl | H | $OCH_3$ | $OCH_2CH_2F$ | CH | |
| $CH_3$ | O-cyclopropyl | H | $CH_3$ | $OCH_2CH_2F$ | N | |
| $CH_3$ | O-cyclopropyl | $CH_3$ | $OCH_3$ | $OCH_2CH_2F$ | N | |
| $CH_3$ | O-cyclopropyl | $CH_3$ | Cl | $OCH_3$ | CH | |
| $CH_3$ | O-cyclopropyl | H | Cl | $N(CH_3)_2$ | CH | |
| $CH_3$ | O-cyclopropyl | H | Cl | $OC_2H_5$ | CH | |
| $C_2H_5$ | O-cyclopropyl | H | $CH_3$ | $CH_3$ | CH | |
| $C_2H_5$ | O-cyclopropyl | H | $OCH_3$ | $CH_3$ | CH | |
| $C_2H_5$ | O-cyclopropyl | H | $OCH_3$ | $OCH_3$ | CH | |
| $C_2H_5$ | O-cyclopropyl | H | Cl | $OCH_3$ | CH | |
| $C_2H_5$ | O-cyclopropyl | H | $CH_3$ | $CH_3$ | N | |
| $C_2H_5$ | O-cyclopropyl | H | $OCH_3$ | $CH_3$ | N | |
| $C_2H_5$ | O-cyclopropyl | H | $OCH_3$ | $OCH_3$ | N | |
| $C_2H_5$ | O-cyclopropyl | H | Br | $OCH_3$ | CH | |
| $C_2H_5$ | O-cyclopropyl | H | $OCH_3$ | $N(CH_3)_2$ | CH | |
| $C_2H_5$ | O-cyclopropyl | H | $OCH_3$ | $N(CH_3)_2$ | N | |
| $C_2H_5$ | O-cyclopropyl | H | $OCH_3$ | $NHCH_3$ | N | |
| $C_2H_5$ | O-cyclopropyl | H | $OCH_3$ | $C_2H_5$ | CH | |
| $C_2H_5$ | O-cyclopropyl | H | $OCH_3$ | $C_2H_5$ | N | |
| $C_2H_5$ | O-cyclopropyl | H | $CH_3$ | $OC_2H_5$ | CH | |
| $C_2H_5$ | O-cyclopropyl | H | $CH_3$ | $OC_2H_5$ | N | |
| $C_2H_5$ | O-cyclopropyl | H | $OCH_3$ | $OC_2H_5$ | CH | |
| $C_2H_5$ | O-cyclopropyl | H | $OCH_3$ | $OC_2H_5$ | N | |
| $C_2H_5$ | O-cyclopropyl | H | $CH_3$ | $OCH_2CF_3$ | CH | |
| $C_2H_5$ | O-cyclopropyl | H | $CH_3$ | $OCH_2CF_3$ | N | |
| $C_2H_5$ | O-cyclopropyl | H | $OCH_3$ | $OCH_2CF_3$ | CH | |
| $C_2H_5$ | O-cyclopropyl | H | $OCH_3$ | $OCH_2CF_3$ | N | |

Table I (Continued)

| $R_1$ | $R_2$ | R | X | Y | Z | m.p. (°C) |
|---|---|---|---|---|---|---|
| $C_2H_5$ | O-cyclopropyl | H | $OCH_3$ | $OCH_2CHF_2$ | CH | |
| $C_2H_5$ | O-cyclopropyl | H | $OCH_3$ | $OCH_2CHF_2$ | N | |
| $C_2H_5$ | O-cyclopropyl | H | $CH_3$ | $OCH_2CHF_2$ | N | |
| $C_2H_5$ | O-cyclopropyl | H | $OCH_3$ | $OCH_2CH_2F$ | CH | |
| $C_2H_5$ | O-cyclopropyl | H | $OCH_3$ | $OCH_2CH_2F$ | N | |
| $C_2H_5$ | O-cyclopropyl | $CH_3$ | $OCH_3$ | $OCH_3$ | CH | |
| $C_2H_5$ | O-cyclopropyl | $CH_3$ | $CH_3$ | $OCH_3$ | N | |
| $C_2H_5$ | O-cyclopropyl | $CH_3$ | $OCH_3$ | $OCH_3$ | N | |
| n-$C_3H_7$ | O-cyclopropyl | H | $OCH_3$ | $OCH_3$ | CH | |
| n-$C_3H_7$ | O-cyclopropyl | H | $CH_3$ | $OCH_3$ | N | |
| $CH(CH_3)_2$ | O-cyclopropyl | H | $OCH_3$ | $CH_3$ | CH | |
| $CH(CH_3)_2$ | O-cyclopropyl | H | $OCH_3$ | $OCH_3$ | CH | |
| $CH(CH_3)_2$ | O-cyclopropyl | H | Cl | $OCH_3$ | CH | |
| $CH(CH_3)_2$ | O-cyclopropyl | H | $OCH_3$ | $CH_3$ | N | |
| $CH(CH_3)_2$ | O-cyclopropyl | H | $OCH_3$ | $OCH_3$ | N | |
| $CH(CH_3)_2$ | O-cyclopropyl | H | $OCH_3$ | $N(CH_3)_2$ | N | |
| $CH_3$ | $OCH_2$-cyclopropyl | H | $OCH_3$ | $OCH_3$ | CH | |
| $CH_3$ | $OCH_2$-cyclopropyl | H | $OCH_3$ | $CH_3$ | CH | |
| $CH_3$ | $OCH_2$-cyclopropyl | H | $OCH_3$ | Cl | CH | |
| $CH_3$ | $OCH_2$-cyclopropyl | H | $CH_3$ | $CH_3$ | CH | |
| $CH_3$ | $OCH_2$-cyclopropyl | H | $OCH_3$ | $OCH_3$ | N | |
| $CH_3$ | $OCH_2$-cyclopropyl | H | $OCH_3$ | $CH_3$ | N | |
| $CH_3$ | O-cyclobutyl | H | $OCH_3$ | $OCH_3$ | CH | |
| $CH_3$ | O-cyclobutyl | H | $OCH_3$ | $CH_3$ | CH | |
| $CH_3$ | O-cyclobutyl | H | $OCH_3$ | Cl | CH | |
| $CH_3$ | O-cyclobutyl | H | $CH_3$ | $CH_3$ | CH | |
| $CH_3$ | O-cyclobutyl | H | $OCH_3$ | $OCH_3$ | N | |
| $CH_2CH_3$ | O-cyclobutyl | H | $OCH_3$ | $CH_3$ | N | |
| $CH_3$ | $OCH_2CF_2CF_3$ | H | $OCH_3$ | $OCH_3$ | CH | |
| $CH_3$ | $OCH_2CF_2CF_3$ | H | $OCH_3$ | $CH_3$ | CH | |
| $CH_3$ | $OCH_2CF_2CF_3$ | H | $OCH_3$ | Cl | CH | |
| $CH_3$ | $OCH_2CF_2CF_3$ | H | $CH_3$ | $CH_3$ | CH | |
| $CH_3$ | $OCH_2CF_2CF_3$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $OCH_2CF_2CF_3$ | H | $OCH_3$ | $CH_3$ | N | |
| $CH_3$ | $OCH(CF_3)CH_3$ | H | $OCH_3$ | $OCH_3$ | CH | |
| $CH_3$ | $OCH(CF_3)CH_3$ | H | $OCH_3$ | $CH_3$ | CH | |
| $CH_3$ | $OCH(CF_3)CH_3$ | H | $OCH_3$ | Cl | CH | |
| $CH_3$ | $OCH(CF_3)CH_3$ | H | $CH_3$ | $CH_3$ | CH | |
| $CH_3$ | $OCH(CF_3)CH_3$ | H | $OCH_3$ | $OCH_3$ | N | |
| $CH_3$ | $OCH(CF_3)CH_3$ | H | $OCH_3$ | $CH_3$ | N | |
| $CH_3$ | $OCH_2CF_2CF_2H$ | H | $OCH_3$ | $OCH_3$ | CH | |
| $CH_3$ | $OCH_2CF_2CF_2H$ | H | $OCH_3$ | $CH_3$ | CH | |
| $CH_3$ | $OCH_2CF_2CF_2H$ | H | $OCH_3$ | Cl | CH | |
| $CH_3$ | $OCH_2CF_2CF_2H$ | H | $CH_3$ | $CH_3$ | CH | |
| $CH_3$ | $OCH_2CF_2CF_2H$ | H | $OCH_3$ | $OCH_3$ | N | |
| $CH_2CH_3$ | $OCH_2CF_2CF_2H$ | H | $OCH_3$ | $CH_3$ | N | |
| $CH_3$ | $OCH(CH_2F)_2$ | H | $OCH_3$ | $OCH_3$ | CH | |
| $CH_3$ | $OCH(CH_2F)_2$ | H | $OCH_3$ | $CH_3$ | CH | |
| $CH_3$ | $OCH(CH_2F)_2$ | H | $OCH_3$ | Cl | CH | |
| $CH_3$ | $OCH(CH_2F)_2$ | H | $CH_3$ | $CH_3$ | CH | |
| $CH_3$ | $OCH(CH_2F)_2$ | H | $OCH_3$ | $OCH_3$ | N | |
| $CH_3$ | $OCH(CH_2F)_2$ | H | $OCH_3$ | $CH_3$ | N | |
| $CH_3$ | $OCH(CH_2F)CH_3$ | H | $OCH_3$ | $OCH_3$ | CH | |
| $CH_3$ | $OCH(CH_2F)CH_3$ | H | $OCH_3$ | $CH_3$ | CH | |
| $CH_3$ | $OCH(CH_2F)CH_3$ | H | $OCH_3$ | Cl | CH | |
| $CH_3$ | $OCH(CH_2F)CH_3$ | H | $CH_3$ | $CH_3$ | CH | |
| $CH_3$ | $OCH(CH_2F)CH_3$ | H | $OCH_3$ | $OCH_3$ | N | |
| $CH_3$ | $OCH(CH_2F)CH_3$ | H | $OCH_3$ | $CH_3$ | N | |
| $CH_3$ | $O(CH_2)_5CH_2Cl$ | H | $OCH_3$ | $OCH_3$ | CH | |
| $CH_3$ | $O(CH_2)_5CH_2Cl$ | H | $OCH_3$ | $CH_3$ | CH | |
| $CH_3$ | $O(CH_2)_5CH_2Cl$ | H | $OCH_3$ | Cl | CH | |
| $CH_3$ | $O(CH_2)_5CH_2Cl$ | H | $CH_3$ | $CH_3$ | CH | |
| $CH_3$ | $O(CH_2)_5CH_2Cl$ | H | $OCH_3$ | $OCH_3$ | N | |
| $CH_2CH_3$ | $O(CH_2)_5CH_2Cl$ | H | $OCH_3$ | $CH_3$ | N | |
| $CH_3$ | $OC(Cl)=CHCl$ | H | $OCH_3$ | $OCH_3$ | CH | |
| $CH_3$ | $OC(Cl)=CHCl$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | OC(Cl)=CHCl | H | $OCH_3$ | Cl | CH | |
| $CH_3$ | OC(Cl)=CHCl | H | $CH_3$ | $CH_3$ | CH | |
| $CH_3$ | OC(Cl)=CHCl | H | $OCH_3$ | $OCH_3$ | N | |
| $CH_3$ | OC(Cl)=CHCl | H | $OCH_3$ | $CH_3$ | N | |
| $CH_3$ | $OCH_2C(Cl)=CH_2$ | H | $OCH_3$ | $OCH_3$ | CH | |
| $CH_3$ | $OCH_2C(Cl)=CH_2$ | H | $OCH_3$ | $CH_3$ | CH | |
| $CH_3$ | $OCH_2C(Cl)=CH_2$ | H | $OCH_3$ | Cl | CH | |
| $CH_3$ | $OCH_2C(Cl)=CH_2$ | H | $CH_3$ | $CH_3$ | CH | |
| $CH_3$ | $OCH_2C(Cl)=CH_2$ | H | $OCH_3$ | $OCH_3$ | N | |
| $CH_3$ | $OCH_2C(Cl)=CH_2$ | H | $OCH_3$ | $CH_3$ | N | |
| $CH_3$ | $OCH_2C\equiv CCH_2Cl$ | H | $OCH_3$ | $OCH_3$ | CH | |
| $CH_3$ | $OCH_2C\equiv CCH_2Cl$ | H | $OCH_3$ | $CH_3$ | CH | |
| $CH_3$ | $OCH_2C\equiv CCH_2Cl$ | H | $OCH_3$ | Cl | CH | |
| $CH_3$ | $OCH_2C\equiv CCH_2Cl$ | H | $CH_3$ | $CH_3$ | CH | |
| $CH_3$ | $OCH_2C\equiv CCH_2Cl$ | H | $OCH_3$ | $OCH_3$ | N | |
| $CH_2CH_3$ | $OCH_2C\equiv CCH_2Cl$ | H | $OCH_3$ | $CH_3$ | N | |
| $CH_3$ | $OCH_2OCH_3$ | H | $OCH_3$ | $OCH_3$ | CH | |
| $CH_3$ | $OCH_2OCH_3$ | H | $OCH_3$ | $CH_3$ | CH | |
| $CH_3$ | $OCH_2OCH_3$ | H | $OCH_3$ | Cl | CH | |
| $CH_3$ | $OCH_2OCH_3$ | H | $CH_3$ | $CH_3$ | CH | |
| $CH_3$ | $OCH_2OCH_3$ | H | $OCH_3$ | $OCH_3$ | N | |
| $CH_3$ | $OCH_2OCH_3$ | H | $OCH_3$ | $CH_3$ | N | |
| $CH_3$ | $OCH_2OCH_2CF_3$ | H | $OCH_3$ | $OCH_3$ | CH | |
| $CH_3$ | $OCH_2OCH_2CF_3$ | H | $OCH_3$ | $CH_3$ | CH | |
| $CH_3$ | $OCH_2OCH_2CF_3$ | H | $OCH_3$ | Cl | CH | |
| $CH_3$ | $OCH_2OCH_2CF_3$ | H | $CH_3$ | $CH_3$ | CH | |
| $CH_3$ | $OCH_2OCH_2CF_3$ | H | $OCH_3$ | $OCH_3$ | N | |
| $CH_3$ | $OCH_2OCH_2CF_3$ | H | $OCH_3$ | $CH_3$ | N | |
| $CH_3$ | $OCH_2SCH_3$ | H | $OCH_3$ | $OCH_3$ | CH | |
| $CH_3$ | $OCH_2SCH_3$ | H | $OCH_3$ | $CH_3$ | CH | |
| $CH_3$ | $OCH_2SCH_3$ | H | $OCH_3$ | Cl | CH | |
| $CH_3$ | $OCH_2SCH_3$ | H | $CH_3$ | $CH_3$ | CH | |
| $CH_3$ | $OCH_2SCH_3$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_2CH_3$ | $OCH_2SCH_3$ | H | $OCH_3$ | $CH_3$ | N | |
| $CH_3$ | $OCH_2CN$ | H | $OCH_3$ | $OCH_3$ | CH | |
| $CH_3$ | $OCH_2CN$ | H | $OCH_3$ | $CH_3$ | CH | |
| $CH_3$ | $OCH_2CN$ | H | $OCH_3$ | Cl | CH | |
| $CH_3$ | $OCH_2CN$ | H | $CH_3$ | $CH_3$ | CH | |
| $CH_3$ | $OCH_2CN$ | H | $OCH_3$ | $OCH_3$ | N | |
| $CH_3$ | $OCH_2CN$ | H | $OCH_3$ | $CH_3$ | N | |
| $CH_3$ | $OCH_2C(O)CH_3$ | H | $OCH_3$ | $OCH_3$ | CH | |
| $CH_3$ | $OCH_2C(O)CH_3$ | H | $OCH_3$ | $CH_3$ | CH | |
| $CH_3$ | $OCH_2C(O)CH_3$ | H | $OCH_3$ | Cl | CH | |
| $CH_3$ | $OCH_2C(O)CH_3$ | H | $CH_3$ | $CH_3$ | CH | |
| $CH_3$ | $OCH_2C(O)CH_3$ | H | $OCH_3$ | $OCH_3$ | N | |
| $CH_3$ | $OCH_2C(O)CH_3$ | H | $OCH_3$ | $CH_3$ | N | |
| $CH_3$ | $OCH_2CH_2N(CH_3)_2$ | H | $OCH_3$ | $OCH_3$ | CH | |
| $CH_3$ | $OCH_2CH_2N(CH_3)_2$ | H | $OCH_3$ | $CH_3$ | CH | |
| $CH_3$ | $OCH_2CH_2N(CH_3)_2$ | H | $OCH_3$ | Cl | CH | |
| $CH_3$ | $OCH_2CH_2N(CH_3)_2$ | H | $CH_3$ | $CH_3$ | CH | |
| $CH_3$ | $OCH_2CH_2N(CH_3)_2$ | H | $OCH_3$ | $OCH_3$ | N | |
| $CH_2CH_3$ | $OCH_2CH_2N(CH_3)_2$ | H | $OCH_3$ | $CH_3$ | N | |
| $CH_3$ | $OCH_2CH_2OCH_2CF_3$ | H | $OCH_3$ | $OCH_3$ | CH | |
| $CH_3$ | $OCH_2CH_2OCH_2CF_3$ | H | $OCH_3$ | $CH_3$ | CH | |
| $CH_3$ | $OCH_2CH_2OCH_2CF_3$ | H | $OCH_3$ | Cl | CH | |
| $CH_3$ | $OCH_2CH_2OCH_2CF_3$ | H | $CH_3$ | $CH_3$ | CH | |
| $CH_3$ | $OCH_2CH_2OCH_2CF_3$ | H | $OCH_3$ | $OCH_3$ | N | |
| $CH_3$ | $OCH_2CH_2OCH_2CF_3$ | H | $OCH_3$ | $CH_3$ | N | |
| $CH_3$ | $OCH_2CH_2SCH_2CF_3$ | H | $OCH_3$ | $OCH_3$ | CH | |
| $CH_3$ | $OCH_2CH_2SCH_2CF_3$ | H | $OCH_3$ | $CH_3$ | CH | |
| $CH_3$ | $OCH_2CH_2SCH_2CF_3$ | H | $OCH_3$ | Cl | CH | |
| $CH_3$ | $OCH_2CH_2SCH_2CF_3$ | H | $CH_3$ | $CH_3$ | CH | |
| $CH_3$ | $OCH_2CH_2SCH_2CF_3$ | H | $OCH_3$ | $OCH_3$ | N | |
| $CH_3$ | $OCH_2CH_2SCH_2CF_3$ | H | $OCH_3$ | $CH_3$ | N | |
| $CH_3$ | $OCF_2CF_2H$ | H | $OCH_3$ | $OCH_3$ | CH | |
| $CH_3$ | $OCF_2CF_2H$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $OCF_2CF_2H$ | H | $OCH_3$ | Cl | CH | |
| $CH_3$ | $OCF_2CF_2H$ | H | $CH_3$ | $CH_3$ | CH | |
| $CH_3$ | $OCF_2CF_2H$ | H | $OCH_3$ | $OCH_3$ | N | |
| $CH_2CH_3$ | $OCF_2CF_2H$ | H | $OCH_3$ | $CH_3$ | N | |
| $CH_3$ | $OCH_2CH_2S(O)CH_2CF_3$ | H | $OCH_3$ | $OCH_3$ | CH | |
| $CH_3$ | $OCH_2CH_2S(O)CH_2CF_3$ | H | $OCH_3$ | $CH_3$ | CH | |
| $CH_3$ | $OCH_2CH_2S(O)CH_2CF_3$ | H | $OCH_3$ | Cl | CH | |
| $CH_3$ | $OCH_2CH_2S(O)CH_2CF_3$ | H | $CH_3$ | $CH_3$ | CH | |
| $CH_3$ | $OCH_2CH_2S(O)CH_2CF_3$ | H | $OCH_3$ | $OCH_3$ | N | |
| $CH_3$ | $OCH_2CH_2S(O)CH_2CF_3$ | H | $OCH_3$ | $CH_3$ | N | |
| $CH_3$ | $OCH_2CH_2S(O)_2CH_2CF_3$ | H | $OCH_3$ | $OCH_3$ | CH | |
| $CH_3$ | $OCH_2CH_2S(O)_2CH_2CF_3$ | H | $OCH_3$ | $CH_3$ | CH | |
| $CH_3$ | $OCH_2CH_2S(O)_2CH_2CF_3$ | H | $OCH_3$ | Cl | CH | |
| $CH_3$ | $OCH_2CH_2S(O)_2CH_2CF_3$ | H | $CH_3$ | $CH_3$ | CH | |
| $CH_3$ | $OCH_2CH_2S(O)_2CH_2CF_3$ | H | $OCH_3$ | $OCH_3$ | N | |
| $CH_3$ | $OCH_2CH_2S(O)_2CH_2CF_3$ | H | $OCH_3$ | $CH_3$ | N | |
| $CH_3$ | $OCH_2CH_2CN$ | H | $OCH_3$ | $OCH_3$ | CH | |
| $CH_3$ | $OCH_2CH_2CN$ | H | $OCH_3$ | $CH_3$ | CH | |
| $CH_3$ | $OCH_2CH_2CN$ | H | $OCH_3$ | Cl | CH | |
| $CH_3$ | $OCH_2CH_2CN$ | H | $CH_3$ | $CH_3$ | CH | |
| $CH_3$ | $OCH_2CH_2CN$ | H | $OCH_3$ | $OCH_3$ | N | |
| $CH_2CH_3$ | $OCH_2CH_2CN$ | H | $OCH_3$ | $CH_3$ | N | |
| $CH_3$ | S-cyclopropyl | H | $OCH_3$ | $OCH_3$ | CH | |
| $CH_3$ | S-cyclopropyl | H | $OCH_3$ | $CH_3$ | CH | |
| $CH_3$ | S-cyclopropyl | H | $OCH_3$ | Cl | CH | |
| $CH_3$ | S-cyclopropyl | H | $CH_3$ | $CH_3$ | CH | |
| $CH_3$ | S-cyclopropyl | H | $OCH_3$ | $OCH_3$ | N | |
| $CH_3$ | S-cyclopropyl | H | $OCH_3$ | $CH_3$ | N | |
| $CH_3$ | $SCH_2$-cyclopropyl | H | $OCH_3$ | $OCH_3$ | CH | |
| $CH_3$ | $SCH_2$-cyclopropyl | H | $OCH_3$ | $CH_3$ | CH | |
| $CH_3$ | $SCH_2$-cyclopropyl | H | $OCH_3$ | Cl | CH | |
| $CH_3$ | $SCH_2$-cyclopropyl | H | $CH_3$ | $CH_3$ | CH | |
| $CH_3$ | $SCH_2$-cyclopropyl | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $SCH_2$-cyclopropyl | H | $OCH_3$ | $CH_3$ | N | |
| $CH_3$ | S-cyclobutyl | H | $OCH_3$ | $OCH_3$ | CH | |
| $CH_3$ | S-cyclobutyl | H | $OCH_3$ | $CH_3$ | CH | |
| $CH_3$ | S-cyclobutyl | H | $OCH_3$ | Cl | CH | |
| $CH_3$ | S-cyclobutyl | H | $CH_3$ | $CH_3$ | CH | |
| $CH_3$ | S-cyclobutyl | H | $OCH_3$ | $OCH_3$ | N | |
| $CH_2CH_3$ | S-cyclobutyl | H | $OCH_3$ | $CH_3$ | N | |
| $CH_3$ | $SCH_2CF_2CF_3$ | H | $OCH_3$ | $OCH_3$ | CH | |
| $CH_3$ | $SCH_2CF_2CF_3$ | H | $OCH_3$ | $CH_3$ | CH | |
| $CH_3$ | $SCH_2CF_2CF_3$ | H | $OCH_3$ | Cl | CH | |
| $CH_3$ | $SCH_2CF_2CF_3$ | H | $CH_3$ | $CH_3$ | CH | |
| $CH_3$ | $SCH_2CF_2CF_3$ | H | $OCH_3$ | $OCH_3$ | N | |
| $CH_3$ | $SCH_2CF_2CF_3$ | H | $OCH_3$ | $CH_3$ | N | |
| $CH_3$ | $SCH(CF_3)CH_3$ | H | $OCH_3$ | $OCH_3$ | CH | |
| $CH_3$ | $SCH(CF_3)CH_3$ | H | $OCH_3$ | $CH_3$ | CH | |
| $CH_3$ | $SCH(CF_3)CH_3$ | H | $OCH_3$ | Cl | CH | |
| $CH_3$ | $SCH(CF_3)CH_3$ | H | $CH_3$ | $CH_3$ | CH | |
| $CH_3$ | $SCH(CF_3)CH_3$ | H | $OCH_3$ | $OCH_3$ | N | |
| $CH_3$ | $SCH(CF_3)CH_3$ | H | $OCH_3$ | $CH_3$ | N | |
| $CH_3$ | $SCH_2CF_2CF_2H$ | H | $OCH_3$ | $OCH_3$ | CH | |
| $CH_3$ | $SCH_2CF_2CF_2H$ | H | $OCH_3$ | $CH_3$ | CH | |
| $CH_3$ | $SCH_2CF_2CF_2H$ | H | $OCH_3$ | Cl | CH | |
| $CH_3$ | $SCH_2CF_2CF_2H$ | H | $CH_3$ | $CH_3$ | CH | |
| $CH_3$ | $SCH_2CF_2CF_2H$ | H | $OCH_3$ | $OCH_3$ | N | |
| $CH_2CH_3$ | $SCH_2CF_2CF_2H$ | H | $OCH_3$ | $CH_3$ | N | |
| $CH_3$ | $SCH(CH_2F)_2$ | H | $OCH_3$ | $OCH_3$ | CH | |
| $CH_3$ | $SCH(CH_2F)_2$ | H | $OCH_3$ | $CH_3$ | CH | |
| $CH_3$ | $SCH(CH_2F)_2$ | H | $OCH_3$ | Cl | CH | |
| $CH_3$ | $SCH(CH_2F)_2$ | H | $CH_3$ | $CH_3$ | CH | |
| $CH_3$ | $SCH(CH_2F)_2$ | H | $OCH_3$ | $OCH_3$ | N | |
| $CH_3$ | $SCH(CH_2F)_2$ | H | $OCH_3$ | $CH_3$ | N | |
| $CH_3$ | $SCH(CH_2F)CH_3$ | H | $OCH_3$ | $OCH_3$ | CH | |
| $CH_3$ | $SCH(CH_2F)CH_3$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $SCH(CH_2F)CH_3$ | H | $OCH_3$ | Cl | CH | |
| $CH_3$ | $SCH(CH_2F)CH_3$ | H | $CH_3$ | $CH_3$ | CH | |
| $CH_3$ | $SCH(CH_2F)CH_3$ | H | $OCH_3$ | $OCH_3$ | N | |
| $CH_3$ | $SCH(CH_2F)CH_3$ | H | $OCH_3$ | $CH_3$ | N | |
| $CH_3$ | $S(CH_2)_5CH_2Cl$ | H | $OCH_3$ | $OCH_3$ | CH | |
| $CH_3$ | $S(CH_2)_5CH_2Cl$ | H | $OCH_3$ | $CH_3$ | CH | |
| $CH_3$ | $S(CH_2)_5CH_2Cl$ | H | $OCH_3$ | Cl | CH | |
| $CH_3$ | $S(CH_2)_5CH_2Cl$ | H | $CH_3$ | $CH_3$ | CH | |
| $CH_3$ | $S(CH_2)_5CH_2Cl$ | H | $OCH_3$ | $OCH_3$ | N | |
| $CH_2CH_3$ | $S(CH_2)_5CH_2Cl$ | H | $OCH_3$ | $CH_3$ | N | |
| $CH_3$ | $SC(Cl){=}CHCl$ | H | $OCH_3$ | $OCH_3$ | CH | |
| $CH_3$ | $SC(Cl){=}CHCl$ | H | $OCH_3$ | $CH_3$ | CH | |
| $CH_3$ | $SC(Cl){=}CHCl$ | H | $OCH_3$ | Cl | CH | |
| $CH_3$ | $SC(Cl){=}CHCl$ | H | $CH_3$ | $CH_3$ | CH | |
| $CH_3$ | $SC(Cl){=}CHCl$ | H | $OCH_3$ | $OCH_3$ | N | |
| $CH_3$ | $SC(Cl){=}CHCl$ | H | $OCH_3$ | $CH_3$ | N | |
| $CH_3$ | $SCH_2C(Cl){=}CH_2$ | H | $OCH_3$ | $OCH_3$ | CH | |
| $CH_3$ | $SCH_2C(Cl){=}CH_2$ | H | $OCH_3$ | $CH_3$ | CH | |
| $CH_3$ | $SCH_2C(Cl){=}CH_2$ | H | $OCH_3$ | Cl | CH | |
| $CH_3$ | $SCH_2C(Cl){=}CH_2$ | H | $CH_3$ | $CH_3$ | CH | |
| $CH_3$ | $SCH_2C(Cl){=}CH_2$ | H | $OCH_3$ | $OCH_3$ | N | |
| $CH_3$ | $SCH_2C(Cl){=}CH_2$ | H | $OCH_3$ | $CH_3$ | N | |
| $CH_3$ | $SCH_2C{\equiv}CCH_2Cl$ | H | $OCH_3$ | $OCH_3$ | CH | |
| $CH_3$ | $SCH_2C{\equiv}CCH_2Cl$ | H | $OCH_3$ | $CH_3$ | CH | |
| $CH_3$ | $SCH_2C{\equiv}CCH_2Cl$ | H | $OCH_3$ | Cl | CH | |
| $CH_3$ | $SCH_2C{\equiv}CCH_2Cl$ | H | $CH_3$ | $CH_3$ | CH | |
| $CH_3$ | $SCH_2C{\equiv}CCH_2Cl$ | H | $OCH_3$ | $OCH_3$ | N | |
| $CH_2CH_3$ | $SCH_2C{\equiv}CCH_2Cl$ | H | $OCH_3$ | $CH_3$ | N | |
| $CH_3$ | $SCH_2OCH_3$ | H | $OCH_3$ | $OCH_3$ | CH | |
| $CH_3$ | $SCH_2OCH_3$ | H | $OCH_3$ | $CH_3$ | CH | |
| $CH_3$ | $SCH_2OCH_3$ | H | $OCH_3$ | Cl | CH | |
| $CH_3$ | $SCH_2OCH_3$ | H | $CH_3$ | $CH_3$ | CH | |
| $CH_3$ | $SCH_2OCH_3$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $SCH_2OCH_3$ | H | $OCH_3$ | $CH_3$ | N | |
| $CH_3$ | $SCH_2OCH_2CF_3$ | H | $OCH_3$ | $OCH_3$ | CH | |
| $CH_3$ | $SCH_2OCH_2CF_3$ | H | $OCH_3$ | $CH_3$ | CH | |
| $CH_3$ | $SCH_2OCH_2CF_3$ | H | $OCH_3$ | Cl | CH | |
| $CH_3$ | $SCH_2OCH_2CF_3$ | H | $CH_3$ | $CH_3$ | CH | |
| $CH_3$ | $SCH_2OCH_2CF_3$ | H | $OCH_3$ | $OCH_3$ | N | |
| $CH_3$ | $SCH_2OCH_2CF_3$ | H | $OCH_3$ | $CH_3$ | N | |
| $CH_3$ | $SCH_2SCH_3$ | H | $OCH_3$ | $OCH_3$ | CH | |
| $CH_3$ | $SCH_2SCH_3$ | H | $OCH_3$ | $CH_3$ | CH | |
| $CH_3$ | $SCH_2SCH_3$ | H | $OCH_3$ | Cl | CH | |
| $CH_3$ | $SCH_2SCH_3$ | H | $CH_3$ | $CH_3$ | CH | |
| $CH_3$ | $SCH_2SCH_3$ | H | $OCH_3$ | $OCH_3$ | N | |
| $CH_2CH_3$ | $SCH_2SCH_3$ | H | $OCH_3$ | $CH_3$ | N | |
| $CH_3$ | $SCH_2CN$ | H | $OCH_3$ | $OCH_3$ | CH | |
| $CH_3$ | $SCH_2CN$ | H | $OCH_3$ | $CH_3$ | CH | |
| $CH_3$ | $SCH_2CN$ | H | $OCH_3$ | Cl | CH | |
| $CH_3$ | $SCH_2CN$ | H | $CH_3$ | $CH_3$ | CH | |
| $CH_3$ | $SCH_2CN$ | H | $OCH_3$ | $OCH_3$ | N | |
| $CH_3$ | $SCH_2CN$ | H | $OCH_3$ | $CH_3$ | N | |
| $CH_3$ | $SCH_2C(O)CH_3$ | H | $OCH_3$ | $OCH_3$ | CH | |
| $CH_3$ | $SCH_2C(O)CH_3$ | H | $OCH_3$ | $CH_3$ | CH | |
| $CH_3$ | $SCH_2C(O)CH_3$ | H | $OCH_3$ | Cl | CH | |
| $CH_3$ | $SCH_2C(O)CH_3$ | H | $CH_3$ | $CH_3$ | CH | |
| $CH_3$ | $SCH_2C(O)CH_3$ | H | $OCH_3$ | $OCH_3$ | N | |
| $CH_3$ | $SCH_2C(O)CH_3$ | H | $OCH_3$ | $CH_3$ | N | |
| $CH_3$ | $SCH_2CH_2N(CH_3)_2$ | H | $OCH_3$ | $OCH_3$ | CH | |
| $CH_3$ | $SCH_2CH_2N(CH_3)_2$ | H | $OCH_3$ | $CH_3$ | CH | |
| $CH_3$ | $SCH_2CH_2N(CH_3)_2$ | H | $OCH_3$ | Cl | CH | |
| $CH_3$ | $SCH_2CH_2N(CH_3)_2$ | H | $CH_3$ | $CH_3$ | CH | |
| $CH_3$ | $SCH_2CH_2N(CH_3)_2$ | H | $OCH_3$ | $OCH_3$ | N | |
| $CH_2CH_3$ | $SCH_2CH_2N(CH_3)_2$ | H | $OCH_3$ | $CH_3$ | N | |
| $CH_3$ | $SCH_2CH_2OCH_2CF_3$ | H | $OCH_3$ | $OCH_3$ | CH | |
| $CH_3$ | $SCH_2CH_2OCH_2CF_3$ | H | $OCH_3$ | $CH_3$ | CH | |

**Table I (Continued)**

| $R_1$ | $R_2$ | R | X | Y | Z | m.p.(°C) |
|---|---|---|---|---|---|---|
| $CH_3$ | $SCH_2CH_2OCH_2CF_3$ | H | $OCH_3$ | Cl | CH | |
| $CH_3$ | $SCH_2CH_2OCH_2CF_3$ | H | $CH_3$ | $CH_3$ | CH | |
| $CH_3$ | $SCH_2CH_2OCH_2CF_3$ | H | $OCH_3$ | $OCH_3$ | N | |
| $CH_3$ | $SCH_2CH_2OCH_2CF_3$ | H | $OCH_3$ | $CH_3$ | N | |
| $CH_3$ | $SCH_2CH_2SCH_2CF_3$ | H | $OCH_3$ | $OCH_3$ | CH | |
| $CH_3$ | $SCH_2CH_2SCH_2CF_3$ | H | $OCH_3$ | $CH_3$ | CH | |
| $CH_3$ | $SCH_2CH_2SCH_2CF_3$ | H | $OCH_3$ | Cl | CH | |
| $CH_3$ | $SCH_2CH_2SCH_2CF_3$ | H | $CH_3$ | $CH_3$ | CH | |
| $CH_3$ | $SCH_2CH_2SCH_2CF_3$ | H | $OCH_3$ | $OCH_3$ | N | |
| $CH_3$ | $SCH_2CH_2SCH_2CF_3$ | H | $OCH_3$ | $CH_3$ | N | |
| $CH_3$ | $SCF_2CF_2H$ | H | $OCH_3$ | $OCH_3$ | CH | |
| $CH_3$ | $SCF_2CF_2H$ | H | $OCH_3$ | $CH_3$ | CH | |
| $CH_3$ | $SCF_2CF_2H$ | H | $OCH_3$ | Cl | CH | |
| $CH_3$ | $SCF_2CF_2H$ | H | $CH_3$ | $CH_3$ | CH | |
| $CH_3$ | $SCF_2CF_2H$ | H | $OCH_3$ | $OCH_3$ | N | |
| $CH_2CH_3$ | $SCF_2CF_2H$ | H | $OCH_3$ | $CH_3$ | N | |
| $CH_3$ | $SCH_2CH_2CN$ | H | $OCH_3$ | $OCH_3$ | CH | |
| $CH_3$ | $SCH_2CH_2CN$ | H | $OCH_3$ | $CH_3$ | CH | |
| $CH_3$ | $SCH_2CH_2CN$ | H | $OCH_3$ | Cl | CH | |
| $CH_3$ | $SCH_2CH_2CN$ | H | $CH_3$ | $CH_3$ | CH | |
| $CH_3$ | $SCH_2CH_2CN$ | H | $OCH_3$ | $OCH_3$ | N | |
| $CH_2CH_3$ | $SCH_2CH_2CN$ | H | $OCH_3$ | $CH_3$ | N | |
| $CH_3$ | $SCH_2CH_2F$ | H | $OCH_3$ | $OCH_3$ | CH | |
| $CH_3$ | $SCH_2CH_2F$ | H | $OCH_3$ | $CH_3$ | CH | |
| $CH_3$ | $SCH_2CH_2F$ | H | $OCH_3$ | Cl | CH | |
| $CH_3$ | $SCH_2CH_2F$ | H | $CH_3$ | $CH_3$ | CH | |
| $CH_3$ | $SCH_2CH_2F$ | H | $OCH_3$ | $OCH_3$ | N | |
| $CH_3$ | $SCH_2CH_2F$ | H | $OCH_3$ | $CH_3$ | N | |
| $CH_3$ | $SCH_2CHF_2$ | H | $OCH_3$ | $OCH_3$ | CH | |
| $CH_3$ | $SCH_2CHF_2$ | H | $OCH_3$ | $CH_3$ | CH | |
| $CH_3$ | $SCH_2CHF_2$ | H | $OCH_3$ | Cl | CH | |
| $CH_3$ | $SCH_2CHF_2$ | H | $CH_3$ | $CH_3$ | CH | |
| $CH_3$ | $SCH_2CHF_2$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $SCH_2CHF_2$ | H | $OCH_3$ | $CH_3$ | N | |
| $CH_3$ | $SCH_2CF_3$ | H | $OCH_3$ | $OCH_3$ | CH | |
| $CH_3$ | $SCH_2CF_3$ | H | $OCH_3$ | $CH_3$ | CH | |
| $CH_3$ | $SCH_2CF_3$ | H | $OCH_3$ | Cl | CH | |
| $CH_3$ | $SCH_2CF_3$ | H | $CH_3$ | $CH_3$ | CH | |
| $CH_3$ | $SCH_2CF_3$ | H | $OCH_3$ | $OCH_3$ | N | |
| $CH_2CH_3$ | $SCH_2CF_3$ | H | $OCH_3$ | $CH_3$ | N | |
| $CH_3$ | S(O)-cyclopropyl | H | $OCH_3$ | $OCH_3$ | CH | |
| $CH_3$ | S(O)-cyclopropyl | H | $OCH_3$ | $CH_3$ | CH | |
| $CH_3$ | S(O)-cyclopropyl | H | $OCH_3$ | Cl | CH | |
| $CH_3$ | S(O)-cyclopropyl | H | $CH_3$ | $CH_3$ | CH | |
| $CH_3$ | S(O)-cyclopropyl | H | $OCH_3$ | $OCH_3$ | N | |
| $CH_3$ | S(O)-cyclopropyl | H | $OCH_3$ | $CH_3$ | N | |
| $CH_3$ | $S(O)CH_2$-cyclopropyl | H | $OCH_3$ | $OCH_3$ | CH | |
| $CH_3$ | $S(O)CH_2$-cyclopropyl | H | $OCH_3$ | $CH_3$ | CH | |
| $CH_3$ | $S(O)CH_2$-cyclopropyl | H | $OCH_3$ | Cl | CH | |
| $CH_3$ | $S(O)CH_2$-cyclopropyl | H | $CH_3$ | $CH_3$ | CH | |
| $CH_3$ | $S(O)CH_2$-cyclopropyl | H | $OCH_3$ | $OCH_3$ | N | |
| $CH_3$ | $S(O)CH_2$-cyclopropyl | H | $OCH_3$ | $CH_3$ | N | |
| $CH_3$ | S(O)-cyclobutyl | H | $OCH_3$ | $OCH_3$ | CH | |
| $CH_3$ | S(O)-cyclobutyl | H | $OCH_3$ | $CH_3$ | CH | |
| $CH_3$ | S(O)-cyclobutyl | H | $OCH_3$ | Cl | CH | |
| $CH_3$ | S(O)-cyclobutyl | H | $CH_3$ | $CH_3$ | CH | |
| $CH_3$ | S(O)-cyclobutyl | H | $OCH_3$ | $OCH_3$ | N | |
| $CH_2CH_3$ | S(O)-cyclobutyl | H | $OCH_3$ | $CH_3$ | N | |
| $CH_3$ | $S(O)CH_2CF_2CF_3$ | H | $OCH_3$ | $OCH_3$ | CH | |
| $CH_3$ | $S(O)CH_2CF_2CF_3$ | H | $OCH_3$ | $CH_3$ | CH | |
| $CH_3$ | $S(O)CH_2CF_2CF_3$ | H | $OCH_3$ | Cl | CH | |
| $CH_3$ | $S(O)CH_2CF_2CF_3$ | H | $CH_3$ | $CH_3$ | CH | |
| $CH_3$ | $S(O)CH_2CF_2CF_3$ | H | $OCH_3$ | $OCH_3$ | N | |
| $CH_3$ | $S(O)CH_2CF_2CF_3$ | H | $OCH_3$ | $CH_3$ | N | |
| $CH_3$ | $S(O)CH(CF_3)CH_3$ | H | $OCH_3$ | $OCH_3$ | CH | |
| $CH_3$ | $S(O)CH(CF_3)CH_3$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $S(O)CH(CF_3)CH_3$ | H | $OCH_3$ | Cl | CH | |
| $CH_3$ | $S(O)CH(CF_3)CH_3$ | H | $CH_3$ | $CH_3$ | CH | |
| $CH_3$ | $S(O)CH(CF_3)CH_3$ | H | $OCH_3$ | $OCH_3$ | N | |
| $CH_3$ | $S(O)CH(CF_3)CH_3$ | H | $OCH_3$ | $CH_3$ | N | |
| $CH_3$ | $S(O)CH_2CF_2CF_2H$ | H | $OCH_3$ | $OCH_3$ | CH | |
| $CH_3$ | $S(O)CH_2CF_2CF_2H$ | H | $OCH_3$ | $CH_3$ | CH | |
| $CH_3$ | $S(O)CH_2CF_2CF_2H$ | H | $OCH_3$ | Cl | CH | |
| $CH_3$ | $S(O)CH_2CF_2CF_2H$ | H | $CH_3$ | $CH_3$ | CH | |
| $CH_3$ | $S(O)CH_2CF_2CF_2H$ | H | $OCH_3$ | $OCH_3$ | N | |
| $CH_2CH_3$ | $S(O)CH_2CF_2CF_2H$ | H | $OCH_3$ | $CH_3$ | N | |
| $CH_3$ | $S(O)CH(CH_2F)_2$ | H | $OCH_3$ | $OCH_3$ | CH | |
| $CH_3$ | $S(O)CH(CH_2F)_2$ | H | $OCH_3$ | $CH_3$ | CH | |
| $CH_3$ | $S(O)CH(CH_2F)_2$ | H | $OCH_3$ | Cl | CH | |
| $CH_3$ | $S(O)CH(CH_2F)_2$ | H | $CH_3$ | $CH_3$ | CH | |
| $CH_3$ | $S(O)CH(CH_2F)_2$ | H | $OCH_3$ | $OCH_3$ | N | |
| $CH_3$ | $S(O)CH(CH_2F)_2$ | H | $OCH_3$ | $CH_3$ | N | |
| $CH_3$ | $S(O)CH(CH_2F)CH_3$ | H | $OCH_3$ | $OCH_3$ | CH | |
| $CH_3$ | $S(O)CH(CH_2F)CH_3$ | H | $OCH_3$ | $CH_3$ | CH | |
| $CH_3$ | $S(O)CH(CH_2F)CH_3$ | H | $OCH_3$ | Cl | CH | |
| $CH_3$ | $S(O)CH(CH_2F)CH_3$ | H | $CH_3$ | $CH_3$ | CH | |
| $CH_3$ | $S(O)CH(CH_2F)CH_3$ | H | $OCH_3$ | $OCH_3$ | N | |
| $CH_3$ | $S(O)CH(CH_2F)CH_3$ | H | $OCH_3$ | $CH_3$ | N | |
| $CH_3$ | $S(O)(CH_2)_5CH_2Cl$ | H | $OCH_3$ | $OCH_3$ | CH | |
| $CH_3$ | $S(O)(CH_2)_5CH_2Cl$ | H | $OCH_3$ | $CH_3$ | CH | |
| $CH_3$ | $S(O)(CH_2)_5CH_2Cl$ | H | $OCH_3$ | Cl | CH | |
| $CH_3$ | $S(O)(CH_2)_5CH_2Cl$ | H | $CH_3$ | $CH_3$ | CH | |
| $CH_3$ | $S(O)(CH_2)_5CH_2Cl$ | H | $OCH_3$ | $OCH_3$ | N | |
| $CH_2CH_3$ | $S(O)(CH_2)_5CH_2Cl$ | H | $OCH_3$ | $CH_3$ | N | |
| $CH_3$ | $S(O)C(Cl)=CHCl$ | H | $OCH_3$ | $OCH_3$ | CH | |
| $CH_3$ | $S(O)C(Cl)=CHCl$ | H | $OCH_3$ | $CH_3$ | CH | |
| $CH_3$ | $S(O)C(Cl)=CHCl$ | H | $OCH_3$ | Cl | CH | |
| $CH_3$ | $S(O)C(Cl)=CHCl$ | H | $CH_3$ | $CH_3$ | CH | |
| $CH_3$ | $S(O)C(Cl)=CHCl$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $S(O)C(Cl)=CHCl$ | H | $OCH_3$ | $CH_3$ | N | |
| $CH_3$ | $S(O)CH_2C(Cl)=CH_2$ | H | $OCH_3$ | $OCH_3$ | CH | |
| $CH_3$ | $S(O)CH_2C(Cl)=CH_2$ | H | $OCH_3$ | $CH_3$ | CH | |
| $CH_3$ | $S(O)CH_2C(Cl)=CH_2$ | H | $OCH_3$ | Cl | CH | |
| $CH_3$ | $S(O)CH_2C(Cl)=CH_2$ | H | $CH_3$ | $CH_3$ | CH | |
| $CH_3$ | $S(O)CH_2C(Cl)=CH_2$ | H | $OCH_3$ | $OCH_3$ | N | |
| $CH_3$ | $S(O)CH_2C(Cl)=CH_2$ | H | $OCH_3$ | $CH_3$ | N | |
| $CH_3$ | $S(O)CH_2C\equiv CCH_2Cl$ | H | $OCH_3$ | $OCH_3$ | CH | |
| $CH_3$ | $S(O)CH_2C\equiv CCH_2Cl$ | H | $OCH_3$ | $CH_3$ | CH | |
| $CH_3$ | $S(O)CH_2C\equiv CCH_2Cl$ | H | $OCH_3$ | Cl | CH | |
| $CH_3$ | $S(O)CH_2C\equiv CCH_2Cl$ | H | $CH_3$ | $CH_3$ | CH | |
| $CH_3$ | $S(O)CH_2C\equiv CCH_2Cl$ | H | $OCH_3$ | $OCH_3$ | N | |
| $CH_2CH_3$ | $S(O)CH_2C\equiv CCH_2Cl$ | H | $OCH_3$ | $CH_3$ | N | |
| $CH_3$ | $S(O)CH_2OCH_3$ | H | $OCH_3$ | $OCH_3$ | CH | |
| $CH_3$ | $S(O)CH_2OCH_3$ | H | $OCH_3$ | $CH_3$ | CH | |
| $CH_3$ | $S(O)CH_2OCH_3$ | H | $OCH_3$ | Cl | CH | |
| $CH_3$ | $S(O)CH_2OCH_3$ | H | $CH_3$ | $CH_3$ | CH | |
| $CH_3$ | $S(O)CH_2OCH_3$ | H | $OCH_3$ | $OCH_3$ | N | |
| $CH_3$ | $S(O)CH_2OCH_3$ | H | $OCH_3$ | $CH_3$ | N | |
| $CH_3$ | $S(O)CH_2OCH_2CF_3$ | H | $OCH_3$ | $OCH_3$ | CH | |
| $CH_3$ | $S(O)CH_2OCH_2CF_3$ | H | $OCH_3$ | $CH_3$ | CH | |
| $CH_3$ | $S(O)CH_2OCH_2CF_3$ | H | $OCH_3$ | Cl | CH | |
| $CH_3$ | $S(O)CH_2OCH_2CF_3$ | H | $CH_3$ | $CH_3$ | CH | |
| $CH_3$ | $S(O)CH_2OCH_2CF_3$ | H | $OCH_3$ | $OCH_3$ | N | |
| $CH_3$ | $S(O)CH_2OCH_2CF_3$ | H | $OCH_3$ | $CH_3$ | N | |
| $CH_3$ | $S(O)CH_2CN$ | H | $OCH_3$ | $OCH_3$ | CH | |
| $CH_3$ | $S(O)CH_2CN$ | H | $OCH_3$ | $CH_3$ | CH | |
| $CH_3$ | $S(O)CH_2CN$ | H | $OCH_3$ | Cl | CH | |
| $CH_3$ | $S(O)CH_2CN$ | H | $CH_3$ | $CH_3$ | CH | |
| $CH_3$ | $S(O)CH_2CN$ | H | $OCH_3$ | $OCH_3$ | N | |
| $CH_3$ | $S(O)CH_2CN$ | H | $OCH_3$ | $CH_3$ | N | |
| $CH_3$ | $S(O)CH_2C(O)CH_3$ | H | $OCH_3$ | $OCH_3$ | CH | |
| $CH_3$ | $S(O)CH_2C(O)CH_3$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $S(O)CH_2C(O)CH_3$ | H | $OCH_3$ | Cl | CH | |
| $CH_3$ | $S(O)CH_2C(O)CH_3$ | H | $CH_3$ | $CH_3$ | CH | |
| $CH_3$ | $S(O)CH_2C(O)CH_3$ | H | $OCH_3$ | $OCH_3$ | N | |
| $CH_3$ | $S(O)CH_2C(O)CH_3$ | H | $OCH_3$ | $CH_3$ | N | |
| $CH_3$ | $S(O)CH_2CH_2N(CH_3)_2$ | H | $OCH_3$ | $OCH_3$ | CH | |
| $CH_3$ | $S(O)CH_2CH_2N(CH_3)_2$ | H | $OCH_3$ | $CH_3$ | CH | |
| $CH_3$ | $S(O)CH_2CH_2N(CH_3)_2$ | H | $OCH_3$ | Cl | CH | |
| $CH_3$ | $S(O)CH_2CH_2N(CH_3)_2$ | H | $CH_3$ | $CH_3$ | CH | |
| $CH_3$ | $S(O)CH_2CH_2N(CH_3)_2$ | H | $OCH_3$ | $OCH_3$ | N | |
| $CH_2CH_3$ | $S(O)CH_2CH_2N(CH_3)_2$ | H | $OCH_3$ | $CH_3$ | N | |
| $CH_3$ | $S(O)CH_2CH_2OCH_2CF_3$ | H | $OCH_3$ | $OCH_3$ | CH | |
| $CH_3$ | $S(O)CH_2CH_2OCH_2CF_3$ | H | $OCH_3$ | $CH_3$ | CH | |
| $CH_3$ | $S(O)CH_2CH_2OCH_2CF_3$ | H | $OCH_3$ | Cl | CH | |
| $CH_3$ | $S(O)CH_2CH_2OCH_2CF_3$ | H | $CH_3$ | $CH_3$ | CH | |
| $CH_3$ | $S(O)CH_2CH_2OCH_2CF_3$ | H | $OCH_3$ | $OCH_3$ | N | |
| $CH_3$ | $S(O)CH_2CH_2OCH_2CF_3$ | H | $OCH_3$ | $CH_3$ | N | |
| $CH_3$ | $S(O)CF_2CF_2H$ | H | $OCH_3$ | $OCH_3$ | CH | |
| $CH_3$ | $S(O)CF_2CF_2H$ | H | $OCH_3$ | $CH_3$ | CH | |
| $CH_3$ | $S(O)CF_2CF_2H$ | H | $OCH_3$ | Cl | CH | |
| $CH_3$ | $S(O)CF_2CF_2H$ | H | $CH_3$ | $CH_3$ | CH | |
| $CH_3$ | $S(O)CF_2CF_2H$ | H | $OCH_3$ | $OCH_3$ | N | |
| $CH_2CH_3$ | $S(O)CF_2CF_2H$ | H | $OCH_3$ | $CH_3$ | N | |
| $CH_3$ | $S(O)CH_2CH_2CN$ | H | $OCH_3$ | $OCH_3$ | CH | |
| $CH_3$ | $S(O)CH_2CH_2CN$ | H | $OCH_3$ | $CH_3$ | CH | |
| $CH_3$ | $S(O)CH_2CH_2CN$ | H | $OCH_3$ | Cl | CH | |
| $CH_3$ | $S(O)CH_2CH_2CN$ | H | $CH_3$ | $CH_3$ | CH | |
| $CH_3$ | $S(O)CH_2CH_2CN$ | H | $OCH_3$ | $OCH_3$ | N | |
| $CH_2CH_3$ | $S(O)CH_2CH_2CN$ | H | $OCH_3$ | $CH_3$ | N | |
| $CH_3$ | $S(O)CH_2CH_2F$ | H | $OCH_3$ | $OCH_3$ | CH | |
| $CH_3$ | $S(O)CH_2CH_2F$ | H | $OCH_3$ | $CH_3$ | CH | |
| $CH_3$ | $S(O)CH_2CH_2F$ | H | $OCH_3$ | Cl | CH | |
| $CH_3$ | $S(O)CH_2CH_2F$ | H | $CH_3$ | $CH_3$ | CH | |
| $CH_3$ | $S(O)CH_2CH_2F$ | H | $OCH_3$ | $OCH_3$ | N | |

**Table I (Continued)**

| $R_1$ | $R_2$ | R | X | Y | Z | m.p.(°C) |
|---|---|---|---|---|---|---|
| $CH_3$ | $S(O)CH_2CH_2F$ | H | $OCH_3$ | $CH_3$ | N | |
| $CH_3$ | $S(O)CH_2CHF_2$ | H | $OCH_3$ | $OCH_3$ | CH | |
| $CH_3$ | $S(O)CH_2CHF_2$ | H | $OCH_3$ | $CH_3$ | CH | |
| $CH_3$ | $S(O)CH_2CHF_2$ | H | $OCH_3$ | Cl | CH | |
| $CH_3$ | $S(O)CH_2CHF_2$ | H | $CH_3$ | $CH_3$ | CH | |
| $CH_3$ | $S(O)CH_2CHF_2$ | H | $OCH_3$ | $OCH_3$ | N | |
| $CH_3$ | $S(O)CH_2CHF_2$ | H | $OCH_3$ | $CH_3$ | N | |
| $CH_3$ | $S(O)CH_2CF_3$ | H | $OCH_3$ | $OCH_3$ | CH | |
| $CH_3$ | $S(O)CH_2CF_3$ | H | $OCH_3$ | $CH_3$ | CH | |
| $CH_3$ | $S(O)CH_2CF_3$ | H | $OCH_3$ | Cl | CH | |
| $CH_3$ | $S(O)CH_2CF_3$ | H | $CH_3$ | $CH_3$ | CH | |
| $CH_3$ | $S(O)CH_2CF_3$ | H | $OCH_3$ | $OCH_3$ | N | |
| $CH_2CH_3$ | $S(O)CH_2CF_3$ | H | $OCH_3$ | $CH_3$ | N | |
| $CH_3$ | $S(O)_2$-cyclopropyl | H | $OCH_3$ | $OCH_3$ | CH | |
| $CH_3$ | $S(O)_2$-cyclopropyl | H | $OCH_3$ | $CH_3$ | CH | |
| $CH_3$ | $S(O)_2$-cyclopropyl | H | $OCH_3$ | Cl | CH | |
| $CH_3$ | $S(O)_2$-cyclopropyl | H | $CH_3$ | $CH_3$ | CH | |
| $CH_3$ | $S(O)_2$-cyclopropyl | H | $OCH_3$ | $OCH_3$ | N | |
| $CH_3$ | $S(O)_2$-cyclopropyl | H | $OCH_3$ | $CH_3$ | N | |
| $CH_3$ | $S(O)_2CH_2$-cyclopropyl | H | $OCH_3$ | $OCH_3$ | CH | |
| $CH_3$ | $S(O)_2CH_2$-cyclopropyl | H | $OCH_3$ | $CH_3$ | CH | |
| $CH_3$ | $S(O)_2CH_2$-cyclopropyl | H | $OCH_3$ | Cl | CH | |
| $CH_3$ | $S(O)_2CH_2$-cyclopropyl | H | $CH_3$ | $CH_3$ | CH | |
| $CH_3$ | $S(O)_2CH_2$-cyclopropyl | H | $OCH_3$ | $OCH_3$ | N | |
| $CH_3$ | $S(O)_2CH_2$-cyclopropyl | H | $OCH_3$ | $CH_3$ | N | |
| $CH_3$ | $S(O)_2$-cyclobutyl | H | $OCH_3$ | $OCH_3$ | CH | |
| $CH_3$ | $S(O)_2$-cyclobutyl | H | $OCH_3$ | $CH_3$ | CH | |
| $CH_3$ | $S(O)_2$-cyclobutyl | H | $OCH_3$ | Cl | CH | |
| $CH_3$ | $S(O)_2$-cyclobutyl | H | $CH_3$ | $CH_3$ | CH | |
| $CH_3$ | $S(O)_2$-cyclobutyl | H | $OCH_3$ | $OCH_3$ | N | |
| $CH_2CH_3$ | $S(O)_2$-cyclobutyl | H | $OCH_3$ | $CH_3$ | N | |
| $CH_3$ | $S(O)_2CH_2CF_2CF_3$ | H | $OCH_3$ | $OCH_3$ | CH | |
| $CH_3$ | $S(O)_2CH_2CF_2CF_3$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $S(O)_2CH_2CF_2CF_3$ | H | $OCH_3$ | Cl | CH | |
| $CH_3$ | $S(O)_2CH_2CF_2CF_3$ | H | $CH_3$ | $CH_3$ | CH | |
| $CH_3$ | $S(O)_2CH_2CF_2CF_3$ | H | $OCH_3$ | $OCH_3$ | N | |
| $CH_3$ | $S(O)_2CH_2CF_2CF_3$ | H | $OCH_3$ | $CH_3$ | N | |
| $CH_3$ | $S(O)_2CH(CF_3)CH_3$ | H | $OCH_3$ | $OCH_3$ | CH | |
| $CH_3$ | $S(O)_2CH(CF_3)CH_3$ | H | $OCH_3$ | $CH_3$ | CH | |
| $CH_3$ | $S(O)_2CH(CF_3)CH_3$ | H | $OCH_3$ | Cl | CH | |
| $CH_3$ | $S(O)_2CH(CF_3)CH_3$ | H | $CH_3$ | $CH_3$ | CH | |
| $CH_3$ | $S(O)_2CH(CF_3)CH_3$ | H | $OCH_3$ | $OCH_3$ | N | |
| $CH_3$ | $S(O)_2CH(CF_3)CH_3$ | H | $OCH_3$ | $CH_3$ | N | |
| $CH_3$ | $S(O)_2CH_2CF_2CF_2H$ | H | $OCH_3$ | $OCH_3$ | CH | |
| $CH_3$ | $S(O)_2CH_2CF_2CF_2H$ | H | $OCH_3$ | $CH_3$ | CH | |
| $CH_3$ | $S(O)_2CH_2CF_2CF_2H$ | H | $OCH_3$ | Cl | CH | |
| $CH_3$ | $S(O)_2CH_2CF_2CF_2H$ | H | $CH_3$ | $CH_3$ | CH | |
| $CH_3$ | $S(O)_2CH_2CF_2CF_2H$ | H | $OCH_3$ | $OCH_3$ | N | |
| $CH_2CH_3$ | $S(O)_2CH_2CF_2CF_2H$ | H | $OCH_3$ | $CH_3$ | N | |
| $CH_3$ | $S(O)_2CH(CH_2F)_2$ | H | $OCH_3$ | $OCH_3$ | CH | |
| $CH_3$ | $S(O)_2CH(CH_2F)_2$ | H | $OCH_3$ | $CH_3$ | CH | |
| $CH_3$ | $S(O)_2CH(CH_2F)_2$ | H | $OCH_3$ | Cl | CH | |
| $CH_3$ | $S(O)_2CH(CH_2F)_2$ | H | $CH_3$ | $CH_3$ | CH | |
| $CH_3$ | $S(O)_2CH(CH_2F)_2$ | H | $OCH_3$ | $OCH_3$ | N | |
| $CH_3$ | $S(O)_2CH(CH_2F)_2$ | H | $OCH_3$ | $CH_3$ | N | |
| $CH_3$ | $S(O)_2CH(CH_2F)CH_3$ | H | $OCH_3$ | $OCH_3$ | CH | |
| $CH_3$ | $S(O)_2CH(CH_2F)CH_3$ | H | $OCH_3$ | $CH_3$ | CH | |
| $CH_3$ | $S(O)_2CH(CH_2F)CH_3$ | H | $OCH_3$ | Cl | CH | |
| $CH_3$ | $S(O)_2CH(CH_2F)CH_3$ | H | $CH_3$ | $CH_3$ | CH | |
| $CH_3$ | $S(O)_2CH(CH_2F)CH_3$ | H | $OCH_3$ | $OCH_3$ | N | |
| $CH_3$ | $S(O)_2CH(CH_2F)CH_3$ | H | $OCH_3$ | $CH_3$ | N | |
| $CH_3$ | $S(O)_2(CH_2)_5CH_2Cl$ | H | $OCH_3$ | $OCH_3$ | CH | |
| $CH_3$ | $S(O)_2(CH_2)_5CH_2Cl$ | H | $OCH_3$ | $CH_3$ | CH | |
| $CH_3$ | $S(O)_2(CH_2)_5CH_2Cl$ | H | $OCH_3$ | Cl | CH | |
| $CH_3$ | $S(O)_2(CH_2)_5CH_2Cl$ | H | $CH_3$ | $CH_3$ | CH | |
| $CH_3$ | $S(O)_2(CH_2)_5CH_2Cl$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_2CH_3$ | $S(O)_2(CH_2)_5CH_2Cl$ | H | $OCH_3$ | $CH_3$ | N | |
| $CH_3$ | $S(O)_2C(Cl)=CHCl$ | H | $OCH_3$ | $OCH_3$ | CH | |
| $CH_3$ | $S(O)_2C(Cl)=CHCl$ | H | $OCH_3$ | $CH_3$ | CH | |
| $CH_3$ | $S(O)_2C(Cl)=CHCl$ | H | $OCH_3$ | Cl | CH | |
| $CH_3$ | $S(O)_2C(Cl)=CHCl$ | H | $CH_3$ | $CH_3$ | CH | |
| $CH_3$ | $S(O)_2C(Cl)=CHCl$ | H | $OCH_3$ | $OCH_3$ | N | |
| $CH_3$ | $S(O)_2C(Cl)=CHCl$ | H | $OCH_3$ | $CH_3$ | N | |
| $CH_3$ | $S(O)_2CH_2C(Cl)=CH_2$ | H | $OCH_3$ | $OCH_3$ | CH | |
| $CH_3$ | $S(O)_2CH_2C(Cl)=CH_2$ | H | $OCH_3$ | $CH_3$ | CH | |
| $CH_3$ | $S(O)_2CH_2C(Cl)=CH_2$ | H | $OCH_3$ | Cl | CH | |
| $CH_3$ | $S(O)_2CH_2C(Cl)=CH_2$ | H | $CH_3$ | $CH_3$ | CH | |
| $CH_3$ | $S(O)_2CH_2C(Cl)=CH_2$ | H | $OCH_3$ | $OCH_3$ | N | |
| $CH_3$ | $S(O)_2CH_2C(Cl)=CH_2$ | H | $OCH_3$ | $CH_3$ | N | |
| $CH_3$ | $S(O)_2CH_2C\equiv CCH_2Cl$ | H | $OCH_3$ | $OCH_3$ | CH | |
| $CH_3$ | $S(O)_2CH_2C\equiv CCH_2Cl$ | H | $OCH_3$ | $CH_3$ | CH | |
| $CH_3$ | $S(O)_2CH_2C\equiv CCH_2Cl$ | H | $OCH_3$ | Cl | CH | |
| $CH_3$ | $S(O)_2CH_2C\equiv CCH_2Cl$ | H | $CH_3$ | $CH_3$ | CH | |
| $CH_3$ | $S(O)_2CH_2C\equiv CCH_2Cl$ | H | $OCH_3$ | $OCH_3$ | N | |
| $CH_2CH_3$ | $S(O)_2CH_2C\equiv CCH_2Cl$ | H | $OCH_3$ | $CH_3$ | N | |
| $CH_3$ | $S(O)_2CH_2OCH_3$ | H | $OCH_3$ | $OCH_3$ | CH | |
| $CH_3$ | $S(O)_2CH_2OCH_3$ | H | $OCH_3$ | $CH_3$ | CH | |
| $CH_3$ | $S(O)_2CH_2OCH_3$ | H | $OCH_3$ | Cl | CH | |
| $CH_3$ | $S(O)_2CH_2OCH_3$ | H | $CH_3$ | $CH_3$ | CH | |
| $CH_3$ | $S(O)_2CH_2OCH_3$ | H | $OCH_3$ | $OCH_3$ | N | |
| $CH_3$ | $S(O)_2CH_2OCH_3$ | H | $OCH_3$ | $CH_3$ | N | |
| $CH_3$ | $S(O)_2CH_2OCH_2CF_3$ | H | $OCH_3$ | $OCH_3$ | CH | |
| $CH_3$ | $S(O)_2CH_2OCH_2CF_3$ | H | $OCH_3$ | $CH_3$ | CH | |
| $CH_3$ | $S(O)_2CH_2OCH_2CF_3$ | H | $OCH_3$ | Cl | CH | |
| $CH_3$ | $S(O)_2CH_2OCH_2CF_3$ | H | $CH_3$ | $CH_3$ | CH | |
| $CH_3$ | $S(O)_2CH_2OCH_2CF_3$ | H | $OCH_3$ | $OCH_3$ | N | |
| $CH_3$ | $S(O)_2CH_2OCH_2CF_3$ | H | $OCH_3$ | $CH_3$ | N | |
| $CH_3$ | $S(O)_2CH_2CN$ | H | $OCH_3$ | $OCH_3$ | CH | |
| $CH_3$ | $S(O)_2CH_2CN$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $S(O)_2CH_2CN$ | H | $OCH_3$ | Cl | CH | |
| $CH_3$ | $S(O)_2CH_2CN$ | H | $CH_3$ | $CH_3$ | CH | |
| $CH_3$ | $S(O)_2CH_2CN$ | H | $OCH_3$ | $OCH_3$ | N | |
| $CH_3$ | $S(O)_2CH_2CN$ | H | $OCH_3$ | $CH_3$ | N | |
| $CH_3$ | $S(O)_2CH_2C(O)CH_3$ | H | $OCH_3$ | $OCH_3$ | CH | |
| $CH_3$ | $S(O)_2CH_2C(O)CH_3$ | H | $OCH_3$ | $CH_3$ | CH | |
| $CH_3$ | $S(O)_2CH_2C(O)CH_3$ | H | $OCH_3$ | Cl | CH | |
| $CH_3$ | $S(O)_2CH_2C(O)CH_3$ | H | $CH_3$ | $CH_3$ | CH | |
| $CH_3$ | $S(O)_2CH_2C(O)CH_3$ | H | $OCH_3$ | $OCH_3$ | N | |
| $CH_3$ | $S(O)_2CH_2C(O)CH_3$ | H | $OCH_3$ | $CH_3$ | N | |
| $CH_3$ | $S(O)_2CH_2CH_2N(CH_3)_2$ | H | $OCH_3$ | $OCH_3$ | CH | |
| $CH_3$ | $S(O)_2CH_2CH_2N(CH_3)_2$ | H | $OCH_3$ | $CH_3$ | CH | |
| $CH_3$ | $S(O)_2CH_2CH_2N(CH_3)_2$ | H | $OCH_3$ | Cl | CH | |
| $CH_3$ | $S(O)_2CH_2CH_2N(CH_3)_2$ | H | $CH_3$ | $CH_3$ | CH | |
| $CH_3$ | $S(O)_2CH_2CH_2N(CH_3)_2$ | H | $OCH_3$ | $OCH_3$ | N | |
| $CH_2CH_3$ | $S(O)_2CH_2CH_2N(CH_3)_2$ | H | $OCH_3$ | $CH_3$ | N | |
| $CH_3$ | $S(O)_2CH_2CH_2OCH_2CF_3$ | H | $OCH_3$ | $OCH_3$ | CH | |
| $CH_3$ | $S(O)_2CH_2CH_2OCH_2CF_3$ | H | $OCH_3$ | $CH_3$ | CH | |
| $CH_3$ | $S(O)_2CH_2CH_2OCH_2CF_3$ | H | $OCH_3$ | Cl | CH | |
| $CH_3$ | $S(O)_2CH_2CH_2OCH_2CF_3$ | H | $CH_3$ | $CH_3$ | CH | |
| $CH_3$ | $S(O)_2CH_2CH_2OCH_2CF_3$ | H | $OCH_3$ | $OCH_3$ | N | |
| $CH_3$ | $S(O)_2CH_2CH_2OCH_2CF_3$ | H | $OCH_3$ | $CH_3$ | N | |
| $CH_3$ | $S(O)_2CF_2CF_2H$ | H | $OCH_3$ | $OCH_3$ | CH | |
| $CH_3$ | $S(O)_2CF_2CF_2H$ | H | $OCH_3$ | $CH_3$ | CH | |
| $CH_3$ | $S(O)_2CF_2CF_2H$ | H | $OCH_3$ | Cl | CH | |
| $CH_3$ | $S(O)_2CF_2CF_2H$ | H | $CH_3$ | $CH_3$ | CH | |
| $CH_3$ | $S(O)_2CF_2CF_2H$ | H | $OCH_3$ | $OCH_3$ | N | |
| $CH_2CH_3$ | $S(O)_2CF_2CF_2H$ | H | $OCH_3$ | $CH_3$ | N | |
| $CH_3$ | $S(O)_2CH_2CH_2CN$ | H | $OCH_3$ | $OCH_3$ | CH | |
| $CH_3$ | $S(O)_2CH_2CH_2CN$ | H | $OCH_3$ | $CH_3$ | CH | |
| $CH_3$ | $S(O)_2CH_2CH_2CN$ | H | $OCH_3$ | Cl | CH | |
| $CH_3$ | $S(O)_2CH_2CH_2CN$ | H | $CH_3$ | $CH_3$ | CH | |
| $CH_3$ | $S(O)_2CH_2CH_2CN$ | H | $OCH_3$ | $OCH_3$ | N | |

**Table I (Continued)**

| $R_1$ | $R_2$ | R | X | Y | Z | m.p.(°C) |
|---|---|---|---|---|---|---|
| $CH_2CH_3$ | $S(O)_2CH_2CH_2CN$ | H | $OCH_3$ | $CH_3$ | N | |
| $CH_3$ | $S(O)_2CH_2CH_2F$ | H | $OCH_3$ | $OCH_3$ | CH | |
| $CH_3$ | $S(O)_2CH_2CH_2F$ | H | $OCH_3$ | $CH_3$ | CH | |
| $CH_3$ | $S(O)_2CH_2CH_2F$ | H | $OCH_3$ | Cl | CH | |
| $CH_3$ | $S(O)_2CH_2CH_2F$ | H | $CH_3$ | $CH_3$ | CH | |
| $CH_3$ | $S(O)_2CH_2CH_2F$ | H | $OCH_3$ | $OCH_3$ | N | |
| $CH_3$ | $S(O)_2CH_2CH_2F$ | H | $OCH_3$ | $CH_3$ | N | |
| $CH_3$ | $S(O)_2CH_2CHF_2$ | H | $OCH_3$ | $OCH_3$ | CH | |
| $CH_3$ | $S(O)_2CH_2CHF_2$ | H | $OCH_3$ | $CH_3$ | CH | |
| $CH_3$ | $S(O)_2CH_2CHF_2$ | H | $OCH_3$ | Cl | CH | |
| $CH_3$ | $S(O)_2CH_2CHF_2$ | H | $CH_3$ | $CH_3$ | CH | |
| $CH_3$ | $S(O)_2CH_2CHF_2$ | H | $OCH_3$ | $OCH_3$ | N | |
| $CH_3$ | $S(O)_2CH_2CHF_2$ | H | $OCH_3$ | $CH_3$ | N | |
| $CH_3$ | $S(O)_2CH_2CF_3$ | H | $OCH_3$ | $OCH_3$ | CH | |
| $CH_3$ | $S(O)_2CH_2CF_3$ | H | $OCH_3$ | $CH_3$ | CH | |
| $CH_3$ | $S(O)_2CH_2CF_3$ | H | $OCH_3$ | Cl | CH | |
| $CH_3$ | $S(O)_2CH_2CF_3$ | H | $CH_3$ | $CH_3$ | CH | |
| $CH_3$ | $S(O)_2CH_2CF_3$ | H | $OCH_3$ | $OCH_3$ | N | |
| $CH_2CH_3$ | $S(O)_2CH_2CF_3$ | H | $OCH_3$ | $CH_3$ | N | |
| $CH_3$ | $NHCH_2$-cyclopropyl | H | $OCH_3$ | $OCH_3$ | CH | |
| $CH_3$ | $NHCH_2$-cyclopropyl | H | $OCH_3$ | $CH_3$ | CH | |
| $CH_3$ | $NHCH_2$-cyclopropyl | H | $OCH_3$ | Cl | CH | |
| $CH_3$ | $NHCH_2$-cyclopropyl | H | $CH_3$ | $CH_3$ | CH | |
| $CH_3$ | $NHCH_2$-cyclopropyl | H | $OCH_3$ | $OCH_3$ | N | |
| $CH_3$ | $NHCH_2$-cyclopropyl | H | $OCH_3$ | $CH_3$ | N | |
| $CH_3$ | $NHCH_2CF_2CF_3$ | H | $OCH_3$ | $OCH_3$ | CH | |
| $CH_3$ | $NHCH_2CF_2CF_3$ | H | $OCH_3$ | $CH_3$ | CH | |
| $CH_3$ | $NHCH_2CF_2CF_3$ | H | $OCH_3$ | Cl | CH | |
| $CH_3$ | $NHCH_2CF_2CF_3$ | H | $CH_3$ | $CH_3$ | CH | |
| $CH_3$ | $NHCH_2CF_2CF_3$ | H | $OCH_3$ | $OCH_3$ | N | |
| $CH_3$ | $NHCH_2CF_2CF_3$ | H | $OCH_3$ | $CH_3$ | N | |
| $CH_3$ | $NHCH(CF_3)CH_3$ | H | $OCH_3$ | $OCH_3$ | CH | |
| $CH_3$ | $NHCH(CF_3)CH_3$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $NHCH(CF_3)CH_3$ | H | $OCH_3$ | Cl | CH | |
| $CH_3$ | $NHCH(CF_3)CH_3$ | H | $CH_3$ | $CH_3$ | CH | |
| $CH_3$ | $NHCH(CF_3)CH_3$ | H | $OCH_3$ | $OCH_3$ | N | |
| $CH_3$ | $NHCH(CF_3)CH_3$ | H | $OCH_3$ | $CH_3$ | N | |
| $CH_3$ | $NHCH_2CF_2CF_2H$ | H | $OCH_3$ | $OCH_3$ | CH | |
| $CH_3$ | $NHCH_2CF_2CF_2H$ | H | $OCH_3$ | $CH_3$ | CH | |
| $CH_3$ | $NHCH_2CF_2CF_2H$ | H | $OCH_3$ | Cl | CH | |
| $CH_3$ | $NHCH_2CF_2CF_2H$ | H | $CH_3$ | $CH_3$ | CH | |
| $CH_3$ | $NHCH_2CF_2CF_2H$ | H | $OCH_3$ | $OCH_3$ | N | |
| $CH_2CH_3$ | $NHCH_2CF_2CF_2H$ | H | $OCH_3$ | $CH_3$ | N | |
| $CH_3$ | $NHCH(CH_2F)_2$ | H | $OCH_3$ | $OCH_3$ | CH | |
| $CH_3$ | $NHCH(CH_2F)_2$ | H | $OCH_3$ | $CH_3$ | CH | |
| $CH_3$ | $NHCH(CH_2F)_2$ | H | $OCH_3$ | Cl | CH | |
| $CH_3$ | $NHCH(CH_2F)_2$ | H | $CH_3$ | $CH_3$ | CH | |
| $CH_3$ | $NHCH(CH_2F)_2$ | H | $OCH_3$ | $OCH_3$ | N | |
| $CH_3$ | $NHCH(CH_2F)_2$ | H | $OCH_3$ | $CH_3$ | N | |
| $CH_3$ | $NHCH(CH_2F)CH_3$ | H | $OCH_3$ | $OCH_3$ | CH | |
| $CH_3$ | $NHCH(CH_2F)CH_3$ | H | $OCH_3$ | $CH_3$ | CH | |
| $CH_3$ | $NHCH(CH_2F)CH_3$ | H | $OCH_3$ | Cl | CH | |
| $CH_3$ | $NHCH(CH_2F)CH_3$ | H | $CH_3$ | $CH_3$ | CH | |
| $CH_3$ | $NHCH(CH_2F)CH_3$ | H | $OCH_3$ | $OCH_3$ | N | |
| $CH_3$ | $NHCH(CH_2F)CH_3$ | H | $OCH_3$ | $CH_3$ | N | |
| $CH_3$ | $NH(CH_2)_3CH_2Cl$ | H | $OCH_3$ | $OCH_3$ | CH | |
| $CH_3$ | $NH(CH_2)_3CH_2Cl$ | H | $OCH_3$ | $CH_3$ | CH | |
| $CH_3$ | $NH(CH_2)_3CH_2Cl$ | H | $OCH_3$ | Cl | CH | |
| $CH_3$ | $NH(CH_2)_3CH_2Cl$ | H | $CH_3$ | $CH_3$ | CH | |
| $CH_3$ | $NH(CH_2)_3CH_2Cl$ | H | $OCH_3$ | $OCH_3$ | N | |
| $CH_2CH_3$ | $NH(CH_2)_3CH_2Cl$ | H | $OCH_3$ | $CH_3$ | N | |
| $CH_3$ | NHC(Cl)=CHCl | H | $OCH_3$ | $OCH_3$ | CH | |
| $CH_3$ | NHC(Cl)=CHCl | H | $OCH_3$ | $CH_3$ | CH | |
| $CH_3$ | NHC(Cl)=CHCl | H | $OCH_3$ | Cl | CH | |
| $CH_3$ | NHC(Cl)=CHCl | H | $CH_3$ | $CH_3$ | CH | |
| $CH_3$ | NHC(Cl)=CHCl | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | NHC(Cl)=CHCl | H | $OCH_3$ | $CH_3$ | N | |
| $CH_3$ | $NHCH_2C(Cl)=CH_2$ | H | $OCH_3$ | $OCH_3$ | CH | |
| $CH_3$ | $NHCH_2C(Cl)=CH_2$ | H | $OCH_3$ | $CH_3$ | CH | |
| $CH_3$ | $NHCH_2C(Cl)=CH_2$ | H | $OCH_3$ | Cl | CH | |
| $CH_3$ | $NHCH_2C(Cl)=CH_2$ | H | $CH_3$ | $CH_3$ | CH | |
| $CH_3$ | $NHCH_2C(Cl)=CH_2$ | H | $OCH_3$ | $OCH_3$ | N | |
| $CH_3$ | $NHCH_2C(Cl)=CH_2$ | H | $OCH_3$ | $CH_3$ | N | |
| $CH_3$ | $NHCH_2C\equiv CCH_2Cl$ | H | $OCH_3$ | $OCH_3$ | CH | |
| $CH_3$ | $NHCH_2C\equiv CCH_2Cl$ | H | $OCH_3$ | $CH_3$ | CH | |
| $CH_3$ | $NHCH_2C\equiv CCH_2Cl$ | H | $OCH_3$ | Cl | CH | |
| $CH_3$ | $NHCH_2C\equiv CCH_2Cl$ | H | $CH_3$ | $CH_3$ | CH | |
| $CH_3$ | $NHCH_2C\equiv CCH_2Cl$ | H | $OCH_3$ | $OCH_3$ | N | |
| $CH_2CH_3$ | $NHCH_2C\equiv CCH_2Cl$ | H | $OCH_3$ | $CH_3$ | N | |
| $CH_3$ | $NHCH_2OCH_3$ | H | $OCH_3$ | $OCH_3$ | CH | |
| $CH_3$ | $NHCH_2OCH_3$ | H | $OCH_3$ | $CH_3$ | CH | |
| $CH_3$ | $NHCH_2OCH_3$ | H | $OCH_3$ | Cl | CH | |
| $CH_3$ | $NHCH_2OCH_3$ | H | $CH_3$ | $CH_3$ | CH | |
| $CH_3$ | $NHCH_2OCH_3$ | H | $OCH_3$ | $OCH_3$ | N | |
| $CH_3$ | $NHCH_2OCH_3$ | H | $OCH_3$ | $CH_3$ | N | |
| $CH_3$ | $NHCH_2SCH_3$ | H | $OCH_3$ | $OCH_3$ | CH | |
| $CH_3$ | $NHCH_2SCH_3$ | H | $OCH_3$ | $CH_3$ | CH | |
| $CH_3$ | $NHCH_2SCH_3$ | H | $OCH_3$ | Cl | CH | |
| $CH_3$ | $NHCH_2SCH_3$ | H | $CH_3$ | $CH_3$ | CH | |
| $CH_3$ | $NHCH_2SCH_3$ | H | $OCH_3$ | $OCH_3$ | N | |
| $CH_2CH_3$ | $NHCH_2SCH_3$ | H | $OCH_3$ | $CH_3$ | N | |
| $CH_3$ | $NHCH_2CN$ | H | $OCH_3$ | $OCH_3$ | CH | |
| $CH_3$ | $NHCH_2CN$ | H | $OCH_3$ | $CH_3$ | CH | |
| $CH_3$ | $NHCH_2CN$ | H | $OCH_3$ | Cl | CH | |
| $CH_3$ | $NHCH_2CN$ | H | $CH_3$ | $CH_3$ | CH | |
| $CH_3$ | $NHCH_2CN$ | H | $OCH_3$ | $OCH_3$ | N | |
| $CH_3$ | $NHCH_2CN$ | H | $OCH_3$ | $CH_3$ | N | |
| $CH_3$ | $NHCH_2C(O)CH_3$ | H | $OCH_3$ | $OCH_3$ | CH | |
| $CH_3$ | $NHCH_2C(O)CH_3$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $NHCH_2C(O)CH_3$ | H | $OCH_3$ | Cl | CH | |
| $CH_3$ | $NHCH_2C(O)CH_3$ | H | $CH_3$ | $CH_3$ | CH | |
| $CH_3$ | $NHCH_2C(O)CH_3$ | H | $OCH_3$ | $OCH_3$ | N | |
| $CH_3$ | $NHCH_2C(O)CH_3$ | H | $OCH_3$ | $CH_3$ | N | |
| $CH_3$ | $NHCH_2CH_2N(CH_3)_2$ | H | $OCH_3$ | $OCH_3$ | CH | |
| $CH_3$ | $NHCH_2CH_2N(CH_3)_2$ | H | $OCH_3$ | $CH_3$ | CH | |
| $CH_3$ | $NHCH_2CH_2N(CH_3)_2$ | H | $OCH_3$ | Cl | CH | |
| $CH_3$ | $NHCH_2CH_2N(CH_3)_2$ | H | $CH_3$ | $CH_3$ | CH | |
| $CH_3$ | $NHCH_2CH_2N(CH_3)_2$ | H | $OCH_3$ | $OCH_3$ | N | |
| $CH_2CH_3$ | $NHCH_2CH_2N(CH_3)_2$ | H | $OCH_3$ | $CH_3$ | N | |
| $CH_3$ | $NHCH_2CH_2OCH_3$ | H | $OCH_3$ | $OCH_3$ | CH | |
| $CH_3$ | $NHCH_2CH_2OCH_3$ | H | $OCH_3$ | $CH_3$ | CH | |
| $CH_3$ | $NHCH_2CH_2OCH_3$ | H | $OCH_3$ | Cl | CH | |
| $CH_3$ | $NHCH_2CH_2OCH_3$ | H | $CH_3$ | $CH_3$ | CH | |
| $CH_3$ | $NHCH_2CH_2OCH_3$ | H | $OCH_3$ | $OCH_3$ | N | |
| $CH_3$ | $NHCH_2CH_2OCH_3$ | H | $OCH_3$ | $CH_3$ | N | |
| $CH_3$ | $NHCH_2CH_2CN$ | H | $OCH_3$ | $OCH_3$ | CH | |
| $CH_3$ | $NHCH_2CH_2CN$ | H | $OCH_3$ | $CH_3$ | CH | |
| $CH_3$ | $NHCH_2CH_2CN$ | H | $OCH_3$ | Cl | CH | |
| $CH_3$ | $NHCH_2CH_2CN$ | H | $CH_3$ | $CH_3$ | CH | |
| $CH_3$ | $NHCH_2CH_2CN$ | H | $OCH_3$ | $OCH_3$ | N | |
| $CH_2CH_3$ | $NHCH_2CH_2CN$ | H | $OCH_3$ | $CH_3$ | N | |
| $CH_3$ | $NHCH_2CH_2F$ | H | $OCH_3$ | $OCH_3$ | CH | |
| $CH_3$ | $NHCH_2CH_2F$ | H | $OCH_3$ | $CH_3$ | CH | |
| $CH_3$ | $NHCH_2CH_2F$ | H | $OCH_3$ | Cl | CH | |
| $CH_3$ | $NHCH_2CH_2F$ | H | $CH_3$ | $CH_3$ | CH | |
| $CH_3$ | $NHCH_2CH_2F$ | H | $OCH_3$ | $OCH_3$ | N | |
| $CH_3$ | $NHCH_2CH_2F$ | H | $OCH_3$ | $CH_3$ | N | |
| $CH_3$ | $NHCH_2CHF_2$ | H | $OCH_3$ | $OCH_3$ | CH | |
| $CH_3$ | $NHCH_2CHF_2$ | H | $OCH_3$ | $CH_3$ | CH | |
| $CH_3$ | $NHCH_2CHF_2$ | H | $OCH_3$ | Cl | CH | |
| $CH_3$ | $NHCH_2CHF_2$ | H | $CH_3$ | $CH_3$ | CH | |
| $CH_3$ | $NHCH_2CHF_2$ | H | $OCH_3$ | $OCH_3$ | N | |

## Table I (Continued)

| $R_1$ | $R_2$ | R | X | Y | Z | m.p.(°C) |
|---|---|---|---|---|---|---|
| $CH_3$ | $NHCH_2CHF_2$ | H | $OCH_3$ | $CH_3$ | N | |
| $CH_3$ | $NHCH_2CF_3$ | H | $OCH_3$ | $OCH_3$ | CH | |
| $CH_3$ | $NHCH_2CF_3$ | H | $OCH_3$ | $CH_3$ | CH | |
| $CH_3$ | $NHCH_2CF_3$ | H | $OCH_3$ | Cl | CH | |
| $CH_3$ | $NHCH_2CF_3$ | H | $CH_3$ | $CH_3$ | CH | |
| $CH_3$ | $NHCH_2CF_3$ | H | $OCH_3$ | $OCH_3$ | N | |
| $CH_2CH_3$ | $NHCH_2CF_3$ | H | $OCH_3$ | $CH_3$ | N | |
| $CH_3$ | $NHCH_2CH_2OH$ | H | $OCH_3$ | $OCH_3$ | CH | |
| $CH_3$ | $NHCH_2CH_2OH$ | H | $OCH_3$ | $CH_3$ | CH | |
| $CH_3$ | $NHCH_2CH_2OH$ | H | $OCH_3$ | Cl | CH | |
| $CH_3$ | $NHCH_2CH_2OH$ | H | $CH_3$ | $CH_3$ | CH | |
| $CH_3$ | $NHCH_2CH_2OH$ | H | $OCH_3$ | $OCH_3$ | N | |
| $CH_3$ | $NHCH_2CH_2OH$ | H | $OCH_3$ | $CH_3$ | N | |
| $CH_3$ | $NHCH_2CH_2NH_2$ | H | $OCH_3$ | $OCH_3$ | CH | |
| $CH_3$ | $NHCH_2CH_2NH_2$ | H | $OCH_3$ | $CH_3$ | CH | |
| $CH_3$ | $NHCH_2CH_2NH_2$ | H | $OCH_3$ | Cl | CH | |
| $CH_3$ | $NHCH_2CH_2NH_2$ | H | $CH_3$ | $CH_3$ | CH | |
| $CH_3$ | $NHCH_2CH_2NH_2$ | H | $OCH_3$ | $OCH_3$ | N | |
| $CH_3$ | $NHCH_2CH_2NH_2$ | H | $OCH_3$ | $CH_3$ | N | |
| $CH_3$ | $NHCH_3$ | H | $OCH_3$ | $OCH_3$ | CH | |
| $CH_3$ | $NHCH_3$ | H | $OCH_3$ | $CH_3$ | CH | |
| $CH_3$ | $NHCH_3$ | H | $OCH_3$ | Cl | CH | |
| $CH_3$ | $NHCH_3$ | H | $CH_3$ | $CH_3$ | CH | |
| $CH_3$ | $NHCH_3$ | H | $OCH_3$ | $OCH_3$ | N | |
| $CH_2CH_3$ | $NHCH_3$ | H | $OCH_3$ | $CH_3$ | N | |
| $CH_3$ | $NHCH_2CH_2SCH_3$ | H | $OCH_3$ | $OCH_3$ | CH | |
| $CH_3$ | $NHCH_2CH_2SCH_3$ | H | $OCH_3$ | $CH_3$ | CH | |
| $CH_3$ | $NHCH_2CH_2SCH_3$ | H | $OCH_3$ | Cl | CH | |
| $CH_3$ | $NHCH_2CH_2SCH_3$ | H | $CH_3$ | $CH_3$ | CH | |
| $CH_3$ | $NHCH_2CH_2SCH_3$ | H | $OCH_3$ | $OCH_3$ | N | |
| $CH_3$ | $NHCH_2CH_2SCH_3$ | H | $OCH_3$ | $CH_3$ | N | |
| $CH_3$ | $NHCH_2CH_2S(O)CH_3$ | H | $OCH_3$ | $OCH_3$ | CH | |
| $CH_3$ | $NHCH_2CH_2S(O)CH_3$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $NHCH_2CH_2S(O)CH_3$ | H | $OCH_3$ | Cl | CH | |
| $CH_3$ | $NHCH_2CH_2S(O)CH_3$ | H | $CH_3$ | $CH_3$ | CH | |
| $CH_3$ | $NHCH_2CH_2S(O)CH_3$ | H | $OCH_3$ | $OCH_3$ | N | |
| $CH_3$ | $NHCH_2CH_2S(O)CH_3$ | H | $OCH_3$ | $CH_3$ | N | |
| $CH_3$ | $NHCH_2CH_2S(O)_2CH_3$ | H | $OCH_3$ | $OCH_3$ | CH | |
| $CH_3$ | $NHCH_2CH_2S(O)_2CH_3$ | H | $OCH_3$ | $CH_3$ | CH | |
| $CH_3$ | $NHCH_2CH_2S(O)_2CH_3$ | H | $OCH_3$ | Cl | CH | |
| $CH_3$ | $NHCH_2CH_2S(O)_2CH_3$ | H | $CH_3$ | $CH_3$ | CH | |
| $CH_3$ | $NHCH_2CH_2S(O)_2CH_3$ | H | $OCH_3$ | $OCH_3$ | N | |
| $CH_2CH_3$ | $NHCH_2CH_2S(O)_2CH_3$ | H | $OCH_3$ | $CH_3$ | N | |
| $CH_3$ | $CH_2O$-cyclopropyl | H | $OCH_3$ | $OCH_3$ | CH | |
| $CH_3$ | $CH_2O$-cyclopropyl | H | $OCH_3$ | $CH_3$ | CH | |
| $CH_3$ | $CH_2O$-cyclopropyl | H | $OCH_3$ | Cl | CH | |
| $CH_3$ | $CH_2O$-cyclopropyl | H | $CH_3$ | $CH_3$ | CH | |
| $CH_3$ | $CH_2O$-cyclopropyl | H | $OCH_3$ | $OCH_3$ | N | |
| $CH_3$ | $CH_2O$-cyclopropyl | H | $OCH_3$ | $CH_3$ | N | |
| $CH_3$ | $CH_2OCH_2$-cyclopropyl | H | $OCH_3$ | $OCH_3$ | CH | |
| $CH_3$ | $CH_2OCH_2$-cyclopropyl | H | $OCH_3$ | $CH_3$ | CH | |
| $CH_3$ | $CH_2OCH_2$-cyclopropyl | H | $OCH_3$ | Cl | CH | |
| $CH_3$ | $CH_2OCH_2$-cyclopropyl | H | $CH_3$ | $CH_3$ | CH | |
| $CH_3$ | $CH_2OCH_2$-cyclopropyl | H | $OCH_3$ | $OCH_3$ | N | |
| $CH_3$ | $CH_2OCH_2$-cyclopropyl | H | $OCH_3$ | $CH_3$ | N | |
| $CH_3$ | $CH_2O$-cyclobutyl | H | $OCH_3$ | $OCH_3$ | CH | |
| $CH_3$ | $CH_2O$-cyclobutyl | H | $OCH_3$ | $CH_3$ | CH | |
| $CH_3$ | $CH_2O$-cyclobutyl | H | $OCH_3$ | Cl | CH | |
| $CH_3$ | $CH_2O$-cyclobutyl | H | $CH_3$ | $CH_3$ | CH | |
| $CH_3$ | $CH_2O$-cyclobutyl | H | $OCH_3$ | $OCH_3$ | N | |
| $CH_2CH_3$ | $CH_2O$-cyclobutyl | H | $OCH_3$ | $CH_3$ | N | |
| $CH_3$ | $CH_2OCH_2CF_2CF_3$ | H | $OCH_3$ | $OCH_3$ | CH | |
| $CH_3$ | $CH_2OCH_2CF_2CF_3$ | H | $OCH_3$ | $CH_3$ | CH | |
| $CH_3$ | $CH_2OCH_2CF_2CF_3$ | H | $OCH_3$ | Cl | CH | |
| $CH_3$ | $CH_2OCH_2CF_2CF_3$ | H | $CH_3$ | $CH_3$ | CH | |
| $CH_3$ | $CH_2OCH_2CF_2CF_3$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $CH_2OCH_2CF_2CF_3$ | H | $OCH_3$ | $CH_3$ | N | |
| $CH_3$ | $CH_2OCH(CF_3)CH_3$ | H | $OCH_3$ | $OCH_3$ | CH | |
| $CH_3$ | $CH_2OCH(CF_3)CH_3$ | H | $OCH_3$ | $CH_3$ | CH | |
| $CH_3$ | $CH_2OCH(CF_3)CH_3$ | H | $OCH_3$ | Cl | CH | |
| $CH_3$ | $CH_2OCH(CF_3)CH_3$ | H | $CH_3$ | $CH_3$ | CH | |
| $CH_3$ | $CH_2OCH(CF_3)CH_3$ | H | $OCH_3$ | $OCH_3$ | N | |
| $CH_3$ | $CH_2OCH(CF_3)CH_3$ | H | $OCH_3$ | $CH_3$ | N | |
| $CH_3$ | $CH_2OCH_2CF_2CF_2H$ | H | $OCH_3$ | $OCH_3$ | CH | |
| $CH_3$ | $CH_2OCH_2CF_2CF_2H$ | H | $OCH_3$ | $CH_3$ | CH | |
| $CH_3$ | $CH_2OCH_2CF_2CF_2H$ | H | $OCH_3$ | Cl | CH | |
| $CH_3$ | $CH_2OCH_2CF_2CF_2H$ | H | $CH_3$ | $CH_3$ | CH | |
| $CH_3$ | $CH_2OCH_2CF_2CF_2H$ | H | $OCH_3$ | $OCH_3$ | N | |
| $CH_2CH_3$ | $CH_2OCH_2CF_2CF_2H$ | H | $OCH_3$ | $CH_3$ | N | |
| $CH_3$ | $CH_2OCH(CH_2F)_2$ | H | $OCH_3$ | $OCH_3$ | CH | |
| $CH_3$ | $CH_2OCH(CH_2F)_2$ | H | $OCH_3$ | $CH_3$ | CH | |
| $CH_3$ | $CH_2OCH(CH_2F)_2$ | H | $OCH_3$ | Cl | CH | |
| $CH_3$ | $CH_2OCH(CH_2F)_2$ | H | $CH_3$ | $CH_3$ | CH | |
| $CH_3$ | $CH_2OCH(CH_2F)_2$ | H | $OCH_3$ | $OCH_3$ | N | |
| $CH_3$ | $CH_2OCH(CH_2F)_2$ | H | $OCH_3$ | $CH_3$ | N | |
| $CH_3$ | $CH_2OCH(CH_2F)CH_3$ | H | $OCH_3$ | $OCH_3$ | CH | |
| $CH_3$ | $CH_2OCH(CH_2F)CH_3$ | H | $OCH_3$ | $CH_3$ | CH | |
| $CH_3$ | $CH_2OCH(CH_2F)CH_3$ | H | $OCH_3$ | Cl | CH | |
| $CH_3$ | $CH_2OCH(CH_2F)CH_3$ | H | $CH_3$ | $CH_3$ | CH | |
| $CH_3$ | $CH_2OCH(CH_2F)CH_3$ | H | $OCH_3$ | $OCH_3$ | N | |
| $CH_3$ | $CH_2OCH(CH_2F)CH_3$ | H | $OCH_3$ | $CH_3$ | N | |
| $CH_3$ | $CH_2O(CH_2)_3CH_2Cl$ | H | $OCH_3$ | $OCH_3$ | CH | |
| $CH_3$ | $CH_2O(CH_2)_3CH_2Cl$ | H | $OCH_3$ | $CH_3$ | CH | |
| $CH_3$ | $CH_2O(CH_2)_3CH_2Cl$ | H | $OCH_3$ | Cl | CH | |
| $CH_3$ | $CH_2O(CH_2)_3CH_2Cl$ | H | $CH_3$ | $CH_3$ | CH | |
| $CH_3$ | $CH_2O(CH_2)_3CH_2Cl$ | H | $OCH_3$ | $OCH_3$ | N | |
| $CH_2CH_3$ | $CH_2O(CH_2)_3CH_2Cl$ | H | $OCH_3$ | $CH_3$ | N | |
| $CH_3$ | $CH_2OCH_2C(Cl)=CH_2$ | H | $OCH_3$ | $OCH_3$ | CH | |
| $CH_3$ | $CH_2OCH_2C(Cl)=CH_2$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $CH_2OCH_2C(Cl)=CH_2$ | H | $OCH_3$ | Cl | CH | |
| $CH_3$ | $CH_2OCH_2C(Cl)=CH_2$ | H | $CH_3$ | $CH_3$ | CH | |
| $CH_3$ | $CH_2OCH_2C(Cl)=CH_2$ | H | $OCH_3$ | $OCH_3$ | N | |
| $CH_3$ | $CH_2OCH_2C(Cl)=CH_2$ | H | $OCH_3$ | $CH_3$ | N | |
| $CH_3$ | $CH_2OCH_2C{\equiv}CCH_2Cl$ | H | $OCH_3$ | $OCH_3$ | CH | |
| $CH_3$ | $CH_2OCH_2C{\equiv}CCH_2Cl$ | H | $OCH_3$ | $CH_3$ | CH | |
| $CH_3$ | $CH_2OCH_2C{\equiv}CCH_2Cl$ | H | $OCH_3$ | Cl | CH | |
| $CH_3$ | $CH_2OCH_2C{\equiv}CCH_2Cl$ | H | $CH_3$ | $CH_3$ | CH | |
| $CH_3$ | $CH_2OCH_2C{\equiv}CCH_2Cl$ | H | $OCH_3$ | $OCH_3$ | N | |
| $CH_2CH_3$ | $CH_2OCH_2C{\equiv}CCH_2Cl$ | H | $OCH_3$ | $CH_3$ | N | |
| $CH_3$ | $CH_2OCH_2OCH_3$ | H | $OCH_3$ | $OCH_3$ | CH | |
| $CH_3$ | $CH_2OCH_2OCH_3$ | H | $OCH_3$ | $CH_3$ | CH | |
| $CH_3$ | $CH_2OCH_2OCH_3$ | H | $OCH_3$ | Cl | CH | |
| $CH_3$ | $CH_2OCH_2OCH_3$ | H | $CH_3$ | $CH_3$ | CH | |
| $CH_3$ | $CH_2OCH_2OCH_3$ | H | $OCH_3$ | $OCH_3$ | N | |
| $CH_3$ | $CH_2OCH_2OCH_3$ | H | $OCH_3$ | $CH_3$ | N | |
| $CH_3$ | $CH_2OCH_2OCH_2CF_3$ | H | $OCH_3$ | $OCH_3$ | CH | |
| $CH_3$ | $CH_2OCH_2OCH_2CF_3$ | H | $OCH_3$ | $CH_3$ | CH | |
| $CH_3$ | $CH_2OCH_2OCH_2CF_3$ | H | $OCH_3$ | Cl | CH | |
| $CH_3$ | $CH_2OCH_2OCH_2CF_3$ | H | $CH_3$ | $CH_3$ | CH | |
| $CH_3$ | $CH_2OCH_2OCH_2CF_3$ | H | $OCH_3$ | $OCH_3$ | N | |
| $CH_3$ | $CH_2OCH_2OCH_2CF_3$ | H | $OCH_3$ | $CH_3$ | N | |
| $CH_3$ | $CH_2OCH_2SCH_3$ | H | $OCH_3$ | $OCH_3$ | CH | |
| $CH_3$ | $CH_2OCH_2SCH_3$ | H | $OCH_3$ | $CH_3$ | CH | |
| $CH_3$ | $CH_2OCH_2SCH_3$ | H | $OCH_3$ | Cl | CH | |
| $CH_3$ | $CH_2OCH_2SCH_3$ | H | $CH_3$ | $CH_3$ | CH | |
| $CH_3$ | $CH_2OCH_2SCH_3$ | H | $OCH_3$ | $OCH_3$ | N | |
| $CH_2CH_3$ | $CH_2OCH_2SCH_3$ | H | $OCH_3$ | $CH_3$ | N | |
| $CH_3$ | $CH_2OCH_2CN$ | H | $OCH_3$ | $OCH_3$ | CH | |
| $CH_3$ | $CH_2OCH_2CN$ | H | $OCH_3$ | $CH_3$ | CH | |
| $CH_3$ | $CH_2OCH_2CN$ | H | $OCH_3$ | Cl | CH | |
| $CH_3$ | $CH_2OCH_2CN$ | H | $CH_3$ | $CH_3$ | CH | |
| $CH_3$ | $CH_2OCH_2CN$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $CH_2OCH_2CN$ | H | $OCH_3$ | $CH_3$ | N | |
| $CH_3$ | $CH_2OCH_2CH_2N(CH_3)_2$ | H | $OCH_3$ | $OCH_3$ | CH | |
| $CH_3$ | $CH_2OCH_2CH_2N(CH_3)_2$ | H | $OCH_3$ | $CH_3$ | CH | |
| $CH_3$ | $CH_2OCH_2CH_2N(CH_3)_2$ | H | $OCH_3$ | Cl | CH | |
| $CH_3$ | $CH_2OCH_2CH_2N(CH_3)_2$ | H | $CH_3$ | $CH_3$ | CH | |
| $CH_3$ | $CH_2OCH_2CH_2N(CH_3)_2$ | H | $OCH_3$ | $OCH_3$ | N | |
| $CH_2CH_3$ | $CH_2OCH_2CH_2N(CH_3)_2$ | H | $OCH_3$ | $CH_3$ | N | |
| $CH_3$ | $CH_2OCH_2CH_2F$ | H | $OCH_3$ | $OCH_3$ | CH | 92-95 |
| $CH_3$ | $CH_2OCH_2CH_2F$ | H | $OCH_3$ | $CH_3$ | CH | 115-117 |
| $CH_3$ | $CH_2OCH_2CH_2F$ | H | $OCH_3$ | Cl | CH | |
| $CH_3$ | $CH_2OCH_2CH_2F$ | H | $CH_3$ | $CH_3$ | CH | |
| $CH_3$ | $CH_2OCH_2CH_2F$ | H | $OCH_3$ | $OCH_3$ | N | 150-152 |
| $CH_3$ | $CH_2OCH_2CH_2F$ | H | $OCH_3$ | $CH_3$ | N | 100-103 |
| $CH_2CH_3$ | $CH_2OCH_2CH_2F$ | H | $OCH_3$ | $OCH_3$ | CH | 81-83 |
| $CH_2CH_3$ | $CH_2OCH_2CH_2F$ | H | $OCH_3$ | $CH_3$ | CH | 100-101 |
| $CH_2CH_3$ | $CH_2OCH_2CH_2F$ | H | $OCH_3$ | Cl | CH | |
| $CH_2CH_3$ | $CH_2OCH_2CH_2F$ | H | $CH_3$ | $CH_3$ | CH | |
| $CH_2CH_3$ | $CH_2OCH_2CH_2F$ | H | $OCH_3$ | $OCH_3$ | N | 125-127 |
| $CH_2CH_3$ | $CH_2OCH_2CH_2F$ | H | $OCH_3$ | $CH_3$ | N | 142-144 |
| $CH_3$ | $CH_2OCH_2CHF_2$ | H | $OCH_3$ | $OCH_3$ | CH | |
| $CH_3$ | $CH_2OCH_2CHF_2$ | H | $OCH_3$ | $CH_3$ | CH | |
| $CH_3$ | $CH_2OCH_2CHF_2$ | H | $OCH_3$ | Cl | CH | |
| $CH_3$ | $CH_2OCH_2CHF_2$ | H | $CH_3$ | $CH_3$ | CH | |
| $CH_3$ | $CH_2OCH_2CHF_2$ | H | $OCH_3$ | $OCH_3$ | N | |
| $CH_3$ | $CH_2OCH_2CHF_2$ | H | $OCH_3$ | $CH_3$ | N | |
| $CH_3$ | $CH_2OCH_2CF_3$ | H | $OCH_3$ | $OCH_3$ | CH | 118-120 |
| $CH_3$ | $CH_2OCH_2CF_3$ | H | $OCH_3$ | $CH_3$ | CH | 139-141 |
| $CH_3$ | $CH_2OCH_2CF_3$ | H | $OCH_3$ | Cl | CH | 124-125 |
| $CH_3$ | $CH_2OCH_2CF_3$ | H | $CH_3$ | $CH_3$ | CH | 159-161 |
| $CH_3$ | $CH_2OCH_2CF_3$ | H | $OCH_3$ | $OCH_3$ | N | 128-130 |
| $CH_3$ | $CH_2OCH_2CF_3$ | H | $OCH_3$ | $CH_3$ | N | 106-109 |
| $CH_2CH_3$ | $CH_2OCH_2CF_3$ | H | $OCH_3$ | $OCH_3$ | CH | 85-89 |
| $CH_2CH_3$ | $CH_2OCH_2CF_3$ | H | $OCH_3$ | $CH_3$ | CH | 110-113 |

Table I (Continued)

| $R_1$ | $R_2$ | R | X | Y | Z | m.p.(°C) |
|---|---|---|---|---|---|---|
| $CH_2CH_3$ | $CH_2OCH_2CF_3$ | H | $OCH_3$ | Cl | CH | 106-110 |
| $CH_2CH_3$ | $CH_2OCH_2CF_3$ | H | $CH_3$ | $CH_3$ | CH | 134-136 |
| $CH_2CH_3$ | $CH_2OCH_2CF_3$ | H | $OCH_3$ | $OCH_3$ | N | >250 |
| $CH_2CH_3$ | $CH_2OCH_2CF_3$ | H | $OCH_3$ | $CH_3$ | N | 126-128 |
| $CH_3$ | $CH_2OCH_2C{\equiv}CH$ | H | $OCH_3$ | $OCH_3$ | CH | |
| $CH_3$ | $CH_2OCH_2C{\equiv}CH$ | H | $OCH_3$ | $CH_3$ | CH | |
| $CH_3$ | $CH_2OCH_2C{\equiv}CH$ | H | $OCH_3$ | Cl | CH | |
| $CH_3$ | $CH_2OCH_2C{\equiv}CH$ | H | $CH_3$ | $CH_3$ | CH | |
| $CH_3$ | $CH_2OCH_2C{\equiv}CH$ | H | $OCH_3$ | $OCH_3$ | N | |
| $CH_3$ | $CH_2OCH_2C{\equiv}CH$ | H | $OCH_3$ | $CH_3$ | N | |
| $CH_3$ | $CH_2OC(O)CH_3$ | H | $OCH_3$ | $OCH_3$ | CH | 100-103 |
| $CH_3$ | $CH_2OC(O)CH_3$ | H | $OCH_3$ | $CH_3$ | CH | 165-167 |
| $CH_3$ | $CH_2OC(O)CH_3$ | H | $OCH_3$ | Cl | CH | |
| $CH_3$ | $CH_2OC(O)CH_3$ | H | $CH_3$ | $CH_3$ | CH | |
| $CH_3$ | $CH_2OC(O)CH_3$ | H | $OCH_3$ | $OCH_3$ | N | |
| $CH_3$ | $CH_2OC(O)CH_3$ | H | $OCH_3$ | $CH_3$ | N | 127-130 |
| $CH_3$ | $CH_2OC(O)CH_2Cl$ | H | $OCH_3$ | $OCH_3$ | CH | |
| $CH_3$ | $CH_2OC(O)CH_2Cl$ | H | $OCH_3$ | $CH_3$ | CH | |
| $CH_3$ | $CH_2OC(O)CH_2Cl$ | H | $OCH_3$ | Cl | CH | |
| $CH_3$ | $CH_2OC(O)CH_2Cl$ | H | $CH_3$ | $CH_3$ | CH | |
| $CH_3$ | $CH_2OC(O)CH_2Cl$ | H | $OCH_3$ | $OCH_3$ | N | |
| $CH_2CH_3$ | $CH_2OC(O)CH_2Cl$ | H | $OCH_3$ | $CH_3$ | N | |
| $CH_3$ | $CH_2OC(O)OCH_3$ | H | $OCH_3$ | $OCH_3$ | CH | |
| $CH_3$ | $CH_2OC(O)OCH_3$ | H | $OCH_3$ | $CH_3$ | CH | |
| $CH_3$ | $CH_2OC(O)OCH_3$ | H | $OCH_3$ | Cl | CH | |
| $CH_3$ | $CH_2OC(O)OCH_3$ | H | $CH_3$ | $CH_3$ | CH | |
| $CH_3$ | $CH_2OC(O)OCH_3$ | H | $OCH_3$ | $OCH_3$ | N | |
| $CH_3$ | $CH_2OC(O)OCH_3$ | H | $OCH_3$ | $CH_3$ | N | |
| $CH_3$ | $CH_2OH$ | H | $OCH_3$ | $OCH_3$ | CH | |
| $CH_3$ | $CH_2OH$ | H | $OCH_3$ | $CH_3$ | CH | |
| $CH_3$ | $CH_2OH$ | H | $OCH_3$ | Cl | CH | |
| $CH_3$ | $CH_2OH$ | H | $CH_3$ | $CH_3$ | CH | |
| $CH_3$ | $CH_2OH$ | H | $OCH_3$ | $OCH_3$ | N | |

**Table I (Continued)**

| $R_1$ | $R_2$ | R | X | Y | Z | m.p.(°C) |
|---|---|---|---|---|---|---|
| $CH_3$ | $CH_2OH$ | H | $OCH_3$ | $CH_3$ | N | |
| $CH_3$ | $CH_2OP(O)(OCH_3)_2$ | H | $OCH_3$ | $OCH_3$ | CH | |
| $CH_3$ | $CH_2OP(O)(OCH_3)_2$ | H | $OCH_3$ | $CH_3$ | CH | |
| $CH_3$ | $CH_2OP(O)(OCH_3)_2$ | H | $OCH_3$ | Cl | CH | |
| $CH_3$ | $CH_2OP(O)(OCH_3)_2$ | H | $CH_3$ | $CH_3$ | CH | |
| $CH_3$ | $CH_2OP(O)(OCH_3)_2$ | H | $OCH_3$ | $OCH_3$ | N | |
| $CH_2CH_3$ | $CH_2OP(O)(OCH_3)_2$ | H | $OCH_3$ | $CH_3$ | N | |
| $CH_3$ | $CH_2OSO_2CH_3$ | H | $OCH_3$ | $OCH_3$ | CH | |
| $CH_3$ | $CH_2OSO_2CH_3$ | H | $OCH_3$ | $CH_3$ | CH | |
| $CH_3$ | $CH_2OSO_2CH_3$ | H | $OCH_3$ | Cl | CH | |
| $CH_3$ | $CH_2OSO_2CH_3$ | H | $CH_3$ | $CH_3$ | CH | |
| $CH_3$ | $CH_2OSO_2CH_3$ | H | $OCH_3$ | $OCH_3$ | N | |
| $CH_3$ | $CH_2OSO_2CH_3$ | H | $OCH_3$ | $CH_3$ | N | |
| $CH_3$ | $CH_2OC(O)NHCH_3$ | H | $OCH_3$ | $OCH_3$ | CH | |
| $CH_3$ | $CH_2OC(O)NHCH_3$ | H | $OCH_3$ | $CH_3$ | CH | |
| $CH_3$ | $CH_2OC(O)NHCH_3$ | H | $OCH_3$ | Cl | CH | |
| $CH_3$ | $CH_2OC(O)NHCH_3$ | H | $CH_3$ | $CH_3$ | CH | |
| $CH_3$ | $CH_2OC(O)NHCH_3$ | H | $OCH_3$ | $OCH_3$ | N | |
| $CH_3$ | $CH_2OC(O)NHCH_3$ | H | $OCH_3$ | $CH_3$ | N | |
| $CH_3$ | $CH_2OC(O)N(CH_3)_2$ | H | $OCH_3$ | $OCH_3$ | CH | |
| $CH_3$ | $CH_2OC(O)N(CH_3)_2$ | H | $OCH_3$ | $CH_3$ | CH | |
| $CH_3$ | $CH_2OC(O)N(CH_3)_2$ | H | $OCH_3$ | Cl | CH | |
| $CH_3$ | $CH_2OC(O)N(CH_3)_2$ | H | $CH_3$ | $CH_3$ | CH | |
| $CH_3$ | $CH_2OC(O)N(CH_3)_2$ | H | $OCH_3$ | $OCH_3$ | N | |
| $CH_2CH_3$ | $CH_2OC(O)N(CH_3)_2$ | H | $OCH_3$ | $CH_3$ | N | |
| $CH_3$ | $CH_2OSi(CH_3)_3$ | H | $OCH_3$ | $OCH_3$ | CH | |
| $CH_3$ | $CH_2OSi(CH_3)_3$ | H | $OCH_3$ | $CH_3$ | CH | |
| $CH_3$ | $CH_2OSi(CH_3)_3$ | H | $OCH_3$ | Cl | CH | |
| $CH_3$ | $CH_2OSi(CH_3)_3$ | H | $CH_3$ | $CH_3$ | CH | |
| $CH_3$ | $CH_2OSi(CH_3)_3$ | H | $OCH_3$ | $OCH_3$ | N | |
| $CH_3$ | $CH_2OSi(CH_3)_3$ | H | $OCH_3$ | $CH_3$ | N | |
| $CH_3$ | $CH_2OCH_2CH_2OCH_3$ | H | $OCH_3$ | $OCH_3$ | CH | 168-170 |
| $CH_3$ | $CH_2OCH_2CH_2OCH_3$ | H | $OCH_3$ | $CH_3$ | CH | 90-91 |

Table I (Continued)

| $R_1$ | $R_2$ | R | X | Y | Z | m.p.(°C) |
|---|---|---|---|---|---|---|
| $CH_3$ | $CH_2OCH_2CH_2OCH_3$ | H | $OCH_3$ | Cl | CH | 109-111 |
| $CH_3$ | $CH_2OCH_2CH_2OCH_3$ | H | $CH_3$ | $CH_3$ | CH | 120-122 |
| $CH_3$ | $CH_2OCH_2CH_2OCH_3$ | H | $OCH_3$ | $OCH_3$ | N | 135-137 |
| $CH_3$ | $CH_2OCH_2CH_2OCH_3$ | H | $OCH_3$ | $CH_3$ | N | 139-141 |
| $CH_3$ | $CH_2OCH_2CH_2OCH_3$ | H | $OCH_3$ | $OCH_3$ | CH | 168-170 |
| $CH_3$ | $CH_2OCH_2CH_2OCH_3$ | H | $OCH_3$ | $CH_3$ | CH | 91-92 |
| $CH_3$ | $CH_2OCH_2CH_2OCH_3$ | H | $OCH_3$ | Cl | CH | 109-111 |
| $CH_3$ | $CH_2OCH_2CH_2OCH_3$ | H | $CH_3$ | $CH_3$ | CH | 120-122 |
| $CH_3$ | $CH_2OCH_2CH_2OCH_3$ | H | $OCH_3$ | $OCH_3$ | N | 135-137 |
| $CH_3$ | $CH_2OCH_2CH_2OCH_3$ | H | $OCH_3$ | $CH_3$ | N | 139-141 |
| $CH_3$ | $CH_2OCH_2C(H)=CH_2$ | H | $OCH_3$ | $OCH_3$ | CH | |
| $CH_3$ | $CH_2OCH_2C(H)=CH_2$ | H | $OCH_3$ | $CH_3$ | CH | |
| $CH_3$ | $CH_2OCH_2C(H)=CH_2$ | H | $OCH_3$ | Cl | CH | |
| $CH_3$ | $CH_2OCH_2C(H)=CH_2$ | H | $CH_3$ | $CH_3$ | CH | |
| $CH_3$ | $CH_2OCH_2C(H)=CH_2$ | H | $OCH_3$ | $OCH_3$ | N | |
| $CH_3$ | $CH_2OCH_2C(H)=CH_2$ | H | $OCH_3$ | $CH_3$ | N | |
| $CH_3$ | $CH_2OCH_2C(Cl)=CH_2$ | H | $OCH_3$ | $OCH_3$ | CH | |
| $CH_3$ | $CH_2OCH_2C(Cl)=CH_2$ | H | $OCH_3$ | $CH_3$ | CH | |
| $CH_3$ | $CH_2OCH_2C(Cl)=CH_2$ | H | $OCH_3$ | Cl | CH | |
| $CH_3$ | $CH_2OCH_2C(Cl)=CH_2$ | H | $CH_3$ | $CH_3$ | CH | |
| $CH_3$ | $CH_2OCH_2C(Cl)=CH_2$ | H | $OCH_3$ | $OCH_3$ | N | |
| $CH_2CH_3$ | $CH_2OCH_2C(Cl)=CH_2$ | H | $OCH_3$ | $CH_3$ | N | |
| $CH_3$ | $CH_2S$-cyclopropyl | H | $OCH_3$ | $OCH_3$ | CH | |
| $CH_3$ | $CH_2S$-cyclopropyl | H | $OCH_3$ | $CH_3$ | CH | |
| $CH_3$ | $CH_2S$-cyclopropyl | H | $OCH_3$ | Cl | CH | |
| $CH_3$ | $CH_2S$-cyclopropyl | H | $CH_3$ | $CH_3$ | CH | |
| $CH_3$ | $CH_2S$-cyclopropyl | H | $OCH_3$ | $OCH_3$ | N | |
| $CH_3$ | $CH_2S$-cyclopropyl | H | $OCH_3$ | $CH_3$ | N | |
| $CH_3$ | $CH_2SCH_2$-cyclopropyl | H | $OCH_3$ | $OCH_3$ | CH | |
| $CH_3$ | $CH_2SCH_2$-cyclopropyl | H | $OCH_3$ | $CH_3$ | CH | |
| $CH_3$ | $CH_2SCH_2$-cyclopropyl | H | $OCH_3$ | Cl | CH | |
| $CH_3$ | $CH_2SCH_2$-cyclopropyl | H | $CH_3$ | $CH_3$ | CH | |
| $CH_3$ | $CH_2SCH_2$-cyclopropyl | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $CH_2SCH_2$-cyclopropyl | H | $OCH_3$ | $CH_3$ | N | |
| $CH_3$ | $CH_2S$-cyclobutyl | H | $OCH_3$ | $OCH_3$ | CH | |
| $CH_3$ | $CH_2S$-cyclobutyl | H | $OCH_3$ | $CH_3$ | CH | |
| $CH_3$ | $CH_2S$-cyclobutyl | H | $OCH_3$ | Cl | CH | |
| $CH_3$ | $CH_2S$-cyclobutyl | H | $CH_3$ | $CH_3$ | CH | |
| $CH_3$ | $CH_2S$-cyclobutyl | H | $OCH_3$ | $OCH_3$ | N | |
| $CH_2CH_3$ | $CH_2S$-cyclobutyl | H | $OCH_3$ | $CH_3$ | N | |
| $CH_3$ | $CH_2SCH_2CF_2CF_3$ | H | $OCH_3$ | $OCH_3$ | CH | |
| $CH_3$ | $CH_2SCH_2CF_2CF_3$ | H | $OCH_3$ | $CH_3$ | CH | |
| $CH_3$ | $CH_2SCH_2CF_2CF_3$ | H | $OCH_3$ | Cl | CH | |
| $CH_3$ | $CH_2SCH_2CF_2CF_3$ | H | $CH_3$ | $CH_3$ | CH | |
| $CH_3$ | $CH_2SCH_2CF_2CF_3$ | H | $OCH_3$ | $OCH_3$ | N | |
| $CH_3$ | $CH_2SCH_2CF_2CF_3$ | H | $OCH_3$ | $CH_3$ | N | |
| $CH_3$ | $CH_2SCH(CF_3)CH_3$ | H | $OCH_3$ | $OCH_3$ | CH | |
| $CH_3$ | $CH_2SCH(CF_3)CH_3$ | H | $OCH_3$ | $CH_3$ | CH | |
| $CH_3$ | $CH_2SCH(CF_3)CH_3$ | H | $OCH_3$ | Cl | CH | |
| $CH_3$ | $CH_2SCH(CF_3)CH_3$ | H | $CH_3$ | $CH_3$ | CH | |
| $CH_3$ | $CH_2SCH(CF_3)CH_3$ | H | $OCH_3$ | $OCH_3$ | N | |
| $CH_3$ | $CH_2SCH(CF_3)CH_3$ | H | $OCH_3$ | $CH_3$ | N | |
| $CH_3$ | $CH_2SCH_2CF_2CF_2H$ | H | $OCH_3$ | $OCH_3$ | CH | |
| $CH_3$ | $CH_2SCH_2CF_2CF_2H$ | H | $OCH_3$ | $CH_3$ | CH | |
| $CH_3$ | $CH_2SCH_2CF_2CF_2H$ | H | $OCH_3$ | Cl | CH | |
| $CH_3$ | $CH_2SCH_2CF_2CF_2H$ | H | $CH_3$ | $CH_3$ | CH | |
| $CH_3$ | $CH_2SCH_2CF_2CF_2H$ | H | $OCH_3$ | $OCH_3$ | N | |
| $CH_2CH_3$ | $CH_2SCH_2CF_2CF_2H$ | H | $OCH_3$ | $CH_3$ | N | |
| $CH_3$ | $CH_2SCH(CH_2F)_2$ | H | $OCH_3$ | $OCH_3$ | CH | |
| $CH_3$ | $CH_2SCH(CH_2F)_2$ | H | $OCH_3$ | $CH_3$ | CH | |
| $CH_3$ | $CH_2SCH(CH_2F)_2$ | H | $OCH_3$ | Cl | CH | |
| $CH_3$ | $CH_2SCH(CH_2F)_2$ | H | $CH_3$ | $CH_3$ | CH | |
| $CH_3$ | $CH_2SCH(CH_2F)_2$ | H | $OCH_3$ | $OCH_3$ | N | |
| $CH_3$ | $CH_2SCH(CH_2F)_2$ | H | $OCH_3$ | $CH_3$ | N | |
| $CH_3$ | $CH_2SCH(CH_2F)CH_3$ | H | $OCH_3$ | $OCH_3$ | CH | |
| $CH_3$ | $CH_2SCH(CH_2F)CH_3$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $CH_2SCH(CH_2F)CH_3$ | H | $OCH_3$ | Cl | CH | |
| $CH_3$ | $CH_2SCH(CH_2F)CH_3$ | H | $CH_3$ | $CH_3$ | CH | |
| $CH_3$ | $CH_2SCH(CH_2F)CH_3$ | H | $OCH_3$ | $OCH_3$ | N | |
| $CH_3$ | $CH_2SCH(CH_2F)CH_3$ | H | $OCH_3$ | $CH_3$ | N | |
| $CH_3$ | $CH_2S(CH_2)_3CH_2Cl$ | H | $OCH_3$ | $OCH_3$ | CH | |
| $CH_3$ | $CH_2S(CH_2)_3CH_2Cl$ | H | $OCH_3$ | $CH_3$ | CH | |
| $CH_3$ | $CH_2S(CH_2)_3CH_2Cl$ | H | $OCH_3$ | Cl | CH | |
| $CH_3$ | $CH_2S(CH_2)_3CH_2Cl$ | H | $CH_3$ | $CH_3$ | CH | |
| $CH_3$ | $CH_2S(CH_2)_3CH_2Cl$ | H | $OCH_3$ | $OCH_3$ | N | |
| $CH_2CH_3$ | $CH_2S(CH_2)_3CH_2Cl$ | H | $OCH_3$ | $CH_3$ | N | |
| $CH_3$ | $CH_2SCH_2CH_2OCH_3$ | H | $OCH_3$ | $OCH_3$ | CH | |
| $CH_3$ | $CH_2SCH_2CH_2OCH_3$ | H | $OCH_3$ | $CH_3$ | CH | |
| $CH_3$ | $CH_2SCH_2CH_2OCH_3$ | H | $OCH_3$ | Cl | CH | |
| $CH_3$ | $CH_2SCH_2CH_2OCH_3$ | H | $CH_3$ | $CH_3$ | CH | |
| $CH_3$ | $CH_2SCH_2CH_2OCH_3$ | H | $OCH_3$ | $OCH_3$ | N | |
| $CH_3$ | $CH_2SCH_2CH_2OCH_3$ | H | $OCH_3$ | $CH_3$ | N | |
| $CH_3$ | $CH_2SCH_2C(Cl)=CH_2$ | H | $OCH_3$ | $OCH_3$ | CH | |
| $CH_3$ | $CH_2SCH_2C(Cl)=CH_2$ | H | $OCH_3$ | $CH_3$ | CH | |
| $CH_3$ | $CH_2SCH_2C(Cl)=CH_2$ | H | $OCH_3$ | Cl | CH | |
| $CH_3$ | $CH_2SCH_2C(Cl)=CH_2$ | H | $CH_3$ | $CH_3$ | CH | |
| $CH_3$ | $CH_2SCH_2C(Cl)=CH_2$ | H | $OCH_3$ | $OCH_3$ | N | |
| $CH_3$ | $CH_2SCH_2C(Cl)=CH_2$ | H | $OCH_3$ | $CH_3$ | N | |
| $CH_3$ | $CH_2SCH_2C\equiv CCH_2Cl$ | H | $OCH_3$ | $OCH_3$ | CH | |
| $CH_3$ | $CH_2SCH_2C\equiv CCH_2Cl$ | H | $OCH_3$ | $CH_3$ | CH | |
| $CH_3$ | $CH_2SCH_2C\equiv CCH_2Cl$ | H | $OCH_3$ | Cl | CH | |
| $CH_3$ | $CH_2SCH_2C\equiv CCH_2Cl$ | H | $CH_3$ | $CH_3$ | CH | |
| $CH_3$ | $CH_2SCH_2C\equiv CCH_2Cl$ | H | $OCH_3$ | $OCH_3$ | N | |
| $CH_2CH_3$ | $CH_2SCH_2C\equiv CCH_2Cl$ | H | $OCH_3$ | $CH_3$ | N | |
| $CH_3$ | $CH_2SCH_2OCH_3$ | H | $OCH_3$ | $OCH_3$ | CH | |
| $CH_3$ | $CH_2SCH_2OCH_3$ | H | $OCH_3$ | $CH_3$ | CH | |
| $CH_3$ | $CH_2SCH_2OCH_3$ | H | $OCH_3$ | Cl | CH | |
| $CH_3$ | $CH_2SCH_2OCH_3$ | H | $CH_3$ | $CH_3$ | CH | |
| $CH_3$ | $CH_2SCH_2OCH_3$ | H | $OCH_3$ | $OCH_3$ | N | |

**Table I (Continued)**

| $R_1$ | $R_2$ | R | X | Y | Z | m.p.(°C) |
|---|---|---|---|---|---|---|
| $CH_3$ | $CH_2SCH_2OCH_3$ | H | $OCH_3$ | $CH_3$ | N | |
| $CH_3$ | $CH_2SCH_2OCH_2CF_3$ | H | $OCH_3$ | $OCH_3$ | CH | |
| $CH_3$ | $CH_2SCH_2OCH_2CF_3$ | H | $OCH_3$ | $CH_3$ | CH | |
| $CH_3$ | $CH_2SCH_2OCH_2CF_3$ | H | $OCH_3$ | Cl | CH | |
| $CH_3$ | $CH_2SCH_2OCH_2CF_3$ | H | $CH_3$ | $CH_3$ | CH | |
| $CH_3$ | $CH_2SCH_2OCH_2CF_3$ | H | $OCH_3$ | $OCH_3$ | N | |
| $CH_3$ | $CH_2SCH_2OCH_2CF_3$ | H | $OCH_3$ | $CH_3$ | N | |
| $CH_3$ | $CH_2SCH_2SCH_3$ | H | $OCH_3$ | $OCH_3$ | CH | |
| $CH_3$ | $CH_2SCH_2SCH_3$ | H | $OCH_3$ | $CH_3$ | CH | |
| $CH_3$ | $CH_2SCH_2SCH_3$ | H | $OCH_3$ | Cl | CH | |
| $CH_3$ | $CH_2SCH_2SCH_3$ | H | $CH_3$ | $CH_3$ | CH | |
| $CH_3$ | $CH_2SCH_2SCH_3$ | H | $OCH_3$ | $OCH_3$ | N | |
| $CH_2CH_3$ | $CH_2SCH_2SCH_3$ | H | $OCH_3$ | $CH_3$ | N | |
| $CH_3$ | $CH_2SCH_2CN$ | H | $OCH_3$ | $OCH_3$ | CH | |
| $CH_3$ | $CH_2SCH_2CN$ | H | $OCH_3$ | $CH_3$ | CH | |
| $CH_3$ | $CH_2SCH_2CN$ | H | $OCH_3$ | Cl | CH | |
| $CH_3$ | $CH_2SCH_2CN$ | H | $CH_3$ | $CH_3$ | CH | |
| $CH_3$ | $CH_2SCH_2CN$ | H | $OCH_3$ | $OCH_3$ | N | |
| $CH_3$ | $CH_2SCH_2CN$ | H | $OCH_3$ | $CH_3$ | N | |
| $CH_3$ | $CH_2SCH_2CH_2N(CH_3)_2$ | H | $OCH_3$ | $OCH_3$ | CH | |
| $CH_3$ | $CH_2SCH_2CH_2N(CH_3)_2$ | H | $OCH_3$ | $CH_3$ | CH | |
| $CH_3$ | $CH_2SCH_2CH_2N(CH_3)_2$ | H | $OCH_3$ | Cl | CH | |
| $CH_3$ | $CH_2SCH_2CH_2N(CH_3)_2$ | H | $CH_3$ | $CH_3$ | CH | |
| $CH_3$ | $CH_2SCH_2CH_2N(CH_3)_2$ | H | $OCH_3$ | $OCH_3$ | N | |
| $CH_2CH_3$ | $CH_2SCH_2CH_2N(CH_3)_2$ | H | $OCH_3$ | $CH_3$ | N | |
| $CH_3$ | $CH_2SCH_2CH_2F$ | H | $OCH_3$ | $OCH_3$ | CH | |
| $CH_3$ | $CH_2SCH_2CH_2F$ | H | $OCH_3$ | $CH_3$ | CH | |
| $CH_3$ | $CH_2SCH_2CH_2F$ | H | $OCH_3$ | Cl | CH | |
| $CH_3$ | $CH_2SCH_2CH_2F$ | H | $CH_3$ | $CH_3$ | CH | |
| $CH_3$ | $CH_2SCH_2CH_2F$ | H | $OCH_3$ | $OCH_3$ | N | |
| $CH_3$ | $CH_2SCH_2CH_2F$ | H | $OCH_3$ | $CH_3$ | N | |
| $CH_3$ | $CH_2SCH_2CHF_2$ | H | $OCH_3$ | $OCH_3$ | CH | |
| $CH_3$ | $CH_2SCH_2CHF_2$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $CH_2SCH_2CHF_2$ | H | $OCH_3$ | Cl | CH | |
| $CH_3$ | $CH_2SCH_2CHF_2$ | H | $CH_3$ | $CH_3$ | CH | |
| $CH_3$ | $CH_2SCH_2CHF_2$ | H | $OCH_3$ | $OCH_3$ | N | |
| $CH_3$ | $CH_2SCH_2CHF_2$ | H | $OCH_3$ | $CH_3$ | N | |
| $CH_3$ | $CH_2SCH_2CF_3$ | H | $OCH_3$ | $OCH_3$ | CH | |
| $CH_3$ | $CH_2SCH_2CF_3$ | H | $OCH_3$ | $CH_3$ | CH | |
| $CH_3$ | $CH_2SCH_2CF_3$ | H | $OCH_3$ | Cl | CH | |
| $CH_3$ | $CH_2SCH_2CF_3$ | H | $CH_3$ | $CH_3$ | CH | |
| $CH_3$ | $CH_2SCH_2CF_3$ | H | $OCH_3$ | $OCH_3$ | N | |
| $CH_2CH_3$ | $CH_2SCH_2CF_3$ | H | $OCH_3$ | $CH_3$ | N | |
| $CH_3$ | $CH_2SCH_2C{\equiv}CH$ | H | $OCH_3$ | $OCH_3$ | CH | |
| $CH_3$ | $CH_2SCH_2C{\equiv}CH$ | H | $OCH_3$ | $CH_3$ | CH | |
| $CH_3$ | $CH_2SCH_2C{\equiv}CH$ | H | $OCH_3$ | Cl | CH | |
| $CH_3$ | $CH_2SCH_2C{\equiv}CH$ | H | $CH_3$ | $CH_3$ | CH | |
| $CH_3$ | $CH_2SCH_2C{\equiv}CH$ | H | $OCH_3$ | $OCH_3$ | N | |
| $CH_3$ | $CH_2SCH_2C{\equiv}CH$ | H | $OCH_3$ | $CH_3$ | N | |
| $CH_3$ | $CH_2SH$ | H | $OCH_3$ | $OCH_3$ | CH | |
| $CH_3$ | $CH_2SH$ | H | $OCH_3$ | $CH_3$ | CH | |
| $CH_3$ | $CH_2SH$ | H | $OCH_3$ | Cl | CH | |
| $CH_3$ | $CH_2SH$ | H | $CH_3$ | $CH_3$ | CH | |
| $CH_3$ | $CH_2SH$ | H | $OCH_3$ | $OCH_3$ | N | |
| $CH_3$ | $CH_2SH$ | H | $OCH_3$ | $CH_3$ | N | |
| $CH_3$ | $CH_2SP(O)(OCH_3)_2$ | H | $OCH_3$ | $OCH_3$ | CH | |
| $CH_3$ | $CH_2SP(O)(OCH_3)_2$ | H | $OCH_3$ | $CH_3$ | CH | |
| $CH_3$ | $CH_2SP(O)(OCH_3)_2$ | H | $OCH_3$ | Cl | CH | |
| $CH_3$ | $CH_2SP(O)(OCH_3)_2$ | H | $CH_3$ | $CH_3$ | CH | |
| $CH_3$ | $CH_2SP(O)(OCH_3)_2$ | H | $OCH_3$ | $OCH_3$ | N | |
| $CH_2CH_3$ | $CH_2SP(O)(OCH_3)_2$ | H | $OCH_3$ | $CH_3$ | N | |
| $CH_3$ | $CH_2SCH_2CH_2OCH_3$ | H | $OCH_3$ | $OCH_3$ | CH | |
| $CH_3$ | $CH_2SCH_2CH_2OCH_3$ | H | $OCH_3$ | $CH_3$ | CH | |
| $CH_3$ | $CH_2SCH_2CH_2OCH_3$ | H | $OCH_3$ | Cl | CH | |
| $CH_3$ | $CH_2SCH_2CH_2OCH_3$ | H | $CH_3$ | $CH_3$ | CH | |
| $CH_3$ | $CH_2SCH_2CH_2OCH_3$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $CH_2SCH_2CH_2OCH_3$ | H | $OCH_3$ | $CH_3$ | N | |
| $CH_3$ | $CH_2SCH_2C(H)=CH_2$ | H | $OCH_3$ | $OCH_3$ | CH | |
| $CH_3$ | $CH_2SCH_2C(H)=CH_2$ | H | $OCH_3$ | $CH_3$ | CH | |
| $CH_3$ | $CH_2SCH_2C(H)=CH_2$ | H | $OCH_3$ | Cl | CH | |
| $CH_3$ | $CH_2SCH_2C(H)=CH_2$ | H | $CH_3$ | $CH_3$ | CH | |
| $CH_3$ | $CH_2SCH_2C(H)=CH_2$ | H | $OCH_3$ | $OCH_3$ | N | |
| $CH_3$ | $CH_2SCH_2C(H)=CH_2$ | H | $OCH_3$ | $CH_3$ | N | |
| $CH_3$ | $CH_2SCH_2C(Cl)=CH_2$ | H | $OCH_3$ | $OCH_3$ | CH | |
| $CH_3$ | $CH_2SCH_2C(Cl)=CH_2$ | H | $OCH_3$ | $CH_3$ | CH | |
| $CH_3$ | $CH_2SCH_2C(Cl)=CH_2$ | H | $OCH_3$ | Cl | CH | |
| $CH_3$ | $CH_2SCH_2C(Cl)=CH_2$ | H | $CH_3$ | $CH_3$ | CH | |
| $CH_3$ | $CH_2SCH_2C(Cl)=CH_2$ | H | $OCH_3$ | $OCH_3$ | N | |
| $CH_2CH_3$ | $CH_2SCH_2C(Cl)=CH_2$ | H | $OCH_3$ | $CH_3$ | N | |
| $CH_3$ | $CH_2S(O)$-cyclopropyl | H | $OCH_3$ | $OCH_3$ | CH | |
| $CH_3$ | $CH_2S(O)$-cyclopropyl | H | $OCH_3$ | $CH_3$ | CH | |
| $CH_3$ | $CH_2S(O)$-cyclopropyl | H | $OCH_3$ | Cl | CH | |
| $CH_3$ | $CH_2S(O)$-cyclopropyl | H | $CH_3$ | $CH_3$ | CH | |
| $CH_3$ | $CH_2S(O)$-cyclopropyl | H | $OCH_3$ | $OCH_3$ | N | |
| $CH_3$ | $CH_2S(O)$-cyclopropyl | H | $OCH_3$ | $CH_3$ | N | |
| $CH_3$ | $CH_2S(O)CH_2$-cyclopropyl | H | $OCH_3$ | $OCH_3$ | CH | |
| $CH_3$ | $CH_2S(O)CH_2$-cyclopropyl | H | $OCH_3$ | $CH_3$ | CH | |
| $CH_3$ | $CH_2S(O)CH_2$-cyclopropyl | H | $OCH_3$ | Cl | CH | |
| $CH_3$ | $CH_2S(O)CH_2$-cyclopropyl | H | $CH_3$ | $CH_3$ | CH | |
| $CH_3$ | $CH_2S(O)CH_2$-cyclopropyl | H | $OCH_3$ | $OCH_3$ | N | |
| $CH_3$ | $CH_2S(O)CH_2$-cyclopropyl | H | $OCH_3$ | $CH_3$ | N | |
| $CH_3$ | $CH_2S(O)$-cyclobutyl | H | $OCH_3$ | $OCH_3$ | CH | |
| $CH_3$ | $CH_2S(O)$-cyclobutyl | H | $OCH_3$ | $CH_3$ | CH | |
| $CH_3$ | $CH_2S(O)$-cyclobutyl | H | $OCH_3$ | Cl | CH | |
| $CH_3$ | $CH_2S(O)$-cyclobutyl | H | $CH_3$ | $CH_3$ | CH | |
| $CH_3$ | $CH_2S(O)$-cyclobutyl | H | $OCH_3$ | $OCH_3$ | N | |
| $CH_2CH_3$ | $CH_2S(O)$-cyclobutyl | H | $OCH_3$ | $CH_3$ | N | |
| $CH_3$ | $CH_2S(O)CH_2CF_2CF_3$ | H | $OCH_3$ | $OCH_3$ | CH | |
| $CH_3$ | $CH_2S(O)CH_2CF_2CF_3$ | H | $OCH_3$ | $CH_3$ | CH | |

**Table I (Continued)**

| $R_1$ | $R_2$ | R | X | Y | Z | m.p.(°C) |
|---|---|---|---|---|---|---|
| $CH_3$ | $CH_2S(O)CH_2CF_2CF_3$ | H | $OCH_3$ | Cl | CH | |
| $CH_3$ | $CH_2S(O)CH_2CF_2CF_3$ | H | $CH_3$ | $CH_3$ | CH | |
| $CH_3$ | $CH_2S(O)CH_2CF_2CF_3$ | H | $OCH_3$ | $OCH_3$ | N | |
| $CH_3$ | $CH_2S(O)CH_2CF_2CF_3$ | H | $OCH_3$ | $CH_3$ | N | |
| $CH_3$ | $CH_2S(O)CH(CF_3)CH_3$ | H | $OCH_3$ | $OCH_3$ | CH | |
| $CH_3$ | $CH_2S(O)CH(CF_3)CH_3$ | H | $OCH_3$ | $CH_3$ | CH | |
| $CH_3$ | $CH_2S(O)CH(CF_3)CH_3$ | H | $OCH_3$ | Cl | CH | |
| $CH_3$ | $CH_2S(O)CH(CF_3)CH_3$ | H | $CH_3$ | $CH_3$ | CH | |
| $CH_3$ | $CH_2S(O)CH(CF_3)CH_3$ | H | $OCH_3$ | $OCH_3$ | N | |
| $CH_3$ | $CH_2S(O)CH(CF_3)CH_3$ | H | $OCH_3$ | $CH_3$ | N | |
| $CH_3$ | $CH_2S(O)CH_2CF_2CF_2H$ | H | $OCH_3$ | $OCH_3$ | CH | |
| $CH_3$ | $CH_2S(O)CH_2CF_2CF_2H$ | H | $OCH_3$ | $CH_3$ | CH | |
| $CH_3$ | $CH_2S(O)CH_2CF_2CF_2H$ | H | $OCH_3$ | Cl | CH | |
| $CH_3$ | $CH_2S(O)CH_2CF_2CF_2H$ | H | $CH_3$ | $CH_3$ | CH | |
| $CH_3$ | $CH_2S(O)CH_2CF_2CF_2H$ | H | $OCH_3$ | $OCH_3$ | N | |
| $CH_2CH_3$ | $CH_2S(O)CH_2CF_2CF_2H$ | H | $OCH_3$ | $CH_3$ | N | |
| $CH_3$ | $CH_2S(O)CH(CH_2F)_2$ | H | $OCH_3$ | $OCH_3$ | CH | |
| $CH_3$ | $CH_2S(O)CH(CH_2F)_2$ | H | $OCH_3$ | $CH_3$ | CH | |
| $CH_3$ | $CH_2S(O)CH(CH_2F)_2$ | H | $OCH_3$ | Cl | CH | |
| $CH_3$ | $CH_2S(O)CH(CH_2F)_2$ | H | $CH_3$ | $CH_3$ | CH | |
| $CH_3$ | $CH_2S(O)CH(CH_2F)_2$ | H | $OCH_3$ | $OCH_3$ | N | |
| $CH_3$ | $CH_2S(O)CH(CH_2F)_2$ | H | $OCH_3$ | $CH_3$ | N | |
| $CH_3$ | $CH_2S(O)CH(CH_2F)CH_3$ | H | $OCH_3$ | $OCH_3$ | CH | |
| $CH_3$ | $CH_2S(O)CH(CH_2F)CH_3$ | H | $OCH_3$ | $CH_3$ | CH | |
| $CH_3$ | $CH_2S(O)CH(CH_2F)CH_3$ | H | $OCH_3$ | Cl | CH | |
| $CH_3$ | $CH_2S(O)CH(CH_2F)CH_3$ | H | $CH_3$ | $CH_3$ | CH | |
| $CH_3$ | $CH_2S(O)CH(CH_2F)CH_3$ | H | $OCH_3$ | $OCH_3$ | N | |
| $CH_3$ | $CH_2S(O)CH(CH_2F)CH_3$ | H | $OCH_3$ | $CH_3$ | N | |
| $CH_3$ | $CH_2S(O)(CH_2)_3CH_2Cl$ | H | $OCH_3$ | $OCH_3$ | CH | |
| $CH_3$ | $CH_2S(O)(CH_2)_3CH_2Cl$ | H | $OCH_3$ | $CH_3$ | CH | |
| $CH_3$ | $CH_2S(O)(CH_2)_3CH_2Cl$ | H | $OCH_3$ | Cl | CH | |
| $CH_3$ | $CH_2S(O)(CH_2)_3CH_2Cl$ | H | $CH_3$ | $CH_3$ | CH | |
| $CH_3$ | $CH_2S(O)(CH_2)_3CH_2Cl$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_2CH_3$ | $CH_2S(O)(CH_2)_3CH_2Cl$ | H | $OCH_3$ | $CH_3$ | N | |
| $CH_3$ | $CH_2S(O)CH_2CH_2OCH_3$ | H | $OCH_3$ | $OCH_3$ | CH | |
| $CH_3$ | $CH_2S(O)CH_2CH_2OCH_3$ | H | $OCH_3$ | $CH_3$ | CH | |
| $CH_3$ | $CH_2S(O)CH_2CH_2OCH_3$ | H | $OCH_3$ | Cl | CH | |
| $CH_3$ | $CH_2S(O)CH_2CH_2OCH_3$ | H | $CH_3$ | $CH_3$ | CH | |
| $CH_3$ | $CH_2S(O)CH_2CH_2OCH_3$ | H | $OCH_3$ | $OCH_3$ | N | |
| $CH_3$ | $CH_2S(O)CH_2CH_2OCH_3$ | H | $OCH_3$ | $CH_3$ | N | |
| $CH_3$ | $CH_2S(O)CH_2C(Cl)=CH_2$ | H | $OCH_3$ | $OCH_3$ | CH | |
| $CH_3$ | $CH_2S(O)CH_2C(Cl)=CH_2$ | H | $OCH_3$ | $CH_3$ | CH | |
| $CH_3$ | $CH_2S(O)CH_2C(Cl)=CH_2$ | H | $OCH_3$ | Cl | CH | |
| $CH_3$ | $CH_2S(O)CH_2C(Cl)=CH_2$ | H | $CH_3$ | $CH_3$ | CH | |
| $CH_3$ | $CH_2S(O)CH_2C(Cl)=CH_2$ | H | $OCH_3$ | $OCH_3$ | N | |
| $CH_3$ | $CH_2S(O)CH_2C(Cl)=CH_2$ | H | $OCH_3$ | $CH_3$ | N | |
| $CH_3$ | $CH_2S(O)CH_2C\equiv CCH_2Cl$ | H | $OCH_3$ | $OCH_3$ | CH | |
| $CH_3$ | $CH_2S(O)CH_2C\equiv CCH_2Cl$ | H | $OCH_3$ | $CH_3$ | CH | |
| $CH_3$ | $CH_2S(O)CH_2C\equiv CCH_2Cl$ | H | $OCH_3$ | Cl | CH | |
| $CH_3$ | $CH_2S(O)CH_2C\equiv CCH_2Cl$ | H | $CH_3$ | $CH_3$ | CH | |
| $CH_3$ | $CH_2S(O)CH_2C\equiv CCH_2Cl$ | H | $OCH_3$ | $OCH_3$ | N | |
| $CH_2CH_3$ | $CH_2S(O)CH_2C\equiv CCH_2Cl$ | H | $OCH_3$ | $CH_3$ | N | |
| $CH_3$ | $CH_2S(O)CH_2CN$ | H | $OCH_3$ | $OCH_3$ | CH | |
| $CH_3$ | $CH_2S(O)CH_2CN$ | H | $OCH_3$ | $CH_3$ | CH | |
| $CH_3$ | $CH_2S(O)CH_2CN$ | H | $OCH_3$ | Cl | CH | |
| $CH_3$ | $CH_2S(O)CH_2CN$ | H | $CH_3$ | $CH_3$ | CH | |
| $CH_3$ | $CH_2S(O)CH_2CN$ | H | $OCH_3$ | $OCH_3$ | N | |
| $CH_3$ | $CH_2S(O)CH_2CN$ | H | $OCH_3$ | $CH_3$ | N | |
| $CH_3$ | $CH_2S(O)CH_2CH_2N(CH_3)_2$ | H | $OCH_3$ | $OCH_3$ | CH | |
| $CH_3$ | $CH_2S(O)CH_2CH_2N(CH_3)_2$ | H | $OCH_3$ | $CH_3$ | CH | |
| $CH_3$ | $CH_2S(O)CH_2CH_2N(CH_3)_2$ | H | $OCH_3$ | Cl | CH | |
| $CH_3$ | $CH_2S(O)CH_2CH_2N(CH_3)_2$ | H | $CH_3$ | $CH_3$ | CH | |
| $CH_3$ | $CH_2S(O)CH_2CH_2N(CH_3)_2$ | H | $OCH_3$ | $OCH_3$ | N | |
| $CH_2CH_3$ | $CH_2S(O)CH_2CH_2N(CH_3)_2$ | H | $OCH_3$ | $CH_3$ | N | |
| $CH_3$ | $CH_2S(O)CH_2CH_2F$ | H | $OCH_3$ | $OCH_3$ | CH | |
| $CH_3$ | $CH_2S(O)CH_2CH_2F$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $CH_2S(O)CH_2CH_2F$ | H | $OCH_3$ | Cl | CH | |
| $CH_3$ | $CH_2S(O)CH_2CH_2F$ | H | $CH_3$ | $CH_3$ | CH | |
| $CH_3$ | $CH_2S(O)CH_2CH_2F$ | H | $OCH_3$ | $OCH_3$ | N | |
| $CH_3$ | $CH_2S(O)CH_2CH_2F$ | H | $OCH_3$ | $CH_3$ | N | |
| $CH_3$ | $CH_2S(O)CH_2CHF_2$ | H | $OCH_3$ | $OCH_3$ | CH | |
| $CH_3$ | $CH_2S(O)CH_2CHF_2$ | H | $OCH_3$ | $CH_3$ | CH | |
| $CH_3$ | $CH_2S(O)CH_2CHF_2$ | H | $OCH_3$ | Cl | CH | |
| $CH_3$ | $CH_2S(O)CH_2CHF_2$ | H | $CH_3$ | $CH_3$ | CH | |
| $CH_3$ | $CH_2S(O)CH_2CHF_2$ | H | $OCH_3$ | $OCH_3$ | N | |
| $CH_3$ | $CH_2S(O)CH_2CHF_2$ | H | $OCH_3$ | $CH_3$ | N | |
| $CH_3$ | $CH_2S(O)CH_2CF_3$ | H | $OCH_3$ | $OCH_3$ | CH | |
| $CH_3$ | $CH_2S(O)CH_2CF_3$ | H | $OCH_3$ | $CH_3$ | CH | |
| $CH_3$ | $CH_2S(O)CH_2CF_3$ | H | $OCH_3$ | Cl | CH | |
| $CH_3$ | $CH_2S(O)CH_2CF_3$ | H | $CH_3$ | $CH_3$ | CH | |
| $CH_3$ | $CH_2S(O)CH_2CF_3$ | H | $OCH_3$ | $OCH_3$ | N | |
| $CH_2CH_3$ | $CH_2S(O)CH_2CF_3$ | H | $OCH_3$ | $CH_3$ | N | |
| $CH_3$ | $CH_2S(O)CH_2C{\equiv}CH$ | H | $OCH_3$ | $OCH_3$ | CH | |
| $CH_3$ | $CH_2S(O)CH_2C{\equiv}CH$ | H | $OCH_3$ | $CH_3$ | CH | |
| $CH_3$ | $CH_2S(O)CH_2C{\equiv}CH$ | H | $OCH_3$ | Cl | CH | |
| $CH_3$ | $CH_2S(O)CH_2C{\equiv}CH$ | H | $CH_3$ | $CH_3$ | CH | |
| $CH_3$ | $CH_2S(O)CH_2C{\equiv}CH$ | H | $OCH_3$ | $OCH_3$ | N | |
| $CH_3$ | $CH_2S(O)CH_2C{\equiv}CH$ | H | $OCH_3$ | $CH_3$ | N | |
| $CH_3$ | $CH_2S(O)CH_2CH_2OCH_3$ | H | $OCH_3$ | $OCH_3$ | CH | |
| $CH_3$ | $CH_2S(O)CH_2CH_2OCH_3$ | H | $OCH_3$ | $CH_3$ | CH | |
| $CH_3$ | $CH_2S(O)CH_2CH_2OCH_3$ | H | $OCH_3$ | Cl | CH | |
| $CH_3$ | $CH_2S(O)CH_2CH_2OCH_3$ | H | $CH_3$ | $CH_3$ | CH | |
| $CH_3$ | $CH_2S(O)CH_2CH_2OCH_3$ | H | $OCH_3$ | $OCH_3$ | N | |
| $CH_3$ | $CH_2S(O)CH_2CH_2OCH_3$ | H | $OCH_3$ | $CH_3$ | N | |
| $CH_3$ | $CH_2S(O)CH_2C(H){=}CH_2$ | H | $OCH_3$ | $OCH_3$ | CH | |
| $CH_3$ | $CH_2S(O)CH_2C(H){=}CH_2$ | H | $OCH_3$ | $CH_3$ | CH | |
| $CH_3$ | $CH_2S(O)CH_2C(H){=}CH_2$ | H | $OCH_3$ | Cl | CH | |
| $CH_3$ | $CH_2S(O)CH_2C(H){=}CH_2$ | H | $CH_3$ | $CH_3$ | CH | |
| $CH_3$ | $CH_2S(O)CH_2C(H){=}CH_2$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $CH_2S(O)CH_2C(H)=CH_2$ | H | $OCH_3$ | $CH_3$ | N | |
| $CH_3$ | $CH_2S(O)CH_2C(Cl)=CH_2$ | H | $OCH_3$ | $OCH_3$ | CH | |
| $CH_3$ | $CH_2S(O)CH_2C(Cl)=CH_2$ | H | $OCH_3$ | $CH_3$ | CH | |
| $CH_3$ | $CH_2S(O)CH_2C(Cl)=CH_2$ | H | $OCH_3$ | Cl | CH | |
| $CH_3$ | $CH_2S(O)CH_2C(Cl)=CH_2$ | H | $CH_3$ | $CH_3$ | CH | |
| $CH_3$ | $CH_2S(O)CH_2C(Cl)=CH_2$ | H | $OCH_3$ | $OCH_3$ | N | |
| $CH_2CH_3$ | $CH_2S(O)CH_2C(Cl)=CH_2$ | H | $OCH_3$ | $CH_3$ | N | |
| $CH_3$ | $CH_2S(O)_2$-cyclopropyl | H | $OCH_3$ | $OCH_3$ | CH | |
| $CH_3$ | $CH_2S(O)_2$-cyclopropyl | H | $OCH_3$ | $CH_3$ | CH | |
| $CH_3$ | $CH_2S(O)_2$-cyclopropyl | H | $OCH_3$ | Cl | CH | |
| $CH_3$ | $CH_2S(O)_2$-cyclopropyl | H | $CH_3$ | $CH_3$ | CH | |
| $CH_3$ | $CH_2S(O)_2$-cyclopropyl | H | $OCH_3$ | $OCH_3$ | N | |
| $CH_3$ | $CH_2S(O)_2$-cyclopropyl | H | $OCH_3$ | $CH_3$ | N | |
| $CH_3$ | $CH_2S(O)_2CH_2$-cyclopropyl | H | $OCH_3$ | $OCH_3$ | CH | |
| $CH_3$ | $CH_2S(O)_2CH_2$-cyclopropyl | H | $OCH_3$ | $CH_3$ | CH | |
| $CH_3$ | $CH_2S(O)_2CH_2$-cyclopropyl | H | $OCH_3$ | Cl | CH | |
| $CH_3$ | $CH_2S(O)_2CH_2$-cyclopropyl | H | $CH_3$ | $CH_3$ | CH | |
| $CH_3$ | $CH_2S(O)_2CH_2$-cyclopropyl | H | $OCH_3$ | $OCH_3$ | N | |
| $CH_3$ | $CH_2S(O)_2CH_2$-cyclopropyl | H | $OCH_3$ | $CH_3$ | N | |
| $CH_3$ | $CH_2S(O)_2$-cyclobutyl | H | $OCH_3$ | $OCH_3$ | CH | |
| $CH_3$ | $CH_2S(O)_2$-cyclobutyl | H | $OCH_3$ | $CH_3$ | CH | |
| $CH_3$ | $CH_2S(O)_2$-cyclobutyl | H | $OCH_3$ | Cl | CH | |
| $CH_3$ | $CH_2S(O)_2$-cyclobutyl | H | $CH_3$ | $CH_3$ | CH | |
| $CH_3$ | $CH_2S(O)_2$-cyclobutyl | H | $OCH_3$ | $OCH_3$ | N | |
| $CH_2CH_3$ | $CH_2S(O)_2$-cyclobutyl | H | $OCH_3$ | $CH_3$ | N | |
| $CH_3$ | $CH_2S(O)_2CH_2CF_2CF_3$ | H | $OCH_3$ | $OCH_3$ | CH | |
| $CH_3$ | $CH_2S(O)_2CH_2CF_2CF_3$ | H | $OCH_3$ | $CH_3$ | CH | |
| $CH_3$ | $CH_2S(O)_2CH_2CF_2CF_3$ | H | $OCH_3$ | Cl | CH | |
| $CH_3$ | $CH_2S(O)_2CH_2CF_2CF_3$ | H | $CH_3$ | $CH_3$ | CH | |
| $CH_3$ | $CH_2S(O)_2CH_2CF_2CF_3$ | H | $OCH_3$ | $OCH_3$ | N | |
| $CH_3$ | $CH_2S(O)_2CH_2CF_2CF_3$ | H | $OCH_3$ | $CH_3$ | N | |
| $CH_3$ | $CH_2S(O)_2CH(CF_3)CH_3$ | H | $OCH_3$ | $OCH_3$ | CH | |
| $CH_3$ | $CH_2S(O)_2CH(CF_3)CH_3$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $CH_2S(O)_2CH(CF_3)CH_3$ | H | $OCH_3$ | Cl | CH | |
| $CH_3$ | $CH_2S(O)_2CH(CF_3)CH_3$ | H | $CH_3$ | $CH_3$ | CH | |
| $CH_3$ | $CH_2S(O)_2CH(CF_3)CH_3$ | H | $OCH_3$ | $OCH_3$ | N | |
| $CH_3$ | $CH_2S(O)_2CH(CF_3)CH_3$ | H | $OCH_3$ | $CH_3$ | N | |
| $CH_3$ | $CH_2S(O)_2CH_2CF_2CF_2H$ | H | $OCH_3$ | $OCH_3$ | CH | |
| $CH_3$ | $CH_2S(O)_2CH_2CF_2CF_2H$ | H | $OCH_3$ | $CH_3$ | CH | |
| $CH_3$ | $CH_2S(O)_2CH_2CF_2CF_2H$ | H | $OCH_3$ | Cl | CH | |
| $CH_3$ | $CH_2S(O)_2CH_2CF_2CF_2H$ | H | $CH_3$ | $CH_3$ | CH | |
| $CH_3$ | $CH_2S(O)_2CH_2CF_2CF_2H$ | H | $OCH_3$ | $OCH_3$ | N | |
| $CH_2CH_3$ | $CH_2S(O)_2CH_2CF_2CF_2H$ | H | $OCH_3$ | $CH_3$ | N | |
| $CH_3$ | $CH_2S(O)_2CH(CH_2F)_2$ | H | $OCH_3$ | $OCH_3$ | CH | |
| $CH_3$ | $CH_2S(O)_2CH(CH_2F)_2$ | H | $OCH_3$ | $CH_3$ | CH | |
| $CH_3$ | $CH_2S(O)_2CH(CH_2F)_2$ | H | $OCH_3$ | Cl | CH | |
| $CH_3$ | $CH_2S(O)_2CH(CH_2F)_2$ | H | $CH_3$ | $CH_3$ | CH | |
| $CH_3$ | $CH_2S(O)_2CH(CH_2F)_2$ | H | $OCH_3$ | $OCH_3$ | N | |
| $CH_3$ | $CH_2S(O)_2CH(CH_2F)_2$ | H | $OCH_3$ | $CH_3$ | N | |
| $CH_3$ | $CH_2S(O)_2CH(CH_2F)CH_3$ | H | $OCH_3$ | $OCH_3$ | CH | |
| $CH_3$ | $CH_2S(O)_2CH(CH_2F)CH_3$ | H | $OCH_3$ | $CH_3$ | CH | |
| $CH_3$ | $CH_2S(O)_2CH(CH_2F)CH_3$ | H | $OCH_3$ | Cl | CH | |
| $CH_3$ | $CH_2S(O)_2CH(CH_2F)CH_3$ | H | $CH_3$ | $CH_3$ | CH | |
| $CH_3$ | $CH_2S(O)_2CH(CH_2F)CH_3$ | H | $OCH_3$ | $OCH_3$ | N | |
| $CH_3$ | $CH_2S(O)_2CH(CH_2F)CH_3$ | H | $OCH_3$ | $CH_3$ | N | |
| $CH_3$ | $CH_2S(O)_2(CH_2)_3CH_2Cl$ | H | $OCH_3$ | $OCH_3$ | CH | |
| $CH_3$ | $CH_2S(O)_2(CH_2)_3CH_2Cl$ | H | $OCH_3$ | $CH_3$ | CH | |
| $CH_3$ | $CH_2S(O)_2(CH_2)_3CH_2Cl$ | H | $OCH_3$ | Cl | CH | |
| $CH_3$ | $CH_2S(O)_2(CH_2)_3CH_2Cl$ | H | $CH_3$ | $CH_3$ | CH | |
| $CH_3$ | $CH_2S(O)_2(CH_2)_3CH_2Cl$ | H | $OCH_3$ | $OCH_3$ | N | |
| $CH_2CH_3$ | $CH_2S(O)_2(CH_2)_3CH_2Cl$ | H | $OCH_3$ | $CH_3$ | N | |
| $CH_3$ | $CH_2S(O)CH_2CH_2OCH_3$ | H | $OCH_3$ | $OCH_3$ | CH | |
| $CH_3$ | $CH_2S(O)CH_2CH_2OCH_3$ | H | $OCH_3$ | $CH_3$ | CH | |
| $CH_3$ | $CH_2S(O)CH_2CH_2OCH_3$ | H | $OCH_3$ | Cl | CH | |
| $CH_3$ | $CH_2S(O)CH_2CH_2OCH_3$ | H | $CH_3$ | $CH_3$ | CH | |
| $CH_3$ | $CH_2S(O)CH_2CH_2OCH_3$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_3$ | $CH_2S(O)CH_2CH_2OCH_3$ | H | $OCH_3$ | $CH_3$ | N | |
| $CH_3$ | $CH_2S(O)_2CH_2C(Cl)=CH_2$ | H | $OCH_3$ | $OCH_3$ | CH | |
| $CH_3$ | $CH_2S(O)_2CH_2C(Cl)=CH_2$ | H | $OCH_3$ | $CH_3$ | CH | |
| $CH_3$ | $CH_2S(O)_2CH_2C(Cl)=CH_2$ | H | $OCH_3$ | Cl | CH | |
| $CH_3$ | $CH_2S(O)_2CH_2C(Cl)=CH_2$ | H | $CH_3$ | $CH_3$ | CH | |
| $CH_3$ | $CH_2S(O)_2CH_2C(Cl)=CH_2$ | H | $OCH_3$ | $OCH_3$ | N | |
| $CH_3$ | $CH_2S(O)_2CH_2C(Cl)=CH_2$ | H | $OCH_3$ | $CH_3$ | N | |
| $CH_3$ | $CH_2S(O)_2CH_2C{\equiv}CCH_2Cl$ | H | $OCH_3$ | $OCH_3$ | CH | |
| $CH_3$ | $CH_2S(O)_2CH_2C{\equiv}CCH_2Cl$ | H | $OCH_3$ | $CH_3$ | CH | |
| $CH_3$ | $CH_2S(O)_2CH_2C{\equiv}CCH_2Cl$ | H | $OCH_3$ | Cl | CH | |
| $CH_3$ | $CH_2S(O)_2CH_2C{\equiv}CCH_2Cl$ | H | $CH_3$ | $CH_3$ | CH | |
| $CH_3$ | $CH_2S(O)_2CH_2C{\equiv}CCH_2Cl$ | H | $OCH_3$ | $OCH_3$ | N | |
| $CH_2CH_3$ | $CH_2S(O)_2CH_2C{\equiv}CCH_2Cl$ | H | $OCH_3$ | $CH_3$ | N | |
| $CH_3$ | $CH_2S(O)_2CH_2C{\equiv}CH$ | H | $OCH_3$ | $OCH_3$ | CH | |
| $CH_3$ | $CH_2S(O)_2CH_2C{\equiv}CH$ | H | $OCH_3$ | $CH_3$ | CH | |
| $CH_3$ | $CH_2S(O)_2CH_2C{\equiv}CH$ | H | $OCH_3$ | Cl | CH | |
| $CH_3$ | $CH_2S(O)_2CH_2C{\equiv}CH$ | H | $CH_3$ | $CH_3$ | CH | |
| $CH_3$ | $CH_2S(O)_2CH_2C{\equiv}CH$ | H | $OCH_3$ | $OCH_3$ | N | |
| $CH_3$ | $CH_2S(O)_2CH_2C{\equiv}CH$ | H | $OCH_3$ | $CH_3$ | N | |
| $CH_3$ | $CH_2S(O)_2CH_2CN$ | H | $OCH_3$ | $OCH_3$ | CH | |
| $CH_3$ | $CH_2S(O)_2CH_2CN$ | H | $OCH_3$ | $CH_3$ | CH | |
| $CH_3$ | $CH_2S(O)_2CH_2CN$ | H | $OCH_3$ | Cl | CH | |
| $CH_3$ | $CH_2S(O)_2CH_2CN$ | H | $CH_3$ | $CH_3$ | CH | |
| $CH_3$ | $CH_2S(O)_2CH_2CN$ | H | $OCH_3$ | $OCH_3$ | N | |
| $CH_3$ | $CH_2S(O)_2CH_2CN$ | H | $OCH_3$ | $CH_3$ | N | |
| $CH_3$ | $CH_2S(O)_2CH_2CH_2N(CH_3)_2$ | H | $OCH_3$ | $OCH_3$ | CH | |
| $CH_3$ | $CH_2S(O)_2CH_2CH_2N(CH_3)_2$ | H | $OCH_3$ | $CH_3$ | CH | |
| $CH_3$ | $CH_2S(O)_2CH_2CH_2N(CH_3)_2$ | H | $OCH_3$ | Cl | CH | |
| $CH_3$ | $CH_2S(O)_2CH_2CH_2N(CH_3)_2$ | H | $CH_3$ | $CH_3$ | CH | |
| $CH_3$ | $CH_2S(O)_2CH_2CH_2N(CH_3)_2$ | H | $OCH_3$ | $OCH_3$ | N | |
| $CH_2CH_3$ | $CH_2S(O)_2CH_2CH_2N(CH_3)_2$ | H | $OCH_3$ | $CH_3$ | N | |
| $CH_3$ | $CH_2S(O)_2CH_2CH_2F$ | H | $OCH_3$ | $OCH_3$ | CH | |
| $CH_3$ | $CH_2S(O)_2CH_2CH_2F$ | H | $OCH_3$ | $CH_3$ | CH | |

Table I (Continued)

| $R_1$ | $R_2$ | R | X | Y | Z | m.p.(°C) |
|---|---|---|---|---|---|---|
| $CH_3$ | $CH_2S(O)_2CH_2CH_2F$ | H | $OCH_3$ | Cl | CH | |
| $CH_3$ | $CH_2S(O)_2CH_2CH_2F$ | H | $CH_3$ | $CH_3$ | CH | |
| $CH_3$ | $CH_2S(O)_2CH_2CH_2F$ | H | $OCH_3$ | $OCH_3$ | N | |
| $CH_3$ | $CH_2S(O)_2CH_2CH_2F$ | H | $OCH_3$ | $CH_3$ | N | |
| $CH_3$ | $CH_2S(O)_2CH_2CHF_2$ | H | $OCH_3$ | $OCH_3$ | CH | |
| $CH_3$ | $CH_2S(O)_2CH_2CHF_2$ | H | $OCH_3$ | $CH_3$ | CH | |
| $CH_3$ | $CH_2S(O)_2CH_2CHF_2$ | H | $OCH_3$ | Cl | CH | |
| $CH_3$ | $CH_2S(O)_2CH_2CHF_2$ | H | $CH_3$ | $CH_3$ | CH | |
| $CH_3$ | $CH_2S(O)_2CH_2CHF_2$ | H | $OCH_3$ | $OCH_3$ | N | |
| $CH_3$ | $CH_2S(O)_2CH_2CHF_2$ | H | $OCH_3$ | $CH_3$ | N | |
| $CH_3$ | $CH_2S(O)_2CH_2CF_3$ | H | $OCH_3$ | $OCH_3$ | CH | |
| $CH_3$ | $CH_2S(O)_2CH_2CF_3$ | H | $OCH_3$ | $CH_3$ | CH | |
| $CH_3$ | $CH_2S(O)_2CH_2CF_3$ | H | $OCH_3$ | Cl | CH | |
| $CH_3$ | $CH_2S(O)_2CH_2CF_3$ | H | $CH_3$ | $CH_3$ | CH | |
| $CH_3$ | $CH_2S(O)_2CH_2CF_3$ | H | $OCH_3$ | $OCH_3$ | N | |
| $CH_2CH_3$ | $CH_2S(O)_2CH_2CF_3$ | H | $OCH_3$ | $CH_3$ | N | |
| $CH_3$ | $CH_2S(O)_2CH_2CH_2OCH_3$ | H | $OCH_3$ | $OCH_3$ | CH | |
| $CH_3$ | $CH_2S(O)_2CH_2CH_2OCH_3$ | H | $OCH_3$ | $CH_3$ | CH | |
| $CH_3$ | $CH_2S(O)_2CH_2CH_2OCH_3$ | H | $OCH_3$ | Cl | CH | |
| $CH_3$ | $CH_2S(O)_2CH_2CH_2OCH_3$ | H | $CH_3$ | $CH_3$ | CH | |
| $CH_3$ | $CH_2S(O)_2CH_2CH_2OCH_3$ | H | $OCH_3$ | $OCH_3$ | N | |
| $CH_3$ | $CH_2S(O)_2CH_2CH_2OCH_3$ | H | $OCH_3$ | $CH_3$ | N | |
| $CH_3$ | $CH_2S(O)_2CH_2C(H)=CH_2$ | H | $OCH_3$ | $OCH_3$ | CH | |
| $CH_3$ | $CH_2S(O)_2CH_2C(H)=CH_2$ | H | $OCH_3$ | $CH_3$ | CH | |
| $CH_3$ | $CH_2S(O)_2CH_2C(H)=CH_2$ | H | $OCH_3$ | Cl | CH | |
| $CH_3$ | $CH_2S(O)_2CH_2C(H)=CH_2$ | H | $CH_3$ | $CH_3$ | CH | |
| $CH_3$ | $CH_2S(O)_2CH_2C(H)=CH_2$ | H | $OCH_3$ | $OCH_3$ | N | |
| $CH_3$ | $CH_2S(O)_2CH_2C(H)=CH_2$ | H | $OCH_3$ | $CH_3$ | N | |
| $CH_3$ | $CH_2S(O)_2CH_2C(Cl)=CH_2$ | H | $OCH_3$ | $OCH_3$ | CH | |
| $CH_3$ | $CH_2S(O)_2CH_2C(Cl)=CH_2$ | H | $OCH_3$ | $CH_3$ | CH | |
| $CH_3$ | $CH_2S(O)_2CH_2C(Cl)=CH_2$ | H | $OCH_3$ | Cl | CH | |
| $CH_3$ | $CH_2S(O)_2CH_2C(Cl)=CH_2$ | H | $CH_3$ | $CH_3$ | CH | |
| $CH_3$ | $CH_2S(O)_2CH_2C(Cl)=CH_2$ | H | $OCH_3$ | $OCH_3$ | N | |

Table I (Continued)

| $R_1$ | $R_2$ | R | X | Y | Z | m.p.(°C) |
|---|---|---|---|---|---|---|
| $CH_2CH_3$ | $CH_2S(O)_2CH_2C(Cl)=CH_2$ | H | $OCH_3$ | $CH_3$ | N | |
| $CH_3$ | $C(H)=CH_2$ | H | $OCH_3$ | $OCH_3$ | CH | |
| $CH_3$ | $C(H)=CH_2$ | H | $OCH_3$ | $CH_3$ | CH | |
| $CH_3$ | $C(H)=CH_2$ | H | $OCH_3$ | Cl | CH | |
| $CH_3$ | $C(H)=CH_2$ | H | $CH_3$ | $CH_3$ | CH | |
| $CH_3$ | $C(H)=CH_2$ | H | $OCH_3$ | $OCH_3$ | N | |
| $CH_3$ | $C(H)=CH_2$ | H | $OCH_3$ | $CH_3$ | N | |
| $CH_3$ | $C(Cl)=CH_2$ | H | $OCH_3$ | $OCH_3$ | CH | |
| $CH_3$ | $C(Cl)=CH_2$ | H | $OCH_3$ | $CH_3$ | CH | |
| $CH_3$ | $C(Cl)=CH_2$ | H | $OCH_3$ | Cl | CH | |
| $CH_3$ | $C(Cl)=CH_2$ | H | $CH_3$ | $CH_3$ | CH | |
| $CH_3$ | $C(Cl)=CH_2$ | H | $OCH_3$ | $OCH_3$ | N | |
| $CH_3$ | $C(Cl)=CH_2$ | H | $OCH_3$ | $CH_3$ | N | |
| $CH_3$ | $CH_2C(O)NHCH_3$ | H | $OCH_3$ | $OCH_3$ | CH | |
| $CH_3$ | $CH_2C(O)NHCH_3$ | H | $OCH_3$ | $CH_3$ | CH | |
| $CH_3$ | $CH_2C(O)NHCH_3$ | H | $OCH_3$ | Cl | CH | |
| $CH_3$ | $CH_2C(O)NHCH_3$ | H | $CH_3$ | $CH_3$ | CH | |
| $CH_3$ | $CH_2C(O)NHCH_3$ | H | $OCH_3$ | $OCH_3$ | N | |
| $CH_3$ | $CH_2C(O)NHCH_3$ | H | $OCH_3$ | $CH_3$ | N | |
| $CH_3$ | $CH_2C(O)N(CH_3)_2$ | H | $OCH_3$ | $OCH_3$ | CH | |
| $CH_3$ | $CH_2C(O)N(CH_3)_2$ | H | $OCH_3$ | $CH_3$ | CH | |
| $CH_3$ | $CH_2C(O)N(CH_3)_2$ | H | $OCH_3$ | Cl | CH | |
| $CH_3$ | $CH_2C(O)N(CH_3)_2$ | H | $CH_3$ | $CH_3$ | CH | |
| $CH_3$ | $CH_2C(O)N(CH_3)_2$ | H | $OCH_3$ | $OCH_3$ | N | |
| $CH_3$ | $CH_2C(O)N(CH_3)_2$ | H | $OCH_3$ | $CH_3$ | N | |
| $CH_3$ | $OCF_3$ | H | $CH_3$ | $OCH_3$ | CH | |
| $CH_3$ | $OCF_3$ | H | $OCH_3$ | $OCH_3$ | CH | |

Table I (Continued)

| $R_1$ | $R_2$ | R | X | Y | Z | m.p.(°C) |
|---|---|---|---|---|---|---|
| $CH_3$ | $OCF_3$ | H | Cl | $OCH_3$ | CH | |
| $CH_3$ | $OCF_3$ | H | $CH_3$ | $OCH_3$ | N | |
| $CH_3$ | $OCF_3$ | H | $OCH_3$ | $OCH_3$ | N | |
| $CH_3$ | $SCF_3$ | H | $CH_3$ | $OCH_3$ | CH | |
| $CH_3$ | $SCF_3$ | H | $OCH_3$ | $OCH_3$ | CH | |
| $CH_3$ | $SCF_3$ | H | Cl | $OCH_3$ | CH | |
| $CH_3$ | $SCF_3$ | H | $CH_3$ | $OCH_3$ | N | |
| $CH_3$ | $SCF_3$ | H | $OCH_3$ | $OCH_3$ | N | |
| $CH_3$ | $OCH_2CH_2Br$ | H | $CH_3$ | $OCH_3$ | CH | |
| $CH_3$ | $OCH_2CH_2I$ | H | $OCH_3$ | $OCH_3$ | CH | |
| $CH_3$ | $C{\equiv}CI$ | H | $CH_3$ | $OCH_3$ | N | |
| $CH_3$ | $CH_2CBr_3$ | H | $OCH_3$ | $OCH_3$ | CH | |
| $CH_3$ | $N(CH_3)(CH_2CH_3)$ | H | $CH_3$ | $OCH_3$ | CH | |
| $CH_3$ | $CH_2Cl$ | H | $CH_3$ | $CH_3$ | CH | 176-180(d) |
| $CH_3$ | $CH_2Cl$ | H | $CH_3$ | $OCH_3$ | CH | 167-170 |
| $CH_3$ | $CH_2Cl$ | H | $OCH_3$ | $OCH_3$ | CH | 149-153(d) |
| $CH_3$ | $CH_2Cl$ | H | Cl | $OCH_3$ | CH | 164-166 |
| $CH_3$ | $CH_2Cl$ | H | $CH_3$ | $OCH_3$ | N | 148-150 |
| $CH_3$ | $CH_2Cl$ | H | $OCH_3$ | $OCH_3$ | N | 161-162 |
| $CH_2CH_3$ | $CH_2Cl$ | H | $CH_3$ | $CH_3$ | CH | 158-162 |
| $CH_2CH_3$ | $CH_2Cl$ | H | $CH_3$ | $OCH_3$ | CH | |
| $CH_2CH_3$ | $CH_2Cl$ | H | $OCH_3$ | $OCH_3$ | CH | 141-144 |
| $CH_2CH_3$ | $CH_2Cl$ | H | Cl | $OCH_3$ | CH | 158-162 |
| $CH_2CH_3$ | $CH_2Cl$ | H | $CH_3$ | $OCH_3$ | N | 134-142 |
| $CH_2CH_3$ | $CH_2Cl$ | H | $OCH_3$ | $OCH_3$ | N | Gum |
| $CH_2CH_3$ | $CHBr_2$ | H | $OCH_3$ | $OCH_3$ | CH | 181-182 |
| $CH_3$ | $CHBr_2$ | H | $OCH_3$ | $OCH_3$ | CH | |
| $CH_3$ | $CH_2Br$ | H | $OCH_3$ | $OCH_3$ | CH | |
| $CH_3$ | $CHCl_2$ | H | $OCH_3$ | $OCH_3$ | CH | |
| $CH_3$ | $OC(O)CH_3$ | H | $CH_3$ | $CH_3$ | CH | |
| $CH_3$ | $OC(O)CH_3$ | H | $CH_3$ | $OCH_3$ | CH | |
| $CH_3$ | $OC(O)CH_3$ | H | $OCH_3$ | $OCH_3$ | CH | 188-190 |
| $CH_3$ | $OC(O)CH_3$ | H | Cl | $OCH_3$ | CH | |

**Table I (Continued)**

| $R_1$ | $R_2$ | R | X | Y | Z | m.p.(°C) |
|---|---|---|---|---|---|---|
| $CH_3$ | $OC(O)CH_3$ | H | $CH_3$ | $OCH_3$ | N | |
| $CH_3$ | $OC(O)CH_3$ | H | $OCH_3$ | $OCH_3$ | N | |

## Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

**Table X**

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0° C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended

uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science". John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 10
Wettable Powder
4-(2,2,2-trifluoroethylthio)-2-[[(4,6-dimethoxypryrimidin-2-yl)aminocarbonyl]aminosulfonyl]benzoic acid, methyl ester 80%
sodium alkylnaphthalenesulfonate 2%
sodium ligninsulfonate 2%
synthetic amorphous silica 3%
kaolinite 13%

The ingredients are blended, hammer-milled until all the solids are essentially under 50 microns, reblended, and packaged.

Example 11
Wettable Powder
4-(2,2,2-trifluoroethylthio)-2-[[(4,6-dimethoxypyrimidin-2-yl)aminocarbonyl]aminosulfonyl]benzoic acid, methyl ester 50%
sodium alkylnaphthalenesulfonate 2%
low viscosity methyl cellulose 2%
diatomaceous earth 46%

The ingredients are blended, coarsely hammer-milled and then air-milled to produce particles essentially all below 10 microns in diameter. The product is reblended before packaging.

Example 12
Granule
Wettable Powder of Example 11 5%
attapulgite granules 95%
(U.S.S. 20-40 mesh; 0.84-0.42 mm)

A slurry of wettable powder containing 25% solids is sprayed on the surface of attapulgite granules while tumbling in a double-cone blender. The granules are dried and packaged.

Example 13
Extruded Pellet
4-(2,2,2-trifluoroethylthio)-2-[[(4,6-dimethoxypyrimidin-2-yl)aminocarbonyl]aminosulfonyl]benzoic acid, methyl ester 25%
anhydrous sodium sulfate 10%

crude calcium ligninsulfonate 5%
sodium alkylnaphthalenesulfonate 1%
calcium/magnesium bentonite 59%

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 14
Oil Suspension
4-(2,2,2-trifluoroethylthio)-2-[[(4,6-dimethoxypyrimidin-2-yl)aminocarbonyl]aminosulfonyl]benzoic acid, methyl ester 25%
polyoxyethylene sorbitol hexaoleate 5%
highly aliphatic hydrocarbon oil 70%

The ingredients are ground together in a sand mill until the solid particles have been reduced to under about 5 microns. The resulting thick suspension may be applied directly, but preferably after being extended with oils or emulsified in water.

Example 15
Wettable Powder
4-(2,2,2-trifluoroethylthio)-2-[[(4,6-dimethoxypyrimidin-2-yl)aminocarbonyl]aminosulfonyl]benzoic acid methyl ester 20%
sodium alkylnaphthalenesulfonate 4%
sodium ligninsulfonate 4%
low viscosity methyl cellulose 3%
attapulgite 69%

The ingredients are thoroughly blended. After grinding in a hammer-mill to produce particles essentially all below 100 microns, the material is reblended and sifted through a U.S.S. No. 50 sieve (0.3 mm opening) and packaged.

Example 16
Low Strength Granule
4-(2,2,2-trifluoroethylthio)-2-[[(4,6-dimethoxypyrimidin-2-yl)aminocarbonyl]aminosulfonyl]benzoic acid, methyl ester 1%
N,N-dimethylformamide 9%
attapulgite granules 90%
(U.S.S. 20-40 sieve)

The active ingredient is dissolved in the solvent and the solution is sprayed upon dedusted granules in a double cone blender. After spraying of the solution has been completed, the blender is allowed to run for a short period and then the granules are packaged.

Example 17
Aqueous Suspension
4-(2,2,2-trifluoroethylthio)-2-[[(4,6-dimethoxypyrimidin-2-yl)aminocarbonyl]aminosulfonyl]benzoic acid, methyl ester 40%
polyacrylic acid thickener 0.3%
dodecylphenol polyethylene glycol ether 0.5%
disodium phosphate 1%
monosodium phosphate 0.5%
polyvinyl alcohol 1.0%
water 56.7%

The ingredients are blended and ground together in a sand mill to produce particles essentially all

under 5 microns in size.

Example 18
Solution
4-(2,2,2-trifluoroethylthio)-2-[[(4,6-dimethoxypyrimidin-2-yl)aminocarbonyl]aminosulfonyl]benzoic acid, methyl ester 5%
water 95%

The salt is added directly to the water with stirring to produce the solution, which may then be packaged for use.

Example 19
Low Strength Granule
4-(2,2,2-trifluoroethylthio)-2-[[(4,6-dimethoxypyrimidin-2-yl)aminocarbonyl]aminosulfonyl]benzoic acid, methyl ester 0.1%
attapulgite granules 99.9%
(U.S.S. 20-40 mesh)

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 20
Granule
4-(2,2,2-trifluoroethylthio)-2-[[(4,6-dimethoxypyrimidin-2-yl)aminocarbonyl]aminosulfonyl]benzoic acid, methyl ester 80%
wetting agent 1%
crude ligninsulfonate salt (containing 5-20% of the natural sugars) 10%
attapulgite clay 9%

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 21
High Strength Concentrate
4-(2,2,2-trifluoroethylthio)-2-[[(4,6-dimethoxypyrimidin-2-yl)aminocarbonyl]aminosulfonyl]benzoic acid, methyl ester 99%
silica aerogel 0.5%
synthetic amorphous silica 0.5%

The ingredients are blended and ground in a hammer-mill to produce a material essentially all passing a U.S.S. No. 50 screen (0.3 mm opening). The concentrate may be formulated further if necessary.

Example 22
Wettable Powder
4-(2,2,2-trifluoroethylthio)-2-[[(4,6-dimethoxypyrimidin-2-yl)aminocarbonyl]aminosulfonyl]benzoic acid, methyl ester 90%
dioctyl sodium sulfosuccinate 0.1%
synthetic fine silica 9.9%

The ingredients are blended and ground in a hammer-mill to produce particles essentially all below 100 microns. The material is sifted through a U.S.S. No. 50 screen and then packaged.

Example 23

Wettable Powder

4-(2,2,2-trifluoroethylthio)-2-[[(4,6-dimethoxypyrimidin-2-yl)aminocarbonyl]aminosulfonyl]benzoic acid, methyl ester 40%
sodium ligninsulfonate 20%
montmorillonite clay 40%

The ingredients are thoroughly blended, coarsely hammer-milled and then air-milled to produce particles essentially all below 10 microns in size. The material is reblended and then packaged.

Example 24

Oil Suspension

4-(2,2,2-trifluoroethylthio)-2-[[(4,6-dimethoxypyrimidin-2-yl)aminocarbonyl]aminosulfonyl]benzoic acid, methyl ester 35%
blend of polyalcohol carboxylic esters and oil soluble petroleum sulfonates 6%
xylene 59%

The ingredients are combined and ground together in a sand mill to produce particles essentially all below 5 microns. The product can be used directly, extended with oils, or emulsified in water.

Example 25

Dust

4-(2,2,2-trifluoroethylthio)-2-[[(4,6-dimethoxypyrimidin-2-yl)aminocarbonyl]aminosulfonyl]benzoic acid, methyl ester 10%
attapulgite 10%
pyrophyllite 80%

The active ingredient is blended with attapulgite and then passed through a hammer-mill to produce particles substantially all below 200 microns. The ground concentrate is then blended with powdered pyrophyllite until homogeneous.

Example 26

Emulsifiable Concentrate

4-(2,2,2-trifluoroethylthio)-2-[[(4,6-dimethoxypyrimidin-2-yl)aminocarbonyl]aminosulfonyl]benzoic acid, methyl ester 20%
chlorobenzene 74%
sorbitan monostearate and polyoxyethylene condensates thereof 6%

The ingredients are combined and stirred to produce a solution which can be emulsified in water for application.

Utility

Test results indicate that the compounds of the present invention are highly active preemergent or postemergent herbicides or plant growth regulants. Many of them have utility for broad-spectrum pre-and/or postemergence weed control in areas where complete control of all vegetation is desired, such as around fuel storage tanks, ammunition depots, industrial storage areas, parking lots, drive-in theaters, around billboards, highway and railroad structures. Some of the compounds have utility for selective weed control in crops such as wheat, barley, corn, cotton, soybean, sugar beets, and rice. With regard to usage in rice, this includes especially Japonica rice or paddy rice. The compound identified herein as Compound 88 is especially effective in control of undesired vegetation in paddy rice and the control of blackgrass in wheat and barley. Alternatively, the subject compounds are useful to modify plant growth.

The rates of application for the compounds of the invention are determined by a number of factors, including their use as plant growth modifiers or as herbicides, the crop species involved, the types of weeds to be controlled, weather and climate, formulations selected, mode of application, amount of foliage present, etc. In general terms, the subject compounds should be applied at levels of around 0.004 to 10 kg/ha, the lower rates being suggested for use on lighter soils and/or those having a low organic matter content, for plant growth modification or for situations where only short-term persistence is required.

The compounds of the invention may be used in combination with any other commercial herbicides.

They are particularly useful in combination with the following herbicides.

| Common Name | Chemical Name |
|---|---|
| acifluorfen | 5-[2-chloro-4-(trifluoromethyl)phenoxy]-2-nitrobenzoic acid |
| acrolein | acrolein |
| alachlor | 2-chloro-2',6'-diethyl-N-(methoxymethyl)-acetanilide |
| ametryn | 2-(ethylamino)-4-(isopropylamino)-6-methylthio)-s-triazine |
| amitrole | 3-amino-s-triazole |
| AMS | ammonium sulfamate |
| asulam | methyl sulfanilylcarbamate |
| atrazine | 2-chloro-4-(ethylamino)-6-(isopropyl-amino)-s-triazine |
| barban | 4-chloro-2-butynyl m-chlorocarbanilate |
| benefin | N-butyl-N-ethyl-α,α,α-trifluoro-2,6-dinitro-p-toluidine |
| bensulide | O,O-diisopropyl phosphorodithioate S-ester with N-(2-mercaptoethyl)-benzenesulfonamide |
| bentazon | 3-isopropyl-1H-2,1,3-benzothiadiazin-4(3H)-one 2,2-dioxide |
| benzipram | 3,5-dimethyl-N-(1-methylethyl)-N-(phenylmethyl)benzamide |
| benzoylprop | N-benzoyl-N-(3,4-dichlorophenyl)-DL-alaine |
| bifenox | methyl 5-(2,4-dichlorophenoxy)-2-nitrobenzoate |
| bromacil | 5-bromo-3-sec-butyl-6-methyluracil |
| bromoxynil | 3,5-dibromo-4-hydroxybenzonitrile |
| butachlor | N-(butoxymethyl)-2-chloro-2',6'-diethylacetanilide |
| butam | 2,2-dimethyl-N-(1-methylethyl)-N-(phenylmethyl)propanamide |

| Common Name | Chemical Name |
|---|---|
| buthidazole | 3-[5-(1,1-dimethylethyl)-1,3,4-thiadiazol-2-yl]-4-hydroxy-1-methyl-2-imidazolidinone |
| butralin | 4-(1,1-dimethylethyl)-N-(1-methylpropyl)-2,6-dinitrobenzenamine |
| butylate | S-ethyl-diisobutylthiocarbamate |
| cacodylic acid | hydroxydimethylarsine oxide |
| carbetamide | D-N-ethyllactamide carbanilate (ester) |
| CDAA | N-N-diallyl-2-chloroacetamide |
| CDEC | 2-chloroallyl diethyldithiocarbamate |
| chlorbromuron | 3-(4-bromo-3-chlorophenyl)-1-methoxy-1-methylurea |
| chloroxuron | 3-[p-(p-chlorophenoxy)phenyl]-1,1-dimethylurea |
| chlorpropham | isopropyl m-chlorocarbanilate |
| chlorsulfuron | 2-chloro-N-[(4-methoxy-6-methyl-1,3,5-triazin-2-yl)aminocarbonyl]benzenesulfonamide |
| chlortoluron | N'-(3-chloro-4-methylphenyl-N',N'-dimethylurea |
| cisanilide | cis-2,5-dimethyl-N-phenyl-1-pyrrolidinecarboxamide |
| CMA | calcium methanearsonate |
| cyanazine | 2-[[4-chloro-6-(ethylamino)-s-triazin-2-yl]amino]-2-methylpropionitrile |
| cycloate | S-ethyl N-ethylthiocyclohexanecarbamate |
| cycluron | 3-cyclooctyl-1,1-dimethylurea |
| cyperquat | 1-methyl-4-phenylpyridinium |
| cyprazine | 2-chloro-4-(cyclopropylamino)-6-(isopropylamino)-s-triazine |

| Common Name | Chemical Name |
| --- | --- |
| cyprazole | N-[5-(2-chloro-1,1-dimethylethyl)-1,3,4-thiadiazol-2-yl] cyclopropanecarboxamide |
| cypromid | 3',4'-dichlorocyclopropanecarboxanilide |
| dalapon | 2,2-dichloropropionic acid |
| dazomet | tetrahydro-3,5-dimethyl-2H-1,3,5-thiadiazine-2-thione |
| DCPA | dimethyl tetrachloroterephthalate |
| desmetryn | 2-(isopropylamino)-4-(methylamino)-6-methylthio)-s-triazine |
| diallate | S-(2,3-dichloroallyl)diisopropylthiocarbamate |
| dicamba | 3,6-dichloro-o-anisic acid |
| dichlobenil | 2,6-dichlorobenzonitrile |
| dichlorprop | 2-(2,4-dichlorophenoxy)propionic acid |
| diclofop | 2-[4-(2,4-dichlorophenoxy)phenoxy]-propanoic acid |
| diethatyl | N-(chloroacetyl)-N-(2,6-diethylphenyl)-glycine |
| difenzoquat | 1,2-dimethyl-3,5-diphenyl-1H-pyrazolium |
| dinitramine | $N^4$,$N^4$-diethyl-α,α,α-trifluoro-3,5-dinitrotoluene-2,4-diamine |
| dinoseb | 2-sec-butyl-4,6-dinitrophenol |
| diphenamide | N,N-dimethyl-2,2-diphenylacetamide |
| dipropetryn | 2-(ethylthio)-4,6-bis(isopropylamino)-s-triazine |
| diquat | 6,7-dihydrodipyrido[1,2-α:2',1'-c]-pyrazinediium ion |
| diuron | 3-(3,4-dichlorophenyl)-1,1-dimethylurea |
| DSMA | disodium methanearsonate |

| Common Name | Chemical Name |
|---|---|
| endothall | 7-oxabicyclo[2.2.1]heptane-2,3-dicarboxylic acid |
| erbon | 2-(2,4,5-trichlorophenoxy)ethyl 2,2-dichloropropionate |
| ethafluralin | N-ethyl-N-(2-methyl-2-propenyl)-2,6-dinitro-4-(trifluoromethyl)benzenamine |
| ethofumesate | (±)-2-ethoxy-2,3-dihydro-3,3-dimethyl-5-benzofuranyl methanesulfonate |
| fenac | (2,3,6-trichlorophenyl)acetic acid |
| fenoxaprop ethyl | ethyl 2-(4-(6-chloro-2-benzoxazolyloxy)phenoxy)propanoate |
| fenuron | 1,1-dimethyl-3-phenylurea |
| fenuron TCA | 1,1-dimethyl-3-phenylurea mono(trichloroacetate) |
| flamprop | N-benzoyl-N-(3-chloro-4-fluorophenyl)-DL-alanine |
| fluchloralin | N-(2-chloroethyl)-2,6-dinitro-N-propyl-4-(trifluoromethyl)aniline |
| fluometuron | 1,1-dimethyl-3-(α,α,α-trifluoro-m-tolyl)-urea |
| fluorodifen | p-nitrophenyl α,α,α-trifluoro-2-nitro-p-tolyl ether |
| fluridone | 1-methyl-3-phenyl-5-[3-(trifluoromethyl)phenyl]-4(1H)-pyridinone |
| fomesafen | 5-(2-chloro-4-trifluoromethylphenoxy)-N-methylsulfonyl-2-nitrobenzamide |
| fosamine | ethyl hydrogen (aminocarbonyl)phosphonate |
| glyphosate | N-(phosphonomethyl)glycine |
| hexaflurate | potassium hexafluoroarsenate |
| hexazinone | 3-cyclohexyl-6-(dimethylamino)-1-methyl-1,3,5-triazine-2,4(1H,3H)-dione |

| Common Name | Chemical Name |
|---|---|
| imazaquin | 2-(4,5-dihydro-4-methyl-4-(1-methylethyl)-5-oxo-1H-imidazol-2-yl)-3-quinolinecarboxylic acid |
| ioxynil | 4-hydroxy-3,5-diiodobenzonitrile |
| isopropalin | 2,6-dinitro-N,N-dipropylcumidine |
| karbutilate | tert-butylcarbamic acid ester with 3-(m-hydroxyphenyl)-1,1-dimethylurea |
| lactofen | 1'-(carboethoxy)ethyl-5-(2-chloro-4-(trifluoromethyl)phenoxy)-2-nitrobenzoate |
| lenacil | 3-cyclohexyl-6,7-dihydro-1H-cyclopentapyrimidine-2,4(3H,5H)-dione |
| linuron | 3-(3,4-dichlorophenyl)-1-methoxy-1-methylurea |
| MAA | methanearsonic acid |
| MAMA | monoammonium methanearsonate |
| MCPA | [(4-chloro-o-tolyl)oxy]acetic acid |
| MCPB | 4-[(4-chloro-o-tolyl)oxy]butyric acid |
| mecoprop | 2-[(4-chloro-o-tolyl)oxy]propionic acid |
| mefluidide | N-[(2,4-dimethyl-5-[[(trifluoromethyl)-sulfonyl]amino]phenyl]acetamide |
| methalpropalin | N-(2-methyl-2-propenyl)-2,6-dinitro-N-propyl-4-(trifluoromethyl)benzenamide |
| methabenzthiazuron | 1,3-dimethyl-3-(2-benzothiazolyl)urea |
| metham | sodium methyldithiocarbamate |
| methazole | 2-(3,4-dichlorophenyl)-4-methyl-1,2,4-oxadiazolidine-3,5-dione |
| methoxuron | N'-(3-chloro-4-methoxyphenyl)N,N-dimethylurea |
| metolachlor | 2-chloro-N-(2-ethyl-6-methylphenyl)-N-(2-methoxy-1-methylethyl)acetamide |

| Common Name | Chemical Name |
|---|---|
| metribuzin | 4-amino-6-tert-butyl-3-(methylthio)-as-triazine-5(4H)-one |
| metsulfuron methyl | 2-[[(4-methoxy-6-methyl-1,3,5-triazine-2-yl)aminocarbonyl]aminosulfonyl]benzoic acid, methyl ester |
| molinate | S-ethyl hexahydro-1H-azepine-1-carbothioate |
| monolinuron | 3-(p-chlorophenyl)-1-methoxy-1-methylurea |
| monuron | 3-(p-chlorophenyl)-1,1-dimethylurea |
| monuron TCA | 3-(p-chlorophenyl)-1,1-dimethylurea mono(trichloroacetate) |
| MSMA | monosodium methanearsonate |
| napropamide | 2-(α-naphthoxy)-N,N-diethylpropionamide |
| naptalam | N-1-naphthylphthalamic acid |
| neburon | 1-butyl-3-(3,4-dichlorophenyl)-1-methylurea |
| nitralin | 4-(methylsulfonyl)-2,6-dinitro-N,N-dipropylaniline |
| nitrofen | 2,4-dichlorophenyl p-nitrophenyl ether |
| nitrofluorfen | 2-chloro-1-(4-nitrophenoxy)-4-(trifluoromethyl)benzene |
| norea | 3-(hexahydro-4,7-methanoindan-5-yl)-1,1-dimethylurea |
| norflurazon | 4-chloro-5-(methylamino)-2-(α,α,α-trifluoro-m-tolyl)-3(2H)-pyridazinone |
| oryzalin | 3,4-dinitro-N,N-dipropylsulfanilamide |
| oxadiazon | 2-tert-butyl-4-(2,4-dichloro-5-isopropoxyphenyl)$\Delta^2$-1,3,4-oxadiazolin-5-one |
| oxyfluorfen | 2-chloro-1-(3-ethoxy-4-nitrophenoxy)-4-(trifluoromethyl)benzene |

| Common Name | Chemical Name |
|---|---|
| paraquat | 1,1'-dimethyl-4,4'-bipyridinium ion |
| PBA | chlorinated benzoic acid |
| pendimethalin | N-(1-ethylpropyl)-3,4-dimethyl-2,6-dinitrobenzenamine |
| perfluidone | 1,1,1-trifluoro-N-[2-methyl-4-(phenylsulfonyl)phenyl]methanesulfonamide |
| picloram | 4-amino-3,5,6-trichloropicolinic acid |
| procyazine | 2-[[4-chloro-6-(cyclopropylamino)-1,3,5-triazine-2-yl]amino]-2-methylpropanenitrile |
| profluralin | N-(cyclopropylmethyl)-α,α,α-trifluoro-2,6-dinitro-N-propyl-p-toluidine |
| prometon | 2,4-bis(isopropylamino)-6-methoxy-s-triazine |
| prometryn | 2,4-bis(isopropylamino)-6-(methylthio)-s-triazine |
| pronamide | 3,5-dichloro N-(1,1-dimethyl-2-propynyl)benzamide |
| propachlor | 2-chloro-N-isopropylacetanilide |
| propanil | 3',4'-dichloropropionalide |
| propazine | 2-chloro-4,6-bis(isopropylamino)-s-triazine |
| propham | isopropyl carbanilate |
| prosulfalin | N-[[4-(dipropylamino)-3,5-dinitrophenyl]sulfonyl]-S,S-dimethylsulfilimine |
| prynachlor | 2-chloro-N-(1-methyl-2-propynyl)acetanilide |
| quinofop ethyl | 2-[4-(6-chloroquinoxalin-2-yloxy)phenoxypropanoic acid, ethyl ester |
| secbumeton | N-ethyl-6-methoxy-N'(1-methylpropyl)-1,3,5-triazine-2,4-diamine |

| Common Name | Chemical Name |
|---|---|
| sethoxydim | 2-[1-(ethoxyimino)butyl]-5-[2-(ethylthio)propyl]-3-hydroxy-2-cyclohexene-1-one |
| siduron | 1-(2-methylcyclohexyl)-3-phenylurea |
| simazine | 2-chloro-4,6-bis(ethylamino)-s-triazine |
| simetryn | 2,4-bis(ethylamino)-6-(methylthio)-s-triazine |
| supriox | 2-[1-(2,5-dimethylphenyl)ethylsulfonyl]-pyridine-N-oxide |
| TCA | trichloroacetic acid |
| tebuthiuron | N-[5-(1,1-dimethylethyl)-1,3,4-thiadiazol-2-yl]-N,N'-dimethylurea |
| terbacil | 3-tert-butyl-5-chloro-6-methyluracil |
| terbuchlor | N-(butoxymethyl)-2-chloro-N-[2-(1,1-dimethylethyl)-6-methylphenyl]-acetamide |
| terbuthylazine | 2-(tert-butylamino)-4-chloro-6-(ethylamino)-s-triazine |
| terbutol | 2,6-di-tert-butyl-p-tolyl methylcarbamate |
| terbutryn | 2-(tert-butylamino)-4-(ethylamino)-6-(methylthio)-s-triazine |
| tetrafluron | N,N-dimethyl-N'-[3-(1,1,2,2-tetrafluoroethoxy)phenyl]urea |
| thiobencarb | S-[(4-chlorophenyl)methyl] diethylcarbamothioate |
| triallate | S-(2,3,3-trichloroallyl)diisopropylthiocarbamate |
| trifluralin | α,α,α-trifluoro-2,6-dinitro-N,N-propyl-p-toluidine |
| trimeturon | 1-(p-chlorophenyl)-2,3,3-trimethylpseudourea |
| vernolate | S-propyl dipropylthiocarbamate<br>ethyl 5-[2-chloro-4-(trifluoromethyl)-phenoxy]-2-nitrobenzoic acid |

| Common Name | Chemical Name |
|---|---|
| 2,3,6-TBA[b] | 2,3,6-trichlorobenzoic acid |
| 2,4-D | (2,4-dichlorophenoxy)acetic acid |
| 2,4-DB | 4-(2,4-dichlorophenoxy)butyric acid |
| 2,4-DEP | tris[2-(2,4-dichlorophenoxy)ethyl] phosphite |

| Trade Name or Code Number | Chemical Name |
|---|---|
| "Cinch" | exo-1-methyl-4-(1-methylethyl)-2-[(2-methylphenyl)methoxy]-7-oxabicyclo-[2.2.1]heptane |
| AC 263,499 | 2-[4,5-dihydro-4-methyl-4-(1-methylethyl)-5-oxo-1H-imidazol-2-yl]-5-ethyl-3-pyridinecarboxylic acid |
| Harmony™ | 3-[[(4-methoxy-6-methyl-1,3,5-triazin-2-yl)aminocarbonyl]aminosulfonyl]-2-thiophenecarboxylic acid, methyl ester |
| PPG-1013 | 5-(2-chloro-4-(trifluoromethyl)phenoxy)-2-nitroacetophenone oxime-O-acetic acid, methyl ester |
| DOWCO 453 ME | 2-(4-(3-chloro-5-trifluoromethylpyridin-2-yloxy)phenoxy)propanoic acid, methyl ester |
| FMC 57020 | 2-(2'-chlorophenyl)methyl-4,4-dimethyl-3-isoxazolidinone |
| AC 222,293 | 6-(4-isopropyl-4-methyl-5-oxo-2-imidazolin-2-yl)-*m*-toluic acid, methyl ester and 6-(4-isopropyl-4-methyl-5-oxo-2-imidazolin-2-yl-*p*-toluic acid, methyl ester |
| AC 252,925 | 2-(4-isopropyl-4-methyl-5-oxo-2-imidazolin-2-yl)nicotinic acid with isopropyl amine (1:1) |
| DPX-L5300 | 2-[[N-(4-methoxy-6-methyl-1,3,5-triazine-2-yl)-N-methylaminocarbonyl]-aminosulfonyl]benzoic acid, methyl ester |

Compounds

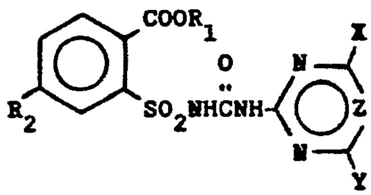

| Cmpd. No. | $R_1$ | $R_2$ | X | Y | Z | m.p.(°C) |
|---|---|---|---|---|---|---|
| 1 | $CH_2CH_3$ | $CH_2Cl$ | $OCH_3$ | $OCH_3$ | CH | 141-144 |
| 2 | $CH_2CH_3$ | $CH_2Cl$ | $CH_3$ | $CH_3$ | CH | 158-162 |
| 3 | $CH_2CH_3$ | $CH_2Cl$ | Cl | $OCH_3$ | CH | 158-162 |
| 4 | $CH_2CH_3$ | $CH_2Cl$ | $OCH_3$ | $OCH_3$ | N | gum |
| 5 | $CH_2CH_3$ | $CH_2Cl$ | $CH_3$ | $OCH_3$ | N | 134-142 |
| 6 | $CH_3$ | $OCH_2$ cyclopropyl | $OCH_3$ | $OCH_3$ | CH | 183-185(dec) |
| 7 | $CH_3$ | $OCH_2$ cyclopropyl | $CH_3$ | $OCH_3$ | N | 153-156(dec) |
| 8 | $CH_3$ | $OCH_2$ cyclopropyl | Cl | $OCH_3$ | CH | 146-149(dec) |
| 9 | $CH(CH_3)_2$ | $CH_2Cl$ | $OCH_3$ | $OCH_3$ | CH | 146-148 |
| 10 | $CH(CH_3)_2$ | $CH_2Cl$ | $CH_3$ | $OCH_3$ | CH | 147-148 |
| 11 | $CH(CH_3)_2$ | $CH_2Cl$ | $CH_3$ | $CH_3$ | CH | 166-169 |
| 12 | $CH(CH_3)_2$ | $CH_2Cl$ | Cl | $OCH_3$ | CH | 171-173 |
| 13 | $CH(CH_3)_2$ | $CH_2Cl$ | $OCH_3$ | $OCH_3$ | N | 76-81 |
| 14 | $CH(CH_3)_2$ | $CH_2Cl$ | $CH_3$ | $OCH_3$ | N | 57-60 |
| 15 | $CH_2CH_3$ | $SCH_2CF_3$ | $OCH_3$ | $OCH_3$ | N | 147-151 |
| 16 | $CH_2CH_3$ | $SCH_2CF_3$ | $CH_3$ | $OCH_3$ | N | 136-142 |
| 17 | $CH_2CH_3$ | $SCH_2CF_3$ | $OCH_3$ | $OCH_3$ | CH | 127-130 |
| 18 | $CH_2CH_3$ | $SCH_2CF_3$ | $CH_3$ | $OCH_3$ | CH | 139-142 |
| 19 | $CH_2CH_3$ | $SCH_2CF_3$ | Cl | $OCH_3$ | CH | 98-106 |
| 20 | $CH_2CH_3$ | $SCH_2CF_3$ | $CH_3$ | $CH_3$ | CH | 166-170 |
| 21 | $CH_2CH_3$ | $SCH_2CF_3$ | $OCH_2CH_3$ | $NHCH_3$ | N | 147-168 |
| 22 | $CH_3$ | $SCH_2CF_3$ | $OCH_3$ | $OCH_3$ | N | 150-152 |
| 23 | $CH_3$ | $SCH_2CF_3$ | $CH_3$ | $OCH_3$ | N | 142-162 |
| 24 | $CH_3$ | $SCH_2CF_3$ | $OCH_3$ | $OCH_3$ | CH | 171-173 |
| 25 | $CH_3$ | $SCH_2CF_3$ | $CH_3$ | $OCH_3$ | CH | 143-147 |
| 26 | $CH_3$ | $SCH_2CF_3$ | Cl | $OCH_3$ | CH | 139-147 |

Compounds (continued)

| Cmpd. No. | $R_1$ | $R_2$ | X | Y | Z | m.p.(°C) |
|---|---|---|---|---|---|---|
| 27 | $CH_3$ | $SCH_2CF_3$ | $CH_3$ | $CH_3$ | CH | 172-174 |
| 28 | $CH_3$ | $SCH_2CF_3$ | $OCH_2CH_3$ | $NHCH_3$ | N | 189-196 |
| 29 | $CH_2CH_2CH_3$ | $CH_2Cl$ | $OCH_3$ | $OCH_3$ | CH | 127-128 |
| 30 | $CH_2CH_2CH_3$ | $CH_2Cl$ | $CH_3$ | $OCH_3$ | CH | 126-128 |
| 31 | $CH_2CH_2CH_3$ | $CH_2Cl$ | $CH_3$ | $CH_3$ | CH | 154-156 |
| 32 | $CH_2CH_2CH_3$ | $CH_2Cl$ | Cl | $OCH_3$ | CH | 169-170 |
| 33 | $CH_2CH_2CH_3$ | $CH_2Cl$ | $OCH_3$ | $OCH_3$ | N | 104-106 |
| 34 | $CH_2CH_2CH_3$ | $CH_2Cl$ | $CH_3$ | $OCH_3$ | N | oil |
| 35 | $CH_2CH_3$ | $CHBr_2$ | $OCH_3$ | $OCH_3$ | CH | 181-182 |
| 36 | $CH_2CH_3$ | $NHCH_3$ | $OCH_3$ | $OCH_3$ | N | 140-143 |
| 37 | $CH_2CH_3$ | $NHCH_3$ | $CH_3$ | $OCH_3$ | N | 135-178 |
| 38 | $CH_2CH_3$ | $NHCH_3$ | $OCH_3$ | $OCH_3$ | CH | 180-186 |
| 39 | $CH_2CH_3$ | $NHCH_3$ | $CH_3$ | $OCH_3$ | CH | 169-170 |
| 40 | $CH_2CH_3$ | $NHCH_3$ | $CH_3$ | $CH_3$ | CH | 178-182 |
| 41 | $CH_2CH_3$ | $NHCH_3$ | $OCH_2CH_3$ | $NHCH_3$ | N | 115-170 |
| 42 | $CH_3$ | $NHCH_3$ | $OCH_3$ | $OCH_3$ | N | 124-145 |
| 43 | $CH_3$ | $NHCH_3$ | $CH_3$ | $OCH_3$ | N | 138-186 |
| 44 | $CH_3$ | $NHCH_3$ | $OCH_3$ | $OCH_3$ | CH | 184-193 |
| 45 | $CH_3$ | $NHCH_3$ | $OCH_3$ | $CH_3$ | CH | 180-185 |
| 46 | $CH_3$ | $NHCH_3$ | Cl | $OCH_3$ | CH | 148-163 |
| 47 | $CH_3$ | $NHCH_3$ | $CH_3$ | $CH_3$ | CH | 179-186 |
| 48 | $CH_3$ | $NHCH_3$ | $OCH_2CH_3$ | $NHCH_3$ | N | 160-194 |
| 49 | $CH_2CH_2OCH_3$ | $OCH_2CF_3$ | $OCH_3$ | $OCH_3$ | N | 180-181 |
| 50 | $CH_2CH_2OCH_3$ | $OCH_2CF_3$ | $CH_3$ | $OCH_3$ | N | 140-142 |
| 51 | $CH_2CH_2OCH_3$ | $OCH_2CF_3$ | $OCH_3$ | $OCH_3$ | CH | 136-166 |
| 52 | $CH_2CH_2OCH_3$ | $OCH_2CF_3$ | $CH_3$ | $CH_3$ | CH | 144-145 |
| 53 | $CH_2CH_2OCH_3$ | $OCH_2CF_3$ | Cl | $OCH_3$ | CH | 159-162 |
| 54 | $CH_2CH_2OCH_3$ | $OCH_2CF_3$ | $CH_3$ | $CH_3$ | CH | 170-171 |
| 55 | $CH_2CH_2OCH_3$ | $OCH_2CF_3$ | $OCH_2CH_3$ | $NHCH_3$ | N | 187-190 |
| 56 | $CH_2CH_2OCH_3$ | $OCHF_2$ | $OCH_3$ | $OCH_3$ | CH | 121-125 |
| 57 | $CH_2CH_2OCH_3$ | $OCHF_2$ | $CH_3$ | $OCH_3$ | CH | 126-128 |
| 58 | $CH_2CH_2OCH_3$ | $OCHF_2$ | Cl | $OCH_3$ | CH | 133-134 |

Compounds (continued)

| Cmpd. No. | $R_1$ | $R_2$ | X | Y | Z | m.p.(°C) |
|---|---|---|---|---|---|---|
| 59 | $CH_2CH_2OCH_3$ | $OCHF_2$ | $OCH_2CH_3$ | $NHCH_3$ | N | 170-180 |
| 60 | $CH_3$ | $CH_2OC(O)CH_3$ | $CH_3$ | $OCH_3$ | N | 127-130 |
| 61 | $CH_3$ | $CH_2OC(O)CH_3$ | $CH_3$ | $CH_3$ | CH | 165-167 |
| 62 | $CH_3$ | $CH_2OCH_2CH_2OCH_3$ | $OCH_3$ | $OCH_3$ | N | 135-137 |
| 63 | $CH_3$ | $CH_2OCH_2CH_2OCH_3$ | $CH_3$ | $OCH_3$ | N | 139-141 |
| 64 | $CH_3$ | $CH_2OCH_2CH_2OCH_3$ | $OCH_3$ | $OCH_3$ | CH | 168-170 |
| 65 | $CH_3$ | $CH_2OCH_2CH_2OCH_3$ | $OCH_3$ | $CH_3$ | CH | 91-92 |
| 66 | $CH_3$ | $CH_2OCH_2CH_2OCH_3$ | Cl | $OCH_3$ | CH | 109-111 |
| 67 | $CH_3$ | $CH_2OCH_2CH_2OCH_3$ | $CH_3$ | $CH_3$ | CH | 120-122 |
| 68 | $CH_3$ | $CH_2OCH_2CF_3$ | $OCH_3$ | $OCH_3$ | N | 128-130 |
| 69 | $CH_3$ | $CH_2OCH_2CF_3$ | $CH_3$ | $OCH_3$ | N | 106-109 |
| 70 | $CH_3$ | $CH_2OCH_2CF_3$ | $OCH_3$ | $OCH_3$ | CH | 118-120 |
| 71 | $CH_3$ | $CH_2OCH_2CF_3$ | $CH_3$ | $OCH_3$ | CH | 139-141 |
| 72 | $CH_3$ | $CH_2OCH_2CF_3$ | Cl | $OCH_3$ | CH | 124-125 |
| 73 | $CH_3$ | $CH_2OCH_2CF_3$ | $CH_3$ | $CH_3$ | CH | 159-161 |
| 74 | $CH_3$ | $CH_2Cl$ | $OCH_3$ | $OCH_3$ | CH | 149-153(dec) |
| 75 | $CH_3$ | $CH_2Cl$ | $CH_3$ | $OCH_3$ | CH | 167-170 |
| 76 | $CH_3$ | $CH_2Cl$ | $CH_3$ | $CH_3$ | CH | 176-180(dec) |
| 77 | $CH_3$ | $CH_2Cl$ | Cl | $OCH_3$ | CH | 164-166 |
| 78 | $CH_3$ | $CH_2Cl$ | $OCH_3$ | $OCH_3$ | N | 161-162 |
| 79 | $CH_3$ | $CH_2Cl$ | $CH_3$ | $OCH_3$ | N | 148-150 |
| 80 | $CH_3$ | O-cyclopentyl | $CH_3$ | $OCH_3$ | N | 141-146 |
| 81 | $CH_3$ | O-cyclopentyl | $OCH_3$ | $OCH_3$ | N | 143-148 |
| 82 | $CH_3$ | O-cyclopentyl | $CH_3$ | $OCH_3$ | CH | 115-122 |
| 83 | $CH_3$ | O-cyclopentyl | $OCH_3$ | $OCH_3$ | CH | 160-166 |

## Compounds (continued)

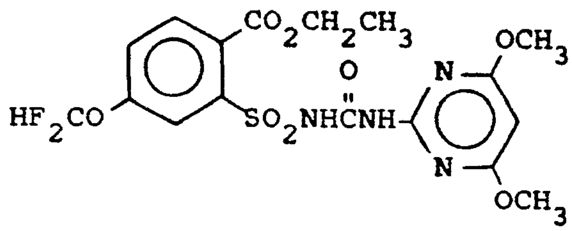

| Cmpd. No. | m.p.(°C) |
|---|---|
| 84 ~~88~~ | 146-149 |

The herbicidal properties of the subject compounds were discovered in a number of greenhouse tests. The test procedures and results follow.

### Test A

Seeds of crabgrass (Digitaria sp.). barnyard-grass (Echinochloa crusgalli), wild oats (Avena fatua), sicklepod (Cassia obtusifolia), morningglory (Ipomoea spp.). cocklebur (Xanthium pensylvanicum), sorghum, corn, soybean, sugar beet, cotton, rice, wheat and purple nutsedge (Cyperus rotundus) tubers were planted and treated preemergence with the test chemicals dissolved in a non-phytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings are based on a numerical scale extending from 0 = no injury, to 10 = complete kill. The accompanying descriptive symbols have the following meanings:

| | |
|---|---|
| B | = burn; |
| C | = chlorosis/necrosis; |
| D | = defoliation; |
| E | = emergence inhibition; |
| G | = growth retardation; |
| H | = formative effects; |
| S | = albinism; |
| U | = unusual pigmentation; |
| X | = axillary stimulation; and |
| 6Y | = abscised buds or flowers. |
| - | = no test |

TEST A

| RATE RATE=KG/HA | CMPD 1 0.05 | CMPD 1 0.01 | CMPD 2 0.05 | CMPD 2 0.01 | CMPD 3 0.05 | CMPD 3 0.01 | CMPD 4 0.05 | CMPD 4 0.01 |
|---|---|---|---|---|---|---|---|---|
| POSTEMERGENCE | | | | | | | | |
| COTTON | 2C,6G | 2G | 0 | 0 | 4G | 0 | 0 | 0 |
| MORNING GLORY | 10C | 3C,8G | 3C,7G | 2C | 1C,2G | 0 | 10C | 3C,7H |
| COCKLEBUR | 9C | 3C,8H | 3C,8H | 0 | 3C,8H | 0 | 2C,6G | 0 |
| NUTSEDGE | 0 | 0 | 0 | 0 | 0 | 0 | 0 | 0 |
| CRABGRASS | 2G | 0 | 2G | 0 | 0 | 0 | 0 | 0 |
| BARNYARD GRASS | 2C,9H | 3G | 3C,7H | 2C,3G | 2C,7G | 4G | 0 | 0 |
| WILD OATS | 0 | 0 | 0 | 0 | 0 | 0 | 0 | 0 |
| WHEAT | 0 | 0 | 0 | 0 | 0 | 0 | 0 | 0 |
| CORN | 4C,9H | 1C,4G | 2C | 0 | 0 | 0 | 0 | 0 |
| SOYBEAN | 2C,4G | 0 | 2H | 0 | 0 | 0 | 0 | 0 |
| RICE | 3C,6G | 2G | 2C,6G | 0 | 0 | 0 | 0 | 0 |
| SORGHUM | 8G | 1C,5H | 2C,5G | 2C,4G | 2C,8H | 0 | 0 | 0 |
| CHEATGRASS | 0 | 0 | 0 | 0 | 0 | 0 | 0 | 0 |
| SUGAR BEETS | 3C,8G | 2G | 5G | 2G | 2G | 0 | 6G | 0 |
| VELVETLEAF | 2C,6G | 2C | 0 | 0 | 0 | 0 | 0 | 0 |
| GIANT FOXTAIL | 4G | 0 | 2G | 0 | 0 | 0 | 0 | 0 |
| BARLEY | 5G | 2G | 0 | 0 | 0 | 0 | 0 | 0 |
| PREEMERGENCE | | | | | | | | |
| COTTON | 0 | 0 | 0 | 0 | 0 | 0 | 0 | 0 |
| MORNING GLORY | 2H | 0 | 0 | 0 | 0 | 0 | 0 | 0 |
| COCKLEBUR | 2H | 0 | 0 | 0 | 0 | 0 | 0 | 0 |
| NUTSEDGE | 0 | 0 | 4G | 0 | 0 | 0 | 0 | 0 |
| CRABGRASS | 0 | 0 | 4G | 0 | 0 | 0 | 0 | 0 |
| BARNYARD GRASS | 0 | 0 | 0 | 0 | 0 | 0 | 0 | 0 |
| WILD OATS | 2G | 0 | 0 | 0 | 0 | 0 | 0 | 0 |
| WHEAT | 0 | 0 | 0 | 0 | 0 | 0 | 0 | 0 |
| CORN | 6G | 0 | 2C | 0 | 0 | 0 | 0 | 0 |
| SOYBEAN | 0 | 0 | 0 | 0 | 0 | 0 | 0 | 0 |
| RICE | 7G | 0 | 2C,6G | 0 | 0 | 0 | 0 | 0 |
| SORGHUM | 3C,8H | 4G | 2C,5H | 0 | 2C,7H | 2C,4G | 0 | 0 |
| CHEATGRASS | 0 | 0 | 4G | 0 | 0 | 0 | 0 | 0 |
| SUGAR BEETS | 0 | 0 | 2G | 0 | 0 | 0 | 0 | 0 |
| VELVETLEAF | 0 | 0 | 0 | 0 | 0 | 0 | 0 | 0 |
| GIANT FOXTAIL | 0 | 0 | 0 | 0 | 0 | 0 | 0 | 0 |
| BARLEY | 3G | 0 | 0 | 0 | 0 | 0 | 0 | 0 |

TEST A

| | CMPD 5 | | CMPD 6 | | CMPD 7 | | CMPD 8 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 0 | 0 | 10C | 10C | 10C | 10C | 10C | 10C |
| MORNING GLORY | 4C,9G | 2C,6G | 10C | 10C | 10C | 10C | 10C | 5C,9G |
| COCKLEBUR | 5G | 0 | 10C | 10C | 10C | 10C | 10C | 10C |
| NUTSEDGE | 0 | 0 | 10C | 5C,9G | 2C,8G | 3C,9G | 9C | 6G |
| CRABGRASS | 0 | 0 | 2G | 0 | 2C,7G | 0 | 0 | 0 |
| BARNYARD GRASS | 0 | 0 | 2C,9H | 2C,5G | 9C | 4C,9G | 2C,7G | 0 |
| WILD OATS | 0 | 0 | 2G | 0 | 9G | 2C,5G | 0 | 0 |
| WHEAT | 0 | 0 | 2G | 0 | 8G | 2G | 0 | 0 |
| CORN | 0 | 0 | 0 | 0 | 9H | 0 | 0 | 0 |
| SOYBEAN | 0 | 0 | 6C,9G | 5C,9G | 4C,9H | 5C,9G | 3C,8H | 0 |
| RICE | 2C,6G | 0 | 2C,5G | 2G | 5C,9G | 5C,9G | 3G | 0 |
| SORGHUM | 0 | 0 | 2C,7H | 5G | 9G | 9H | 7G | 3G |
| CHEATGRASS | 0 | 0 | 3C,8G | 5G | 9C | 4C,9G | 8G | 0 |
| SUGAR BEETS | 4G | 2G | 5C,9G | 10C | 10C | 10C | 10C | 2C,7G |
| VELVETLEAF | 0 | 0 | 10C | 10C | 10C | 5C,9G | 5C,9G | 5C,9G |
| GIANT FOXTAIL | 0 | 0 | 2G | 0 | 2C,8G | 0 | 3G | 0 |
| BARLEY | 0 | 0 | 3G | 0 | 9G | 7G | 0 | 0 |
| PREEMERGENCE | | | | | | | | |
| COTTON | 0 | 0 | 3C,8G | 0 | 2C,6G | 0 | 2C,4G | 0 |
| MORNING GLORY | 0 | 0 | 2H | 2H | 2C,8G | - | 4G | 0 |
| COCKLEBUR | 0 | 0 | 2H | 2H | 2C,7G | 2C,4H | 2C,5G | 0 |
| NUTSEDGE | 0 | 0 | 2G | 0 | 6G | 0 | 0 | 0 |
| CRABGRASS | 0 | 0 | 0 | 0 | 2C,7G | 0 | 0 | 0 |
| BARNYARD GRASS | 0 | 0 | 2G | 0 | 3C,8H | 0 | 0 | 0 |
| WILD OATS | 0 | 0 | 2G | 0 | 2C,7G | 6G | 0 | 0 |
| WHEAT | 0 | 0 | 0 | 0 | 8G | 5G | 0 | 0 |
| CORN | 0 | 0 | 3C,7H | 3C,5G | 3C,6G | 0 | 3G | 0 |
| SOYBEAN | 0 | 0 | 0 | 0 | 3C,8H | 1C,3G | 0 | 0 |
| RICE | 0 | 0 | 2C,8G | 3G | 9C | 2C,8G | 8G | 0 |
| SORGHUM | 0 | 0 | 9G | 2G | 2C,9G | 2C,7H | 2C,8H | 0 |
| CHEATGRASS | 0 | 0 | 8G | 0 | 9C | 2C,8G | 6G | 0 |
| SUGAR BEETS | 0 | 0 | 3C,8G | 5G | 5C,9G | 4C,9G | 7G | 0 |
| VELVETLEAF | 0 | 0 | 4C,8G | 2C,5G | 4C,8G | 3C,7G | 6G | 0 |
| GIANT FOXTAIL | 0 | 0 | 0 | 0 | 0 | 0 | 0 | 0 |
| BARLEY | 0 | 0 | 0 | 0 | 7G | 5G | 0 | 0 |

TEST A

| | CMPD 9 | | CMPD 10 | | CMPD 11 | | CMPD 12 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 5G | 2C | 2G | 2G | 2G | 0 | 0 | 0 |
| MORNING GLORY | 3C,7H | 1C,2G | 3C,7G | 1C | 5G | 0 | 2G | 0 |
| COCKLEBUR | 3C,7H | 3H | 3C,8H | 1C,3H | 2G | 0 | 0 | 0 |
| NUTSEDGE | 4G | 3G | 5G | 0 | 4G | 0 | 5G | 0 |
| CRABGRASS | 4G | 0 | 0 | 0 | 0 | 0 | 0 | 0 |
| BARNYARD GRASS | 9H | 8H | 9H | 2C,5H | 7H | 0 | 2H | 0 |
| WILD OATS | 0 | 0 | 0 | 0 | 0 | 0 | 0 | 0 |
| WHEAT | 0 | 0 | 0 | 0 | 0 | 0 | 0 | 0 |
| CORN | 8H | 7H | 3C,7H | 2C,4H | 3C,7G | 2G | 0 | 0 |
| SOYBEAN | 3C,7H | 3C,4H | 3C,4H | 1H | 3H | 0 | 0 | 0 |
| RICE | 2C,5G | 2G | 2C,7G | 3G | 3C,8G | 7G | 2G | 0 |
| SORGHUM | 2C,8G | 3C,7G | 3C,7H | 3C,4G | 7G | 0 | 2C,6G | 0 |
| CHEATGRASS | 0 | 0 | 0 | 0 | 0 | 0 | 0 | 0 |
| SUGAR BEETS | 2C | 2H | 3G | 1C | 0 | 0 | 0 | 0 |
| VELVETLEAF | 5G | 1H | 2C,7H | 3H | 2G | 0 | 0 | 0 |
| GIANT FOXTAIL | 3G | 0 | 2H | 0 | 2G | 0 | 0 | 0 |
| BARLEY | 0 | 0 | 3G | 0 | 2C,3G | 0 | 0 | 0 |
| PREEMERGENCE | | | | | | | | |
| COTTON | 6G | 2G | 3G | 0 | 0 | 0 | 2G | 0 |
| MORNING GLORY | 2G | 1H | 3G | 0 | 0 | 3C,3H | 0 | 0 |
| COCKLEBUR | 3C,3H | 2H | - | 0 | 0 | - | 0 | - |
| NUTSEDGE | 0 | 0 | 0 | 0 | 0 | 0 | 0 | 0 |
| CRABGRASS | 0 | 0 | 3G | 0 | 0 | 0 | 0 | 0 |
| BARNYARD GRASS | 2G | 0 | 0 | 0 | 0 | 0 | 0 | 0 |
| WILD OATS | 0 | 0 | 0 | 0 | 0 | 0 | 0 | 0 |
| WHEAT | 0 | 0 | 0 | 0 | 0 | 0 | 0 | 0 |
| CORN | 3C,7G | 2G | 3C,6G | 2G | 2G | 0 | 0 | 0 |
| SOYBEAN | 2G | 2G | 2H | 0 | 2G | 2H | 0 | 0 |
| RICE | 5G | 3G | 5G | 0 | 2G | 0 | 0 | 0 |
| SORGHUM | 7G | 2G | 3C,9H | 3G | 2C,4G | 2G | 2C,9H | 0 |
| CHEATGRASS | 0 | 0 | 0 | 0 | 0 | 0 | 0 | 0 |
| SUGAR BEETS | 2G | 0 | 0 | 0 | 0 | 0 | 2H | 0 |
| VELVETLEAF | 2G | 0 | 0 | 0 | 0 | 0 | 0 | 0 |
| GIANT FOXTAIL | 0 | 0 | 2G | 0 | 0 | 0 | 0 | 0 |
| BARLEY | 0 | 0 | 0 | 0 | 0 | 0 | 0 | 0 |

TEST A

| | CMPD 13 | | CMPD 14 | | CMPD 15 | | CMPD 16 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 0 | 0 | 5H | 0 | 9C | 9C | 10C | 10C |
| MORNING GLORY | 2C,6G | 0 | 3C,7H | 0 | 9C | 5C,9G | 9C | 9C |
| COCKLEBUR | 2H | 0 | 4H | 0 | 10C | 5C,9G | 9C | 9C |
| NUTSEDGE | 0 | 0 | 4G | 0 | 0 | 7G | 3G | 0 |
| CRABGRASS | 0 | 0 | 4G | 0 | 0 | 0 | 2G | 0 |
| BARNYARD GRASS | 0 | 0 | 2G | 0 | 3H | 2H | 5C,9H | 3C,6H |
| WILD OATS | 0 | 0 | 0 | 0 | 4C,9G | 2C,8G | 3C,9G | 3C,9G |
| WHEAT | 0 | 0 | 0 | 0 | 2C,9G | 2C,8G | 3C,9G | 2C,9G |
| CORN | 0 | 0 | 2G | 0 | 3C,8H | 3G | 3C,9H | 5H |
| SOYBEAN | 3C,8G | 2C,2H | 3C,7G | 2H | 4C,9G | 9C | 9C | 9C |
| RICE | 6G | 2G | 3C,9G | 2G | 5C,9G | 7G | 9C | 4C,9G |
| SORGHUM | - | 0 | 2C | 0 | 4C,9H | 2C,6G | 4C,9G | 4C,8H |
| CHEATGRASS | 0 | 0 | 0 | 0 | 3C,8G | 3G | 3C,8G | 5G |
| SUGAR BEETS | 3C,7G | 2H | 3C,8H | 0 | 9C | 3C,3H | 3C,7G | 3C,7H |
| VELVETLEAF | 0 | 0 | 5G | 0 | 5C,8G | 6G | 5C,9H | 3C,7G |
| GIANT FOXTAIL | 0 | 0 | 2G | 0 | 4G | 2G | 3C,6G | 2G |
| BARLEY | 0 | 0 | 0 | 0 | 4C,8G | 3C,8G | 3C,9G | 4C,9G |
| PREEMERGENCE | | | | | | | | |
| COTTON | 0 | 0 | 0 | 0 | 2G | 0 | 6G | 0 |
| MORNING GLORY | 3G | 0 | 0 | 0 | 8H | 8G | 9G | 9G |
| COCKLEBUR | 0 | 0 | 0 | 0 | 5H | 2H | - | 2C,4H |
| NUTSEDGE | 0 | 0 | 0 | 0 | 0 | 0 | 5G | 0 |
| CRABGRASS | 0 | 0 | 0 | 0 | 0 | 0 | 0 | 0 |
| BARNYARD GRASS | 0 | 0 | 0 | 0 | 2G | 0 | 7H | 0 |
| WILD OATS | 0 | 0 | 0 | 0 | 3C,7G | 0 | 3C,9H | 2G |
| WHEAT | 0 | 0 | 0 | 0 | 4C,9H | 0 | 9H | 4G |
| CORN | 0 | 0 | 0 | 0 | 2C,5G | 0 | 4C,7G | 3G |
| SOYBEAN | 0 | 0 | 0 | 0 | 3C,5H | 0 | 8H | 2C,3H |
| RICE | 0 | 0 | 0 | 0 | 9H | 5G | 9H | 8G |
| SORGHUM | 0 | 0 | 0 | 0 | 10E | 2C,5G | 8H | 3C,8G |
| CHEATGRASS | 0 | 0 | 0 | 0 | 8G | 0 | 8H | 0 |
| SUGAR BEETS | 0 | 0 | 0 | 0 | 6G | 2H | 6G | 2H |
| VELVETLEAF | 0 | 0 | 0 | 0 | 7G | 0 | 7G | 1H |
| GIANT FOXTAIL | 0 | 0 | 0 | 0 | 0 | 0 | 0 | 0 |
| BARLEY | 0 | 0 | 0 | 0 | 2C,8G | 2G | 9G | 4G |

TEST A

| | CMPD 17 | | CMPD 18 | | CMPD 19 | | CMPD 20 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 10C | 10C | 10C | 10C | 10C | 9C | 10C | 10C |
| MORNING GLORY | 9C | 9C | 10C | 10C | 10C | 3C,8G | 5C,9G | 5C,9G |
| COCKLEBUR | 10C | 10C | 10C | 10C | 10C | 4C,9H | 9C | 4C,9H |
| NUTSEDGE | 9G | 3C,6G | 7G | 5G | 0 | 0 | 3G | 0 |
| CRABGRASS | 2G | 0 | 0 | 0 | 4G | 0 | 2G | 0 |
| BARNYARD GRASS | 3C,8H | 3C,6H | 5C,9H | 3C,9H | 2C,5H | 0 | 4C,9H | 2C,5H |
| WILD OATS | 4G | 4G | 4C,9G | 2C,5G | 2G | 0 | 3C,9G | 4G |
| WHEAT | 5C,9G | 9G | 4C,9G | 9G | 8G | 3G | 3C,9G | 5G |
| CORN | 3U,9H | 3C,9H | 3C,9H | 3C,7H | 3H | 2G | 3C,8G | 3G |
| SOYBEAN | 9C | 10C | 9C | 9C | 3C,6H | 2H | 9C | 9C |
| RICE | 7G | 4G | 8G | 5G | 8G | 4G | 4C,9G | 2C,5G |
| SORGHUM | 4C,9G | 4C,9H | 5C,9G | 5C,9G | 4C,9G | 4C,9H | 4C,9H | 2C,7H |
| CHEATGRASS | 4C,9G | 3C,9G | 9G | 7G | 2C,8G | 0 | 7G | 4G |
| SUGAR BEETS | 9C | 9C | 10C | 9C | 9C | 3C,8G | 9C | 5C,9G |
| VELVETLEAF | 9C | 9C | 10C | 9C | 5C,9G | 3H | 10C | 4C,9H |
| GIANT FOXTAIL | 4G | 2G | 3C,7G | 2G | 3G | 0 | 2C,7G | 2G |
| BARLEY | 5C,9G | 3C,8G | 4C,9G | 7G | 7G | 2G | 3C,8G | 3G |
| PREEMERGENCE | | | | | | | | |
| COTTON | 2G | 0 | 8H | 2C | 2G | 0 | 4G | 0 |
| MORNING GLORY | 6H | 0 | 7H | 3C,3H | 8G | 0 | 2C | 1H |
| COCKLEBUR | 8H | 0 | 8H | 2C,2H | 3C,5H | 0 | 8H | 1H |
| NUTSEDGE | 0 | 0 | 5G | 0 | 0 | 0 | 0 | 0 |
| CRABGRASS | 0 | 0 | 3G | 0 | 0 | 0 | 0 | 0 |
| BARNYARD GRASS | 8H | 0 | 9H | 5H | 2H | 0 | 3C,8H | 3G |
| WILD OATS | 0 | 0 | 3G | 0 | 2C | 0 | 5G | 0 |
| WHEAT | 8H | 2G | 9H | 6G | 8G | 2G | 7G | 0 |
| CORN | 3C,4G | 0 | 9H | 2C,2H | 3G | 0 | 2C,5G | 2G |
| SOYBEAN | 2C,3H | 0 | 3C,7H | 2C,6H | 0 | 0 | 0 | 0 |
| RICE | 8H | 2G | 4C,8G | 5G | 7G | 2G | 4C,9H | 3G |
| SORGHUM | 3C,9H | 2G | 9H | 3C,5G | 3C,8G | 3G | 4C,9H | 5G |
| CHEATGRASS | 8G | 2G | 8H | 2G | 0 | 0 | 3G | 0 |
| SUGAR BEETS | 4C,9G | 3G | 4C,9G | 3C,9G | 3C,5G | 3G | 8G | 5G |
| VELVETLEAF | 7G | 0 | 4C,8G | 3C,8H | 5G | 0 | 8G | 1H |
| GIANT FOXTAIL | 0 | 0 | 2G | 0 | 0 | 0 | 2G | 0 |
| BARLEY | 8G | 0 | 9G | 4G | 7G | 2G | 7G | 0 |

TEST A

| | CMPD 21 | | CMPD 22 | | CMPD 23 | | CMPD 24 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 3C,7G | 4G | 10C | 9C | 10C | 10C | 10C | 10C |
| MORNING GLORY | 10C | 5C,9G | 10C | 9C | 10C | 10C | 9C | 10C |
| COCKLEBUR | 5C,9H | 4C,8H | 10C | 10C | 10C | 9C | 10C | 10C |
| NUTSEDGE | 0 | 0 | 0 | 0 | 5G | 0 | 4C,9G | 2C,5G |
| CRABGRASS | 0 | 0 | 0 | 0 | 5G | 2G | 4G | 0 |
| BARNYARD GRASS | 3H | 3H | 3C,8H | 0 | 9C | 3C,7H | 4C,9H | 6H |
| WILD OATS | 2C,6G | 4G | 6C,9G | 3C,8G | 9C | 4C,9G | 5C,9G | 8G |
| WHEAT | 3C,9G | 6G | 4C,9G | 3C,8G | 5C,9G | 3C,9G | 9C | 9G |
| CORN | 0 | 0 | 4C,9H | 0 | 2C,9G | 5G | 4C,9H | 6H |
| SOYBEAN | 9C | 9C | 9C | 9C | 9C | 9C | 10C | 9C |
| RICE | 7G | 4G | 5C,9G | 4C,9G | 9C | 5C,9G | 8G | 5G |
| SORGHUM | 3C,7G | 3G | 4C,9G | 4C,8G | 5C,9G | 3C,9H | 9C | 4C,9H |
| CHEATGRASS | 4G | 0 | 5C,9G | 4C,9G | 9C | 4C,9G | 5C,9G | 3C,9G |
| SUGAR BEETS | 4C,9H | 4C,8G | 9C | 3C,6G | 4C,9G | 3C,6G | 9C | 9C |
| VELVETLEAF | 2C,5G | 5G | 4C,9H | 3C,7G | 4C,9H | 3C,7G | 10C | 9C |
| GIANT FOXTAIL | 0 | 0 | 3G | 2G | 3C,7G | 3G | 7G | 3G |
| BARLEY | 2C,8G | 6G | 4C,9G | 7G | 7G | 7G | 3C,9G | 7G |
| PREEMERGENCE | | | | | | | | |
| COTTON | 0 | 0 | 4G | 2G | 8G | 4G | 4G | 0 |
| MORNING GLORY | 3G | 0 | 9C | 9G | 9G | 9G | 9H | 3G |
| COCKLEBUR | - | 0 | 6H | 7H | 9H | 6H | 7H | 3H |
| NUTSEDGE | 0 | 0 | 0 | 0 | 5G | 0 | 0 | 0 |
| CRABGRASS | 0 | 0 | 0 | 0 | 0 | 0 | 2G | 0 |
| BARNYARD GRASS | 0 | 0 | 3G | 0 | 5G | 2G | 6H | 0 |
| WILD OATS | 0 | 0 | 9H | 3G | 8H | 7G | 3C,6G | 2G |
| WHEAT | 0 | 0 | 9H | 2G | 9H | 8G | 8G | 4G |
| CORN | 0 | 0 | 2C,6G | 3G | 8G | 3G | 2G | 0 |
| SOYBEAN | 0 | 0 | 8H | 7H | 8H | 7H | 4H | 0 |
| RICE | 0 | 0 | 9H | 8H | 9H | 9H | 9H | 4G |
| SORGHUM | 2C,5G | 0 | 9H | 3C,6G | 3C,9H | 3C,6G | 9H | 9G |
| CHEATGRASS | 0 | 0 | 9H | 6G | 9H | 7G | 8G | 5G |
| SUGAR BEETS | 5G | 0 | 3H | 2G | 5G | 3G | 4C,9G | 7G |
| VELVETLEAF | 2G | 0 | 8G | 6G | 8G | 6G | 6H | 2G |
| GIANT FOXTAIL | 0 | 0 | 0 | 0 | 4G | 0 | 2G | 0 |
| BARLEY | 0 | 0 | 7G | 6G | 2C,9G | 3G | 7G | 6G |

TEST A

| RATE RATE=KG/HA | CMPD 25 0.05 | CMPD 25 0.01 | CMPD 26 0.05 | CMPD 26 0.01 | CMPD 27 0.05 | CMPD 27 0.01 | CMPD 28 0.05 | CMPD 28 0.01 |
|---|---|---|---|---|---|---|---|---|
| POSTEMERGENCE | | | | | | | | |
| COTTON | 10C | 10C | 10C | 10C | 10C | 10C | 5C,9G | 7G |
| MORNING GLORY | 10C | 10C | 9C | 5C,9H | 9C | 9C | 5C,9G | 4C,9G |
| COCKLEBUR | 10C | 10C | 10C | 10C | 10C | 10C | 4C,9H | 8H |
| NUTSEDGE | 8G | 0 | 3G | 0 | 0 | 0 | 2G | 0 |
| CRABGRASS | 4G | 0 | 0 | 0 | 4G | 0 | 2G | 0 |
| BARNYARD GRASS | 4C,9H | 9H | 2H | 2C,5G | 4C,9H | 3C,7H | 5H | 4H |
| WILD OATS | 4C,9G | 9G | 3C,9G | 6G | 3C,9G | 3C,9G | 2C,9G | 9G |
| WHEAT | 5C,9G | 9G | 3C,9G | 7G | 3C,9G | 2C,9G | 9G | 8G |
| CORN | 4C,9H | 6H | 3C,6H | 0 | 3C,8H | 3G | 0 | 0 |
| SOYBEAN | 9C | 9C | 9C | 4C,8G | 9C | 9C | 9C | 9C |
| RICE | 9G | 5G | 3C,6G | 2G | 5C,9G | 4C,9G | 3G | 0 |
| SORGHUM | 9C | 4C,9G | 9C | 4C,9G | 4C,9G | 4C,9G | 5C,9G | 2G |
| CHEATGRASS | 4C,9G | 4C,9G | 2C,8G | 8G | 4C,9G | 3C,8G | 4C,9G | 6G |
| SUGAR BEETS | 9C | 9C | 9C | 5C,9G | 10C | 9C | 3C,8G | 4C,6H |
| VELVETLEAF | 10C | 9C | 2C,7G | 2C,5G | 9C | 9C | 3C,7G | 3G |
| GIANT FOXTAIL | 3C,8G | 6G | 2G | 0 | 4C,8G | 7G | 2G | 0 |
| BARLEY | 4C,9G | 8G | 6G | 5G | 8G | 5G | 7G | 5G |
| PREEMERGENCE | | | | | | | | |
| COTTON | 8H | 0 | 2G | 2H | 9G | 2G | 0 | 0 |
| MORNING GLORY | 8H | 2H | 4G | 0 | 6H | 2H | 6H | 2H |
| COCKLEBUR | 8H | 2H | 0 | 0 | - | 5H | 9H | - |
| NUTSEDGE | 5G | 0 | 0 | 0 | 5G | 0 | 0 | 0 |
| CRABGRASS | 2G | 0 | 2G | 0 | 7G | 0 | 0 | 0 |
| BARNYARD GRASS | 7H | 6H | 5G | 0 | 9H | 3G | 3G | 0 |
| WILD OATS | 3C,8G | 2C,5G | 2C,6G | 0 | 3C,8H | 6G | 3C,4G | 0 |
| WHEAT | 9H | 8G | 9H | 6G | 9H | 5G | 6G | 0 |
| CORN | 3C,7H | 1H | 4G | 0 | 3C,5G | 2G | 2G | 0 |
| SOYBEAN | 9H | 2C,4G | 0 | 0 | 3C,5H | 1H | 0 | 0 |
| RICE | 4C,9H | 6G | 8H | 0 | 9H | 7H | 0 | 0 |
| SORGHUM | 9H | 3C,7H | 5C,9H | 8G | 10E | 2C,7G | 3C,8G | 0 |
| CHEATGRASS | 7G | 7G | 5G | 0 | 7G | 4G | 0 | 0 |
| SUGAR BEETS | 8G | 8G | 9G | 6G | 8G | - | 6H | 0 |
| VELVETLEAF | 3C,8G | 5G | 2H | 0 | 8G | 2G | 0 | 0 |
| GIANT FOXTAIL | 5G | 0 | 3G | 0 | 3G | 0 | 0 | 0 |
| BARLEY | 9G | 5G | 8G | 0 | 3C,8G | 2G | 5G | 0 |

TEST A

| | CMPD 29 | | CMPD 30 | | CMPD 31 | | CMPD 32 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 0 | - | 6G | - | 0 | - | 0 | - |
| MORNING GLORY | 4C,9G | - | 2C,7H | - | 3G | - | 1H,5G | - |
| COCKLEBUR | 3C,9G | - | 2C,9H | - | 0 | - | 1H | - |
| NUTSEDGE | 0 | - | 0 | - | 0 | - | 0 | - |
| CRABGRASS | 0 | - | 0 | - | 0 | - | 0 | - |
| BARNYARD GRASS | 4C,8H | - | 4C,6H | - | 2C | - | 0 | - |
| WILD OATS | 0 | - | 0 | - | 0 | - | 0 | - |
| WHEAT | 0 | - | 0 | - | 0 | - | 0 | - |
| CORN | 8H | - | 2C,7H | - | 0 | - | 0 | - |
| SOYBEAN | 3C,8H | - | 3C,7H | - | 3H | - | 0 | - |
| RICE | 9G | - | 0 | - | 0 | - | 0 | - |
| SORGHUM | 1C,8G | - | 3C,7G | - | 0 | - | 0 | - |
| CHEATGRASS | 0 | - | 0 | - | 0 | - | 0 | - |
| SUGAR BEETS | 5G | - | 2C,7G | - | 0 | - | 0 | - |
| VELVETLEAF | 5H | - | 2G | - | 0 | - | 2G | - |
| GIANT FOXTAIL | 0 | - | 0 | - | 0 | - | 0 | - |
| BARLEY | 6G | - | 4G | - | 0 | - | 0 | - |
| PREEMERGENCE | | | | | | | | |
| COTTON | 0 | - | 0 | - | 0 | - | 0 | - |
| MORNING GLORY | 2G | - | 2G | - | 0 | - | 0 | - |
| COCKLEBUR | - | - | 3H | - | 0 | - | 9H | - |
| NUTSEDGE | 0 | - | 0 | - | 0 | - | 0 | - |
| CRABGRASS | 0 | - | 0 | - | 0 | - | 0 | - |
| BARNYARD GRASS | 0 | - | 0 | - | 0 | - | 0 | - |
| WILD OATS | 0 | - | 0 | - | 0 | - | 0 | - |
| WHEAT | 0 | - | 0 | - | 0 | - | 0 | - |
| CORN | 2C,5H | - | 4C | - | 0 | - | 0 | - |
| SOYBEAN | 2G | - | 2G | - | 0 | - | 0 | - |
| RICE | 7G | - | 1S | - | 0 | - | 0 | - |
| SORGHUM | 1C,7G | - | 2C,7G | - | 0 | - | 0 | - |
| CHEATGRASS | 0 | - | 0 | - | 0 | - | 0 | - |
| SUGAR BEETS | 1C | - | 0 | - | 3H | - | 0 | - |
| VELVETLEAF | 0 | - | 0 | - | 0 | - | 0 | - |
| GIANT FOXTAIL | 0 | - | 0 | - | 0 | - | 0 | - |
| BARLEY | 0 | - | 4G | - | 0 | - | 0 | - |

TEST A

| | CMPD 33 | | CMPD 34 | | CMPD 35 | | CMPD 36 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 0 | - | 2G | - | 4C,9G | - | 10C | 2C,9H |
| MORNING GLORY | 3C,9G | - | 3C,9G | - | 3C,8G | - | 5C,9G | 3C,8H |
| COCKLEBUR | 2C,8H | - | 3C,9G | - | 5C,9G | - | 3C,9G | 3C,9H |
| NUTSEDGE | 0 | - | 0 | - | 5G | - | 3C,7G | 7G |
| CRABGRASS | 1C | - | 0 | - | 0 | - | 5G | 0 |
| BARNYARD GRASS | 0 | - | 4G | - | 9C | - | 9C | 6C,9G |
| WILD OATS | 0 | - | 0 | - | 0 | - | 3C,8G | 7G |
| WHEAT | 0 | - | 0 | - | 5G | - | 8G | 7G |
| CORN | 0 | - | 0 | - | 5C,9G | - | 3C,7H | 0 |
| SOYBEAN | 7H | - | 2C,8H | - | 4C,9G | - | 6C,9G | 3C,8G |
| RICE | 2C | - | 1C,7G | - | 4C,9G | - | 3C,9G | 3C,9G |
| SORGHUM | 5G | - | 1C,4H | - | 9C | - | 6C,9G | 3C,9G |
| CHEATGRASS | 0 | - | 0 | - | 5G | - | 2C,7G | 3G |
| SUGAR BEETS | 3C,9G | - | 4C,9G | - | 5G | - | 9C | 5C,9G |
| VELVETLEAF | 0 | - | 2G | - | 5C,9G | - | 3C,8G | 2C,6G |
| GIANT FOXTAIL | 0 | - | 2G | - | 2G | - | 3C,8G | 2G |
| BARLEY | 0 | - | 0 | - | 3C,8G | - | 4C,8G | 8G |
| PREEMERGENCE | | | | | | | | |
| COTTON | 0 | - | 0 | - | - | - | 3G | 0 |
| MORNING GLORY | 0 | - | 0 | - | - | - | 0 | 0 |
| COCKLEBUR | 0 | - | - | - | - | - | 1C,7G | 3G |
| NUTSEDGE | 0 | - | 0 | - | - | - | 0 | 0 |
| CRABGRASS | 2G | - | 0 | - | - | - | 0 | 0 |
| BARNYARD GRASS | 0 | - | 0 | - | - | - | 0 | 0 |
| WILD OATS | 0 | - | 0 | - | - | - | 0 | 0 |
| WHEAT | 0 | - | 0 | - | - | - | 0 | 0 |
| CORN | 0 | - | 0 | - | - | - | 0 | 0 |
| SOYBEAN | 0 | - | 0 | - | - | - | 0 | 0 |
| RICE | 0 | - | 3G | - | - | - | 5G | 0 |
| SORGHUM | 0 | - | 0 | - | - | - | 4G | 0 |
| CHEATGRASS | 0 | - | 0 | - | - | - | 0 | 0 |
| SUGAR BEETS | 0 | - | 3H | - | - | - | 2C,6G | 0 |
| VELVETLEAF | 0 | - | 0 | - | - | - | 0 | 0 |
| GIANT FOXTAIL | 3G | - | 0 | - | - | - | 0 | 0 |
| BARLEY | 0 | - | 0 | - | - | - | 0 | 0 |

TEST A

| | CMPD 37 | | CMPD 38 | | CMPD 39 | | CMPD 40 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 9C | 2C,4G | 9C | 3C,9G | 10C | 10C | 9C | 10C |
| MORNING GLORY | 3C,8G | 4C,9H | 10C | 6C,9G | 10C | 2C,5G | 10C | 4C,9G |
| COCKLEBUR | 10C | 3C,9H | 10C | 4C,9G | 10C | 2C,9H | 10C | 3C,9H |
| NUTSEDGE | 3C,9G | 9G | 2C,8G | 8G | 6G | 2G | 2C,8G | 2C,9G |
| CRABGRASS | 5G | 5G | 7G | 4G | 7G | 0 | 8G | 3G |
| BARNYARD GRASS | 9C | 6C,9G | 9C | 4C,8G | 5C,9G | 5C,9G | 10C | 9C |
| WILD OATS | 6C,9G | 2C,7G | 9C | 2C,8G | 3C,7G | 2C,7G | 9C | 3C,7G |
| WHEAT | 4C,9G | 7G | 3C,9G | 7G | 4C,9G | 7G | 5C,9G | 2C,9G |
| CORN | 4C,8H | 3C,5G | 5U,8G | 3C,7H | 9C | 3C,9H | 9C | 2U,9G |
| SOYBEAN | 6C,9G | 6C,9G | 6C,9G | 6C,9G | 9C | 3C,8H | 9C | 4C,9G |
| RICE | 4C,9G | 2C,9G | 4C,9G | 3C,8G | 5C,9G | 6G | 9C | 3C,9G |
| SORGHUM | 9C | 2C,9G | 9C | 4C,9G | 5C,9G | 3C,9G | 10C | 9C |
| CHEATGRASS | 2C,7G | 0 | 2C,7G | 3G | 3C,8G | 2G | 3C,8G | 2C,6G |
| SUGAR BEETS | 4C,9G | 5C,9G | 9C | 9C | 10C | 4C,9H | 9C | 9C |
| VELVETLEAF | 4C,9G | 2C,7G | 10C | 4C,9G | 9C | 2C,8G | 10C | 3C,9G |
| GIANT FOXTAIL | 4C,9G | 3C,7G | 2C,7G | 3G | 5C,9G | 2C,5G | 9C | 4C,9G |
| BARLEY | 9C | 3C,8G | 5C,8G | 4C,8G | 9C | 4C,9H | 9C | 9C |
| PREEMERGENCE | | | | | | | | |
| COTTON | 5G | 0 | 0 | 0 | 0 | 0 | 6G | 4G |
| MORNING GLORY | 9G | 0 | 2G | 2G | 2G | 0 | 6G | 3G |
| COCKLEBUR | 2C,3H | 8G | 2G | - | 2C | 1C | 7G | 4G |
| NUTSEDGE | 4G | 0 | 7G | 0 | 5G | 0 | 0 | 0 |
| CRABGRASS | 2G | 0 | 0 | 0 | 0 | 0 | 3G | 5G |
| BARNYARD GRASS | 5H | 0 | 0 | 0 | 8G | 0 | 8H | 7G |
| WILD OATS | 4G | 0 | 0 | 0 | 2C,3G | 0 | 2C,5G | 2G |
| WHEAT | 2G | 0 | 0 | 0 | 2C,5G | 0 | 6G | 0 |
| CORN | 0 | 0 | 0 | 0 | 2G | 0 | 2C,8H | 0 |
| SOYBEAN | 2C | 0 | 0 | 0 | 2H | 0 | 5H | 0 |
| RICE | 1C,5G | 0 | 0 | 0 | 2G | 0 | 8G | 7G |
| SORGHUM | 3C,8H | 0 | 0 | 0 | 9H | 0 | 3C,9H | 3C,8H |
| CHEATGRASS | 2G | 0 | 0 | 0 | 5G | 0 | 5G | 5G |
| SUGAR BEETS | 3C,6G | 0 | 2C,7G | 4G | 9G | 0 | 9G | 7G |
| VELVETLEAF | 2C,9G | 0 | 2C,5G | 2G | 2G | 1H | 8G | 0 |
| GIANT FOXTAIL | 1C,7G | 0 | 0 | 0 | 7G | 2G | 5G | 3G |
| BARLEY | 2G | 0 | 0 | 0 | 2G | 0 | 5G | 0 |

TEST A

| | CMPD 41 | | CMPD 42 | | CMPD 43 | | CMPD 44 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 3G | 0 | 10C | 10C | 10C | 10C | 10C | 10C |
| MORNING GLORY | 4C,8H | 1H | 10C | 9C | 10C | 9C | 10C | 10C |
| COCKLEBUR | 4C,9H | 2H | 10C | 9C | 10C | 10C | 10C | 10C |
| NUTSEDGE | 0 | 0 | 3C,8G | 5G | 6C,9G | 2C,8G | 9C | 2C,8G |
| CRABGRASS | 0 | 0 | 3C,7G | 2G | 3C,8G | 5G | 5G | 4G |
| BARNYARD GRASS | 3C,9H | 6H | 9C | 9C | 10C | 9C | 9C | 4C,9H |
| WILD OATS | 3C,8G | 2C | 9C | 5C,9G | 9C | 5C,9G | 9C | 4C,7G |
| WHEAT | 2C,9G | 2G | 6C,9G | 9G | 9C | 2C,9G | 5C,9G | 5G |
| CORN | 1H | 0 | 5C,9G | 6H | 10C | 4C,9H | 9C | 2U,9H |
| SOYBEAN | 3C,9G | 2C,7G | 9C | 9C | 9C | 9C | 9C | 9C |
| RICE | 3C,8G | 2G | 9C | 6C,9G | 9C | 5C,9G | 9C | 5C,9G |
| SORGHUM | 3C,8H | 2G | 9C | 2C,8G | 9C | 9C | 10C | 9C |
| CHEATGRASS | 0 | 0 | 6C,9G | 3C,9G | 6C,9G | 2C,9G | 9C | 3C,8G |
| SUGAR BEETS | 4C,9G | 2C,5H | 10C | 9C | 10C | 9C | 9C | 9C |
| VELVETLEAF | 1H | 1H | 2C,8G | 6G | 10C | 2C,8G | 10C | 2C,8G |
| GIANT FOXTAIL | 3G | 0 | 9C | 2C,6G | 9C | 9C | 9C | 3C,7H |
| BARLEY | 3C,8G | 5G | 9C | 3C,9G | 9C | 9C | 9C | 3C,9G |
| PREEMERGENCE | | | | | | | | |
| COTTON | 0 | 0 | 7G | 5G | 8G | 2G | - | 4G |
| MORNING GLORY | 0 | 0 | 8H | 3G | 9G | 5G | 5H | 2G |
| COCKLEBUR | - | 0 | 5H | 2H | 8H | 5G | 2C,5G | 1H |
| NUTSEDGE | 0 | 0 | 0 | 0 | 0 | 5G | 8G | 0 |
| CRABGRASS | 0 | 0 | 0 | 0 | 2G | 0 | 0 | 0 |
| BARNYARD GRASS | 0 | 0 | 2C,8H | 0 | 9H | 2G | 2C,4G | 0 |
| WILD OATS | 7G | 0 | 3G | 0 | 3C,9G | 2C | 5G | 0 |
| WHEAT | 2G | 0 | 5G | 0 | 8G | 2G | 4G | 0 |
| CORN | 0 | 0 | 2C,4G | 0 | 7G | 0 | 3G | 0 |
| SOYBEAN | 1H | 0 | 3C,7H | 0 | 3C,7G | 2G | 7H | 0 |
| RICE | 0 | 0 | 3C,9H | 7G | 3C,8H | 3G | 9G | 5G |
| SORGHUM | 0 | 0 | 2C,9H | 5G | 2C,9G | 5G | 8H | 0 |
| CHEATGRASS | 0 | 0 | 8G | 4G | 2C,8G | 2G | 7G | 0 |
| SUGAR BEETS | 3H | 0 | 9G | 8G | 5C,9G | 7G | 8G | 6G |
| VELVETLEAF | 2G | 0 | 2H | 0 | 8G | 2G | 5G | 0 |
| GIANT FOXTAIL | 0 | 0 | 2G | 0 | 0 | 0 | 2C | 0 |
| BARLEY | 0 | 0 | 0 | 0 | 6G | 2G | 0 | 0 |

TEST A

| RATE RATE=KG/HA | CMPD 45 0.05 | CMPD 45 0.01 | CMPD 46 0.05 | CMPD 46 0.01 | CMPD 47 0.05 | CMPD 47 0.01 | CMPD 48 0.05 | CMPD 48 0.01 |
|---|---|---|---|---|---|---|---|---|
| POSTEMERGENCE | | | | | | | | |
| COTTON | 10C | 10C | 10C | 4C,9G | 10C | 5C,9G | 10C | 4C,9G |
| MORNING GLORY | 10C | 10C | 10C | 3C,7G | 10C | 3C,8H | 10C | 3C,8H |
| COCKLEBUR | 10C | 5C,9H | 10C | 5C,9G | 10C | 9C | 3C,9H | 2C,5H |
| NUTSEDGE | 3C,9G | 2C,8G | 4C,9G | 4C,9G | 5C,9G | 4C,9G | 5G | 0 |
| CRABGRASS | 3C,7G | 5G | 5G | 4G | 4C,9G | 6G | 5G | 0 |
| BARNYARD GRASS | 9C | 9C | 9C | 9C | 10C | 9C | 9C | 4C,9H |
| WILD OATS | 9C | 3C,5G | 6C,9G | 2C,3G | 9C | 9C | 5C,9G | 2C,2H |
| WHEAT | 5C,9G | 2C,9G | 9G | 3G | 6C,9G | 4C,9G | 9G | 2C,5G |
| CORN | 9C | 2C,9H | 3C,9G | 2C,6H | 9C | 6C,9G | 3C,9H | 3C,6H |
| SOYBEAN | 9C | 9C | 9C | 9C | 9C | 5C,9G | 5C,9G | 4C,8G |
| RICE | 9C | 6C,9G | 9C | 6C,9G | 9C | 6C,9G | 5C,9G | 3C,9G |
| SORGHUM | 9C | 4C,9H | 9C | 3C,8G | 5C,9G | 5C,9G | 4C,9G | 3C,9H |
| CHEATGRASS | 6C,9G | 3C,9G | 5C,9G | 2C,3G | 9C | 5C,9G | 4C,9G | 3C,8G |
| SUGAR BEETS | 10C | 9C | 10C | 4C,8H | 10C | 9C | 5C,9H | 4C,8H |
| VELVETLEAF | 10C | 8G | 10C | 2C,8H | 10C | 9C | 3C,7G | 1C |
| GIANT FOXTAIL | 9C | 3C,8G | 9C | 2C,5G | 9C | 5C,9G | 3C,8H | 3C,6G |
| BARLEY | 9C | 5C,9H | 9C | 3C,7G | 6C,9G | 9C | 5C,9H | 3C,7G |
| PREEMERGENCE | | | | | | | | |
| COTTON | 9G | 2G | 6G | 3G | 8G | 5G | 3G | 2G |
| MORNING GLORY | 3G | 0 | 2G | 7G | 7G | 3G | 5G | 0 |
| COCKLEBUR | 6H | - | 2C,5G | 2C,2H | 9H | 2C,5G | 2G | 0 |
| NUTSEDGE | 8G | 0 | 0 | 0 | 10E | 2C,5G | 0 | 0 |
| CRABGRASS | 2G | 0 | 0 | 0 | 4G | 3G | 0 | 0 |
| BARNYARD GRASS | 3C,8H | 0 | 2C,8G | 0 | 9H | 3C,7H | 2C,5G | 0 |
| WILD OATS | 2C,5G | 2C | 2C,4G | 0 | 7G | 2C,4G | 2C,5G | 1C |
| WHEAT | 5G | 3G | 2C,3G | 0 | 7G | 5G | 2C,5G | 0 |
| CORN | 2C,8H | 0 | 2G | 0 | 3C,9H | 3G | 2C,2G | 0 |
| SOYBEAN | 3C,8H | 1H | 1H | 1H | 8H | 3C,3H | 3C,5H | 1H |
| RICE | 9H | 5G | 9G | 5G | 5C,9H | 9H | 5G | 4G |
| SORGHUM | 3C,9H | 2C,6G | 9H | 5G | 9H | 9H | 3C,5G | 2C,3G |
| CHEATGRASS | 8G | 4G | 5G | 0 | 8G | 7G | 6G | 2G |
| SUGAR BEETS | 9G | 7G | 8G | 8G | 9G | 9G | 3C,5G | 3H |
| VELVETLEAF | 3C,8G | 2H | 3H | 1H | 8G | 4H | 3G | 0 |
| GIANT FOXTAIL | 3C,5H | 0 | 3G | 0 | 3C,5H | 2G | 2G | 0 |
| BARLEY | 2C,2G | 0 | 0 | 0 | 8G | 4G | 2G | 0 |

TEST A

| | CMPD 49 | | CMPD 50 | | CMPD 51 | | CMPD 52 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 5G | 5G | 9C | 1C,5G | 10C | 10C | 10C | 4C,9G |
| MORNING GLORY | 10C | 10C | 9C | 10C | 10C | 10C | 10C | 10C |
| COCKLEBUR | 10C | 10C | 10C | 10C | 10C | 10C | 10C | 9C |
| NUTSEDGE | 9G | 7G | 3C,9G | 3C,8G | 5C,9G | 9G | 3C,9G | 4C,9G |
| CRABGRASS | 4G | 0 | 1H | 4G | 3G | 0 | 2C,5G | 3G |
| BARNYARD GRASS | 9H | 3C,8H | 6C,9H | 9H | 4C,9H | 3C,8H | 4C,9H | 9H |
| WILD OATS | 9C | 4C,9G | 9C | 5C,9G | 3C,6G | 2G | 4C,9H | 3C,7G |
| WHEAT | 4C,9G | 3C,9G | 9C | 4C,9G | 9G | 8G | 9G | 9G |
| CORN | 5C,9G | 3C,9H | 9C | 2C,9G | 9G | 3C,8H | 2C,9G | 9H |
| SOYBEAN | 6C,9G | 4C,9G | 6C,9G | 5C,9G | 5C,9G | 4C,9G | 6C,9G | 4C,9G |
| RICE | 9C | 5C,9G | 9C | 9C | 6C,9G | 4C,9G | 9C | 4C,9G |
| SORGHUM | 6C,9H | 3C,9G | 6C,9H | 3C,9H | 3C,9H | 3C,9H | 2C,9G | 3C,9H |
| CHEATGRASS | 6C,9G | 9G | 9C | 4C,9G | 3C,9G | 8G | 4C,9G | 9G |
| SUGAR BEETS | 9C | 9C | 10C | 10C | 10C | 9C | 10C | 9C |
| VELVETLEAF | 9C | 4C,8G | 9C | 3C,8H | 10C | 10C | 10C | 10C |
| GIANT FOXTAIL | 4C,9G | 3C,7G | 6C,9G | 4C,8G | 4C,9H | 3C,7G | 4C,9G | 3C,9G |
| BARLEY | 5C,9G | 2C,9G | 9C | 2C,9G | 3C,8G | 2C,7G | 9H | 3C,8G |
| PREEMERGENCE | | | | | | | | |
| COTTON | 3G | 0 | 6G | 2G | 0 | 0 | 2H | 0 |
| MORNING GLORY | 9G | 9G | 9G | 9G | 2C,8G | 2C,5G | 8H | 2C,4G |
| COCKLEBUR | 9H | 8H | 9H | - | 8H | 9H | 9H | 7H |
| NUTSEDGE | 10E | 3C,8G | 8G | 5G | 9G | 4C,9G | 9G | 7G |
| CRABGRASS | 1C | 0 | 2G | 0 | 3G | 0 | 2C,5G | 0 |
| BARNYARD GRASS | 4C,8H | 3G | 3C,9H | 3C,8G | 3C,8H | 3G | 9H | 3C,6G |
| WILD OATS | 4C,9H | 2C,8G | 3C,9H | 4C,9G | 3C,7G | 2C | 4C,8H | 3C,6G |
| WHEAT | 4C,9H | 9H | 3C,9H | 9H | 3C,9H | 2C,9G | 3C,9H | 8G |
| CORN | 9H | 9H | 5C,9H | 3C,9H | 3C,9H | 3C,8H | 3C,9H | 3C,8H |
| SOYBEAN | 9H | 4C,7H | 9H | 4C,8H | 2C,6H | 3C,5H | 4C,8H | 3C,6H |
| RICE | 9H | 3C,7H | 9H | 4C,8H | 9H | 3C,6G | 4C,9H | 3C,7H |
| SORGHUM | 10H | 9H | 10H | 5C,9H | 9H | 9H | 3C,9H | 9H |
| CHEATGRASS | 9H | 8G | 9H | 3C,8H | 9H | 8G | 9H | 8G |
| SUGAR BEETS | 4C,9G | 4C,9G | 5C,9G | 4C,9H | 9G | 8G | 5C,9H | 4C,9G |
| VELVETLEAF | 4C,8H | 3G | 4C,8H | 3C,5H | 8H | 5H | 3C,7H | 4H |
| GIANT FOXTAIL | 5C,9H | 5G | 9H | 2C,8G | 3C,9H | 5G | 9H | 3C,7G |
| BARLEY | 9G | 9G | 4C,9H | 2C,9G | 2C,7G | 2C,3G | 2C,7G | 3C,7G |

TEST A

| RATE RATE=KG/HA | CMPD 53 0.05 | CMPD 53 0.01 | CMPD 54 0.05 | CMPD 54 0.01 | CMPD 55 0.05 | CMPD 55 0.01 | CMPD 56 0.05 | CMPD 56 0.01 |
|---|---|---|---|---|---|---|---|---|
| POSTEMERGENCE | | | | | | | | |
| COTTON | 4C,9G | 0 | 4C,8H | 5G | 3G | 9G | 10C | 10C |
| MORNING GLORY | 10C | 9C | 9C | 4C,8H | 5C,9G | 4C,9H | 10C | 10C |
| COCKLEBUR | 10C | 10C | 10C | 5C,9H | 10C | 4C,9H | 10C | 10C |
| NUTSEDGE | 4C,8G | 2C,5G | 4C,9G | 2G | 5G | 0 | 2C,8G | 8G |
| CRABGRASS | 2G | 0 | 5G | 0 | 0 | 0 | 4G | 2G |
| BARNYARD GRASS | 3C,5H | 1H | 4C,9H | 3C,7H | 3C,9H | 7H | 9H | 6H |
| WILD OATS | 3C,5G | 2G | 4C,9G | 3C,7G | 3C,9G | 3G | 3G | 0 |
| WHEAT | 9G | 8G | 9G | 9G | 9G | 4G | 3G | 0 |
| CORN | 5G | 0 | 3C,9H | 2C,5H | 3C,7H | 2C,3G | 8H | 7G |
| SOYBEAN | 3C,5H | 2C,5G | 5C,9G | 4C,9G | 5C,9G | 4C,9G | 4C,9H | 5C,9H |
| RICE | 5C,9G | 9G | 6C,9G | 4C,9G | 4C,9G | 4C,9G | 5G | 0 |
| SORGHUM | 9G | 9G | 3C,9H | 4C,9H | 4C,9H | 3C,9H | 9H | 9H |
| CHEATGRASS | 2C,5G | 0 | 4C,9G | 8G | 3C,8G | 3G | 5C,9G | 9C |
| SUGAR BEETS | 4C,9G | 3C,7G | 10C | 5C,9H | 3C,8H | 3C,7H | 9C | 5C,9G |
| VELVETLEAF | 5C,9G | 7H | 9C | 4C,9H | 3C,8G | 7G | 10C | 10C |
| GIANT FOXTAIL | 3C,7G | 2C,5G | 4C,9G | 3C,7H | 2C,6G | 2H | 7G | 4G |
| BARLEY | 7G | 3G | 3C,8G | 2C,3G | 5C,9G | 2C,7G | 3C,6G | 0 |
| PREEMERGENCE | | | | | | | | |
| COTTON | 0 | 0 | 0 | 0 | 0 | 0 | 8G | 5G |
| MORNING GLORY | 3C,7G | 3G | 2C,6H | 1C | 8H | 1C,4G | 9G | 3H |
| COCKLEBUR | 9H | 2C,5G | - | 0 | 8H | - | 8H | 8H |
| NUTSEDGE | 5G | 0 | 5G | 0 | 0 | 0 | 9G | 8G |
| CRABGRASS | 0 | 0 | 0 | 0 | 0 | 0 | 0 | 0 |
| BARNYARD GRASS | 2G | 0 | 3C,4H | 0 | 2G | 0 | 7H | 2G |
| WILD OATS | 2G | 0 | 3C,8G | 0 | 2C,6G | 0 | 2C,3G | 0 |
| WHEAT | 9G | 7G | 8G | 3G | 8G | 0 | 6G | 3G |
| CORN | 2C,5G | 2G | 3C,5G | 0 | 3C,8H | 2G | 9H | 3C,3G |
| SOYBEAN | 1C | 3G | 3C,3G | 1C | 3C,5H | 3G | 8H | 7H |
| RICE | 8H | 5G | 3C,5G | 0 | 3C,6H | 3G | 7G | 4G |
| SORGHUM | 9H | 3C,9H | 3C,8H | 3C,5G | 9H | 3C,7G | 9H | 7G |
| CHEATGRASS | 7G | 3G | 7G | 0 | 7G | 0 | 9H | 8G |
| SUGAR BEETS | 9G | 2C,5H | 4C,9G | 6H | 6H | 3G | 9G | 8G |
| VELVETLEAF | 5H | 0 | 2C,5H | 0 | 2H | 0 | 4C,9G | 6H |
| GIANT FOXTAIL | 5G | 0 | 7G | 0 | 3G | 0 | 7G | 0 |
| BARLEY | 7G | 5G | 6G | 3G | 7G | 6G | 5G | 0 |

TEST A

| | CMPD 57 | | CMPD 58 | | CMPD 59 | | CMPD 60 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 9C | 5C,9G | 9C | 4C,9G | 3G | 2G | 10C | 10C |
| MORNING GLORY | 10C | 9C | 10C | 2C,7H | 5C,9G | 3C,8H | 10C | 10C |
| COCKLEBUR | 10C | 10C | 10C | 10C | 10C | 5C,9G | 10C | 10C |
| NUTSEDGE | 9G | 3C,9G | 8G | 0 | 2G | 0 | 4C,9G | 5G |
| CRABGRASS | 5G | 0 | 3G | 0 | 3C,5G | 0 | 3G | 0 |
| BARNYARD GRASS | 3C,9H | 7H | 7H | 3H | 8H | 4G | 9C | 4C,9H |
| WILD OATS | 7G | 2C,5G | 2G | 0 | 8G | 2G | 2C,7G | 2C,5G |
| WHEAT | 9G | 5G | 5G | 0 | 9G | 3G | 3C,9G | 4C,9G |
| CORN | 3C,9H | 2C,7H | 7H | 0 | 7H | 3G | 9C | 2C,9G |
| SOYBEAN | 9C | 3C,8H | 2C,7H | 2C,2H | 5C,9G | 4C,9G | 9C | 5C,9G |
| RICE | 6G | 0 | 5G | 2G | 4C,9G | 3C,8G | 5C,9G | 2C,7G |
| SORGHUM | 3C,9G | 3C,9G | 3C,9H | 3C,8H | 9G | 3C,9G | 5C,9G | 4C,9G |
| CHEATGRASS | 5C,9G | 8G | 8G | 3C,5G | 3C,8G | 4G | 4C,9G | 7G |
| SUGAR BEETS | 9C | 4C,9G | 10C | 3C,7G | 3C,7H | 3C,6G | 9C | 9C |
| VELVETLEAF | 10C | 10C | 9C | 3C,8G | 4C,8H | 7G | 10C | 5C,9G |
| GIANT FOXTAIL | 6C,9G | 7G | 7G | 2G | 3C,8G | 6G | 3C,8G | 3C,7G |
| BARLEY | 9G | 2C,6G | 2C,5G | 0 | 8G | 2C,6G | 9H | 9H |
| PREEMERGENCE | | | | | | | | |
| COTTON | 3C,8G | 8G | 8G | 0 | 0 | 0 | 3C,8H | 3H |
| MORNING GLORY | 9G | 5G | 9H | 8G | 9H | 2C | 4C,8G | 3G |
| COCKLEBUR | 9H | - | 8H | 3C,5G | 8H | 2C,2H | 7H | 5H |
| NUTSEDGE | 9G | 5G | 10E | 0 | 0 | 0 | 8G | 2G |
| CRABGRASS | 3G | 1C | 2C | 0 | 5G | 0 | 3G | 0 |
| BARNYARD GRASS | 9H | 3C,3G | 3C,7G | 2G | 6G | 0 | 9H | 7G |
| WILD OATS | 3C,5G | 2C | 2G | 0 | 2C,5G | 0 | 4C,8G | 2C,3G |
| WHEAT | 9H | 6G | 8G | 0 | 8G | 0 | 4C,9H | 3C,8H |
| CORN | 9H | 3C,7G | 8G | 0 | 3C,7H | 0 | 3C,9H | 3C,8H |
| SOYBEAN | 9H | 3C,8H | 7H | 1H | 3C,7H | 3G | 4C,7H | 4H |
| RICE | 3C,8H | 2C,4G | 8G | 0 | 9H | 5G | 5C,9H | 8G |
| SORGHUM | 9H | 9H | 10H | 8G | 9H | 3C,7G | 9H | 4C,9G |
| CHEATGRASS | 9H | 8H | 8G | 3G | 7G | 0 | 5C,9G | 7G |
| SUGAR BEETS | 9G | 4C,9G | 9G | 7G | 9G | 6G | 9C | 3C,8G |
| VELVETLEAF | 5C,9H | 5G | 3C,8G | 0 | 3G | 0 | 7H | 5H |
| GIANT FOXTAIL | 3C,8H | 3C | 2C,5G | 0 | 3G | 0 | 6G | 0 |
| BARLEY | 9G | 6G | 5G | 2G | 9G | 8G | 9G | 5G |

TEST A

| | CMPD 61 | | CMPD 62 | | CMPD 63 | | CMPD 64 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 10C | 5C,9G | 10C | 4C,8G | 10C | 4C,8G | 9C | 5C,9G |
| MORNING GLORY | 9C | 3C,7G | 10C | 10C | 10C | 10C | 10C | 10C |
| COCKLEBUR | 10C | 4C,9H | 10C | 10C | 10C | 9C | 10C | 10C |
| NUTSEDGE | 4C,9G | 3C,6G | 4C,9G | 3C,7G | 6C,9G | 2C,5G | 10C | 9C |
| CRABGRASS | 3C,7G | 0 | 3G | 0 | 3C,7G | 0 | 5C,9G | 3C,7G |
| BARNYARD GRASS | 10C | 5C,9H | 9C | 4C,9H | 10C | 9C | 9C | 9C |
| WILD OATS | 2C,3G | 0 | 9C | 9G | 6C,9G | 5C,9G | 5C,9G | 7G |
| WHEAT | 2C,5G | 0 | 10C | 5C,9G | 10C | 9C | 9C | 3C,8G |
| CORN | 9C | 3C,9H | 10C | 9H | 9C | 9C | 10C | 5C,9G |
| SOYBEAN | 4C,9G | 2C,7H | 9C | 4C,9G | 9C | 6C,9G | 9C | 5C,9G |
| RICE | 9C | 9C | 9C | 9C | 9C | 9C | 9C | 9C |
| SORGHUM | 4C,9H | 4C,9H | 9C | 5C,9G | 9C | 9C | 9C | 10C |
| CHEATGRASS | 4G | 0 | 10C | 9C | 10C | 9C | 10C | 9C |
| SUGAR BEETS | 10C | 2C,5G | 10C | 9C | 10C | 9C | 10C | 10C |
| VELVETLEAF | 10C | 3C,8G | 9C | 4C,8H | 10C | 5C,9H | 10C | 9C |
| GIANT FOXTAIL | 5C,9G | 5G | 9C | 3C,6G | 9C | 4C,8G | 9C | 9C |
| BARLEY | 6C,9G | 4C,8G | 9C | 4C,9G | 9C | 6C,9G | 9C | 5C,9G |
| PREEMERGENCE | | | | | | | | |
| COTTON | - | - | - | - | - | - | - | - |
| MORNING GLORY | - | - | - | - | - | - | - | - |
| COCKLEBUR | - | - | - | - | - | - | - | - |
| NUTSEDGE | - | - | - | - | - | - | - | - |
| CRABGRASS | - | - | - | - | - | - | - | - |
| BARNYARD GRASS | - | - | - | - | - | - | - | - |
| WILD OATS | - | - | - | - | - | - | - | - |
| WHEAT | - | - | - | - | - | - | - | - |
| CORN | - | - | - | - | - | - | - | - |
| SOYBEAN | - | - | - | - | - | - | - | - |
| RICE | - | - | - | - | - | - | - | - |
| SORGHUM | - | - | - | - | - | - | - | - |
| CHEATGRASS | - | - | - | - | - | - | - | - |
| SUGAR BEETS | - | - | - | - | - | - | - | - |
| VELVETLEAF | - | - | - | - | - | - | - | - |
| GIANT FOXTAIL | - | - | - | - | - | - | - | - |
| BARLEY | - | - | - | - | - | - | - | - |

TEST A

| | CMPD 65 | | CMPD 66 | | CMPD 67 | | CMPD 68 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 10C | 5C,9G | 9C | 4C,9G | 9C | 4C,8H | 9C | 10C |
| MORNING GLORY | 10C | 10C | 9C | 9C | 10C | 10C | 10C | 10C |
| COCKLEBUR | 10C | 10C | 10C | 10C | 10C | 5C,9G | 10C | 9C |
| NUTSEDGE | 10C | 4C,9G | 10C | 10C | 10C | 4C,8G | 4C,8G | 3C,7G |
| CRABGRASS | 4C,8H | 3C,6G | 3C,7G | 3C,5G | 4C,8H | 4G | 2C,4G | 0 |
| BARNYARD GRASS | 9C | 4C,9H | 6C,9H | 4C,9H | 10C | 4C,9H | 10C | 3C,9H |
| WILD OATS | 5C,9G | 3C,9G | 3C,4G | 0 | 3C,9G | 2C,9G | 5C,9G | 5C,9G |
| WHEAT | 9C | 3C,9G | 9C | 5G | 9C | 4C,9G | 10C | 10C |
| CORN | 10C | 4C,9H | 9C | 3C,9H | 9C | 5C,9G | 4C,9G | 2C,9G |
| SOYBEAN | 9C | 5C,9G | 2C,8H | 3H,5G | 9C | 5C,9G | 9C | 9C |
| RICE | 9C | 9C | 9C | 9C | 9C | 9C | 9C | 9C |
| SORGHUM | 10C | 5C,9H | 10C | 4C,9H | 10C | 9C | 9C | 10C |
| CHEATGRASS | 9C | 3C,8G | 6C,9G | 2C,5G | 9C | 6G | 9C | 9C |
| SUGAR BEETS | 10C | 10C | 10C | 9C | 10C | 9C | 10C | 5C,9G |
| VELVETLEAF | 10C | 9C | 10C | 5C,9G | 10C | 9C | 10C | 10C |
| GIANT FOXTAIL | 9C | 9C | 9C | 9C | 9C | 9C | 9C | 5C,9G |
| BARLEY | 6C,9G | 4C,9G | 4C,9G | 8G | 4C,9G | 2C,7G | 5C,9G | 5C,9G |
| PREEMERGENCE | | | | | | | | |
| COTTON | - | - | - | - | - | - | - | - |
| MORNING GLORY | - | - | - | - | - | - | - | - |
| COCKLEBUR | - | - | - | - | - | - | - | - |
| NUTSEDGE | - | - | - | - | - | - | - | - |
| CRABGRASS | - | - | - | - | - | - | - | - |
| BARNYARD GRASS | - | - | - | - | - | - | - | - |
| WILD OATS | - | - | - | - | - | - | - | - |
| WHEAT | - | - | - | - | - | - | - | - |
| CORN | - | - | - | - | - | - | - | - |
| SOYBEAN | - | - | - | - | - | - | - | - |
| RICE | - | - | - | - | - | - | - | - |
| SORGHUM | - | - | - | - | - | - | - | - |
| CHEATGRASS | - | - | - | - | - | - | - | - |
| SUGAR BEETS | - | - | - | - | - | - | - | - |
| VELVETLEAF | - | - | - | - | - | - | - | - |
| GIANT FOXTAIL | - | - | - | - | - | - | - | - |
| BARLEY | - | - | - | - | - | - | - | - |

TEST A

| | CMPD 69 | | CMPD 70 | | CMPD 71 | | CMPD 72 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 10C | 10C | 10C | 5C,9G | 10C | 9C | 9C | 9C |
| MORNING GLORY | 10C | 10C | 9C | 10C | 10C | 10C | 10C | 10C |
| COCKLEBUR | 10C | 10C | 9C | 10C | 10C | 10C | 10C | 9C |
| NUTSEDGE | 10C | 3C,8G | 10C | 4C,8G | 9C | 4G | 4C,8G | 5G |
| CRABGRASS | 3C,7G | 2C | 6G | 0 | 2C,8G | 4G | 4G | 0 |
| BARNYARD GRASS | 9C | 10C | 6C,9H | 4C,9H | 9C | 3C,9H | 3C,9H | 4C,9H |
| WILD OATS | 9C | 6C,9G | 3C,7G | 2G | 4C,9G | 3C,6G | 2C,5G | 2G |
| WHEAT | 10C | 9C | 5G | 0 | 4C,9G | 4G | 2C,5G | 2G |
| CORN | 4U,9C | 9C | 3C,9G | 3C,9H | 5C,9G | 4C,9G | 2C,8H | 2C,5H |
| SOYBEAN | 9C | 9C | 9C | 9C | 9C | 4C,9G | 3C,5G | 2H,3G |
| RICE | 9C | 9C | 9C | 9C | 9C | 5C,9G | 9C | 4C,9G |
| SORGHUM | 10C | 9C | 9C | 5C,9G | 5C,9G | 3C,9G | 4C,9G | 4C,9G |
| CHEATGRASS | 10C | 9C | 9C | 4C,9G | 9C | 3C,6G | 4C,9G | 6G |
| SUGAR BEETS | 10C | 9C | 10C | 4C,9G | 10C | 3C,8G | 9C | 2C,5G |
| VELVETLEAF | 10C | 10C | 10C | 10C | 10C | 10C | 10C | 9C |
| GIANT FOXTAIL | 10C | 9C | 9C | 4C,9G | 9C | 5C,9G | 3C,8G | 3C,6G |
| BARLEY | 6C,9G | 9C | 4C,8G | 3C,8G | 4C,9G | 3C,9G | 2C,6G | 2G |
| PREEMERGENCE | | | | | | | | |
| COTTON | - | - | - | - | - | - | - | - |
| MORNING GLORY | - | - | - | - | - | - | - | - |
| COCKLEBUR | - | - | - | - | - | - | - | - |
| NUTSEDGE | - | - | - | - | - | - | - | - |
| CRABGRASS | - | - | - | - | - | - | - | - |
| BARNYARD GRASS | - | - | - | - | - | - | - | - |
| WILD OATS | - | - | - | - | - | - | - | - |
| WHEAT | - | - | - | - | - | - | - | - |
| CORN | - | - | - | - | - | - | - | - |
| SOYBEAN | - | - | - | - | - | - | - | - |
| RICE | - | - | - | - | - | - | - | - |
| SORGHUM | - | - | - | - | - | - | - | - |
| CHEATGRASS | - | - | - | - | - | - | - | - |
| SUGAR BEETS | - | - | - | - | - | - | - | - |
| VELVETLEAF | - | - | - | - | - | - | - | - |
| GIANT FOXTAIL | - | - | - | - | - | - | - | - |
| BARLEY | - | - | - | - | - | - | - | - |

TEST A

| | CMPD 73 | | CMPD 74 | | CMPD 75 | | CMPD 76 | |
|---|---|---|---|---|---|---|---|---|
| RATE RATE=KG/HA | 0.05 | 0.01 | 0.05 | 0.01 | 0.05 | 0.01 | 0.05 | 0.01 |
| POSTEMERGENCE | | | | | | | | |
| COTTON | 9C | 9C | 10C | 2G | 5C,9G | 3G | 4G | 0 |
| MORNING GLORY | 10C | 4C,9G | 10C | 10C | 10C | 2C,7G | 5C,9G | 6G |
| COCKLEBUR | 10C | 5C,9G | 9C | 3C,9G | 9C | 7H | 3C,9H | 5H |
| NUTSEDGE | 4C,8G | 5G | 3G | 2G | 3G | 2G | 2C,5G | 3G |
| CRABGRASS | 4C,9G | 5G | 3G | 0 | 5G | 0 | 0 | 0 |
| BARNYARD GRASS | 9C | 9C | 5C,9H | 3C,9H | 4C,9H | 3C,7H | 3C,9H | 3H |
| WILD OATS | 4C,9G | 4C,9G | 2G | 0 | 0 | 0 | 0 | 0 |
| WHEAT | 2C,8G | 5G | 0 | 0 | 0 | 0 | 0 | 0 |
| CORN | 4C,9G | 2C,9H | 3C,8H | 1C,1H | 3C,9H | 3C,5H | 3C,5G | 0 |
| SOYBEAN | 9C | 5C,9G | 4C,9G | 3C,5G | 3C,8G | 3H | 4H | 2H |
| RICE | 9C | 5C,9G | 9C | 3C,8G | 5C,9G | 5C,9G | 3C,9G | 2C,6G |
| SORGHUM | 10C | 4C,9G | 2C,8H | 3G | 2C,8G | 2G | 3C,9G | 4G |
| CHEATGRASS | 9C | 8G | 2C,5G | 0 | 3G | 0 | 5G | 0 |
| SUGAR BEETS | 10C | 3C,6G | 9C | 4G | 2C,8G | 2G | 6G | 0 |
| VELVETLEAF | 10C | 10C | 9C | 7G | 10C | 2G | 8G | 4G |
| GIANT FOXTAIL | 9C | 4C,9G | 3C,6G | 3G | 5G | 4G | 3C,6G | 2G |
| BARLEY | 4C,9G | 3C,9G | 2C,7G | 2C,5G | 3C,7G | 0 | 8G | 0 |
| PREEMERGENCE | | | | | | | | |
| COTTON | - | - | - | - | - | - | - | - |
| MORNING GLORY | - | - | - | - | - | - | - | - |
| COCKLEBUR | - | - | - | - | - | - | - | - |
| NUTSEDGE | - | - | - | - | - | - | - | - |
| CRABGRASS | - | - | - | - | - | - | - | - |
| BARNYARD GRASS | - | - | - | - | - | - | - | - |
| WILD OATS | - | - | - | - | - | - | - | - |
| WHEAT | - | - | - | - | - | - | - | - |
| CORN | - | - | - | - | - | - | - | - |
| SOYBEAN | - | - | - | - | - | - | - | - |
| RICE | - | - | - | - | - | - | - | - |
| SORGHUM | - | - | - | - | - | - | - | - |
| CHEATGRASS | - | - | - | - | - | - | - | - |
| SUGAR BEETS | - | - | - | - | - | - | - | - |
| VELVETLEAF | - | - | - | - | - | - | - | - |
| GIANT FOXTAIL | - | - | - | - | - | - | - | - |
| BARLEY | - | - | - | - | - | - | - | - |

TEST A

| RATE RATE=KG/HA | CMPD 77 | | CMPD 78 | | CMPD 79 | | CMPD 80 | |
|---|---|---|---|---|---|---|---|---|
| | 0.05 | 0.01 | 0.05 | 0.01 | 0.05 | 0.01 | 0.05 | 0.01 |
| POSTEMERGENCE | | | | | | | | |
| COTTON | 8G | 2G | 4C,9G | 5G | 4C,9G | 4G | 5C,9G | - |
| MORNING GLORY | 10C | 2C,8G | 10C | 4C,9G | 4C,9G | 2C,8G | 4C,8G | - |
| COCKLEBUR | 4C,9H | 2H,5G | 10C | 7G | 10C | 2C,8G | 5C,9G | - |
| CASSIA | - | - | - | - | - | - | - | - |
| NUTSEDGE | 3C,9G | 5G | 5G | 3G | 7G | 0 | 0 | - |
| CRABGRASS | 6G | 0 | 2G | 0 | 3G | 2G | 0 | - |
| BARNYARD GRASS | 4C,9H | 6H | 5H | 0 | 4C,9H | 2H | 3C,3H | - |
| WILD OATS | 0 | 0 | 0 | 0 | 0 | 0 | 0 | - |
| WHEAT | 0 | 0 | 0 | 0 | 0 | 0 | 0 | - |
| CORN | 4G | 0 | 2H | 0 | 1H | 0 | 3C,9H | - |
| SOYBEAN | 5H | 1H | 5C,9G | 3C,8H | 5C,9G | 4H | 4C,9G | - |
| RICE | 4C,9G | 4G | 4C,9G | 6G | 9C | 2C,8G | 4C,9G | - |
| SORGHUM | 3C,9H | 4G | 5G | 0 | 4C,9G | 1H | 3C,8H | - |
| CHEATGRASS | 4G | 0 | 0 | 0 | 4G | 0 | 0 | - |
| SUGAR BEETS | 7G | 2H | 10C | 4C,9G | 9C | 5C,9G | 2C,4H | - |
| VELVETLEAF | 2C,9G | 2G | 1H | 2G | 9C | 5G | 2C,7G | - |
| GIANT FOXTAIL | 4G | 2G | 0 | 0 | 2G | 0 | - | - |
| BARLEY | 6G | 0 | 0 | 0 | 0 | 0 | - | - |
| PREEMERGENCE | | | | | | | | |
| COTTON | - | - | - | - | - | - | - | - |
| MORNING GLORY | - | - | - | - | - | - | - | - |
| COCKLEBUR | - | - | - | - | - | - | - | - |
| NUTSEDGE | - | - | - | - | - | - | - | - |
| CRABGRASS | - | - | - | - | - | - | - | - |
| BARNYARD GRASS | - | - | - | - | - | - | - | - |
| WILD OATS | - | - | - | - | - | - | - | - |
| WHEAT | - | - | - | - | - | - | - | - |
| CORN | - | - | - | - | - | - | - | - |
| SOYBEAN | - | - | - | - | - | - | - | - |
| RICE | - | - | - | - | - | - | - | - |
| SORGHUM | - | - | - | - | - | - | - | - |
| CHEATGRASS | - | - | - | - | - | - | - | - |
| SUGAR BEETS | - | - | - | - | - | - | - | - |
| VELVETLEAF | - | - | - | - | - | - | - | - |
| GIANT FOXTAIL | - | - | - | - | - | - | - | - |
| BARLEY | - | - | - | - | - | - | - | - |

TEST A

| | CMPD 81 | | CMPD 82 | | CMPD 83 | |
|---|---|---|---|---|---|---|
| RATE RATE=KG/HA | 0.05 | 0.01 | 0.05 | 0.01 | 0.05 | 0.01 |
| POSTEMERGENCE | | | | | | |
| COTTON | 5C,9G | - | 5C,9G | - | 9C | - |
| MORNING GLORY | 5C,9G | - | 9C | - | 10C | - |
| COCKLEBUR | 5C,9G | - | 9C | - | 10C | - |
| NUTSEDGE | 0 | - | 2C,3G | - | 0 | - |
| CRABGRASS | 0 | - | 2G | - | 0 | - |
| BARNYARD GRASS | 0 | - | 1C,2H | - | 3G | - |
| WILD OATS | 0 | - | 0 | - | 0 | - |
| WHEAT | 0 | - | 3G | - | 3G | - |
| CORN | 2C,7H | - | 5C,9H | - | 2C,8H | - |
| SOYBEAN | 4C,9G | - | 4C,9H | - | 3C,8H | - |
| RICE | 5C,9G | - | 4C,9G | - | 5C,9G | - |
| SORGHUM | 2C,7H | - | 2C,6G | - | 2C,7H | - |
| CHEATGRASS | 0 | - | 0 | - | 0 | - |
| SUGAR BEETS | 1H | - | 4C,8H | - | 9C | - |
| VELVETLEAF | 2C,7G | - | 5C,9G | - | 9C | - |
| GIANT FOXTAIL | - | - | - | - | - | - |
| BARLEY | - | - | - | - | - | - |
| PREEMERGENCE | | | | | | |
| COTTON | - | - | - | - | - | - |
| MORNING GLORY | - | - | - | - | - | - |
| COCKLEBUR | - | - | - | - | - | - |
| NUTSEDGE | - | - | - | - | - | - |
| CRABGRASS | - | - | - | - | - | - |
| BARNYARD GRASS | - | - | - | - | - | - |
| WILD OATS | - | - | - | - | - | - |
| WHEAT | - | - | - | - | - | - |
| CORN | - | - | - | - | - | - |
| SOYBEAN | - | - | - | - | - | - |
| RICE | - | - | - | - | - | - |
| SORGHUM | - | - | - | - | - | - |
| CHEATGRASS | - | - | - | - | - | - |
| SUGAR BEETS | - | - | - | - | - | - |
| VELVETLEAF | - | - | - | - | - | - |
| GIANT FOXTAIL | - | - | - | - | - | - |
| BARLEY | - | - | - | - | - | - |

Test B

Two ten-inch in diameter plastic pans lined with polyethylene liners were filled with prepared Fallsington silt loam soil. One pan was planted with seeds of wheat (Triticum aestivum), barley (Hordeum vulgare), wild oats (Avena fatua), cheatgrass (Bromus secalinus), blackgrass (Alopecurus myosuroides), annual bluegrass (Poa annua), green foxtail (Setaria viridis), rapeseed (Brassica napus) and Italian ryegrass (Lolium multiflorum). The other pan was planted with seeds of Russian thistle (Salsola kali), kochia (Kochia scoparia), speedwell (Veronica persica), shepherd's purse (Capsella bursa-pastoris), Matricaria inodora, black nightshade (Solanum nigrum), wild buckwheat (Polygonum convolvulus), Galium aparine and sugar beets. The above two pans were treated preemergence. At the same time two pans in which the above plant species were growing were treated postemergence. Plant height at the time of treatment ranged from 1-15 cm depending on plant species.

The compounds applied were diluted with a non-phytotoxic solvent and sprayed over-the-top of the

pans. An untreated control and a solvent alone control were included for comparison. All treatments were maintained in the greenhouse for 20 days at which time the treatments were compared to the controls and the effects visually rated.

CMPD 84

| RATE KG/HA | 0.009 | 0.016 | 0.032 | 0.064 | 0.125 |
|---|---|---|---|---|---|
| PREEMERGENCE | | | | | |
| WHEAT | 0 | 0 | 0 | 0 | 20 |
| BARLEY | 0 | 0 | 0 | 30 | 40 |
| WILD OATS | 0 | 0 | 0 | 0 | 30 |
| DOWNY BROME | - | - | - | - | - |
| CHEATGRASS | 30 | 40 | 70 | 90 | 90 |
| BLACKGRASS | 70 | 80 | 80 | 90 | 90 |
| ANN. BLUEGRASS | 30 | 40 | 60 | 80 | 80 |
| GREEN FOXTAIL | 0 | 20 | 20 | 40 | 70 |
| QUACKGRASS | - | - | - | - | - |
| ITAL. RYEGRASS | 0 | 0 | 20 | 40 | 50 |
| RIPGUT BROME | - | - | - | - | - |
| RAPESEED | 95 | 100 | 100 | 100 | 100 |
| WINTER WHEAT | 0 | 0 | 0 | 0 | 20 |
| WINTER BARLEY | 0 | 0 | 20 | 30 | 50 |
| GOATGRASS | 0 | 0 | 0 | 0 | 40 |

Test C

Sixteen-cm diameter Wagner pots, equipped with a stoppered drain opening near the bottom of the side wall, were filled with Woodstown sandy loam. About 1500 ml of water were added to each pot to bring the water level to a point 3 cm above the soil surface. Japonica and Indica rice seedlings were transplanted. Also, a number of barnyardgrass (Echinochloa crusgalli) seeds were added to each pot. At the same time, seedlings or tubers of the following species were transplanted into the muddy soil: water plaintain (Alisma trivale), Scirpus (Scirpus mucranatus) and Cyperus (Cyperus difformis). The weed species selected for this test are of economic importance in major ricegrowing areas. The chemical treatments were applied within hours after transplanting two additional species selected from: water chestnut (Eleocharis spp.), arrowhead (Sagittaria latifolia) and pond plant (Potamogeton Americanus). Shortly after treatment, the drain hold was opened to drop the water level by two cm. Water was then added to restore the water level to its original height. The following day the draining and refilling process was repeated. The pots were then maintained in the greenhouse. Rates of application and plant response ratings were made 21 days after treatment.

These test results demonstrate the utility of compound 84 for weed control in rice. The results also show utility for Japonica rice and Indica rice. Useful rates of application range from 4-2000 g/ha with 4-32 g being preferred and 8-16 g being most preferred.

Test C

Compound ~~88~~ 84

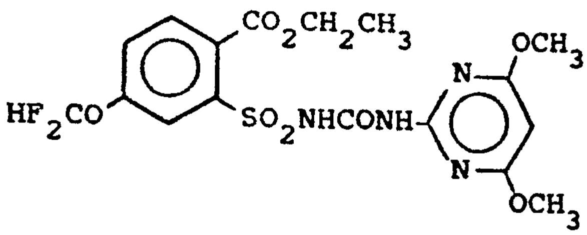

| Rate (g/ha) | 8 | 30 |
|---|---|---|
| Japonica Rice | 5 | 17 |
| Indica Rice | 0 | 20 |
| Barnyardgrass | 65 | 85 |
| Waterchestnut | 87 | 97 |
| Arrowhead | 92 | 95 |
| Scirpus | 65 | 72 |
| Cyperus | 100 | 100 |
| Water Plaintain | 75 | 90 |

| Rate (g/ha) | 8 | 16 | 30 | 63 |
|---|---|---|---|---|
| Japonica Rice | 0 | 5 | 0 | 17 |
| Indica Rice | 7 | 10 | 0 | 32 |
| Barnyardgrass | 80 | 90 | 85 | 95 |
| Arrowhead | 87 | 95 | 95 | 100 |
| Scirpus | 87 | 92 | 87 | 95 |
| Cyperus | 100 | 100 | 100 | 100 |
| Water Plaintain | 90 | 90 | 95 | 100 |
| Pond Plant | 97 | 100 | 100 | 100 |

## Claims

1. A compound of the formula

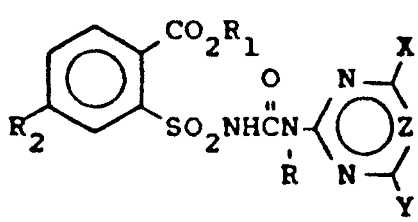

I

wherein

R is H or $CH_3$;

$R_1$ is $C_1$-$C_3$ alkyl, $C_3$-$C_4$ alkoxyalkyl, $C_2$-$C_4$ haloalkyl, $C_3$-$C_4$ alkenyl or $C_3$-$C_4$ alkynyl;

$R_2$ is $C_2$-$C_6$ alkoxy, $C_3$-$C_6$ cycloalkoxy, $C_4$-$C_6$ cycloalkylalkoxy, $C_1$-$C_6$ haloalkoxy, $C_2$-$C_6$ alkenyloxy, $C_2$-$C_6$ haloalkenyloxy, $C_3$-$C_6$ alkynyloxy, $C_3$-$C_6$ haloalkynyloxy, $C_2$-$C_4$ alkoxyalkoxy, $C_2$ -$C_4$ haloalkoxyalkoxy, $C_2$-$C_4$ alkylthioalkoxy, $C_2$-$C_4$ haloalkylthioalkoxy, $C_2$-$C_4$ alkylsulfinylalkoxy, $C_2$-$C_4$ haloalkylsulfinylalkoxy, $C_2$-$C_4$ alkylsulfonylalkoxy, $C_2$-$C_4$ haloalkylsulfonylalkoxy, $C_2$-$C_4$ cyanoalkoxy, $OCH_2C(O)CH_3$,$OCH_2CH_2C(O)CH_3$, $C_2$-$C_4$ aminoalkoxy, $C_1$-$C_8$ alkylthio, $C_3$-$C_6$ cycloalkylthio, $C_4$-$C_6$ cycloalkylalkylthio, $C_1$-$C_8$ haloalkylthio, $C_2$-$C_6$ alkenylthio, $C_2$-$C_6$ haloalkenylthio, $C_3$-$C_6$ alkynylthio, $C_3$-$C_6$ haloalkynylthio, $C_2$-$C_4$ alkoxyalkylthio, $C_2$-$C_4$ haloalkoxyalkylthio, $C_2$-$C_4$ alkylthioalkylthio, $C_2$-$C_4$ haloalkylthioalkylthio, $C_2$-$C_4$ cyanoalkylthio, $SCH_2C(O)CH_3$, $SCH_2CH_2C(O)CH_3$, $C_2$-$C_4$ aminoalkylthio, $SC_6H_5$, $SCH_2C_6H_5$, $C_1$-$C_8$ alkylsulfinyl, $C_3$-$C_6$ cycloalkylsulfinyl, $C_4$-$C_6$ cycloalkylalkylsulfinyl, $C_1$-$C_8$ haloalkylsulfinyl, $C_2$-$C_6$ alkenylsulfinyl, $C_2$-$C_6$ haloalkenylsulfinyl, $C_3$-$C_6$ alkynylsulfinyl, $C_3$-$C_6$ haloalkynylsulfinyl, $C_2$-$C_4$ alkoxyalkylsulfinyl, $C_2$-$C_4$ haloalkoxyalkylsulfinyl, $C_2$-$C_4$ cyanoalkylsulfinyl, $S(O)CH_2C(O)CH_3$, $S(O)CH_2CH_2C(O)CH_3$, $C_2$-$C_4$ aminoalkylsulfinyl, $C_2$-$C_8$ alkylsulfonyl, $C_3$-$C_6$ cycloalkylsulfonyl, $C_4$-$C_6$ cycloalkylalkylsulfonyl, $C_1$-$C_8$ haloalkylsulfonyl, $C_2$-$C_6$ alkenylsulfonyl, $C_2$-$C_6$ haloalkenylsulfonyl, $C_3$-$C_6$ alkynylsulfonyl, $C_3$-$C_6$ haloalkynylsulfonyl, $C_2$-$C_4$ alkoxyalkylsulfonyl, $C_2$-$C_4$ haloalkoxyalkylsulfonyl, $C_2$-$C_4$ cyanoalkylsulfonyl, $SO_2CH_2C(O)CH_3$, $SO_2CH_2CH_2C(O)CH_3$, $C_2$-$C_4$ aminoalkylsulfonyl, $CH_2F$, $CHF_2$, $CH_2Cl$, $CHCl_2$, $CH_2Br$, $CHBr_2$, $C_2$-$C_6$ alkyl substituted with 1-3 atoms of F, Cl or Br, $C_2$-$C_6$ alkenyl, $C_2$-$C_6$ haloalkenyl, $C_2$-$C_6$ haloalkynyl, $OC(O)C_1$-$C_4$ alkyl, $CH_2C(O)NR_aR_b$, $NHCH_3$, $NR_bR_c$ or $C_1$-$C_4$ alkyl substituted with $C_1$-$C_4$ alkoxy, $C_3$-$C_4$ cycloalkoxy, cyclopropylmethoxy, $C_1$-$C_4$ haloalkoxy, $C_2$-$C_4$ alkenyloxy, $C_2$-$C_4$ haloalkenyloxy, $C_3$-$C_4$ alkynyloxy, $C_3$-$C_4$ haloalkynyloxy, $C_2$-$C_4$ alkoxyalkoxy, $C_2$-$C_4$ aminoalkoxy, $C_1$-$C_4$ alkylcarbonyloxy, $C_1$-$C_4$ haloalkylcarbonyloxy, $C_1$-$C_4$ carbamoyloxy, $C_1$-$C_4$ alkoxycarbonyloxy, OH, $OP(O)(OC_1$-$C_2$ alkyl$)_2$, $C_1$-$C_4$ alkylsulfonyloxy, $C_1$-$C_2$ haloalkylsulfonyloxy, $OSi(CH_3)_3$, $OSi(CH_3)_2C(CH_3)_3$, $C_1$-$C_4$ alkylthio, $C_3$-$C_4$ cycloalkylthio, cyclopropylmethylthio, $C_1$-$C_4$ haloalkylthio, $C_2$-$C_4$ alkenylthio, $C_2$-$C_4$ haloalkenylthio, $C_3$-$C_4$ alkynylthio, $C_3$-$C_4$ haloalkynylthio, $C_2$-$C_4$ alkoxyalkylthio, $C_2$-$C_4$ aminoalkylthio, SH, $SP(O)(OC_1$-$C_2$ alkyl$)_2$, $C_1$-$C_4$ alkylsulfinyl, $C_3$-$C_4$ cycloalkylsulfonyl, cyclopropylmethylsulfinyl, $C_1$-$C_4$ haloalkylsulfinyl, $C_2$-$C_4$ alkenylsulfinyl, $C_2$-$C_4$ haloalkenylsulfinyl, $C_3$-$C_4$ alkynylsulfinyl, $C_3$-$C_4$ haloalkynylsulfinyl, $C_2$-$C_4$ alkoxyalkylsulfinyl, $C_2$-$C_4$ aminoalkylsulfinyl, $C_1$-$C_4$ alkylsulfonyl, $C_3$-$C_4$ cycloalkylsulfonyl, cyclopropylmethylsulfonyl, $C_1$-$C_4$ haloalkylsulfonyl, $C_2$-$C_4$ alkenylsulfonyl, $C_2$-$C_4$ haloalkenylsulfonyl, $C_3$-$C_4$ alkynylsulfonyl, $C_3$-$C_4$ haloalkynylsulfonyl, $C_2$-$C_4$ alkoxyalkylsulfonyl or $C_2$-$C_4$ aminoalkylsulfonyl;

$R_a$ and $R_b$ are independently H or $C_1$-$C_3$ alkyl;

$R_c$ is $C_2$-$C_4$ alkyl, cyclopropylmethyl, $C_2$-$C_4$ cyanoalkyl, $CH_2C(O)CH_3$, $CH_2CH_2C(O)CH_3$, $C_1$-$C_4$ haloalkyl, $C_3$-$C_4$ alkenyl, $C_3$-$C_4$ haloalkenyl, $C_3$-$C_4$ alkynyl, $C_3$-$C_4$ haloalkynyl, $C_1$-$C_4$ alkyl substituted with $C_1$-$C_4$ alkoxy, $C_1$-$C_4$ alkylthio, $C_1$-$C_4$ alkylsulfinyl, $C_1$-$C_4$ alkylsulfonyl, OH, $NH_2$, $NHCH_3$ or $N(CH_3)_2$;

X is $CH_3$, $OCH_3$, $OC_2H_5$, Cl or Br;

Y is $C_1$-$C_2$ alkyl, $C_1$-$C_2$ alkoxy, $OCH_2CH_2F$, $OCH_2CHF_2$,$OCH_2CF_3$, $NHCH_3$ or $N(CH_3)_2$; and

Z is CH or N; and

their agriculturally suitable salts;

provided that

1) when X is Cl or Br, then Z is CH and Y is $C_1$-$C_2$ alkoxy, $NHCH_3$ or $N(CH_3)_2$;

2) when $R_1$ is $C_1$-$C_3$ alkyl then $R_2$ is other than $C_2$-$C_6$ alkoxy, $C_1$-$C_8$ alkylthio, $C_1$-$C_8$ alkylsulfinyl, $C_2$-$C_8$ alkylsulfonyl, $C_3$-$C_6$ alkenyloxy, $C_3$-$C_6$ alkynyloxy, $C_3$-$C_6$ alkenylthio, $C_3$-$C_6$ alkenylsulfinyl, $C_3$-$C_6$ alkenylsulfonyl, $C_3$-$C_6$ alkynylthio, $C_3$-$C_6$ alkynylsulfinyl, $C_3$-$C_6$ alkynylsulfonyl, $OCH_2CH_2OCH_3$, $OCH_2CH_2SCH_3$, $OCH_2CH_2S(O)CH_3$, $OCH_2CH_2SO_2CH_3$, $C_2$-$C_6$ alkyl substituted with 1-3 atoms of F, Cl or Br, $CH_2F$, $CHF_2$, $C_1$-$C_4$ alkyl substituted with $C_1$-$C_2$ alkoxy, $C_1$-$C_2$ alkylthio, $C_1$-$C_2$ alkylsulfinyl or $C_1$-$C_2$ alkylsulfonyl, $OCF_2H$, $OCH_2CH_2F$, $OCH_2CHF_2$, $OCH_2CF_3$, $OCH_2CH_2Cl$, S-cyclohexyl, S-$C_6H_5$ or $SCH_2C_6H_5$.

**2.** A compound of Claim 1 wherein

$R_2$ is $C_2$-$C_6$ alkoxy, $C_1$-$C_8$ alkylthio, $C_1$-$C_8$ alkylsulfinyl, $C_2$-$C_8$ alkylsulfonyl, $C_3$-$C_6$ alkenyloxy,

$C_3$-$C_6$ alkynyloxy, $C_3$-$C_6$ alkenylthio, $C_3$-$C_6$ alkenylsulfinyl, $C_3$-$C_6$ alkenylsulfonyl, $C_3$-$C_6$ alkynylthio, $C_3$-$C_6$ alkynylsulfinyl, $C_3$-$C_6$ alkynylsulfonyl, $OCH_2CH_2OCH_3$, $OCH_2CH_2SCH_3$, $OCH_2CH_2S(O)CH_3$, $OCH_2CH_2SO_2CH_3$,$C_2$-$C_6$ alkyl substituted with 1-3 atoms of F, Cl or Br, $CH_2F$, $CHF_2$, $C_1$-$C_4$ alkyl substituted with $C_1$-$C_2$ alkoxy, $C_1$-$C_2$ alkylthio, $C_1$-$C_2$ alkylsulfinyl or $C_1$-$C_2$ alkylsulfonyl, $OCF_2H$, $OCH_2CH_2F$, $OCH_2CHF_2$, $OCH_2CF_3$, $OCH_2CH_2Cl$, S-cyclohexyl, $SC_6H_5$ or $SCH_2C_6H_5$;

3. A compound of Claim 2 wherein
   $R_2$ is $CH_3S$, $C_3$-$C_6$ alkenyloxy, $C_3$-$C_6$ alkynyloxy, $C_3$-$C_6$ alkenylthio, $C_3$-$C_6$ alkenylsulfinyl, $C_3$-$C_6$ alkenylsulfonyl, $C_3$-$C_6$ alkynylthio, $C_3$-$C_6$ alkynylsulfinyl, $C_3$-$C_6$ alkynylsulfonyl, $C_2$-$C_6$ alkyl substituted with 1-3 atoms of F, Cl or Br, $CH_2F$, $CHF_2$, $C_1$-$C_4$ alkyl substituted in a nonbenzylic position with $C_1$-$C_2$ alkoxy, $C_1$-$C_2$ alkylthio, $C_1$-$C_2$ alkylsulfinyl or $C_1$-$C_2$ alkylsulfonyl, $OCH_2CH_2OCH_3$, $OCH_2CH_2SCH_3$, $OCH_2CH_2S(O)CH_3$, $OCN_2CH_2SO_2CH_3$,$OCF_2H$, $OCH_2CH_2F$, $OCH_2CHF_2$, $OCH_2CF_3$, $OCH_2CH_2Cl$, S-cyclohexyl, $SC_6H_5$ or $SCH_2C_6H_5$.

4. A compound of Claim 3 wherein R is H,

5. A compound of Claim 4 wherein
   $R_2$ is $CH_3S$, $C_3$-$C_4$ alkenyloxy, $C_3$-$C_4$ alkenylthio, $C_3$-$C_4$ alkenylsulfinyl, $C_3$-$C_4$ alkenylsulfonyl, $C_2$-$C_3$ alkyl substituted with 1-3 atoms of F or Cl, $CH_2F$, $CHF_2$, $OCHF_2$, $OCH_2CH_2F$, $OCH_2CF_3$ or $OCH_2CH_2Cl$.

6. A compound of Claim 5 wherein
   $R_1$ is $CH_3$ or $CH_2CH_3$;
   X is $CH_3$, $OCH_3$ or Cl; and
   Y is $CH_3$, $OCH_3$, $C_2H_5$ or $OC_2H_5$.

7. An agriculturally suitable composition for controlling the growth of undesired vegetation or for use as a plant growth regulant comprising an effective amount of a compound of any of Claims 1 to 6 and at least one of the following: surfactant, solid or liquid diluent.

8. A method for controlling the growth of undesired vegetation which comprises applying to the locus to be protected an effective amount of a compound of any of Claims 1 to 6.

9. A method for controlling the growth of undesired vegetation which comprises applying to the locus of the rice paddy rice crop to be protected an effective amount of the compound

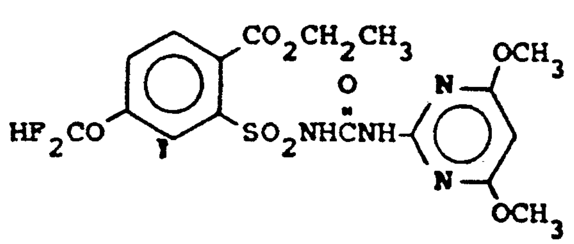

.

10. A method for controlling the growth of blackgrass in wheat and barley which comprises applying to the locus to be protected an effective amount of the compound

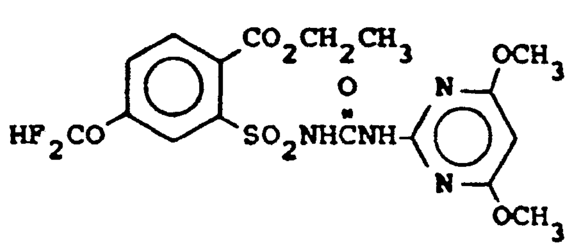

.

**11.** A method for regulating the growth of plants which comprises applying to the locus of such plants an effective but substantially non-phytotoxic amount of plant growth regulant compound of any of claims 1 to 6.

**12.** A process for the preparation of a compound of claim 1 which comprises:

(a) reacting a sulfonyl isocyanate of formula

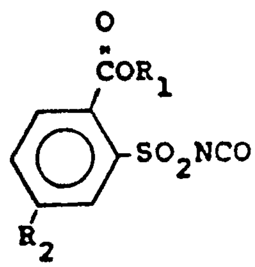

(1)

with an amino or methylamino heterocycle of formula

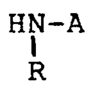

(2);

(b) reacting a phenyl carbamate of formula

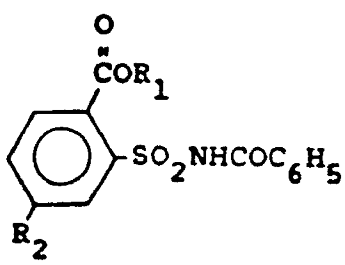

(3)

with said amino or methylamino heterocycle;

(c) reacting a sulfonamide of formula

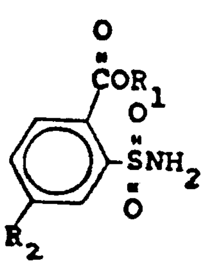

(4)

with a heterocyclic phenyl carbamate of formula

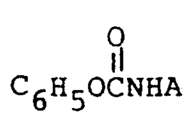

(5);

wherein A, R, $R_1$ and $R_2$ are as defined in claim 1; or

(d) reacting a compound of formula

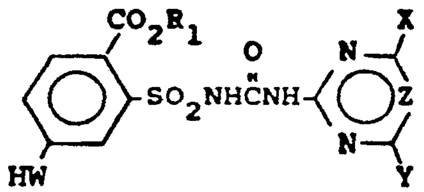

(46)

wherein $R_1$, X, Y and Z are as defined in claim 1; W is S, O or $NR_b$; $R_b$ is as defined in claim 1; with an appropriate alkylating, alkenylating or alkylylating agent of formula

$R_8X_1$

wherein $R_8$ is $C_4$-$C_6$ cycloalkylalkyl, $C_2$-$C_6$ alkenyl, $C_2$-$C_6$ haloalkenyl, $C_3$-$C_6$ alkynyl, $C_3$-$C_6$ haloalkynyl, $C_2$-$C_8$ alkoxyalkyl, $C_2$-$C_4$ haloalkoxyalkyl, $C_2$-$C_8$ alkylthioalkyl $C_2$-$C_4$ haloalkylthioalkyl, $C_2$-$C_4$ cyanoalkyl, $CH_2C(O)CH_3$, $CH_2CH_2C(O)CH_3$ or $C_1$-$C_6$ alkyl;

and $X_1$ is Cl, Br, I, 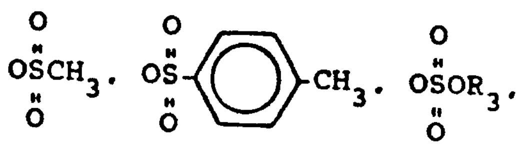

or 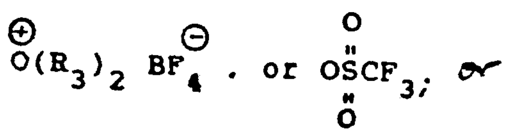

(e) oxidising a compound of formula (I) wherein $R_8$ is alkyl, cycloalkyl, cycloalkylalkyl, haloalkyl, alkenyl, haloalkenyl, alkynyl, haloalkynyl, alkoxyalkyl, haloalkoxyalkyl, cyanoalkyl, $-CH_2C(O)CH_3$, or $-CH_2CH_2C(O)CH_3$ and W is S to obtain a corresponding product wherein W is S(O) or $SO_2$.

**13.** Intermediates suitable for the preparation of a compound of claim 1, having the formula

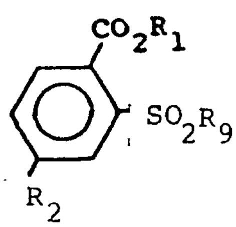

wherein $R_1$ and $R_2$ are as defined in claim 1 (wherein proviso 2 applies) and $R_9$ is NCO, $NH_2$ or $NHCOC_6H_5$

**14.** Intermediates suitable for the preparation of a compound of claim 1, having the formula (46) as defined in claim 12.

## Revendications

**1.** Un composé de la formule

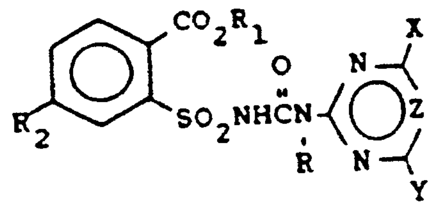

I

dans laquelle

R est H ou $CH_3$ ;

$R_1$ est un groupe alkyle en $C_1$-$C_3$, alcoxyalkyle en $C_3$-$C_4$, halogénalkyle en $C_2$-$C_4$, alcényle en $C_3$-$C_4$ ou alcynyle en $C_3$-$C_4$ ;

$R_2$ est un groupe alcoxy en $C_2$-$C_6$, cycloalcoxy en $C_3$-$C_6$, cycloalkylalcoxy en $C_4$-$C_6$,halogénalcoxy en $C_1$-$C_6$, alcényloxy en $C_2$-$C_6$, halogénalcényloxy en $C_2$-$C_6$, alcynyloxy en $C_3$-$C_6$, halogénalcynyloxy en $C_3$-$C_6$,alcoxyalcoxy en $C_2$-$C_4$, halogénalcoxyalcoxy en $C_2$-$C_4$, alkylthioalcoxy en $C_2$-$C_4$,halogénalkylthioalcoxy en $C_2$-$C_4$, alkylsulfinylalcoxy en $C_2$-$C_4$, halogénalkylsulfinylalcoxy en $C_2$-$C_4$, alkylsulfonylalcoxy en $C_2$-$C_4$, halogénalkylsulfonylalcoxy en $C_2$-$C_4$, cyanoalcoxy en $C_2$-$C_4$, $OCH_2C(O)CH_3$, $OCH_2CH_2C(O)CH_3$, aminoalcoxy en $C_2$-$C_4$, alkylthio en $C_1$-$C_8$, cycloalkylthio en $C_3$-$C_6$, cycloalkylalkylthio en $C_4$-$C_6$, halogénalkylthio en $C_1$-$C_8$, alcénylthio en $C_2$-$C_6$,halogénalcénylthio en $C_2$-$C_6$, alcynylthio en $C_3$-$C_6$, halogénalcynylthio en $C_3$-$C_6$, alcoxyalkylthio en $C_2$-$C_4$, halogénalcoxyalkylthio en $C_2$-$C_4$, alkylthioalkylthio en $C_2$-$C_4$, halogénalkylthioalkylthio en $C_2$-$C_4$, cyanoalkylthio en $C_2$-$C_4$, $SCH_2C(O)CH_3$, $SCH_2CH_2C(O)CH_3$, aminoalkylthio en $C_2$-$C_4$, $SC_6H_5$, $SCH_2C_6H_5$, alkylsulfinyle en $C_1$-$C_8$, cycloalkylsulfinyle en $C_3$-$C_6$, cycloalkylalkylsulfinyle en $C_4$-$C_6$, halogénalkylsulfinyle en $C_1$-$C_8$, alcénylsulfinyle en $C_2$-$C_6$, halogénalcénylsulfinyle en $C_2$-$C_6$, alcynylsulfinyle en $C_3$-$C_6$, halogénalcynylsulfinyle en $C_3$-$C_6$, alcoxyalkylsulfinyle en $C_2$-$C_4$, halogénalcoxyalkylsulfinyle en $C_2$-$C_4$, cyanoalkylsulfinyle en $C_2$-$C_4$, $S(O)CH_2C(O)CH_3$, $S(O)CH_2CH_2C(O)CH_3$, aminoalkylsulfinyle en $C_2$-$C_4$, alkylsulfonyle en $C_2$-$C_8$,cycloalkylsulfonyle en $C_3$-$C_6$, cycloalkylalkylsulfonyle en $C_4$-$C_6$, halogénalkylsulfonyle en $C_1$-$C_8$, alcénylsulfonyle en $C_2$-$C_6$, halogénalcénylsulfonyle en $C_2$-$C_6$, alcynylsulfonyle en $C_3$-$C_6$, halogénalcynylsulfonyle en $C_3$-$C_6$, alcoxyalkylsulfonyle en $C_2$-$C_4$, halogénalcoxyalkylsulfonyle en $C_2$-$C_4$, cyanoalkylsulfonyle en $C_2$-$C_4$, $SO_2CH_2C(O)CH_3$, $SO_2CH_2CH_2C(O)CH_3$, aminoalkylsulfonyle en $C_2$-$C_4$, $CH_2F$, $CHF_2$, $CH_2Cl$, $CH_cl_2$, $CH_2Br$, $CHBr_2$, alkyle en $C_2$-$C_6$ substitué par 1 à 3 atomes de F, Cl ou Br, alcényle en $C_2$-$C_6$, halogénalcényle en $C_2$-$C_6$, halogénalcynyle en $C_2$-$C_6$, OC(O)-alkyle en $C_1$-$C_4$, $CH_2C(O)NR_aR_b$,$NHCH_3$, $NR_bR_c$ ou alkyle en $C_1$-$C_4$ substitué par alcoxy en $C_1$-$C_4$, cycloalcoxy en $C_3$-$C_4$, cyclopropylméthoxy, halogénalcoxy en $C_1$-$C_4$, alcényloxy en $C_2$-$C_4$, halogénalcényloxy en $C_2$-$C_4$, alcynyloxy en $C_3$-$C_4$, halogénalcynyloxy en $C_3$-$C_4$, alcoxyalcoxy en $C_2$-$C_4$, aminoalcoxy en $C_2$-$C_4$, alkylcarbonyloxy en $C_1$-$C_4$, halogénalkylcarbonyloxy en $C_1$-$C_4$, carbamyloxy en $C_1$-$C_4$, alcoxycarbonyloxy en $C_1$-$C_4$, OH, OP(O)(O-alkyle en $C_1$-$C_2$)$_2$, alkylsulfonyloxy en $C_1$-$C_4$, halogénalkylsulfonyloxy en $C_1$-$C_2$, $OSi(CH_3)_3$, $OSi(CH_3)_2C(CH_3)_3$, alkylthio en $C_1$-$C_4$, cycloalkylthio en $C_3$-$C_4$,cyclopropylméthylthio, halogénalkylthio en $C_1$-$C_4$, alcénylthio en $C_2$-$C_4$,halogénalcénylthio en $C_2$-$C_4$, alcynylthio en $C_3$-$C_4$, halogénalcynylthio en $C_3$-$C_4$, alcoxyalkylthio en $C_2$-$C_4$, aminoalkylthio en $C_2$-$C_4$, SH, SP(O)(O-alkyle en $C_1$-$C_2$)$_2$, alkylsulfinyle en $C_1$-$C_4$, cycloalkylsulfonyle en $C_3$-$C_4$, cyclopropylméthylsulfinyle, halogénalkylsulfinyle en $C_1$-$C_4$, alcénylsulfinyle en $C_2$-$C_4$, halogénalcénylsulfinyle en $C_2$-$C_4$, alcynylsulfinyle en $C_3$-$C_4$, halogénalcynylsulfinyle en $C_3$-$C_4$, alcoxyalkylsulfinyle en $C_2$-$C_4$, aminoalkylsulfinyle en $C_2$-$C_4$, alkylsulfonyle an $C_1$-$C_4$, cycloalkylsulfonyle en $C_3$-$C_4$, cyclopropylméthylsulfonyle, halogénalkylsulfonyle en $C_1$-$C_4$, alcénylsulfonyle en $C_2$-$C_4$, halogénalcénylsulfonyle en $C_2$-$C_4$, alcynylsulfonyle en $C_3$-$C_4$, halogénalcynylsulfonyle en $C_3$-$C_4$, alcoxyalkylsulfonyle en $C_2$-$C_4$ ou aminoalkylsulfonyle en $C_2$-$C_4$ ;

$R_a$ et $R_b$ sont indépendamment H ou un groupe alkyle en $C_1$-$C_3$ ;

$R_c$ est un groupe alkyle en $C_2$-$C_4$, cyclopropylméthyle, cyanoalkyle en $C_2$-$C_4$, $CH_2C(O)CH_3$, $CH_2CH_2C(O)CH_3$, halogénalkyle en $C_1$-$C_4$, alcényle en $C_3$-$C_4$, halogénalcényle en $C_3$-$C_4$, alcynyle en $C_3$-$C_4$, halogénalcynyle en $C_3$-$C_4$, alkyle en $C_1$-$C_4$ substitué par

alcoxy en $C_1$-$C_4$, alkylthio en $C_1$-$C_4$, alkylsulfinyle en $C_1$-$C_4$, alkylsulfonyle en $C_1$-$C_4$, OH, $NH_2$, $NHCH_3$ ou $N(CH_3)_2$ ;

x est $CH_3$, $OCH_3$, $OC_2H_5$, Cl ou Br ;

Y est un groupe alkyle en $C_1$-$C_2$, alcoxy en $C_1$-$C_2$, $OCH_2CH_2F$, $OCH_2CHF_2$, $OCH_2CF_3$, $NHCH_3$ ou $N(CH_3)_2$ ; et

Z est CH ou N ; et

ses sels utilisables en agriculture ;

avec les conditions suivantes

1) si X est Cl ou Br, alors Z est CH et Y est un groupe alcoxy en $C_1$-$C_2$, $NHCH_3$ ou $N(CH_3)_2$ ;

2) si $R_1$ est un groupe alkyle en $C_1$-$C_3$, alors $R_2$ est autre chose qu'un groupe alcoxy en $C_2$-$C_6$, alkylthio en $C_1$-$C_8$, alkylsulfinyle en $C_1$-$C_8$, alkylsulfonyle en $C_2$-$C_8$, alcényloxy en $C_3$-$C_6$, alcynyloxy en $C_3$-$C_6$, alcénylthio en $C_3$-$C_6$, alcénylsulfinyle en $C_3$-$C_6$, alcénylsulfonyle en $C_3$-$C_6$, alcynylthio en $C_3$-$C_6$, alcynylsulfinyle en $C_3$-$C_6$, alcynylsulfonyle en $C_3$-$C_6$ $OCH_2CH_2OCH_3$, $OCH_2CH_2SCH_3$, $OCH_2CH_2S(O)CH_3$, $OCH_2CH_2SO_2CH_3$, alkyle en $C_2$-$C_6$ substitué par 1 à 3 atomes de F, Cl ou Br, $CH_2F$, $CHF_2$, alkyle an $C_1$-$C_4$ substitué par alcoxy en $C_1$-$C_2$, alkylthio an $C_1$-$C_2$, alkylsulfinyle en $C_1$-$C_2$ ou alkylsulfonyle en $C_1$-$C_2$, $OCF_2H$, $OCH_2CH_2F$, $OCH_2CHF_2$, $OCH_2CF_3$, $OCH_2CH_2Cl$, S-cyclohexyle, S-$C_6H_5$ ou $SCH_2C_6H_5$.

**2.** Un composé de la revendication 1, dans lequel

$R_2$ est un groupe alcoxy en $C_2$-$C_6$, alkylthio en $C_1$-$C_8$, alkylsulfinyle en $C_1$-$C_8$, alkylsulfonyle en $C_2$-$C_8$, alcényloxy en $C_3$-$C_6$, alcynyloxy en $C_3$-$C_6$, alcénylthio en $C_3$-$C_6$, alcénylsulfinyle an $C_3$-$C_6$, alcénylsulfonyle en $C_3$-$C_6$, alcynylthio en $C_3$-$C_6$, alcynylsulfinyle en $C_3$-$C_6$, alcynylsulfonyle en $C_3$-$C_6$, $OCH_2CH_2OCH_3$, $OCH_2CH_2SCH_3$, $OCH_2CH_2S(O)CH_3$, $OCH_2CH_2SO_2CH_3$,alkyle en $C_2$-$C_6$ substitué par 1 à 3 atomes de F, Cl ou Br, $CH_2F$, $CHF_2$, alkyle en $C_1$-$C_4$ substitué par alcoxy en $C_1$-$C_2$, alkylthio en $C_1$-$C_2$, alkylsulfinyle en $C_1$-$C_2$ ou alkylsulfonyle en $C_1$-$C_2$, $OCF_2H$, $OCH_2CH_2F$, $OCH_2CHF_2$, $OCH_2CF_3$, $OCH_2CH_2Cl$, S-cyclohexyle, $SC_6H_5$ ou $SCH_2C_6H_5$ ;

**3.** Un composé de la revendication 2, dans lequel

$R_2$ est un groupe $CH_3S$, alcényloxy en $C_3$-$C_6$, alcynyloxy en $C_3$-$C_6$, alcénylthio en $C_3$-$C_6$, alcénylsulfinyle en $C_3$-$C_6$, alcénylsulfonyle en $C_3$-$C_6$, alcynylthio en $C_3$-$C_6$, alcynylsulfinyle en $C_3$-$C_6$, alcynylsulfonyle en $C_3$-$C_6$, alkyle en $C_2$-$C_6$ substitué par 1 à 3 atomes de F, Cl ou Br, $CH_2F$, $CHF_2$, alkyle en $C_1$-$C_4$ substitué en une position non benzylique par alcoxy en $C_1$-$C_2$, alkylthio en $C_1$-$C_2$, alkylsulfinyle en $C_1$-$C_2$ ou alkylsulfonyle en $C_1$-$C_2$,$OCH_2CH_2OCH_3$, $OCH_2CH_2SCH_3$, $OCH_2CH_2S(O)CH_3$, $OCH_2CH_2SO_2CH_3$, $OCF_2H$, $OCH_2CH_2F$, $OCH_2CHF_2$,$OCH_2CF_3$, $OCH_2CH_2Cl$, S-cyclohexyle, $SC_6H_5$ ou $SCH_2C_6H_5$.

**4.** Un composé de la revendication 3, dans lequel R est H.

**5.** Un composé de la revendication 4, dans lequel

$R_2$ est un groupe $CH_3S$, alcényloxy en $C_3$-$C_4$, alcénylthio en $C_3$-$C_4$, alcénylsulfinyle en $C_3$-$C_4$, alcénylsulfonyle en $C_3$-$C_4$, alkyle en $C_2$-$C_3$ substitué par 1 à 3 atomes de F ou Cl, $CH_2F$, $CHF_2$, $OCHF_2$, $OCH_2CH_2F$, $OCH_2CF_3$ ou $OCH_2CH_2Cl$.

**6.** Un composé de la revendication 5, dans lequel

$R_1$ est $CH_3$ ou $CH_2CH_3$ ;

X est $CH_3$, $OCH_3$ ou Cl ; et

Y est $CH_3$, $OCH_3$, $C_2H_5$ ou $OC_2H_5$.

**7.** Une composition utilisable en agriculture pour combattre la croissance d'une végétation indésirable ou pour être utilisée comme régulateur de la croissance de plantes, comprenant une quantité efficace d'un composé de l'une quelconque des revendications 1 à 6 et au moins l'un des ingrédients suivants : agent tensio-actif, diluant liquide ou solide.

**8.** Un procédé pour combattre la croissance d'une végétation indésirable, qui consiste à appliquer au lieu à protéger une quantité efficace d'un composé de l'une quelconque des revendications 1 à 6.

**9.** Un procédé pour combattre la croissance d'une végétation indésirable, qui consiste à appliquer au lieu

où se trouve la culture de riz de rizière à protéger une quantité efficace du composé

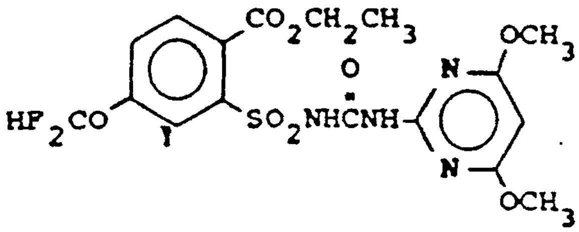

**10.** Un procédé pour combattre la croissance du vulpin des champs dans le blé et l'orge, qui consiste à appliquer à l'emplacement à protéger une quantité efficace du composé

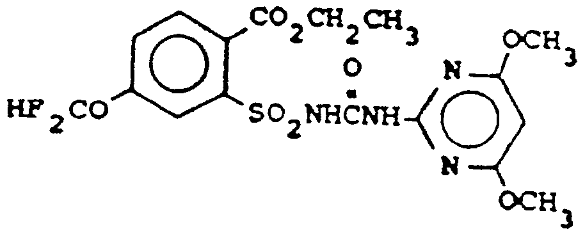

**11.** Un procédé pour réguler la croissance de plantes, qui consiste à appliquer au lieu où se trouvent ces plantes une quantité efficace, mais sensiblement non phytotoxique, d'un composé régulateur de la croissance de plantes de l'une quelconque des revendications 1 à 6.

**12.** Un procédé pour la préparation d'un composé de la revendication 1, qui consiste à :
(a) faire réagir un isocyanate de sulfonyle de formule

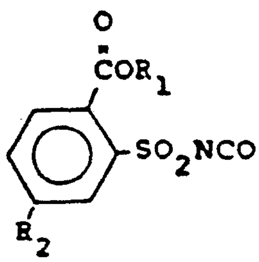

(1)

avec un amino- ou méthylamino-hétérocycle de formule

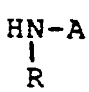

(2) ;

(b) faire réagir un carbamate de phényle de formule

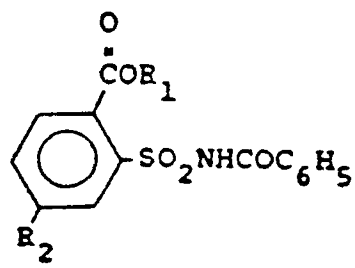

(3)

avec ledit amino- ou méthylamino-hétérocycle ;
(c) faire réagir un sulfonamide de formule

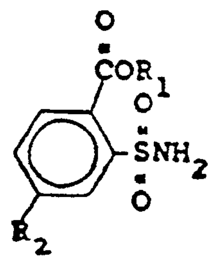

(4)

avec un carbamate de phényle hétérocyclique de formule

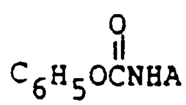

(5) ;

où A, R, $R_1$ et $R_2$ sont tels que définis dans la revendication 1 ; ou
(d) faire réagir un composé de formule

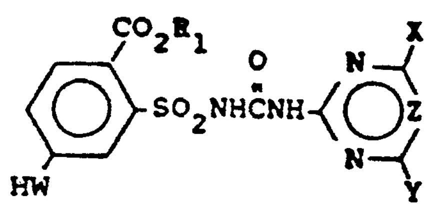

(46)

où $R_1$, X, Y et Z sont tels que définis dans la revendication 1 ; W est S, O ou $NR_b$ ; $R_b$ est tel que défini dans la revendication 1 ;
avec un agent alkylant, alcénylant ou alcynylant approprié de formule

$R_8X_1$

où $R_8$ est un groupe cycloalkylalkyle en $C_4$-$C_6$,alcényle en $C_2$-$C_6$, halogénalcényle en $C_2$-$C_6$, alcynyle en $C_3$-$C_6$, halogénalcynyle en $C_3$-$C_6$, alcoxyalkyle en $C_2$-$C_8$, halogénalcoxyalkyle en $C_2$-$C_4$, alkylthioalkyle en $C_2$-$C_8$,halogénalkylthioalkyle en $C_2$-$C_4$, cyanoalkyle en $C_2$-$C_4$, $CH_2C(O)CH_3$, $CH_2CH_2C(O)CH_3$ ou alkyle en $C_1$-$C_6$ ;

et $X_1$ is Cl, Br, I,

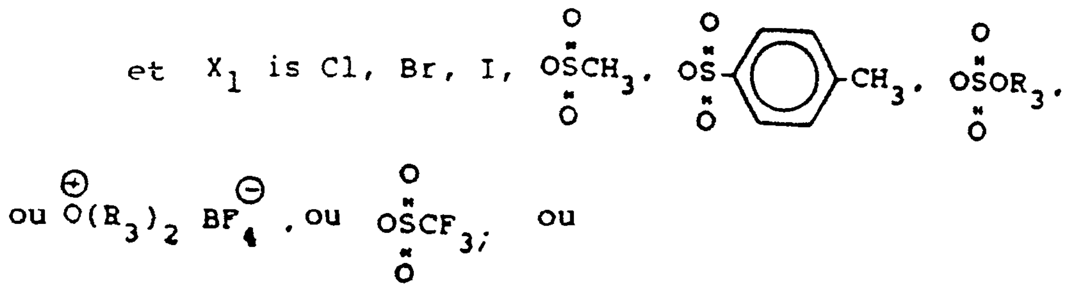

ou

(e) oxyder un composé de formule (I) dans lequel $R_8$ est un groupe alkyle, cycloalkyle, cycloalkylalkyle, halogénalkyle, alcényle, halogénalcényle, alcynyle, halogénalcynyle, alcoxyalkyle, halogénalcoxyalkyle, cyanoalkyle, $-CH_2C(O)CH_3$ ou $-CH_2CH_2C(O)CH_3$ et W est S, pour obtenir un produit correspondant dans lequel W est S(O) ou $SO_2$.

**13.** Composés intermédiaires convenant pour la préparation d'un composé de formule 1, répondant à la formule

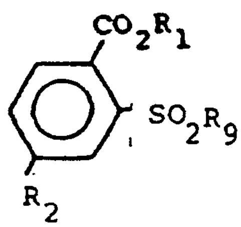

où $R_1$ et $R_2$ sont tels que définis dans la revendication 1 (avec les mêmes conditions) et $R_9$ est NCO, $NH_2$ ou $NHCOC_6H_5$.

**14.** Composés intermédiaires convenant pour la préparation d'un composé de la revendication 1, répondant à la formule (46) telle que définie dans la revendication 12.

## Patentansprüche

**1.** Verbindung der Formel

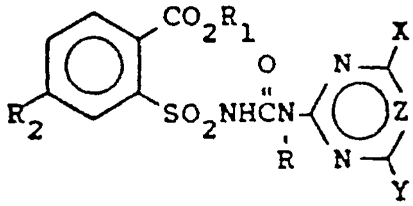

I

worin

| | |
|---|---|
| R | H oder $CH_3$ ist; |
| $R_1$ | $C_1$-$C_3$-Alkyl, $C_3$-$C_4$-Alkoxyalkyl, $C_2$-$C_4$-Halogenalkyl, $C_3$-$C_4$-Alkenyl oder $C_3$-$C_4$-Alkinyl ist; |
| $R_2$ | $C_2$-$C_6$-Alkoxy, $C_3$-$C_6$-Cycloalkoxy, $C_4$-$C_6$-Cycloalkylalkoxy, $C_1$-$C_6$-Halogenalkoxy, $C_2$-$C_6$-Alkenyloxy, $C_2$-$C_6$-Halogenalkenyloxy, $C_3$-$C_6$-Alkinyloxy, $C_3$-$C_6$-Halogenalkinyloxy, $C_2$-$C_4$-Alkoxyalkoxy, $C_2$-$C_4$-Halogenalkoxyalkoxy, $C_2$-$C_4$-Alkylthioalkoxy, $C_2$-$C_4$-Halogenalkylthioalkoxy, $C_2$-$C_4$-Alkylsulfinylalkoxy, $C_2$-$C_4$-Halogenalkylsulfinylalkoxy, $C_2$-$C_4$-Alkylsulfonylalkoxy, $C_2$-$C_4$-Halogenalkylsulfonylalkoxy, $C_2$-$C_4$-Cyanalkoxy, $OCH_2C$- |

(O)$CH_3$, $OCH_2CH_2C(O)CH_3$, $C_2$-$C_4$-Aminoalkoxy, $C_1$-$C_8$-Alkylthio, $C_3$-$C_6$-Cycloalkylthio, $C_4$-$C_6$-Cycloalkylalkylthio, $C_1$-$C_8$-Halogenalkylthio, $C_2$-$C_6$-Alkenylthio, $C_2$-$C_6$-Halogenalkenylthio, $C_3$-$C_6$-Alkinylthio, $C_3$-$C_6$-Halogenalkinylthio, $C_2$-$C_4$-Alkoxyalkylthio, $C_2$-$C_4$-Halogenalkoxyalkylthio, $C_2$-$C_4$-Alkylthioalkylthio, $C_2$-$C_4$-Halogenalkylthioalkylthio, $C_2$-$C_4$-Cyanalkylthio, $SCH_2C(O)CH_3$, $SCH_2CH_2C(O)CH_3$, $C_2$-$C_4$-Aminoalkylthio, $SC_6H_5$, $SCH_2C_6H_5$, $C_1$-$C_8$-Alkylsulfinyl, $C_3$-$C_6$-Cycloalkylsulfinyl, $C_4$-$C_6$-Cycloalkylalkylsulfinyl, $C_1$-$C_8$-Halogenalkylsulfinyl, $C_2$-$C_6$-Alkenylsulfinyl, $C_2$-$C_6$-Halogenalkenylsulfinyl, $C_3$-$C_6$-Alkinylsulfinyl, $C_3$-$C_6$-Halogenalkinylsulfinyl, $C_2$-$C_4$-Alkoxyalkylsulfinyl, $C_2$-$C_4$-Halogenalkoxyalkylsulfinyl, $C_2$-$C_4$-Cyanalkylsulfinyl, $S(O)CH_2C(O)CH_3$, S-(O)$CH_2CH_2C(O)CH_3$, $C_2$-$C_4$-Aminoalkylsulfinyl, $C_2$-$C_8$-Alkylsulfonyl, $C_3$-$C_6$-Cycloalkylsulfonyl, $C_4$-$C_6$-Cycloalkylalkylsulfonyl, $C_1$-$C_8$-Halogenalkylsulfonyl, $C_2$-$C_6$-Alkenylsulfonyl, $C_2$-$C_6$-Halogenalkenylsulfonyl, $C_3$-$C_6$-Alkinylsulfonyl, $C_3$-$C_6$-Halogenalkinylsulfonyl, $C_2$-$C_4$-Alkoxyalkylsulfonyl, $C_2$-$C_4$-Halogenalkoxyalkylsulfonyl, $C_2$-$C_4$-Cyanalkylsulfonyl, $SO_2CH_2C(O)CH_3$, $SO_2CH_2CH_2C(O)CH_3$, $C_2$-$C_4$-Aminoalkylsulfonyl, $CH_2F$, $CHF_2$, $CH_2Cl$, $CHCL_2$, $CH_2Br$, $CHBr_2$, mit 1 bis 3 Atomen aus F, Cl oder Br substituiertes $C_2$-$C_6$-Alkyl, $C_2$-$C_6$-Alkenyl, $C_2$-$C_6$-Halogenalkenyl, $C_2$-$C_6$-Halogenalkinyl, $OC(O)C_1$-$C_4$-Alkyl, $CH_2C(O)NR_aR_b$, $NHCH_3$, $NR_bR_c$ oder mit $C_1$-$C_4$-Alkoxy, $C_3$-$C_4$-Cycloalkoxy, Cyclopropylmethoxy, $C_1$-$C_4$-Halogenalkoxy, $C_2$-$C_4$-Alkenyloxy, $C_2$-$C_4$-Halogenalkenyloxy, $C_3$-$C_4$-Alkinyloxy, $C_3$-$C_4$-Halogenalkinyloxy, $C_2$-$C_4$-Alkoxyalkoxy, $C_2$-$C_4$-Aminoalkoxy, $C_1$-$C_4$-Alkylcarbonyloxy, $C_1$-$C_4$-Halogenalkylcarbonyloxy, $C_1$-$C_4$-Carbamoyloxy, $C_1$-$C_4$-Alkoxycarbonyloxy, OH, $OP(O)(OC_1$-$C_2$-$Alkyl)_2$, $C_1$-$C_4$-Alkylsulfonyloxy, $C_1$-$C_2$-Halogenalkylsulfonyloxy, $OSi(CH_3)_3$, $OSi(CH_3)_2C(CH_3)_3$, $C_1$-$C_4$-Alkylthio, $C_3$-$C_4$-Cycloalkylthio, Cyclopropylmethylthio, $C_1$-$C_4$-Halogenalkylthio, $C_2$-$C_4$-Alkenylthio, $C_2$-$C_4$-Halogenalkenylthio, $C_3$-$C_4$-Alkinylthio, $C_3$-$C_4$-Halogenalkinylthio, $C_2$-$C_4$-Alkoxyalkylthio, $C_2$-$C_4$-Aminoalkylthio, SH, $SP(O)(OC_1$-$C_2$-$Alkyl)_2$, $C_1$-$C_4$-Alkylsulfinyl, $C_3$-$C_4$-Cycloalkylsulfonyl, Cyclopropylmethylsulfinyl, $C_1$-$C_4$-Halogenalkylsulfinyl, $C_2$-$C_4$-Alkenylsulfinyl, $C_2$-$C_4$-Halogenalkenylsulfinyl, $C_3$-$C_4$-Alkinylsulfinyl, $C_3$-$C_4$-Halogenalkinylsulfinyl, $C_2$-$C_4$-Alkoxyalkylsulfinyl, $C_2$-$C_4$-Aminoalkylsulfinyl, $C_1$-$C_4$-Alkylsulfonyl, $C_3$-$C_4$-Cycloalkylsulfonyl, Cyclopropylmethylsulfonyl, $C_1$-$C_4$-Halogenalkylsulfonyl, $C_2$-$C_4$-Alkenylsulfonyl, $C_2$-$C_4$-Halogenalkenylsulfonyl, $C_3$-$C_4$-Alkinylsulfonyl, $C_3$-$C_4$-Halcgenalkinylsulfonyl, $C_2$-$C_4$-Alkoxyalkylsulfonyl oder $C_2$-$C_4$-Aminoalkylsulfonyl substituiertes $C_1$-$C_4$-Alkyl ist;

Ra und $R_b$ unabhängig H oder $C_1$-$C_3$-Alkyl sind;

$R_c$ $C_2$-$C_4$-Alkyl, Cyclopropylmethyl, $C_2$-$C_4$-Cyanalkyl, $CH_2C(O)CH_3$, $CH_2CH_2C(O)CH_3$, $C_1$-$C_4$-Halogenalkyl, $C_3$-$C_4$-Alkenyl, $C_3$-$C_4$-Halogenalkenyl, $C_3$-$C_4$-Alkinyl, mit $C_1$-$C_4$-Alkoxy, $C_1$-$C_4$-Alkylthio, $C_1$-$C_4$-Alkylsulfinyl, $C_1$-$C_4$-Alkylsulfonyl, OH, $NH_2$, $NHCH_3$ oder $N(CH_3)_2$ substituiertes $C_1$-$C_4$-Alkyl ist;

X $CH_3$, $OCH_3$, $OC_2H_5$, Cl oder Br ist;

Y $C_1$-$C_2$-Alkyl, $C_1$-$C_2$-Alkoxy, $OCH_2CH_2F$, $OCH_2CHF_2$, $OCH_2CF_3$, $NHCH_3$ oder $N(CH_3)_2$ ist; und

Z CH oder N ist; sowie

ihre landwirtschaftlich geeigneten Salze, mit der Maßgabe, daß

1) wenn X Cl oder Br ist, dann Z CH ist und Y $C_1$-$C_2$-Alkoxy, $NHCH_3$ oder $N(CH_3)_2$ ist;

2) wenn $R_1$ $C_1$-$C_3$-Alkyl ist, dann $R_2$ von $C_2$-$C_6$-Alkoxy, $C_1$-$C_8$-Alkylthio, $C_1$-$C_8$-Alkylsulfinyl, $C_2$-$C_8$-Alkylsulfonyl, $C_3$-$C_6$-Alkenyloxy, $C_3$-$C_6$-Alkinyloxy, $C_3$-$C_6$-Alkenylthio, $C_3$-$C_6$-Alkenylsulfinyl, $C_3$-$C_6$-Alkenylsulfonyl, $C_3$-$C_6$-Alkinylthio, $C_3$-$C_6$-Alkinylsulfinyl, $C_3$-$C_6$-Alkinylsulfonyl, $OCH_2CH_2OCH_3$, $OCH_2CH_2SCH_3$, $OCH_2CH_2S(O)CH_3$, $OCH_2CH_2SO_2CH_3$, mit 1 bis 3 Atomen aus F, Cl oder Br substituiertem $C_2$-$C_6$-Alkyl, $CH_2F$, $CHF_2$, mit $C_1$-$C_2$-Alkoxy, $C_1$-$C_2$-Alkylthio, $C_1$-$C_2$-Alkylsulfinyl oder $C_1$-$C_2$-Alkylsulfonyl substituiertem $C_1$-$C_4$-Alkyl, $OCF_2H$, $OCH_2CH_2F$, $OCH_2CHF_2$, $OCH_2CF_3$, $OCH_2CH_2Cl$, S-Cyclohexyl, S-$C_6H_5$ oder $SCH_2C_6H_5$ verschieden ist.

**2.** Verbindung nach Anspruch 1, worin

$R_2$ $C_2$-$C_6$-Alkoxy, $C_1$-$C_8$-Alkylthio, $C_1$-$C_8$-Alkylsulfinyl, $C_2$-$C_8$-Alkylsulfonyl, $C_3$-$C_6$-Alkenyloxy, $C_3$-$C_6$-Alkinyloxy, $C_3$-$C_6$-Alkenylthio, $C_3$-$C_6$-Alkenylsulfinyl, $C_3$-$C_6$-Alkenylsulfonyl, $C_3$-$C_6$-Alkinylthio, $C_3$-$C_6$-Alkinylsulfinyl, $C_3$-$C_6$-Alkinylsulfonyl, $OCH_2CH_2OCH_3$, $OCH_2CH_2SCH_3$, $OCH_2CH_2S(O)CH_3$, $OCH_2CH_2SO_2CH_3$, mit 1 bis 3 Atomen aus F, Cl oder Br substituiertes $C_2$-$C_6$-Alkyl, $CH_2F$, $CHF_2$, mit $C_1$-$C_2$-Alkoxy, $C_1$-$C_2$-Aökylthio, $C_1$-$C_2$-Alkylsulfinyl oder $C_1$-$C_2$-Alkylsulfonyl substituiertes $C_1$-$C_4$-Alkyl, $OCF_2H$, $OCH_2CH_2F$, $OCH_2CHF_2$, $OCH_2CF_3$,

$OCH_2CH_2Cl$, S-Cyclohexyl, $SC_6H_5$ oder $SCH_2C_6H_5$ ist.

**3.** Verbindung nach Anspruch 2, worin
$R_2$ $CH_3S$, $C_3$-$C_6$-Alkenyloxy, $C_3$-$C_6$-Alkinyloxy, $C_3$-$C_6$-Alkenylthio, $C_3$-$C_6$-Alkenylsulfinyl, $C_3$-$C_6$-Alkenylsulfonyl, $C_3$-$C_6$-Alkinylthio, $C_3$-$C_6$-Alkinylsulfinyl, $C_3$-$C_6$-Alkinylsulfonyl, mit 1 bis 3 Atomen aus F, Cl oder Br substituiertes $C_2$-$C_6$-Alkyl, $CH_2F$, $CHF_2$, in nicht benzylischer Stellung mit $C_1$-$C_2$-Alkoxy, $C_1$-$C_2$-Alkylthio, $C_1$-$C_2$-Alkylsulfinyl oder $C_1$-$C_2$-Alkylsulfonyl substituiertes $C_1$-$C_4$-Alkyl, $OCH_2CH_2OCH_3$, $OCH_2CH_2SCH_3$, $OCH_2CH_2S(O)CH_3$, $OCH_2CH_2SO_2CH_3$, $OCF_2H$, $OCH_2CH_2F$, $OCH_2CHF_2$, $OCH_2CF_3$, $OCH_2CH_2Cl$, S-Cyclohexyl, $SC_6H_5$ oder $SCH_2C_6H_5$ ist.

**4.** Verbindung nach Anspruch 3, worin R H ist.

**5.** Verbindung nach Anspruch 4, worin
$R_2$ $CH_3S$, $C_3$-$C_4$-Alkenyloxy, $C_3$-$C_4$-Alkenylthio, $C_3$-$C_4$-Alkenylsulfinyl, $C_3$-$C_4$-Alkenylsulfonyl, mit 1 bis 3 Atomen aus F oder Cl substituiertes $C_2$-$C_3$-Alkyl, $CH_2F$, $CHF_2$, $OCHF_2$, $OCH_2CH_2F$, $OCH_2CF_3$ oder $OCH_2CH_2Cl$ ist.

**6.** Verbindung nach Anspruch 5, worin
$R_1$ $CH_3$ oder $CH_2CH_3$ ist;
X $CH_3$, $OCH_3$ oder Cl ist; und
Y $CH_3$, $OCH_3$, $C_2H_5$ oder $OC_2H_5$ ist.

**7.** Landwirtschaftlich geeignete Zusammensetzung zur Bekämpfung des Wachstums unerwünschter Vegetation oder zur Verwendung als Pflanzenwachstumsregulans, welche eine wirksame Menge einer Verbindung nach einem der Ansprüche 1 bis 6 und wenigstens eines der folgenden: oberflächenaktives Mittel, festes oder flüssiges Verdünnungsmittel, umfaßt.

**8.** Verfahren zur Bekämpfung des Wachstums unerwünschter Vegetation, das die Anwendung einer wirksamen Menge einer Verbindung nach einem der Ansprüche 1 bis 6 auf den zu schützenden Ort umfaßt.

**9.** Verfahren zur Bekämpfung des Wachstums unerwünschter Vegetation, das die Anwendung einer wirksamen Menge der Verbindung

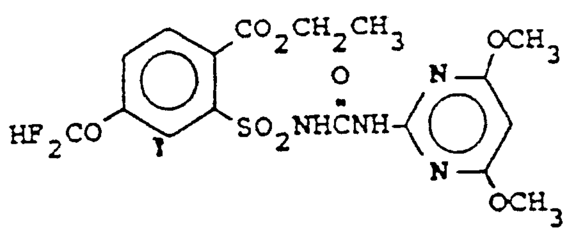

auf den Ort der zu schützenden Reispflanzen eines Reisfeldes umfaßt.

**10.** Verfahren zur Bekämpfung des Wachstums von Wiesenfuchsschwanz in Weizen und Gerste, das die Anwendung einer wirksamen Menge der Verbindung

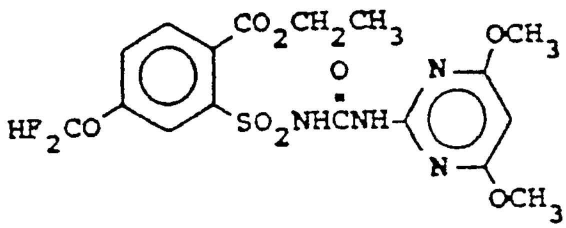

auf den zu schützenden Ort umfaßt.

**11.** Verfahren zur Steuerung des Wachstums von Pflanzen durch Anwendung einer wirksamen, jedoch im wesentlichen nicht phytotoxischen Menge einer das Pflanzenwachstum steuernden Verbindung eines der Ansprüche 1 bis 6 auf den Ort solcher Pflanzen.

**12.** Verfahren zur Herstellung nach einer Verbindung nach Anspruch 1 durch:
(a) Umsetzung eines Sulfonylisocyanats der Formel

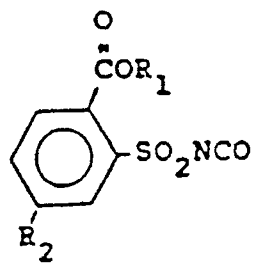

(1)

mit einem Amino- oder Methylaminoheterocyclus der Formel

HN-A
 |
R

(2)

(b) Umsetzung eines Phenylcarbamats der Formel

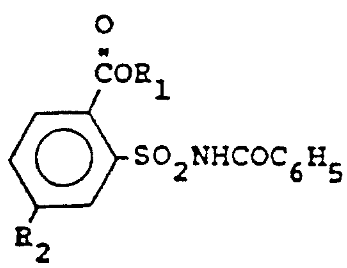

(3)

mit dem Amino- oder Methylaminoheterocyclus;
(c) Umsetzung eines Sulfonamids der Formel

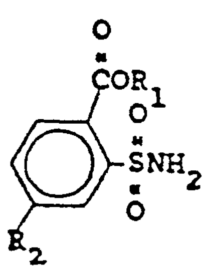

(4)

mit einem heterocyclischen Phenylcarbamat der Formel

$$C_6H_5O\overset{O}{\overset{\|}{C}}NHA$$

(5)

worin A, R, $R_1$ und $R_2$ wie in Anspruch 1 definiert sind, oder
(d) Umsetzung einer Verbindung der Formel

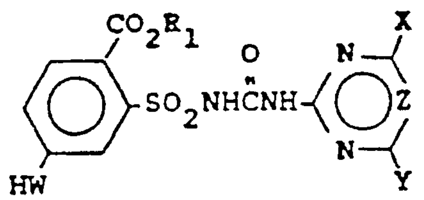

(46)

worin $R_1$, X, Y und Z wie in Anspruch 1 definiert sind, W S, O oder $NR_b$ ist, $R_b$ wie in Anspruch 1 definiert ist, mit einem geeigneten alkylierenden, alkenylierenden und alkinylierenden Mittel der Formel

$R_8X_1$

worin $R_8$ $C_4$-$C_6$-Cycloalkylalkyl, $C_2$-$C_6$-Alkenyl, $C_2$-$C_6$-Halogenalkenyl, $C_3$-$C_6$-Alkinyl, $C_3$-$C_6$-Halogenalkinyl, $C_2$-$C_8$-Alkoxyalkyl, $C_2$-$C_4$-Halogenalkoxyalkyl, $C_2$-$C_8$-Alkylthioalkyl, $C_2$-$C_4$-Halogenalkylthioalkyl, $C_2$-$C_4$-Cyanalkyl, $CH_2C(O)CH_3$, $CH_2CH_2C(O)CH_3$ oder $C_1$-$C_6$-Alkyl ist und

$X_1$ Cl, Br, I, 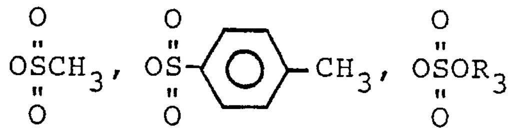 oder

$\overset{\oplus}{O}(R_3)_2$ $BF_4^{\ominus}$ oder 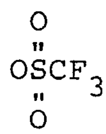 ist, oder

(e) Oxidieren einer Verbindung der Formel (I), worin $R_8$ Alkyl, Cycloalkyl, Cycloalkylalkyl, Halogenalkyl, Alkenyl, Halogenalkenyl, Alkinyl, Halogenalkinyl, Alkoxyalkyl, Halogenalkoxyalkyl, Cyanalkyl, $-CH_2C(O)CH_3$ oder $-CH_2CH_2C(O)CH_3$ ist und W S ist unter Erhalt eines entsprechenden Produkts, worin W S(O) oder $SO_2$ ist.

**13.** Zur Herstellung einer Verbindung nach Anspruch 1 geeignete Zwischenprodukte mit der Formel

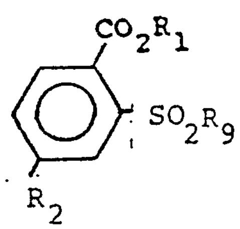

worin $R_1$ und $R_2$ wie in Anspruch 1 definiert sind (wobei Maßgabe 2 gilt) und $R_9$ NCO, $NH_2$ oder $NHCOC_6H_5$ ist.

**14.** Zur Herstellung einer Verbindung nach Anspruch 1 mit der Formel (46), wie in Anspruch 12 definiert, geeignete Zwischenprodukte.